# EUROPEAN PATENT SPECIFICATION

(11) **EP 2 624 916 B1**
(45) Date of publication and mention of the grant of the patent: **17.01.2018**
(21) Application number: 11831498.8
(22) Date of filing: 05.10.2011
(51) Int. Cl.: A61K 31/426, A61K 31/427, A61P 27/02, A61K 31/513, A61K 31/433, A61K 31/496, A61K 31/506, A61K 31/4439, A61K 31/497

(54) **THIADIAZOLE DERIVATIVES FOR USE IN THE TREATMENT OF OCULAR EDEMA**
THIADIAZOL-DERIVATE ZUR VERWENDUNG BEI DER BEHANDLUNG VON AUGENÖDEMEN
DERIVES DU THIADIAZOLE POUR LEUR UTILISATION DANS LE TRAITEMENT D'UN OEDEME OCULAIRE

(30) Priority: 07.10.2010 US 390899 P
(43) Date of publication of application: 14.08.2013
(73) Proprietor: Aerpio Therapeutics, Inc., Cincinnati, OH 45242 (US)
(72) Inventor: PETERS, Kevin, G., Cincinnati, OH 45243 (US); SHALWITZ, Robert, Bexley, OH 43209 (US)
(74) Representative: Avidity IP
(86) International application number: PCT/US2011/054873
(87) International publication number: WO 2012/047966

(56) References cited:
- WO-A1-2011/005330
- WO-A2-2008/002569
- WO-A2-2008/002570
- US-A1- 2007 299 116

## Description

This application claims the benefit of U.S. Provisional Application Serial number 61/390,899 that was filed on October 7, 2010.

### FIELD

Disclosed are methods for the treatment of diseases or conditions of the eye, especially retinopathies, ocular edema and ocular neovascularization. Non-limiting examples of these diseases or conditions include diabetic macular edema, age-related macular degeneration (wet form), choroidal neovascularization, diabetic retinopathy, retinal vein occlusion (central or branch), ocular trauma, surgery induced edema, surgery induced neovascularization, cystoid macular edema, ocular ischemia, uveitis, and the like. These diseases or conditions are characterized by changes in the ocular vasculature whether progressive or non-progressive, whether a result of an acute disease or condition, or a chronic disease or condition.

### BACKGROUND

The eye comprises several structurally and functionally distinct vascular beds, which supply ocular components critical to the maintenance of vision. These include the retinal and choroidal vasculatures, which supply the inner and outer portions of the retina, respectively, and the limbal vasculature located at the periphery of the cornea. Injuries and diseases that impair the normal structure or function of these vascular beds are among the leading causes of visual impairment and blindness. For example, diabetic retinopathy is the most common disease affecting the retinal vasculature, and is the leading cause of vision loss among the working age population in the United States. Vascularization of the cornea secondary to injury or disease is yet another category of ocular vascular disease that can lead to severe impairment of vision.

"Macular degeneration" is a general medical term that applies to any of several disease syndromes which involve a gradual loss or impairment of eyesight due to cell and tissue degeneration of the yellow macular region in the center of the retina. Macular degeneration is often characterized as one of two types, non-exudative (dry form) or exudative (wet form). Although both types are bilateral and progressive, each type may reflect different pathological processes. The wet form of age-related macular degeneration (AMD) is the most common form of choroidal neovascularization and a leading cause of blindness in the elderly. AMD affects millions of Americans over the age of 60, and is the leading cause of new blindness among the elderly.

Choroidal neovascular membrane (CNVM) is a problem that is related to a wide variety of retinal diseases, but is most commonly linked to age-related macular degeneration. With CNVM, abnormal blood vessels stemming from the choroid (the blood vessel-rich tissue layer just beneath the retina) grow up through the retinal layers. These new vessels are very fragile and break easily, causing blood and fluid to pool within the layers of the retina.

Diabetes (diabetes mellitus) is a metabolic disease caused by the inability of the pancreas to produce insulin or to use the insulin that is produced. The most common types of diabetes are type 1 diabetes (often referred to as Juvenile Onset Diabetes Mellitus) and type 2 diabetes (often referred to as Adult Onset Diabetes Mellitus). Type 1 diabetes results from the body's failure to produce insulin due to loss of insulin producing cells, and presently requires the person to inject insulin. Type 2 diabetes generally results from insulin resistance, a condition in which cells fail to use insulin properly. Type 2 diabetes of the has a component of insulin deficiency as well.

Diabetes is directly responsible for a large number of disease conditions, including conditions or diseases of the eye including diabetic retinopathy (DR) and diabetic macular edema (DME) which are leading causes of vision loss and blindness in most developed countries. The increasing number of individuals with diabetes worldwide suggests that DR and DME will continue to be major contributors to vision loss and associated functional impairment for years to come.

Diabetic retinopathy is a complication of diabetes that results from damage to the blood vessels of the light-sensitive tissue at the back of the eye (retina). At first, diabetic retinopathy may cause no symptoms or only mild vision problems. Eventually, however, diabetic retinopathy can result in blindness. Diabetic retinopathy can develop in anyone who has type 1 diabetes or type 2 diabetes.

At its earliest stage, non-proliferative retinopathy, microaneurysms occur in the retina's tiny blood vessels. As the disease progresses, more of these blood vessels become damaged or blocked and these areas of the retina send signals into the regional tissue to grow new blood vessels for nourishment. This stage is called proliferative retinopathy. The new blood vessels grow along the retina and along the surface of the clear, vitreous gel that fills the inside of the eye.

By themselves, these blood vessels do not cause symptoms or vision loss. However, they have thin, fragile walls and without timely treatment, these new blood vessels can leak blood (whole blood or some constituents thereof) which can result in severe vision loss and even blindness.

Also, fluid can leak into the center of the macula, the part of the eye where sharp, straight-ahead vision occurs. The fluid and the associated protein begin to deposit on or under the macula swell the patient's central vision becomes distorted. This condition is called macular edema. It can occur at any stage of diabetic retinopathy, although it is more likely to occur as the disease progresses. About half of the people with proliferative retinopathy also have macular edema.

Uveitis is a condition in which the uvea becomes inflamed. The eye is shaped much like a tennis ball, hollow on the inside with three different layers of tissue surrounding a central cavity. The outermost is the sclera (white coat of the eye) and the innermost is the retina. The middle layer between the sclera and the retina is called the uvea. The uvea contains many of the blood vessels that nourish the eye. Complications of uveitis include glaucoma, cataracts or new blood vessel formation (neovascularization).

The currently available interventions for exudative (wet form) macular degeneration, diabetic retinopathy, diabetic macular edema, choroidal neovascular membrane and complications from uveitis or trauma, include laser photocoagulation therapy, low dose radiation (teletherapy) and surgical removal of neovascular membranes (vitrectomy). Laser therapy has had limited success and selected choroidal neovascular membranes which initially respond to laser therapy have high disease recurrence rates. There is also a potential loss of vision resulting from laser therapy. Low dose radiation has been applied ineffectively to induce regression of choroidal neovascularization. Recently ranibizumab and pegaptinib which are vascular endothelial growth factor (VEGF) antagonist, have been approved for use in age-related macular degeneration.

Retinal vein occlusion (RVO) is the most common retinal vascular disease after diabetic retinopathy. Depending on the area of retinal venous drainage effectively occluded, it is broadly classified as either central retinal vein occlusion (CRVO), hemispheric retinal vein occlusion (HRVO), or branch retinal vein occlusion (BRVO). It has been observed that each of these has two subtypes. Presentation of RVO in general is with variable painless visual loss with any combination of fundal findings consisting of retinal vascular tortuosity, retinal hemorrhages(blot and flame shaped), cotton wool spots, optic disc swelling and macular edema. In a CRVO, retinal hemorrhages will be found in all four quadrants of the fundus, whilst these are restricted to either the superior or inferior fundal hemisphere in a HRVO. In a BRVO, hemorrhages are largely localized to the area drained by the occluded branch retinal vein. Vision loss occurs secondary to macular edema or ischemia.

There is therefore a long felt and substantial need for methods of treating diseases of the eye which are characterized by vascular instability, vascular leakage, and neovascularization.

### BRIEF DESCRIPTION OF THE FIGURES

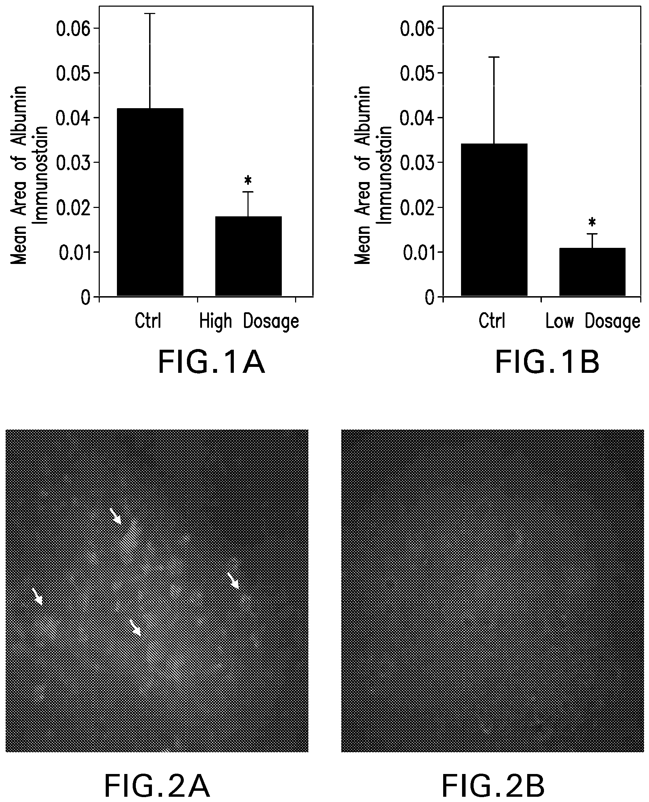

**Figure 1A** is a histogram showing the mean area of albumin deposits that formed in the retinas of rhodopsin/VEGF transgenic mice (control) versus the animals treated with a 10 mg/kg/dose of a compound from Table XXI.
**Figure 1B** is a histogram showing the mean area of albumin deposits that formed in the retinas of the control animals versus animals treated with a 3 mg/kg/dose of a compound from Table XXI.
**Figure 2A** is a micrograph showing the presence of significant focal perivascular albumin deposits (hazy white accumulations indicated by arrows) in the retina of a rhodopsin/VEGF transgenic mouse control.
**Figure 2B** is a micrograph showing the relative absence of perivascular albumin deposits in the retina of a rhodopsin/VEGF transgenic mouse treated with a 3 mg/kg/dose of a compound from Table XXI.

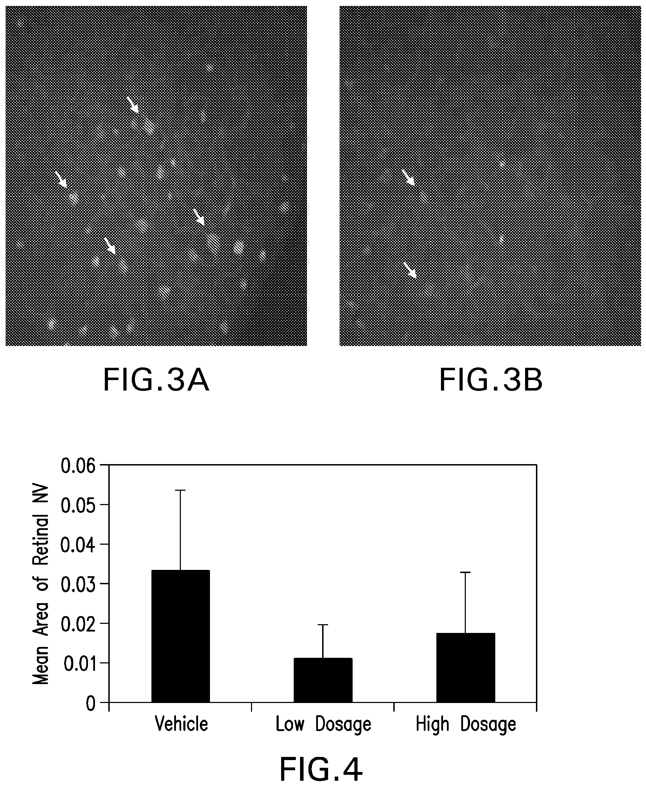

**Figure 3A** is a micrograph showing a significant level of sprouting of new blood vessels (neovascular tufts; white areas indicated by arrows) in the retina of control animals (treated with vehicle) on P21.
**Figure 3B** is a micrograph showing a relative absence of new blood vessels in the retina of animals on P21 that were treated b.i.d. with a 3 mg/kg/dose a compound from Table XXI for 7 days.
**Figure 4** depicts the mean area of retinal neovascular tufts that formed in the retinas of control mice, mice receiving a low dose (3 mg/kg/injection) of a compound from Table XXI, and mice receiving a high dose (10 mg/kg/injection) of a compound from Table XXI.

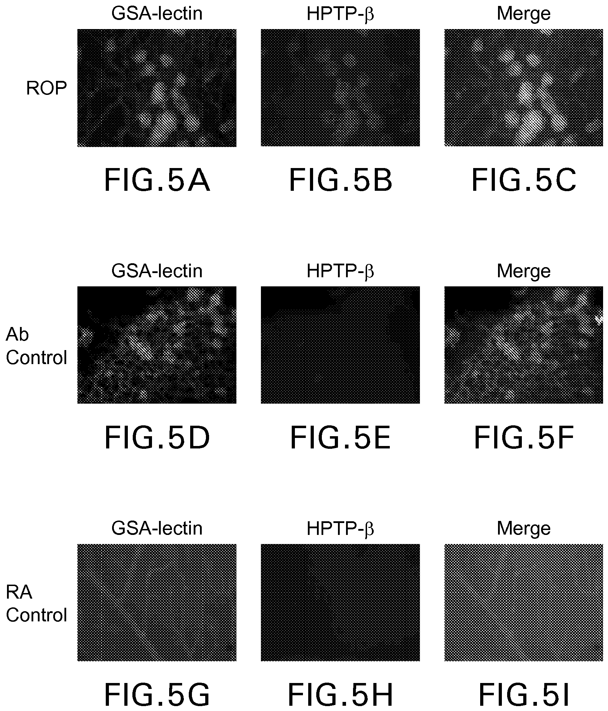

**Figures 5A to 5I** depict micrographs of C57BL/6 mice retinas with oxygen-induced ischemic retinopathy. The retinas were immunostained for VE-PTP/HPTP-β, counterstained with FITC-labeled Griffonia Simplicifolia (GSA) lectin, and flat mounted. Fluorescence microscopy with the green channel showed clumps of GSA-stained NV on the surface of the retina with some faint staining of retinal vessels in the background (**Figures A and D**). The retina from a room air (RA) control mouse showed normal retinal vessels with no neovascularization (**Figure G**). As depicted in **Figures B** and **C**, there was strong staining for HPTP-β in clumps of retinal neovascularization on the surface of the retina and faint staining of some underlying retinal vessels, primarily feeder vessels leading to the neovascularization. As depicted in **Figures H and I,** there was no detectable staining of retinal vessels in the non-ischemic retinas of RA control mice. These data suggest that VE-PTP/ HPTP-β is upregulated in retinal endothelial cells participating in neovascularization.

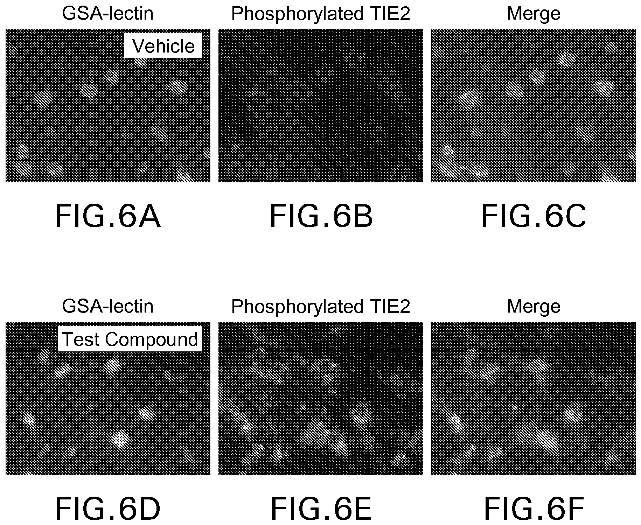

**Figures 6A to 6F** depicts micrographs of hemizygous rho/VEGF transgenic mouse retinas wherein the mice were given single subcutaneous injections of vehicle or 10 mg/kg of a compound from Table XXI at P21. Twelve hours after injection, the mice were euthanized, retinas were removed, stained with FITC-labeled Griffonia Simplicifolia (GSA) lectin, and immunohistochemically stained with anti-phosphoTie2. As depicted in **Figures B and C,** Fluorescence microscopy of retinal flat mounts from vehicle-treated mice showed numerous buds of subretinal neovascularization visualized with GSA lectin (**Figure A**) and faint background staining for anti-phosphoTie2 which was slightly greater in the neovascularization. As depicted in **Frame D,** mice treated with a compound from Table XXI showed GSA-stained buds of subretinal neovascularization. **Figures E and F** depict that these buds also stained strongly for phosphoTie2.

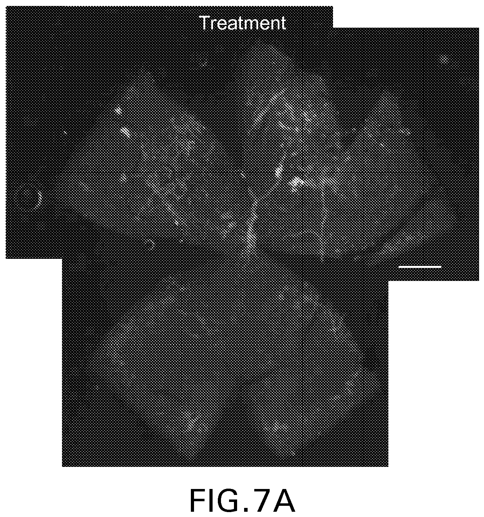

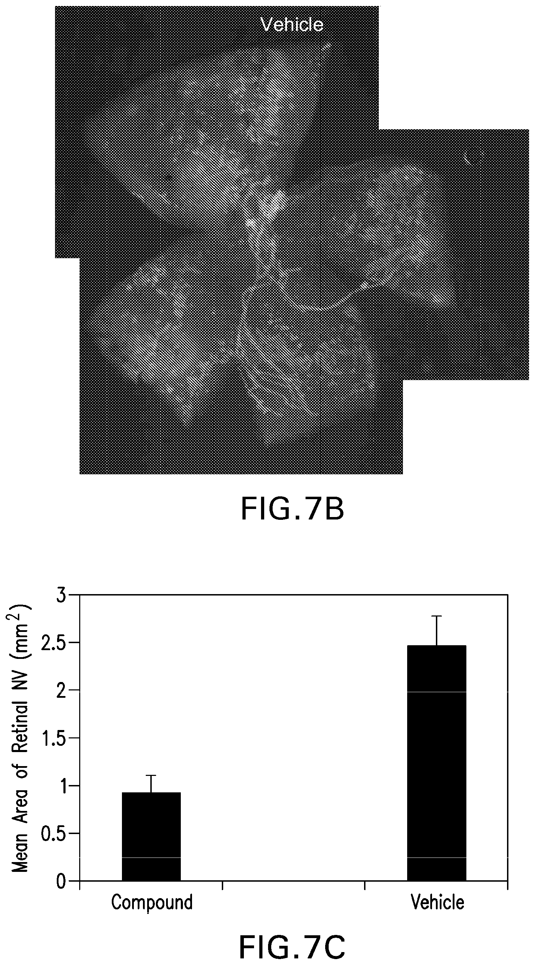

**Figure 7A** and **7B** show the results when mice with oxygen-induced ischemic retinopathy were given an intraocular injection of 3 µg of a compound from Table XXI in one eye and vehicle in the fellow eye. At P17, *in vivo* staining for PECAM-1 showed little neovascularization on the surface of the retina in eyes treated with a compound from Table XXI **(****Figure 7A****)** compared to retinas from eyes treated with vehicle **(****Figure 7B****).**
**Figure 7C** is a graph depicting the measurement of the mean area of retinal neovascularization on the surface of the retina of the treated eye versus the untreated eye as measured by image analysis. These data confirm that intraocular treatment with a compound from Table XXI results in a reduction in retinal neovascularization.

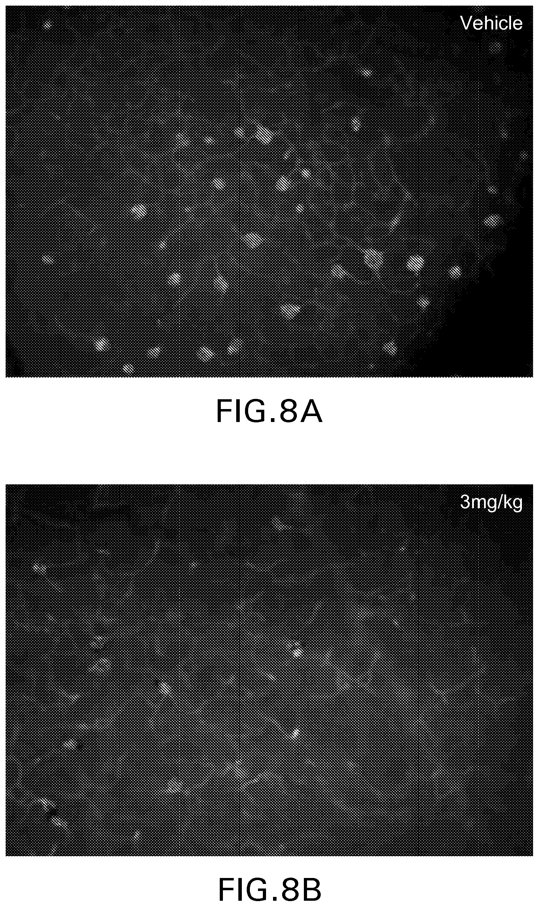

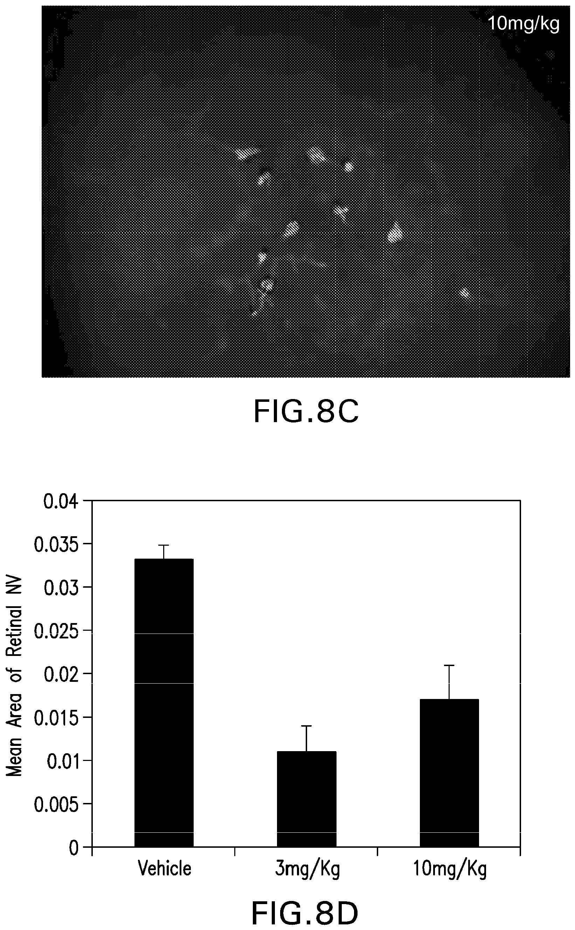

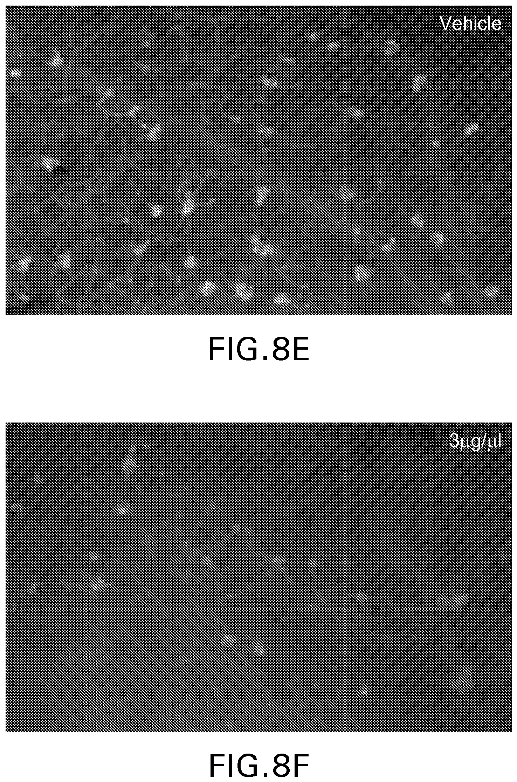

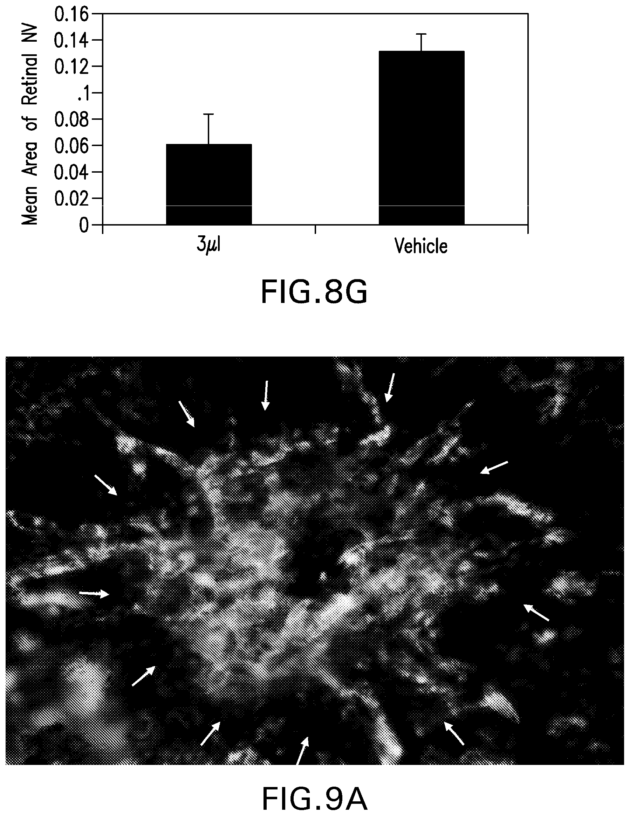

**Figure 8A to Figure 8G** depict the results when hemizygous rho/VEGF transgenic mice were given daily subcutaneous injections of vehicle containing 0,3, or 10 mg/kg of a compound from Table XXI starting at postnatal day (P) 15. At P21 the mice were perfused with fluorescein-labeled dextran and retinal flat mounts were examined by fluorescence microscopy. Micrographs **Figures 8A to 8C** depict the results of this experiment. The retina of a mouse treated with vehicle shows many buds of subretinal neovascularization **(****Figure 8A****)** while retinas from mice treated with 3 mg/kg **(****Figure 8B****)** or 10 mg/kg of a compound from Table XXI **(****Figure 8C****)** had fewer buds of neovascularization. **Figure 8D** is a graph depicting the measurement of the mean area of subretinal neovascularization as measured by image analysis. As see in **Figure 8D****,** compared to mice treated with vehicle, the mean area of subretinal neovascularization was less in mice treated with either dose of a compound from Table XXI. **Figures 8E** and **8F** are micrographs of subsequent experiments wherein rho/VEGF mice were given an injection of 3 µg of a compound from Table XXI in one eye and vehicle in the other eye. As seen in these two photos there were many more buds of subretinal neovascularization in vehicle-injected eyes **(****Figure 8E****)** than those injected with 3 µg of a compound from Table XXI **(****Figure 8F****).** **Figure 8G** is a graph depicting the measurement of the mean area of retinal neovascularization on the surface of the retina of the treated eye versus the untreated eye as measured by image analysis. These data confirm that intraocular treatment with a compound from Table XXI results in a reduction in retinal neovascularization.

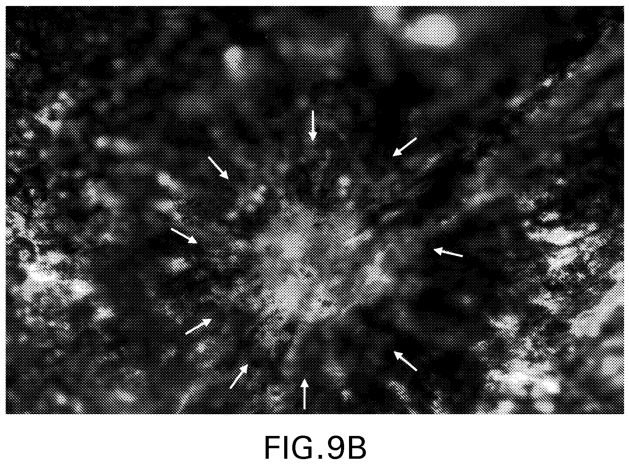

**Figures 9A** **and** **9B** depict the results when C57BL/6 mice had rupture of Bruch's membrane by laser photocoagulation in 3 locations in each eye and then received subcutaneous injections of vehicle (n=8), 20 mg/kg (n=10), or 40 mg/kg of a compound from Table XXI (n=10) twice a day for 14 days. In another experiment the mice (n=6 for each dose) received an injection of 1, 3, or 5 µg of a compound from Table XXI in one eye and vehicle in the fellow eye immediately after and 7 days after laser.. Fourteen days after rupture of Bruch membrane, the mice were perfused with fluorescein-labeled dextran and choroidal flat mounts were examined by fluorescence microscopy. **Figure 9A** depicts a choroidal flat mount from a mouse treated with vehicle shows a large choroidal neovascularization lesion at a Bruch's membrane rupture site, while the choroidal neovascularization is smaller in a choroidal flat mount from a mouse treated with 20 mg/kg of a compound from Table XXI as depicted in **Figure 9B****.**

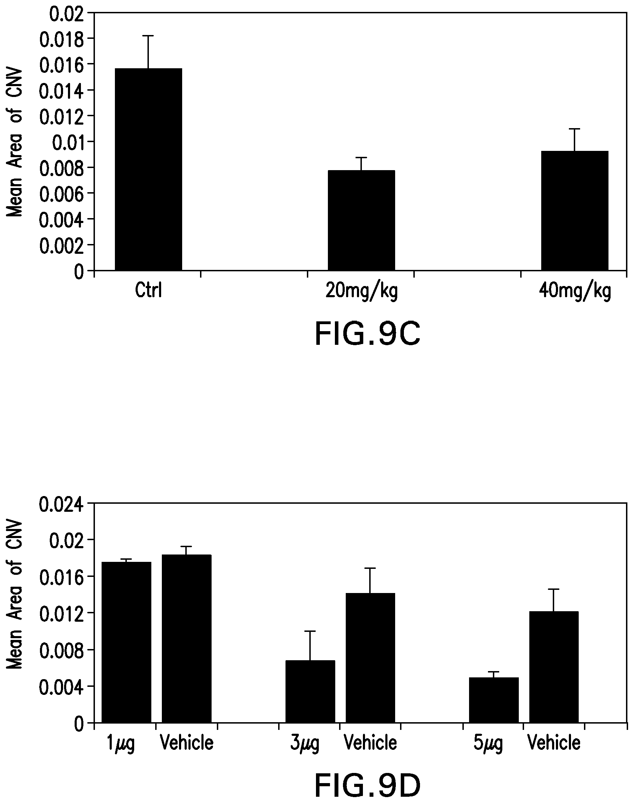

**Figure 9C** shows the results when adult C57BL/6 mice had rupture of Bruch's membrane by laser photocoagulation in 3 locations in each eye and then received subcutaneous injections of vehicle, 20 mg/kg a compound from Table XXI, or 40 mg/kg of a compound from Table XXI twice a day for 14 days. Compared to mice treated with vehicle, the mean area of choroidal neovascularization was significantly less in mice treated with 20 mg/kg or 40 mg/kg of a compound from Table XXI. **Figure 9D** shows that mice given an intraocular injection of 3 µg or 5 µg of a compound from Table XXI, but not mice injected with 1 µg had a significant reduction in mean area of choroidal neovascularization compared to fellow eyes injected with vehicle.

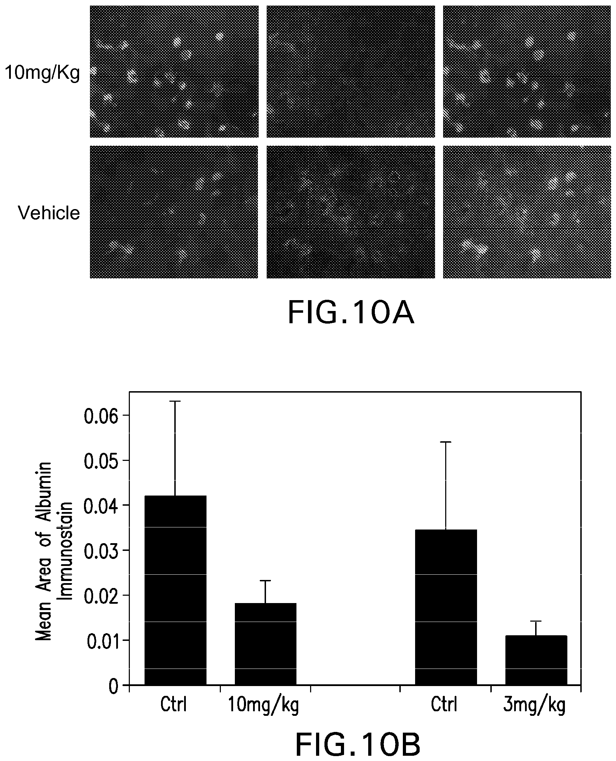

**Figure 10A** shows micrographs of isolated retinas of rho/VEGF mice that at P20 were given a subcutaneous injection of 3 or 10 mg/kg of a compound from Table XXI or vehicle which was repeat 12 hours later. At P21, a third injection was given and then and 2 hours later, mice were euthanized, retinas were dissected, immunofluorescently stained for albumin, and vessels were labeled by counterstaining with GSA lectin. As seen in **Figure 10** **A, Frames A to C,** there was little albumin immunoreactivity seen in the retinas of mice treated with 10 mg/kg of a compound from Table XXI, while as seen in **Figure 10A****, Frames D to F,** the retinas of vehicle-treated mice showed strong staining for albumin surrounding new vessels and causing a red haze throughout the retina. Figure 10B is a graph that shows that the mean area of albumin staining was significantly reduced in mice injected with 3 mg/kg or 10 mg/kg of a compound from Table XXI compared to corresponding controls.

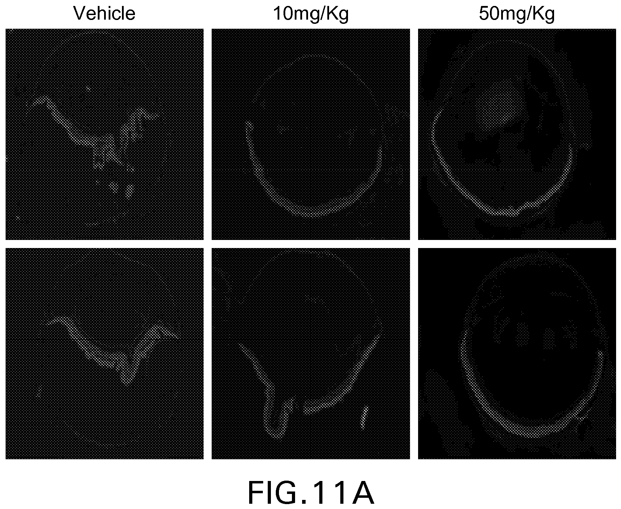

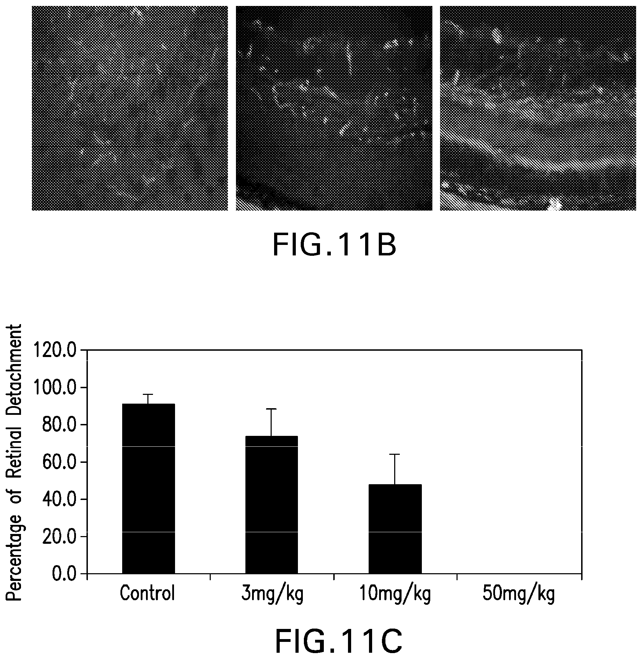

**Figures 11A** **and** **11B** show the results of *Tet*/*opsin*/*VEGF* mice were given twice a day subcutaneous injections of 3, 10, or 50 mg /kg of a compound from Table XXI or vehicle and after 3 days were given an additional daily subcutaneous injection of 50 mg/kg of doxycycline. After an additional 4 days mice were euthanized and frozen ocular sections through the optic nerve were stained with Hoechst (blue) and some were stained with anti-PECAM-1 (green). As seen in Figure 11A, Column 1, the Hoechst-stained retinas from 2 different mice treated with vehicle show complete retinal detachments and Figure 11B, Frame 1, shows that the PECAM-1 stained retina from another vehicle treated mouse indicates a detached, disorganized retina with severe NV in the outer retina.
**Figure 11A****, Column 2,** shows Hoechst-stained retinas from 2 mice treated with 10 mg/kg of a compound from Table XXI; one shows no detachment and the other shows total detachment. **Figure 11B****, Frame 2,** shows a PECAM-1 stained retina from a mouse treated with 10 mg/kg a compound from Table XXI and shows attached retina, but there is prominent neovascularization in the outer retina.
**Figure 11A****, Column 3,** shows the Hoechst-stained retinas from 2 different mice treated with 50 mg/kg of a compound from Table XXI show completely attached retinas and **Figure 11B****, Frame 3,** the PECAM-1 stained retina from another 50 mg/kg-treated mouse show an attached retina with no neovascularization in the outer retina.
**Figure 11C** is a graph of the results of image analysis. All vehicle-treated control mice had complete or near-complete retinal detachments. Compared to vehicle-treated mice, there was a dose-dependent decrease of retinal detachment in mice treated with increasing doses of a compound from Table XXI. All mice treated with 50 mg/kg of a compound from Table XXI had completely attached retinas.

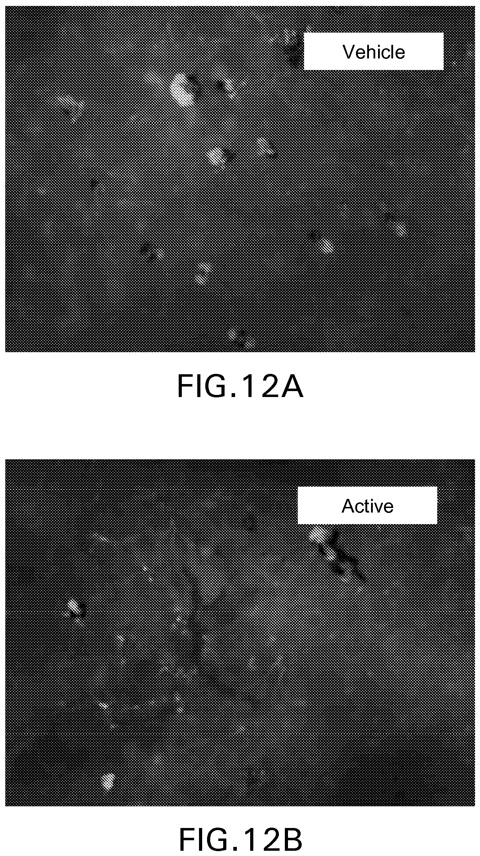

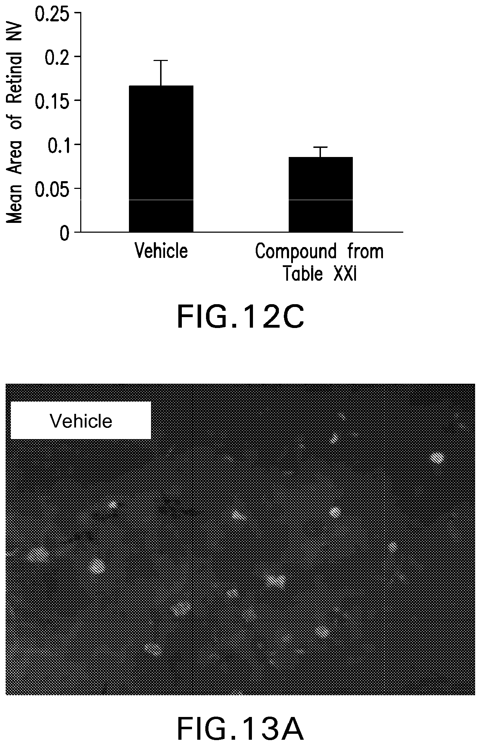

**Figure 12A** depicts the retinal neovascularization in Rho/VEGF mice treated with vehicle beginning on P21 and **Figure 12B** depicts the retinal neovascularization in Rho/VEGF mice treated with 10 mg/kg subcutaneously twice daily with a compound from Table XXII. **Figure 12C** shows the mean area of retinal neovascularization at day 27 for each group.

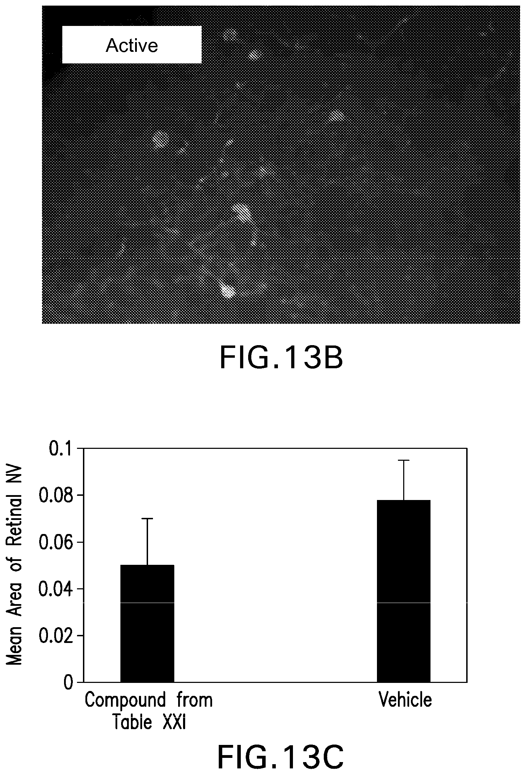

**Figure 13A** depicts the retinal neovascularization in Rho/VEGF mice treated topically with vehicle beginning on P21 and **Figure 13B** depicts the retinal neovascularization in Rho/VEGF mice treated topically with 30 mg/mL subcutaneously three times daily with a compound from Table XXII. **Figure 13C** shows the mean area of retinal neovascularization after 7 days treatment for each group.

### DETAILED DESCRIPTION

The materials, compounds, compositions, articles, and methods described herein may be understood more readily by reference to the following detailed description of specific aspects of the disclosed subject matter and the Examples included therein. Before the present materials, compounds, compositions, articles, devices, and methods are disclosed and described, it is to be understood that the aspects described below are not limited to specific synthetic methods or specific reagents, as such may, of course, vary. It is also to be understood that the terminology used herein is for the purpose of describing particular aspects only and is not intended to be limiting.

Also, throughout this specification, various publications are referenced. The disclosures of these publications in their entireties are hereby incorporated by reference into this application in order to more fully describe the state of the art to which the disclosed matter pertains. The references disclosed are also individually and specifically incorporated by reference herein for the material contained in them that is discussed in the sentence in which the reference is relied upon.

### General Definitions

In this specification and in the claims that follow, reference will be made to a number of terms, which shall be defined to have the following meanings: All percentages, ratios and proportions herein are by weight, unless otherwise specified. All temperatures are in degrees Celsius (° C) unless otherwise specified.

By "pharmaceutically acceptable" is meant a material that is not biologically or otherwise undesirable, i.e., the material can be administered to an individual along with the relevant active compound without causing clinically unacceptable biological effects or interacting in a deleterious manner with any of the other components of the pharmaceutical composition in which it is contained.

Ranges may be expressed herein as from "about" one particular value, and/or to "about" another particular value. When such a range is expressed, another aspect includes from the one particular value and/or to the other particular value. Similarly, when values are expressed as approximations, by use of the antecedent "about," it will be understood that the particular value forms another aspect. It will be further understood that the endpoints of each of the ranges are significant both in relation to the other endpoint, and independently of the other endpoint.

A weight percent of a component, unless specifically stated to the contrary, is based on the total weight of the formulation or composition in which the component is included.

By "effective amount" as used herein means "an amount of one or more of the disclosed compounds, effective at dosages and for periods of time necessary to achieve the desired or therapeutic result." An effective amount may vary according to factors known in the art, such as the disease state, age, sex, and weight of the human or animal being treated. Although particular dosage regimes may be described in examples herein, a person skilled in the art would appreciate that the dosage regime may be altered to provide optimum therapeutic response. For example, several divided doses may be administered daily or the dose may be proportionally reduced as indicated by the exigencies of the therapeutic situation. In addition, the compositions of this disclosure can be administered as frequently as necessary to achieve a therapeutic amount.

"Admixture" or "blend" is generally used herein means a physical combination of two or more different components

"Excipient" is used herein to include any other compound that may be contained in or combined with one or more of the disclosed inhibitors that is not a therapeutically or biologically active compound. As such, an excipient should be pharmaceutically or biologically acceptable or relevant (for example, an excipient should generally be non-toxic to the subject). "Excipient" includes a single such compound and is also intended to include a plurality of excipients.

"HPTP beta" or "HPTP-β" are used interchangeably herein and are abbreviations for human protein tyrosine phosphatase beta.

"Excipient" is used herein to include any other compound that may be contained in or combined with one or more of the disclosed inhibitors that is not a therapeutically or biologically active compound. As such, an excipient should be pharmaceutically or biologically acceptable or relevant (for example, an excipient should generally be non-toxic to the subject). "Excipient" includes a single such compound and is also intended to include a plurality of excipients.

As used herein, by a "subject" is meant an individual. Thus, the "subject" can include domesticated animals (*e.g.,* cats, dogs, etc.), livestock (*e.g.,* cattle, horses, pigs, sheep, goats, etc.), laboratory animals (*e.g.,* mouse, rabbit, rat, guinea pig, etc.), and birds. "Subject" can also include a mammal, such as a primate or a human.

By "reduce" or other forms of the word, such as "reducing" or "reduction," is meant lowering of an event or characteristic (*e.g.,* vascular leakage). It is understood that this is typically in relation to some standard or expected value, in other words it is relative, but that it is not always necessary for the standard or relative value to be referred to.

The term "treat" or other forms of the word such as "treated" or "treatment" is used herein to mean that administration of a compound of the present invention mitigates a disease or a disorder in a host and/or reduces, inhibits, or eliminates a particular characteristic or event associated with a disorder (e.g., vascular leakage). Thus, the term "treatment" includes, preventing a disorder from occurring in a host, particularly when the host is predisposed to acquiring the disease, but has not yet been diagnosed with the disease; inhibiting the disorder; and/or alleviating or reversing the disorder. Insofar as the methods of the present invention are directed to preventing disorders, it is understood that the term "prevent" does not require that the disease state be completely thwarted. Rather, as used herein, the term preventing refers to the ability of the skilled artisan to identify a population that is susceptible to disorders, such that administration of the compounds of the present invention may occur prior to onset of a disease. The term does not imply that the disease state be completely avoided.

The disclosed compounds affect vascular leakage by inhibiting HPTP-β (and the rodent equivalent, VE-PTP). Unless otherwise specified, diabetic retinopathy includes all stages of non-proliferative retinopathy and proliferative retinopathy.

Throughout the description and claims of this specification the word "comprise" and other forms of the word, such as "comprising" and "comprises," means including but not limited to, and is not intended to exclude, for example, other additives, components, integers, or steps.

As used in the description and the appended claims, the singular forms "a," "an," and "the" include plural referents unless the context clearly dictates otherwise. Thus, for example, reference to "a composition" includes mixtures of two or more such compositions, reference to "a phenylsulfamic acid" includes mixtures of two or more such phenylsulfamic acids, reference to "the compound" includes mixtures of two or more such compounds, and the like.

"Optional" or "optionally" means that the subsequently described event or circumstance can or cannot occur, and that the description includes instances where the event or circumstance occurs and instances where it does not.

Ranges can be expressed herein as from "about" one particular value, and/or to "about" another particular value. When such a range is expressed, another aspect includes from the one particular value and/or to the other particular value. Similarly, when values are expressed as approximations, by use of the antecedent "about," it will be understood that the particular value forms another aspect. It will be further understood that the endpoints of each of the ranges are significant both in relation to the other endpoint, and independently of the other endpoint. It is also understood that there are a number of values disclosed herein, and that each value is also herein disclosed as "about" that particular value in addition to the value itself. For example, if the value "10" is disclosed, then "about 10" is also disclosed. It is also understood that when a value is disclosed, then "less than or equal to" the value, "greater than or equal to the value," and possible ranges between values are also disclosed, as appropriately understood by the skilled artisan. For example, if the value "10" is disclosed, then "less than or equal to 10" as well as "greater than or equal to 10" is also disclosed. It is also understood that throughout the application data are provided in a number of different formats and that this data represent endpoints and starting points and ranges for any combination of the data points. For example, if a particular data point "10" and a particular data point "15" are disclosed, it is understood that greater than, greater than or equal to, less than, less than or equal to, and equal to 10 and 15 are considered disclosed as well as between 10 and 15. It is also understood that each unit between two particular units are also disclosed. For example, if 10 and 15 are disclosed, then 11, 12, 13, and 14 are also disclosed.

The following chemical hierarchy is used throughout the specification to describe and enable the scope of the present disclosure and to particularly point out and distinctly claim the units which comprise the compounds of the present disclosure, however, unless otherwise specifically defined, the terms used herein are the same as those of the artisan of ordinary skill. The term "hydrocarbyl" stands for any carbon atom-based unit (organic molecule), said units optionally containing one or more organic functional group, including inorganic atom comprising salts, *inter alia,* carboxylate salts, quaternary ammonium salts. Within the broad meaning of the term "hydrocarbyl" are the classes "acyclic hydrocarbyl" and "cyclic hydrocarbyl" which terms are used to divide hydrocarbyl units into cyclic and non-cyclic classes.

As it relates to the following definitions, "cyclic hydrocarbyl" units can comprise only carbon atoms in the ring (i.e., carbocyclic and aryl rings) or can comprise one or more heteroatoms in the ring (i.e., heterocyclic and heteroaryl rings). For "carbocyclic" rings the lowest number of carbon atoms in a ring are 3 carbon atoms; cyclopropyl. For "aryl" rings the lowest number of carbon atoms in a ring are 6 carbon atoms; phenyl. For "heterocyclic" rings the lowest number of carbon atoms in a ring is 1 carbon atom; diazirinyl. Ethylene oxide comprises 2 carbon atoms and is a C₂ heterocycle. For "heteroaryl" rings the lowest number of carbon atoms in a ring is 1 carbon atom; 1,2,3,4-tetrazolyl. The following is a non-limiting description of the terms "acyclic hydrocarbyl" and "cyclic hydrocarbyl" as used herein.

### A. Substituted and unsubstituted acyclic hydrocarbyl:

For the purposes of the present disclosure the term "substituted and unsubstituted acyclic hydrocarbyl" encompasses 3 categories of units:
1) linear or branched alkyl, non-limiting examples of which include, methyl (C₁), ethyl (C₂), n-propyl (C₃), *iso*-propyl (C₃), n-butyl (C₄), *sec*-butyl (C₄), *iso*-butyl (C₄), *tert-*butyl (C₄), and the like; substituted linear or branched alkyl, non-limiting examples of which includes, hydroxymethyl (C₁), chloromethyl (C₁), trifluoromethyl (C₁), aminomethyl (C₁), 1-chloroethyl (C₂), 2-hydroxyethyl (C₂), 1,2-difluoroethyl (C₂), 3-carboxypropyl (C₃), and the like.
2) linear or branched alkenyl, non-limiting examples of which include, ethenyl (C₂), 3-propenyl (C₃), 1-propenyl (*also* 2-methylethenyl) (C₃), isopropenyl (*also 2-*methylethen-2-yl) (C₃), buten-4-yl (C₄), and the like; substituted linear or branched alkenyl, non-limiting examples of which include, 2-chloroethenyl *(also* 2-chlorovinyl) (C₂), 4-hydroxybuten-1-yl (C₄), 7-hydroxy-7-methyloct-4-en-2-yl (C₉), 7-hydroxy-7-methyloct-3,5-dien-2-yl (C₉), and the like.
3) linear or branched alkynyl, non-limiting examples of which include, ethynyl (C₂), prop-2-ynyl (*also* propargyl) (C₃), propyn-1-yl (C₃), and 2-methyl-hex-4-yn-1-yl (C₇); substituted linear or branched alkynyl, non-limiting examples of which include, 5-hydroxy-5-methylhex-3-ynyl (C₇), 6-hydroxy-6-methylhept-3-yn-2-yl (C₈), 5-hydroxy-5-ethylhept-3-ynyl (C₉), and the like.

### B. Substituted and unsubstituted cyclic hydrocarbyl:

For the purposes of the present disclosure the term "substituted and unsubstituted cyclic hydrocarbyl" encompasses 5 categories of units:
1) The term "carbocyclic" is defined herein as "encompassing rings comprising from 3 to 20 carbon atoms, wherein the atoms which comprise said rings are limited to carbon atoms, and further each ring can be independently substituted with one or more moieties capable of replacing one or more hydrogen atoms." The following are non-limiting examples of "substituted and unsubstituted carbocyclic rings" which encompass the following categories of units:
   i) carbocyclic rings having a single substituted or unsubstituted hydrocarbon ring, non-limiting examples of which include, cyclopropyl (C₃), 2-methylcyclopropyl (C₃), cyclopropenyl (C₃), cyclobutyl (C₄), 2,3-dihydroxycyclobutyl (C₄), cyclobutenyl (C₄), cyclopentyl (C₅), cyclopentenyl (C₅), cyclopentadienyl (C₅), cyclohexyl (C₆), cyclohexenyl (C₆), cycloheptyl (C₇), cyclooctanyl (C₈), 2,5-dimethylcyclopentyl (C₅), 3,5-dichlorocyclohexyl (C₆), 4-hydroxycyclohexyl (C₆), and 3,3,5-trimethylcyclohex-1-yl (C₆).
   ii) carbocyclic rings having two or more substituted or unsubstituted fused hydrocarbon rings, non-limiting examples of which include, octahydropentalenyl (C₈), octahydro-1*H*-indenyl (C₉), 3a,4,5,6,7,7a-hexahydro-3*H*-inden-4-yl (C₉), decahydroazulenyl (C₁₀).
   iii) carbocyclic rings which are substituted or unsubstituted bicyclic hydrocarbon rings, non-limiting examples of which include, bicyclo-[2.1.1]hexanyl, bicyclo[2.2.1]heptanyl, bicyclo[3. 1. 1]heptanyl, 1,3-dimethyl[2.2.1]heptan-2-yl, bicyclo[2.2.2]octanyl, and bicyclo[3.3.3]undecanyl.
2) The term "aryl" is defined herein as "units encompassing at least one phenyl or naphthyl ring and wherein there are no heteroaryl or heterocyclic rings fused to the phenyl or naphthyl ring and further each ring can be independently substituted with one or more moieties capable of replacing one or more hydrogen atoms." The following are non-limiting examples of "substituted and unsubstituted aryl rings" which encompass the following categories of units:
   i) C₆ or C₁₀ substituted or unsubstituted aryl rings; phenyl and naphthyl rings whether substituted or unsubstituted, non-limiting examples of which include, phenyl (C₆), naphthylen-1-yl (C₁₀), naphthylen-2-yl (C₁₀), 4-fluorophenyl (C₆), 2-hydroxyphenyl (C₆), 3-methylphenyl (C₆), 2-amino-4-fluorophenyl (C₆), 2-(*N,N-*diethylamino)phenyl (C₆), 2-cyanophenyl (C₆), 2,6-di-*tert*-butylphenyl (C₆), 3-methoxyphenyl (C₆), 8-hydroxynaphthylen-2-yl (C₁₀), 4,5-dimethoxynaphthylen-1-yl (C₁₀), and 6-cyano-naphthylen-1-yl (C₁₀).
   ii) C₆ or C₁₀ aryl rings fused with 1 or 2 saturated rings to afford C₈-C₂₀ ring systems, non-limiting examples of which include, bicyclo[4.2.0]octa-1,3,5-trienyl (C₈), and indanyl (C₉).
3) The terms "heterocyclic" and/or "heterocycle" are defined herein as "units comprising one or more rings having from 3 to 20 atoms wherein at least one atom in at least one ring is a heteroatom chosen from nitrogen (N), oxygen (O), or sulfur (S), or mixtures of N, O, and S, and wherein further the ring which contains the heteroatom is also not an aromatic ring." The following are non-limiting examples of "substituted and unsubstituted heterocyclic rings" which encompass the following categories of units:
   i) heterocyclic units having a single ring containing one or more heteroatoms, non-limiting examples of which include, diazirinyl (C₁), aziridinyl (C₂), urazolyl (C₂), azetidinyl (C₃), pyrazolidinyl (C₃), imidazolidinyl (C₃), oxazolidinyl (C₃), isoxazolinyl (C₃), thiazolidinyl (C₃), isothiazolinyl (C₃), oxathiazolidinonyl (C₃), oxazolidinonyl (C₃), hydantoinyl (C₃), tetrahydrofuranyl (C₄), pyrrolidinyl (C₄), morpholinyl (C₄), piperazinyl (C₄), piperidinyl (C₄), dihydropyranyl (C₅), tetrahydropyranyl (C₅), piperidin-2-onyl (valerolactam) (C₅), 2,3,4,5-tetrahydro-1*H-*azepinyl (C₆), 2,3-dihydro-1*H*-indole (C₈), and 1,2,3,4-tetrahydroquinoline (C₉).
   ii) heterocyclic units having 2 or more rings one of which is a heterocyclic ring, non-limiting examples of which include hexahydro-1*H*-pyrrolizinyl (C₇), 3a,4,5,6,7,7a-hexahydro-1*H*-benzo[d]imidazolyl (C₇), 3a,4,5,6,7,7a-hexahydro-1*H-*indolyl (C₈), 1,2,3,4-tetrahydroquinolinyl (C₉), and decahydro-1*H-*cycloocta[b]pyrrolyl (C₁₀).
4) The term "heteroaryl" is defined herein as "encompassing one or more rings comprising from 5 to 20 atoms wherein at least one atom in at least one ring is a heteroatom chosen from nitrogen (N), oxygen (O), or sulfur (S), or mixtures of N, O, and S, and wherein further at least one of the rings which comprises a heteroatom is an aromatic ring." The following are non-limiting examples of "substituted and unsubstituted heterocyclic rings" which encompass the following categories of units:
   i) heteroaryl rings containing a single ring, non-limiting examples of which include, 1,2,3,4-tetrazolyl (C₁), [1,2,3]triazolyl (C₂), [1,2,4]triazolyl (C₂), triazinyl (C₃), thiazolyl (C₃), 1*H*-imidazolyl (C₃), oxazolyl (C₃), isoxazolyl (C₃), isothiazolyl (C₃), furanyl (C₄), thiophenyl (C₄), pyrimidinyl (C₄), 2-phenylpyrimidinyl (C₄), pyridinyl (C₅), 3-methylpyridinyl (C₅), and 4-dimethylaminopyridinyl (C₅)
   ii) heteroaryl rings containing 2 or more fused rings one of which is a heteroaryl ring, non-limiting examples of which include: 7*H*-purinyl (C₅), 9*H*-purinyl (C₅), 6-amino-9*H*-purinyl (C₅), 5*H*-pyrrolo[3,2-*d*]pyrimidinyl (C₆), 7*H*-pyrrolo[2,3-*d*]pyrimidinyl (C₆), pyrido[2,3-*d*]pyrimidinyl (C₇), 2-phenylbenzo[d]thiazolyl (C₇), 1*H*-indolyl (C₈), 4,5,6,7-tetrahydro-1-*H*-indolyl (C₈), quinoxalinyl (C₈), 5-methylquinoxalinyl (C₈), quinazolinyl (C₈), quinolinyl (C₉), 8-hydroxy-quinolinyl (C₉), and isoquinolinyl (C₉).
5) C₁-C₆ tethered cyclic hydrocarbyl units (whether carbocyclic units, C₆ or C₁₀ aryl units, heterocyclic units, or heteroaryl units) which connected to another moiety, unit, or core of the molecule by way of a C₁-C₆ alkylene unit. Non-limiting examples of tethered cyclic hydrocarbyl units include benzyl C₁-(C₆) having the formula: 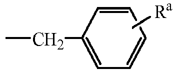 wherein R^{a} is optionally one or more independently chosen substitutions for hydrogen. Further examples include other aryl units, *inter alia*, (2-hydroxyphenyl)hexyl C₆-(C₆); naphthalen-2-ylmethyl C₁-(C₁₀), 4-fluorobenzyl C₁-(C₆), 2-(3-hydroxyphenyl)ethyl C₂-(C₆), as well as substituted and unsubstituted C₃-C₁₀ alkylenecarbocyclic units, for example, cyclopropylmethyl C₁-(C₃), cyclopentylethyl C₂-(C₅), cyclohexylmethyl C₁-(C₆);. Included within this category are substituted and unsubstituted C₁-C₁₀ alkylene-heteroaryl units, for example a 2-picolyl C₁-(C₆) unit having the formula: 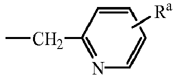 wherein R^{a} is the same as defined above. In addition, C₁-C₁₂ tethered cyclic hydrocarbyl units include C₁-C₁₀ alkyleneheterocyclic units and alkylene-heteroaryl units, non-limiting examples of which include, aziridinylmethyl C₁-(C₂) and oxazol-2-ylmethyl C₁-(C₃).

For the purposes of the present disclosure carbocyclic rings are from C₃ to C₂₀; aryl rings are C₆ or C₁₀; heterocyclic rings are from C₁ to C₉; and heteroaryl rings are from C₁ to C₉.

For the purposes of the present disclosure, and to provide consistency in defining the present disclosure, fused ring units, as well as spirocyclic rings, bicyclic rings and the like, which comprise a single heteroatom will be characterized and referred to herein as being encompassed by the cyclic family corresponding to the heteroatom containing ring, although the artisan may have alternative characterizations. For example, 1,2,3,4-tetrahydroquinoline having the formula: 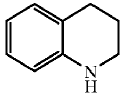 is, for the purposes of the present disclosure, considered a heterocyclic unit. 6,7-Dihydro-5*H*-cyclopentapyrimidine having the formula: 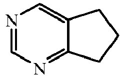 is, for the purposes of the present disclosure, considered a heteroaryl unit. When a fused ring unit contains heteroatoms in both a saturated ring (heterocyclic ring) and an aryl ring (heteroaryl ring), the aryl ring will predominate and determine the type of category to which the ring is assigned herein for the purposes of describing the invention. For example, 1,2,3,4-tetrahydro-[1,8]naphthpyridine having the formula: 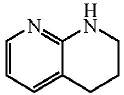 is, for the purposes of the present disclosure, considered a heteroaryl unit.

The term "substituted" is used throughout the specification. The term "substituted" is applied to the units described herein as "substituted unit or moiety is a hydrocarbyl unit or moiety, whether acyclic or cyclic, which has one or more hydrogen atoms replaced by a substituent or several substituents as defined herein below." The units, when substituting for hydrogen atoms are capable of replacing one hydrogen atom, two hydrogen atoms, or three hydrogen atoms of a hydrocarbyl moiety at a time. In addition, these substituents can replace two hydrogen atoms on two adjacent carbons to form said substituent, new moiety, or unit. For example, a substituted unit that requires a single hydrogen atom replacement includes halogen, hydroxyl, and the like. A two hydrogen atom replacement includes carbonyl, oximino, and the like. A two hydrogen atom replacement from adjacent carbon atoms includes epoxy, and the like. Three hydrogen replacement includes cyano, and the like. The term substituted is used throughout the present specification to indicate that a hydrocarbyl moiety, *inter alia,* aromatic ring, alkyl chain; can have one or more of the hydrogen atoms replaced by a substituent. When a moiety is described as "substituted" any number of the hydrogen atoms may be replaced. For example, 4-hydroxyphenyl is a "substituted aromatic carbocyclic ring (aryl ring)", (N,N-dimethyl-5-amino)octanyl is a " substituted C₈ linear alkyl unit, 3-guanidinopropyl is a "substituted C₃ linear alkyl unit," and 2-carboxypyridinyl is a "substituted heteroaryl unit."

The following are non-limiting examples of units which can substitute for hydrogen atoms on a carbocyclic, aryl, heterocyclic, or heteroaryl unit:
i) C₁-C₁₂ linear, branched, or cyclic alkyl, alkenyl, and alkynyl; methyl (C₁), ethyl (C₂), ethenyl (C₂), ethynyl (C₂), n-propyl (C₃), *iso*-propyl (C₃), cyclopropyl (C₃), 3-propenyl (C₃), 1-propenyl (*also* 2-methylethenyl) (C₃), isopropenyl (*also* 2-methylethen-2-yl) (C₃), prop-2-ynyl (*also* propargyl) (C₃), propyn-1-yl (C₃), n-butyl (C₄), *sec*-butyl (C₄), *iso*-butyl (C₄), *tert-butyl* (C₄), cyclobutyl (C₄), buten-4-yl (C₄), cyclopentyl (C₅), cyclohexyl (C₆);
ii) substituted or unsubstituted C₆ or C₁₀ aryl; for example, phenyl, naphthyl (also referred to herein as naphthylen-1-yl (C₁₀) or naphthylen-2-yl (C₁₀));
iii) substituted or unsubstituted C₆ or C₁₀ alkylenearyl; for example, benzyl, 2-phenylethyl, naphthylen-2-ylmethyl;
iv) substituted or unsubstituted C₁-C₉ heterocyclic rings; as described herein below;
v) substituted or unsubstituted C₁-C₉ heteroaryl rings; as described herein below;
vi) -(CR^{102a}R^{102b})ₐOR¹⁰¹; for example, -OH, -CH₂OH, -OCH₃, -CH₂OCH₃, -OCH₂CH₃, -CH₂OCH₂CH₃, -OCH₂CH₂CH₃, and -CH₂OCH₂CH₂CH₃;
vii) -(CR^{102a}R^{102b})ₐC(O)R¹⁰¹; for example, -COCH₃, -CH₂COCH₃, -COCH₂CH₃, -CH₂COCH₂CH₃, -COCH₂CH₂CH₃, and -CH₂COCH₂CH₂CH₃;
viii) -(CR^{102a}R^{102b})ₐC(O)OR¹⁰¹; for example, -CO₂CH₃, -CH₂CO₂CH₃, -CO₂CH₂CH₃, -CH₂CO₂CH₂CH₃, -CO₂CH₂CH₂CH₃, and -CH₂CO₂CH₂CH₂CH₃;
   i) -(CR^{102a}R^{102b})ₐC(O)N(R¹⁰¹)₂; for example, -CONH₂, -CH₂CONH₂, -CONHCH₃, -CH₂CONHCH₃, -CON(CH₃)₂, and -CH₂CON(CH₃)₂;
   ii) -(CR^{102a}R^{101b})ₐN(R¹⁰¹)₂; for example, -NH₂, -CH₂NH₂, -NHCH₃, -CH₂NHCH₃, -N(CH₃)₂, and -CH₂N(CH₃)₂;
   iii) halogen; -F, -Cl, -Br, and -I;
   iv) -(CR^{102a}R^{102b})ₐCN;
   v) -(CR^{102a}R^{102b})ₐNO₂;
   vi) -CHⱼXₖ; wherein X is halogen, the index j is an integer from 0 to 2, j + k = 3; for example, -CH₂F, -CHF₂, -CF₃, -CCl₃, or -CBr₃;
   vii) -(CR^{102a}R^{102b})ₐSR¹⁰¹; -SH, -CH₂SH, -SCH₃, -CH₂SCH₃, -SC₆H₅, and -CH₂SC₆H₅;
   viii) -(CR^{102a}R^{102b})ₐSO₂R¹⁰¹; for example, -SO₂H, -CH₂SO₂H, -SO₂CH₃, -CH₂SO₂CH₃, -SO₂C₆H₅, and -CH₂SO₂C₆H₅; and
   ix) -(CR^{102a}R^{102b})ₐSO₃R¹⁰¹; for example, -SO₃H, -CH₂SO₃H, -SO₃CH₃, -CH₂SO₃CH₃, -SO₃C₆H₅, and -CH₂SO₃C₆H₅;
wherein each R¹⁰¹ is independently hydrogen, substituted or unsubstituted C₁-C₆ linear, branched, or cyclic alkyl, phenyl, benzyl, heterocyclic, or heteroaryl; or two R¹⁰¹ units can be taken together to form a ring comprising 3-7 atoms; R^{102a} and R^{102b} are each independently hydrogen or C₁-C₄ linear or branched alkyl; the index "a" is from 0 to 4.

For the purposes of the present disclosure the terms "compound," "analog," and "composition of matter" stand equally well for each other and are used interchangeably throughout the specification. The disclosed compounds include all enantiomeric forms, diastereomeric forms, salts, and the like.

The compounds disclosed herein include all salt forms, for example, salts of both basic groups, *inter alia,* amines, as well as salts of acidic groups, *inter alia,* carboxylic acids. The following are non-limiting examples of anions that can form salts with protonated basic groups: chloride, bromide, iodide, sulfate, bisulfate, carbonate, bicarbonate, phosphate, formate, acetate, propionate, butyrate, pyruvate, lactate, oxalate, malonate, maleate, succinate, tartrate, fumarate, citrate, and the like. The following are non-limiting examples of cations that can form salts of acidic groups: ammonium, sodium, lithium, potassium, calcium, magnesium, bismuth, lysine, and the like.

The present invention relates to compounds for use in treating an ocular odema of an eye in a subject, wherein the compound has a formula as defined in claim 1 or a pharmaceutically acceptable salt thereof.

The application also discloses other formula not falling within the scope of the invention.

The disclosed compounds have Formula (1): 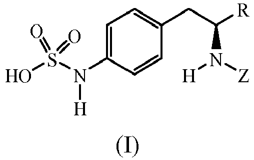 wherein the carbon atom having the amino unit has the (S) stereochemistry as indicated in the following formula: 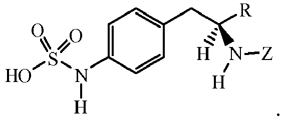 The units which comprise R and Z can comprise units having any configuration, and, as such, the disclosed compounds can be single enantiomers, diastereomeric pairs, or combinations thereof. In addition, the compounds can be isolated as salts or hydrates. In the case of salts, the compounds can comprises more than one cation or anion. In the case of hydrates, any number of water molecules, or fractional part thereof (for example, less than 1 water molecule present for each molecule of analog) can be present.

### R Units

R is a substituted or unsubstituted thiazolyl unit having the formula: 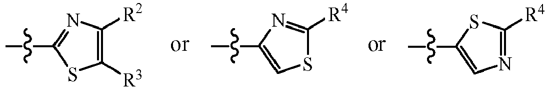 R², R³, and R⁴ are substituent groups that can be independently chosen from a wide variety of non-carbon atom containing units (for example, hydrogen, hydroxyl, amino, halogen, nitro, and the like) or organic substituent units, such as substituted and unsubstituted acyclic hydrocarbyl and cyclic hydrocarbyl units as described herein. The carbon comprising units can comprise from 1 to 12 carbon atoms, or 1 to 10 carbon atoms, or 1 to 6 carbon atoms.

An example of compounds of Formula (I) include compounds wherein R units are thiazol-2-yl units having the formula: 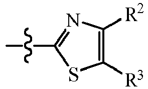 wherein R² and R³ are each independently chosen from:
i) hydrogen;
ii) substituted or unsubstituted C₁-C₆ linear, C₃-C₆ branched, or C₃-C₆ cyclic alkyl;
iii) substituted or unsubstituted C₂-C₆ linear, C₃-C₆ branched, or C₃-C₆ cyclic alkenyl;
iv) substituted or unsubstituted C₂-C₆ linear or C₃-C₆ branched alkynyl;
v) substituted or unsubstituted C₆ or C₁₀ aryl;
vi) substituted or unsubstituted C₁-C₉ heteroaryl;
vii) substituted or unsubstituted C₁-C₉ heterocyclic; or
viii) R² and R³ can be taken together to form a saturated or unsaturated ring having from 5 to 7 atoms; wherein from 1 to 3 atoms can optionally be heteroatoms chosen from oxygen, nitrogen, and sulfur.

The following are non-limiting examples of units that can substitute for one or more hydrogen atoms on the R² and R³ units. The following substituents, as well as others not herein described, are each independently chosen:
i) C₁-C₁₂ linear, C₃-C₁₂ branched, or C₃-C₁₂ cyclic alkyl, alkenyl, and alkynyl; methyl (C₁), ethyl (C₂), ethenyl (C₂), ethynyl (C₂), n-propyl (C₃), *iso*-propyl (C₃), cyclopropyl (C₃), 3-propenyl (C₃), 1-propenyl (*also* 2-methylethenyl) (C₃), isopropenyl (*also* 2-methylethen-2-yl) (C₃), prop-2-ynyl (*also* propargyl) (C₃), propyn-1-yl (C₃), n-butyl (C₄), *sec*-butyl (C₄), *iso*-butyl (C₄), *tert*-butyl (C₄), cyclobutyl (C₄), buten-4-yl (C₄), cyclopentyl (C₅), cyclohexyl (C₆);
ii) substituted or unsubstituted C₆ or C₁₀ aryl; for example, phenyl, naphthyl (also referred to herein as naphthylen-1-yl (C₁₀) or naphthylen-2-yl (C₁₀));
iii) substituted or unsubstituted C₆ or C₁₀ alkylenearyl; for example, benzyl, 2-phenylethyl, naphthylen-2-ylmethyl;
iv) substituted or unsubstituted C₁-C₉ heterocyclic rings; as described herein;
v) substituted or unsubstituted C₁-C₉ heteroaryl rings; as described herein;
vi) -(CR^{21a}R^{21b})ₚOR²⁰; for example, -OH, -CH₂OH, -OCH₃, -CH₂OCH₃, -OCH₂CH₃, -CH₂OCH₂CH₃, -OCH₂CH₂CH₃, and -CH₂OCH₂CH₂CH₃;
vii) -(CR^{21a}R^{21b} )ₚC(O)R²⁰; for example, -COCH₃, -CH₂COCH₃, -COCH₂CH₃, -CH₂COCH₂CH₃, -COCH₂CH₂CH₃, and -CH₂COCH₂CH₂CH₃;
viii) -(CR^{21a}R^{21b})ₚC(O)OR²⁰; for example, -CO₂CH₃, -CH₂CO₂CH₃, -CO₂CH₂CH₃, -CH₂CO₂CH₂CH₃, -CO₂CH₂CH₂CH₃, and -CH₂CO₂CH₂CH₂CH₃;
x) -(CR^{21a}R^{21b})ₚC(O)N(R²⁰)₂; for example, -CONH₂, -CH₂CONH₂, -CONHCH₃, -CH₂CONHCH₃, -CON(CH₃)₂, and -CH₂CON(CH₃)₂;
x) -(CR^{21a}R^{21b})ₚN(R²⁰)₂; for example, -NH₂, -CH₂NH₂, -NHCH₃, -CH₂NHCH₃, -N(CH₃)₂, and -CH₂N(CH₃)₂;
xi) halogen; -F, -Cl, -Br, and -I;
xii) -(CR^{21a}R^{21b})ₚCN;
xiii) -(CR^{21a}R^{21b})ₚNO₂;
xiv) -(CH_{j'}X_{k'})ₕCHⱼXₖ; wherein X is halogen, the index j is an integer from 0 to 2, j + k = 3, the index j' is an integer from 0 to 2, j' + k' = 2, the index h is from 0 to 6; for example, -CH₂F, -CHF₂, -CF₃, -CH₂CF₃, -CHFCF₃, -CCl₃, or -CBr₃;
xv) -(CR^{21a}R^{21b})ₚSR²⁰; -SH, -CH₂SH, -SCH₃, -CH₂SCH₃, -SC₆H₅, and -CH₂SC₆H₅;
xvi) -(CR^{21a}R^{21b})ₚSO₂R²⁰; for example, -SO₂H, -CH₂SO₂H, -SO₂CH₃, -CH₂SO₂CH₃, -SO₂C₆H₅, and -CH₂SO₂C₆H₅; and
xvii) -(CR^{21a}R^{21b})ₚSO₃R²⁰; for example, -SO₃H, -CH₂SO₃H, -SO₃CH₃, -CH₂SO₃CH₃, -SO₃C₆H₅, and -CH₂SO₃C₆H₅;
wherein each R²⁰ is independently hydrogen, substituted or unsubstituted C₁-C₄ linear, C₃-C₄ branched, or C₃-C₄ cyclic alkyl, phenyl, benzyl, heterocyclic, or heteroaryl; or two R²⁰ units can be taken together to form a ring comprising 3-7 atoms; R^{21a} and R^{21b} are each independently hydrogen or C₁-C₄ linear or C₃-C₄ branched alkyl; the index p is from 0 to 4.

An example of compounds of Formula (I) includes R units having the formula: 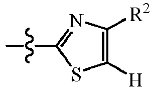 wherein R³ is hydrogen and R² is a unit chosen from methyl (C₁), ethyl (C₂), n-propyl (C₃), iso-propyl (C₃), n-butyl (C₄), *sec*-butyl (C₄), *iso*-butyl (C₄), *tert*-butyl (C₄), n-pentyl (C₅), 1-methylbutyl (C₅), 2-methylbutyl (C₅), 3-methylbutyl (C₅), cyclopropyl (C₃), n-hexyl (C₆), 4-methylpentyl (C₆), and cyclohexyl (C₆).

Another example of compounds of Formula (I) include R units having the formula: 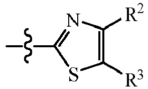 wherein R² is a unit chosen from methyl (C₁), ethyl (C₂), n-propyl (C₃), iso-propyl (C₃), n-butyl (C₄), *sec*-butyl (C₄), *iso*-butyl (C₄), and *tert*-butyl (C₄); and R³ is a unit chosen from methyl (C₁) or ethyl (C₂). Non-limiting examples of this aspect of R includes 4,5-dimethylthiazol-2-yl, 4-ethyl-5-methylthiazol-2-yl, 4-methyl-5-ethylthiazol-2-yl, and 4,5-diethylthiazol-2-yl.

A further example of compounds of Formula (I) includes R units wherein R³ is hydrogen and R² is a substituted alkyl unit, said substitutions chosen from:
i) halogen: -F, -Cl, -Br, and -I;
ii) -N(R¹¹)₂; and
iii) -OR¹¹;
wherein each R¹¹ is independently hydrogen or C₁-C₄ linear or C₃-C₄ branched alkyl. Non-limiting examples of units that can be a substitute for a R² or R³ hydrogen atom on R units include -CH₂F, -CHF₂, -CF₃, -CH₂CF₃, -CH₂CH₂CF₃, -CH₂Cl, -CH₂OH, -CH₂OCH₃, -CH₂CH₂OH, -CH₂CH₂OCH₃, -CH₂NH₂, -CH₂NHCH₃, -CH₂N(CH₃)₂, and -CH₂NH(CH₂CH₃).

Further non-limiting examples of units that can be a substitute for a R² or R³ hydrogen atom on R units include 2,2-difluorocyclopropyl, 2-methoxycyclohexyl, and 4-chlorocyclohexyl.

A yet further example of compounds of Formula (I), R units include units wherein R³ is hydrogen and R² is phenyl or substituted phenyl, wherein non-limiting examples of R² units include phenyl, 3,4-dimethylphenyl, 4-*tert*-butylphenyl, 4-cyclopropylphenyl, 4-diethylaminophenyl, 4-(trifluoromethyl)phenyl, 4-methoxyphenyl, 4-(difluoromethoxy)-phenyl, 4-(trifluoromethoxy)phenyl, 3-chloropheny, 4-chlorophenyl, and 3,4-dichlorophenyl, which when incorporated into the definition of R affords the following R units 4-phenylthiazol-2-yl, 3,4-dimethylphenylthiazol-2-yl, 4-*tert*-butylphenylthiazol-2-yl, 4-cyclopropylphenylthiazol-2-yl, 4-diethylaminophenylthiazol-2-yl, 4-(trifluoromethyl)-phenylthiazol-2-yl, 4-methoxyphenylthiazol-2-yl, 4-(difluoromethoxy)phenylthiazol-2-yl, 4-(trifluoromethoxy)phenylthiazol-2-yl, 3 -chlorophenylthiazol-2-yl, 4-chlorophenylthiazol-2-yl, and 3,4-dichlorophenylthiazol-2-yl.

A still further example of compounds of Formula (I) includes R units wherein R² is chosen from hydrogen, methyl, ethyl, n-propyl, and *iso*-propyl and R³ is phenyl or substituted phenyl. A non-limiting example of a R unit according to the fifth aspect of the first category of R units includes 4-methyl-5-phenylthiazol-2-yl and 4-ethyl-5-phenylthiazol-2-yl.

Another further example of compounds of Formula (I) includes R units wherein R³ is hydrogen and R² is a substituted or unsubstituted heteroaryl unit chosen from 1,2,3,4-tetrazol-1-yl,1,2,3,4-tetrazol-5-yl, [1,2,3]triazol-4-yl, [1,2,3]triazol-5-yl, [1,2,4]triazol-4-yl, [1,2,4]triazol-5-yl, imidazol-2-yl, imidazol-4-yl, pyrrol-2-yl, pyrrol-3-yl, oxazol-2-yl, oxazol-4-yl, oxazol-5-yl, isoxazol-3-yl, isoxazol-4-yl, isoxazol-5-yl, [1,2,4]oxadiazol-3-yl, [1,2,4]oxadiazol-5-yl, [1,3,4]oxadiazol-2-yl, furan-2-yl, furan-3-yl, thiophen-2-yl, thiophen-3-yl, isothiazol-3-yl, isothiazol-4-yl, isothiazol-5-yl, thiazol-2-yl, thiazol-4-yl, thiazol-5-yl, [1,2,4]thiadiazol-3-yl, [1,2,4]thiadiazol-5-yl, and [1,3,4]thiadiazol-2-yl.

Further non-limiting example of compounds of Formula (I) includes R units wherein R² is substituted or unsubstituted thiophen-2-yl, for example thiophen-2-yl, 5-chlorothiophen-2-yl, and 5-methylthiophen-2-yl.

A still further example of compounds of Formula (I) includes R units wherein R² is substituted or unsubstituted thiophen-3-yl, for example thiophen-3-yl, 5-chlorothiophen-3-yl, and 5-methylthiophen-3-yl.

Another example of compounds of Formula (I) includes R units wherein R² and R³ are taken together to form a saturated or unsaturated ring having from 5 to 7 atoms. Non-limiting examples of the sixth aspect of the first category of R units include 5,6-dihydro-4*H*-cyclopenta[*d*]thiazol-2-yl and 4,5,6,7-tetrahydrobenzo[*d*]thiazol-2-yl.

Further examples of compounds of Formula (I) include R units that are thiazol-4-yl or thiazol-5-yl units having the formula: 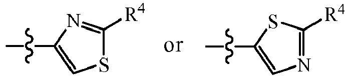 wherein R⁴ is a unit chosen from:
i) hydrogen;
ii) substituted or unsubstituted C₁-C₆ linear, C₃-C₆ branched, or C₃-C₆ cyclic alkyl;
iii) substituted or unsubstituted C₂-C₆ linear, C₃-C₆ branched, or C₃-C₆ cyclic alkenyl;
iv) substituted or unsubstituted C₂-C₆ linear or branched alkynyl;
v) substituted or unsubstituted C₆ or C₁₀ aryl;
vi) substituted or unsubstituted C₁-C₉ heteroaryl; or
vii) substituted or unsubstituted C₁-C₉ heterocyclic.

The following are non-limiting examples of units that can substitute for one or more hydrogen atoms on the R⁴ units. The following substituents, as well as others not herein described, are each independently chosen:
i) C₁-C₁₂ linear, C₃-C₁₂ branched, or C₃-C₁₂ cyclic alkyl, alkenyl, and alkynyl; methyl (C₁), ethyl (C₂), ethenyl (C₂), ethynyl (C₂), n-propyl (C₃), *iso*-propyl (C₃), cyclopropyl (C₃), 3-propenyl (C₃), 1-propenyl (*also* 2-methylethenyl) (C₃), isopropenyl (*also* 2-methylethen-2-yl) (C₃), prop-2-ynyl (*also* propargyl) (C₃), propyn-1-yl (C₃), n-butyl (C₄), *sec*-butyl (C₄), *iso*-butyl (C₄), *tert*-butyl (C₄), cyclobutyl (C₄), buten-4-yl (C₄), cyclopentyl (C₅), cyclohexyl (C₆);
ii) substituted or unsubstituted C₆ or C₁₀ aryl; for example, phenyl, naphthyl (also referred to herein as naphthylen-1-yl (C₁₀) or naphthylen-2-yl (C₁₀));
iii) substituted or unsubstituted C₆ or C₁₀ alkylenearyl; for example, benzyl, 2-phenylethyl, naphthylen-2-ylmethyl;
iv) substituted or unsubstituted C₁-C₉ heterocyclic rings; as described herein below;
v) substituted or unsubstituted C₁-C₉ heteroaryl rings; as described herein below;
vi) -(CR^{21a}R^{21b})ₚOR²⁰; for example, -OH, -CH₂OH, -OCH₃, -CH₂OCH₃, -OCH₂CH₃, -CH₂OCH₂CH₃, -OCH₂CH₂CH₃, and -CH₂OCH₂CH₂CH₃;
vii) -(CR^{21a}R^{21b})ₚC(O)R²⁰; for example, -COCH₃, -CH₂COCH₃, -COCH₂CH₃, -CH₂COCH₂CH₃, -COCH₂CH₂CH₃, and -CH₂COCH₂CH₂CH₃;
viii) _(CR^{21a}R^{21b})ₚC(O)OR²⁰; for example, -CO₂CH₃, -CH₂CO₂CH₃, -CO₂CH₂CH₃, -CH₂CO₂CH₂CH₃, -CO₂CH₂CH₂CH₃, and -CH₂CO₂CH₂CH₂CH₃;
xi) _(CR^{21a}R^{21b})ₚC(O)N(R²⁰)₂; for example, -CONH₂, -CH₂CONH₂, -CONHCH₃, -CH₂CONHCH₃, -CON(CH₃)₂, and -CH₂CON(CH₃)₂;
x) -(CR^{21a}R^{21b})ₚN(R²⁰)₂; for example, -NH₂, -CH₂NH₂, -NHCH₃, -CH₂NHCH₃, -N(CH₃)₂, and -CH₂N(CH₃)₂;
xi) halogen; -F, -Cl, -Br, and -I;
xii) -(CR^{21a}R^{21b})ₚCN;
xiii) -(CR^{21a}R^{21b})ₚNO₂;
xiv) -(CH_{j'}X_{k'})ₕCHⱼXₖ; wherein X is halogen, the index j is an integer from 0 to 2, j + k = 3, the index j' is an integer from 0 to 2, j' + k' = 2, the index h is from 0 to 6; for example, -CH₂F, -CHF₂, -CF₃, -CH₂CF₃, -CHFCF₃, -CCl₃, or -CBr₃;
xv) -(CR^{21a}R^{21b})ₚSR²⁰; -SH, -CH₂SH, -SCH₃, -CH₂SCH₃, -SC₆H₅, and -CH₂SC₆H₅;
xvi) -(CR^{21a}R^{21b})ₚSO₂R²⁰; for example, -SO₂H, -CH₂SO₂H, -SO₂CH₃, -CH₂SO₂CH₃, -SO₂C₆H₅, and -CH₂SO₂C₆H₅; and
xvii) -(CR^{21a}R^{21b})ₚSO₃R²⁰; for example, -SO₃H, -CH₂SO₃H, -SO₃CH₃, -CH₂SO₃CH₃, -SO₃C₆H₅, and -CH₂SO₃C₆H₅;
wherein each R²⁰ is independently hydrogen, substituted or unsubstituted C₁-C₄ linear, C₃-C₄ branched, or C₃-C₄ cyclic alkyl, phenyl, benzyl, heterocyclic, or heteroaryl; or two R²⁰ units can be taken together to form a ring comprising 3-7 atoms; R^{21a} and R^{21b} are each independently hydrogen or C₁-C₄ linear or C₃-C₄ branched alkyl; the index p is from 0 to 4.

An example of compounds of Formula (I) includes R units wherein R⁴ is hydrogen.

A further example of compounds of Formula (I) includes R units wherein R⁴ is a unit chosen from methyl (C₁), ethyl (C₂), n-propyl (C₃), iso-propyl (C₃), n-butyl (C₄), *sec-*butyl (C₄), *iso*-butyl (C₄), and *tert*-butyl (C₄). Non-limiting examples of this aspect of R includes 2-methylthiazol-4-yl, 2-ethylthiazol-4-yl, 2-(n-propyl)thiazol-4-yl, and 2-(*iso-*propyl)thiazol-4-yl.

A still further example of compounds of Formula (I) includes R units wherein R⁴ is substituted or unsubstituted phenyl, non-limiting examples of which include phenyl, 2-fluorophenyl, 2-chlorophenyl, 2-methylphenyl, 2-methoxyphenyl, 3-fluorophenyl, 3-chlorophenyl, 3-methylphenyl, 3-methoxyphenyl, 4-fluorophenyl, 4-chlorophenyl, 4-methylphenyl, and 4-methoxyphenyl.

Yet further example of compounds of Formula (I) includes R units wherein R⁴ is substituted or unsubstituted heteroaryl, non-limiting examples of which include thiophen-2-yl, thiophen-3-yl, thiazol-2-yl, thiazol-4-yl, thiazol-5-yl, 2,5-dimethylthiazol-4-yl, 2,4-dimethylthiazol-5-yl, 4-ethylthiazol-2-yl, oxazol-2-yl, oxazol-4-yl, oxazol-5-yl, and 3-methyl-1,2,4-oxadiazol-5-yl.

Another example of 5-member ring R units includes substituted or unsubstituted imidazolyl units having the formula:

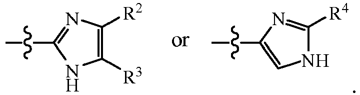

One example of imidazolyl R units includes imidazol-2-yl units having the formula: 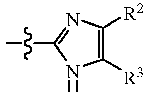 wherein R² and R³ are each independently chosen from:
i) hydrogen;
ii) substituted or unsubstituted C₁-C₆ linear, C₃-C₆ branched, or C₃-C₆ cyclic alkyl;
iii) substituted or unsubstituted C₂-C₆ linear, C₃-C₆ branched, or C₃-C₆ cyclic alkenyl;
iv) substituted or unsubstituted C₂-C₆ linear or branched alkynyl;
v) substituted or unsubstituted C₆ or C₁₀ aryl;
vi) substituted or unsubstituted C₁-C₉ heteroaryl;
vii) substituted or unsubstituted C₁-C₉ heterocyclic; or
viii) R² and R³ can be taken together to form a saturated or unsaturated ring having from 5 to 7 atoms; wherein from 1 to 3 atoms can optionally be heteroatoms chosen from oxygen, nitrogen, and sulfur.

The following are non-limiting examples of units that can substitute for one or more hydrogen atoms on the R² and R³ units. The following substituents, as well as others not herein described, are each independently chosen:
i) C₁-C₁₂ linear, C₃-C₁₂ branched, or C₃-C₁₂ cyclic alkyl, alkenyl, and alkynyl; methyl (C₁), ethyl (C₂), ethenyl (C₂), ethynyl (C₂), n-propyl (C₃), *iso*-propyl (C₃), cyclopropyl (C₃), 3-propenyl (C₃), 1-propenyl (*also* 2-methylethenyl) (C₃), isopropenyl (*also* 2-methylethen-2-yl) (C₃), prop-2-ynyl (*also* propargyl) (C₃), propyn-1-yl (C₃), n-butyl (C₄), *sec*-butyl (C₄), *iso*-butyl (C₄), *tert*-butyl (C₄), cyclobutyl (C₄), buten-4-yl (C₄), cyclopentyl (C₅), cyclohexyl (C₆);
ii) substituted or unsubstituted C₆ or C₁₀ aryl; for example, phenyl, naphthyl (also referred to herein as naphthylen-1-yl (C₁₀) or naphthylen-2-yl (C₁₀));
iii) substituted or unsubstituted C₆ or C₁₀ alkylenearyl; for example, benzyl, 2-phenylethyl, naphthylen-2-ylmethyl;
iv) substituted or unsubstituted C₁-C₉ heterocyclic rings; as described herein;
v) substituted or unsubstituted C₁-C₉ heteroaryl rings; as described herein;
vi) -(CR^{21a}R^{21b})_{z}OR²⁰; for example, -OH, -CH₂OH, -OCH₃, -CH₂OCH₃, -OCH₂CH₃, -CH₂OCH₂CH₃, -OCH₂CH₂CH₃, and -CH₂OCH₂CH₂CH₃;
vii) -(CR^{21a}R^{21b})_{z}C(O)R²⁰; for example, -COCH₃, -CH₂COCH₃, -COCH₂CH₃, -CH₂COCH₂CH₃, -COCH₂CH₂CH₃, and -CH₂COCH₂CH₂CH₃;
viii) -(CR^{21a}R^{21b})_{z}C(O)OR²⁰; for example, -CO₂CH₃, -CH₂CO₂CH₃, -CO₂CH₂CH₃, -CH₂CO₂CH₂CH₃, -CO₂CH₂CH₂CH₃, and -CH₂CO₂CH₂CH₂CH₃;
xii) -(CR^{21a}R^{21b})_{z}C(O)N(R²⁰)₂; for example, -CONH₂, -CH₂CONH₂, -CONHCH₃, -CH₂CONHCH₃, -CON(CH₃)₂, and -CH₂CON(CH₃)₂;
x) -(CR^{21a}R^{21b})_{z}N(R²⁰)₂; for example, -NH₂, -CH₂NH₂, -NHCH₃, -CH₂NHCH₃, -N(CH₃)₂, and -CH₂N(CH₃)₂;
xi) halogen; -F, -Cl, -Br, and -I;
xii) -(CR^{21a}R^{21b})_{z}CN;
xiii) -(CR^{21a}R^{21b})_{z}NO₂;
xiv) -(CH_{j'}X_{k'})ₕCHⱼXₖ; wherein X is halogen, the index j is an integer from 0 to 2, j + k = 3, the index j' is an integer from 0 to 2, j' + k' = 2, the index h is from 0 to 6; for example, -CH₂F, -CHF₂, -CF₃, -CH₂CF₃, -CHFCF₃, -CCl₃, or -CBr₃;
xv) -(CR^{21a}R^{21b})_{z}SR²⁰; -SH, -CH₂SH, -SCH₃, -CH₂SCH₃, -SC₆H₅, and -CH₂SC₆H₅;
xvi) -(CR^{21a}R^{21b})_{z}SO₂R²⁰; for example, -SO₂H, -CH₂SO₂H, -SO₂CH₃, -CH₂SO₂CH₃, -SO₂C₆H₅, and -CH₂SO₂C₆H₅; and
xvii) -(CR^{21a}R^{21b})_{z}SO₃R²⁰; for example, -SO₃H, -CH₂SO₃H, -SO₃CH₃, -CH₂SO₃CH₃, -SO₃C₆H₅, and -CH₂SO₃C₆H₅;
wherein each R²⁰ is independently hydrogen, substituted or unsubstituted C₁-C₄ linear, C₃-C₄ branched, or C₃-C₄ cyclic alkyl, phenyl, benzyl, heterocyclic, or heteroaryl; or two R²⁰ units can be taken together to form a ring comprising 3-7 atoms; R^{21a} and R^{21b} are each independently hydrogen or C₁-C₄ linear or C₃-C₄ branched alkyl; the index p is from 0 to 4.

One example of R units includes compounds wherein R units have the formula: 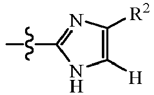 wherein R³ is hydrogen and R² is a unit chosen from methyl (C₁), ethyl (C₂), n-propyl (C₃), iso-propyl (C₃), n-butyl (C₄), *sec*-butyl (C₄), *iso*-butyl (C₄), and *tert*-butyl (C₄).

Another example of R units includes compounds wherein R² is a unit chosen from methyl (C₁), ethyl (C₂), n-propyl (C₃), iso-propyl (C₃), n-butyl (C₄), *sec*-butyl (C₄), *iso*-butyl (C₄), and *tert*-butyl (C₄); and R³ is a unit chosen from methyl (C₁) or ethyl (C₂). Non-limiting examples of this aspect of R includes 4,5-dimethylimidazol-2-yl, 4-ethyl-5-methylimidazol-2-yl, 4-methyl-5-ethylimidazol-2-yl, and 4,5-diethylimidazol-2-yl.

An example of R units includes compounds wherein R³ is hydrogen and R² is a substituted alkyl unit chosen, said substitutions chosen from:
i) halogen: -F, -Cl, -Br, and -I;
ii) -N(R¹¹)₂; and
iii) -OR¹¹;
wherein each R¹¹ is independently hydrogen or C₁-C₄ linear or C₃-C₄ branched alkyl.

Non-limiting examples of units comprising this embodiment of R includes: -CH₂F, -CHF₂, -CF₃, -CH₂CF₃, -CH₂Cl, -CH₂OH, -CH₂OCH₃, -CH₂CH₂OH, -CH₂CH₂OCH₃, -CH₂NH₂, -CH₂NHCH₃, -CH₂N(CH₃)₂, and -CH₂NH(CH₂CH₃).

A yet further example of R units include units wherein R³ is hydrogen and R² is phenyl.

A still further example of R units include units wherein R³ is hydrogen and R² is a heteroaryl unit chosen from 1,2,3,4-tetrazol-1-yl,1,2,3 ,4-tetrazol-5-yl, [1,2,3]triazol-4-yl, [1,2,3]triazol-5-yl, [1,2,4]triazol-4-yl, [1,2,4]triazol-5-yl, imidazol-2-yl, imidazol-4-yl, pyrrol-2-yl, pyrrol-3-yl, oxazol-2-yl, oxazol-4-yl, oxazol-5-yl, isoxazol-3-yl, isoxazol-4-yl, isoxazol-5-yl, [1,2,4]oxadiazol-3-yl, [1,2,4]oxadiazol-5-yl, [1,3,4]oxadiazol-2-yl, furan-2-yl, furan-3-yl, thiophen-2-yl, thiophen-3-yl, isothiazol-3-yl, isothiazol-4-yl, isothiazol-5-yl, thiazol-2-yl, thiazol-4-yl, thiazol-5-yl, [1,2,4]thiadiazol-3-yl, [1,2,4]thiadiazol-5-yl, and [1,3,4]thiadiazol-2-yl.

### Z Units

Z is a unit having the formula:

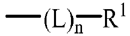

R¹ is chosen from:
i) hydrogen;
ii) hydroxyl;
iii) amino;
iv) substituted or unsubstituted C₁-C₆ linear, C₃-C₆ branched or C₃-C₆ cyclic alkyl;
v) substituted or unsubstituted C₁-C₆ linear, C₃-C₆ branched o C₃-C₆r cyclic alkoxy;
vi) substituted or unsubstituted C₆ or C₁₀ aryl;
vii) substituted or unsubstituted C₁-C₉ heterocyclic rings; or
viii) substituted or unsubstituted C₁-C₉ heteroaryl rings.

The following are non-limiting examples of units that can substitute for one or more hydrogen atoms on the R¹ units. The following substituents, as well as others not herein described, are each independently chosen:
i) C₁-C₁₂ linear, C₃-C₁₂ branched, or C₃-C₁₂ cyclic alkyl, alkenyl, and alkynyl; methyl (C₁), ethyl (C₂), ethenyl (C₂), ethynyl (C₂), n-propyl (C₃), *iso*-propyl (C₃), cyclopropyl (C₃), 3-propenyl (C₃), 1-propenyl (*also* 2-methylethenyl) (C₃), isopropenyl (*also* 2-methylethen-2-yl) (C₃), prop-2-ynyl (*also* propargyl) (C₃), propyn-1-yl (C₃), n-butyl (C₄), *sec*-butyl (C₄), *iso*-butyl (C₄), *tert*-butyl (C₄), cyclobutyl (C₄), buten-4-yl (C₄), cyclopentyl (C₅), cyclohexyl (C₆);
ii) substituted or unsubstituted C₆ or C₁₀ aryl; for example, phenyl, naphthyl (also referred to herein as naphthylen-1-yl (C₁₀) or naphthylen-2-yl (C₁₀));
iii) substituted or unsubstituted C₆ or C₁₀ alkylenearyl; for example, benzyl, 2-phenylethyl, naphthylen-2-ylmethyl;
iv) substituted or unsubstituted C₁-C₉ heterocyclic rings; as described herein;
v) substituted or unsubstituted C₁-C₉ heteroaryl rings; as described herein;
vi) -(CR^{31a}R^{31b})_{q}OR³⁰; for example, -OH, -CH₂OH, -OCH₃, -CH₂OCH₃, -OCH₂CH₃, -CH₂OCH₂CH₃, -OCH₂CH₂CH₃, and -CH₂OCH₂CH₂CH₃;
vii) -(CR^{31a}R^{31b})_{q}C(O)R³⁰; for example, -COCH₃, -CH₂COCH₃, -COCH₂CH₃, -CH₂COCH₂CH₃, -COCH₂CH₂CH₃, and -CH₂COCH₂CH₂CH₃;
viii) -(CR^{31a}R^{31b})_{q}C(O)OR³⁰; for example, -CO₂CH₃, -CH₂CO₂CH₃, -CO₂CH₂CH₃, -CH₂CO₂CH₂CH₃, -CO₂CH₂CH₂CH₃, and -CH₂CO₂CH₂CH₂CH₃;
xiii) -(CR^{31a}R^{31b})_{q}C(O)N(R³⁰)₂; for example, -CONH₂, -CH₂CONH₂, -CONHCH₃, -CH₂CONHCH₃, -CON(CH₃)₂, and -CH₂CON(CH₃)₂;
x) -(CR ^{31a}R^{31b} )_{q}N(R³⁰)₂; for example, -NH₂, -CH₂NH₂, -NHCH₃, -CH₂NHCH₃, -N(CH₃)₂, and -CH₂N(CH₃)₂;
xi) halogen; -F, -Cl, -Br, and -I;
xii) -(CR^{31a}R^{31b})_{q}CN;
xiii) -(CR^{31a}R^{31b})_{q}NO₂;
xiv) -(CH_{j'}X_{k'})ₕCHⱼXₖ; wherein X is halogen, the index j is an integer from 0 to 2, j + k = 3, the index j' is an integer from 0 to 2, j' + k' = 2, the index h is from 0 to 6; for example, -CH₂F, -CHF₂, -CF₃, -CH₂CF₃, -CHFCF₃, -CCl₃, or -CBr₃;
xv) -(CR^{31a}R^{31b})_{q}SR³⁰; -SH, -CH₂SH, -SCH₃, -CH₂SCH₃, -SC₆H₅, and -CH₂SC₆H₅;
xvi) -(CR^{31a}R^{31b} )_{q}SO₂R³⁰; for example, -SO₂H, -CH₂SO₂H, -SO₂CH₃, -CH₂SO₂CH₃, -SO₂C₆H₅, and -CH₂SO₂C₆H₅; and
xvii) -(CR^{31a}R^{31b})_{q}SO₃R³⁰; for example, -SO₃H, -CH₂SO₃H, -SO₃CH₃, -CH₂SO₃CH₃, -SO₃C₆H₅, and -CH₂SO₃C₆H₅;
wherein each R³⁰ is independently hydrogen, substituted or unsubstituted C₁-C₆ linear, C₃-C₆ branched, or C₃-C₆ cyclic alkyl, phenyl, benzyl, heterocyclic, or heteroaryl; or two R³⁰ units can be taken together to form a ring comprising 3-7 atoms; R^{31a} and R^{31b} are each independently hydrogen or C₁-C₄ linear or C₃-C₄ branched alkyl; the index q is from 0 to 4.

One example of R¹ units includes substituted or unsubstituted phenyl (C₆ aryl) units, wherein each substitution is independently chosen from: halogen, C₁-C₄ linear, branched alkyl, or cyclic alkyl, -OR¹¹, -CN, -N(R¹¹)₂, -CO₂R¹¹, -C(O)N(R¹¹)₂, -NR¹¹C(O)R¹¹, -NO₂, and -SO₂R¹¹; each R¹¹ is independently hydrogen; substituted or unsubstituted C₁-C₄ linear, C₃-C₄ branched, C₃-C₄ cyclic alkyl, alkenyl, or alkynyl; substituted or unsubstituted phenyl or benzyl; or two R¹¹ units can be taken together to form a ring comprising from 3-7 atoms.

Another example of R¹ units includes substituted C₆ aryl units chosen from phenyl, 2-fluorophenyl, 3-fluorophenyl, 4-fluorophenyl, 2,3-difluorophenyl, 3,4-difluorophenyl, 3,5-difluorophenyl, 2-chlorophenyl, 3-chlorophenyl, 4-chlorophenyl, 2,3-dichlorophenyl, 3,4-dichlorophenyl, 3,5-dichlorophenyl, 2-hydroxyphenyl, 3-hydroxyphenyl, 4-hydroxyphenyl, 2-methoxyphenyl, 3-methoxyphenyl, 4-methoxyphenyl, 2,3-dimethoxyphenyl, 3,4-dimethoxyphenyl, and 3,5-dimethoxyphenyl.

A further example of R¹ units includes substituted or unsubstituted C₆ aryl units chosen from 2,4-difluorophenyl, 2,5-difluorophenyl, 2,6-difluorophenyl, 2,3,4-trifluorophenyl, 2,3,5-trifluorophenyl, 2,3,6-trifluorophenyl, 2,4,5-trifluorophenyl, 2,4,6-trifluorophenyl, 2,4-dichlorophenyl, 2,5-dichlorophenyl, 2,6-dichlorophenyl, 3,4-dichlorophenyl, 2,3,4-trichlorophenyl, 2,3,5-trichlorophenyl, 2,3,6-trichlorophenyl, 2,4,5-trichlorophenyl, 3,4,5-trichlorophenyl, and 2,4,6-trichlorophenyl.

A yet further example of R¹ units includes substituted C₆ aryl units chosen from 2-methylphenyl, 3-methylphenyl, 4-methylphenyl, 2,3-dimethylphenyl, 2,4-dimethylphenyl, 2,5-dimethylphenyl, 2,6-dimethylphenyl, 3,4-dimethylphenyl, 2,3,4-trimethylphenyl, 2,3,5-trimethylphenyl, 2,3,6-trimethylphenyl, 2,4,5-trimethylphenyl, 2,4,6-trimethylphenyl, 2-ethylphenyl, 3-ethylphenyl, 4-ethylphenyl, 2,3-diethylphenyl, 2,4-diethylphenyl, 2,5-diethylphenyl, 2,6-diethylphenyl, 3,4-diethylphenyl, 2,3,4-triethylphenyl, 2,3,5-triethylphenyl, 2,3,6-triethylphenyl, 2,4,5-triethylphenyl, 2,4,6-triethylphenyl, 2-isopropylphenyl, 3-isopropylphenyl, and 4-isopropylphenyl.

Another still further example of R¹ units includes substituted C₆ aryl units chosen from 2-aminophenyl, 2-(*N*-methylamino)phenyl, 2-(*N*,*N*-dimethylamino)phenyl, 2-(*N-*ethylamino)phenyl, 2-(*N*,*N*-diethylamino)phenyl, 3-aminophenyl, 3-(*N-*methylamino)phenyl, 3-(*N*,*N*-dimethylamino)phenyl, 3-(*N*-ethylamino)phenyl, 3-(*N*,*N-*diethylamino)phenyl, 4-aminophenyl, 4-(*N*-methylamino)phenyl, 4-(*N,N-*dimethylamino)phenyl, 4-(*N*-ethylamino)phenyl, and 4-(*N*,*N*-diethylamino)phenyl.

R¹ can comprise heteroaryl units. Non-limiting examples of C₁-C₉ heteroaryl units include:
i) 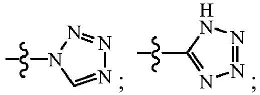
ii) 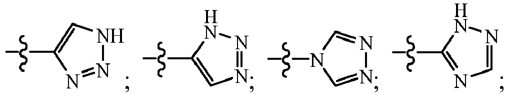
iii) 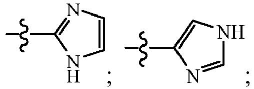
iv) 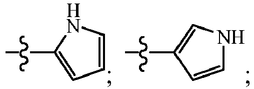
v) 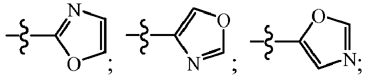
vi) 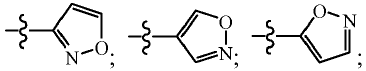
vii) 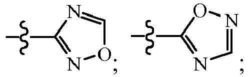
viii) 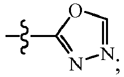
ix) 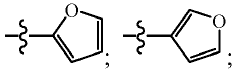
x) 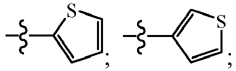
xi) 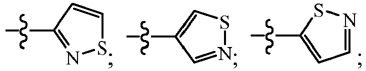
xii) 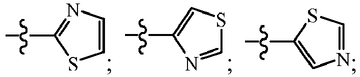
xiii) 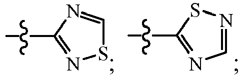 and
xiv)

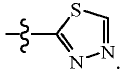

R¹ heteroaryl units can be substituted or unsubstituted. Non-limiting examples of units that can substitute for hydrogen include units chosen from:
i) C₁-C₆ linear, C₃-C₆ branched, and C₃-C₆ cyclic alkyl;
ii) substituted or unsubstituted phenyl and benzyl;
iii) substituted of unsubstituted C₁-C₉ heteroaryl;
iv) -C(O)R⁹; and
v) -NHC(O)R⁹;
wherein R⁹ is C₁-C₆ linear and branched alkyl; C₁-C₆ linear and C₃-C₆ branched alkoxy; or -NHCH₂C(O)R¹⁰; R¹⁰ is chosen from hydrogen, methyl, ethyl, and *tert*-butyl.

An example of R¹ relates to units substituted by an alkyl unit chosen from methyl, ethyl, n-propyl, *iso*-propyl, n-butyl, *iso*-butyl, *sec*-butyl, and *tert*-butyl.

Another example of R¹ includes units that are substituted by substituted or unsubstituted phenyl and benzyl, wherein the phenyl and benzyl substitutions are chosen from one or more:
i) halogen;
ii) C₁-C₃ alkyl;
iii) C₁-C₃ alkoxy;
iv) -CO₂R¹¹; and
v) -NHCOR¹⁶;
wherein R¹¹ and R¹⁶ are each independently hydrogen, methyl, or ethyl.

Another example of R¹ relates to phenyl and benzyl units substituted by a carboxy unit having the formula -C(O)R⁹; R⁹ is chosen from methyl, methoxy, ethyl, and ethoxy.

A further example of R¹ includes phenyl and benzyl units substituted by an amide unit having the formula NHC(O)R⁹; R⁹ is chosen from methyl, methoxy, ethyl, ethoxy, *tert-*butyl*,* and *tert*-butoxy.

A yet further example of R¹ includes phenyl and benzyl units substituted by one or more fluoro or chloro units.

### L Units

L is a linking unit which is present when the index n is equal to 1, but is absent when the index n is equal to 0. L units have the formula:

-[Q]_{y}[C(R^{5a}R^{5b})]ₓ[Q¹]_{z}[C(R^{6a}R^{6b})]_{w}-

wherein Q and Q¹ are each independently:
i) -C(O)-;
ii) -NH-;
iii) -C(O)NH-;
iv) -NHC(O)-;
v) -NHC(O)NH-;
vi) -NHC(O)O-;
vii) -C(O)O-;
viii) -C(O)NHC(O)-;
ix) -O-;
x) -S-;
xi) -SO₂-;
xii) -C(=NH)-;
xiii) -C(=NH)NH-;
xiv) -NHC(=NH)-; or
xv) -NHC(=NH)NH-.
When the index y is equal to 1, Q is present. When the index y is equal to 0, Q is absent. When the index z is equal to 1, Q¹ is present. When the index z is equal to 0, Q¹ is absent.

R^{5a} and R^{5b} are each independently:
i) hydrogen;
ii) hydroxy;
iii) halogen;
iv) substituted or unsubstituted C₁-C₆ linear or C₃-C₆ branched alkyl; or
v) a unit having the formula:

   -[C(R^{7a}R^{7b})]ₜR⁸
wherein R^{7a} and R^{7b} are each independently:
i) hydrogen; or
ii) substituted or unsubstituted C₁-C₆ linear, C₃-C₆ branched, or C₃-C₆ cyclic alkyl.
R⁸ is:
i) hydrogen;
ii) substituted or unsubstituted C₁-C₆ linear, C₃-C₆ branched, or C₃-C₆ cyclic alkyl;
iii) substituted or unsubstituted C₆ or C₁₀ aryl;
iv) substituted or unsubstituted C₁-C₉ heteroaryl; or
v) substituted or unsubstituted C₁-C₉ heterocyclic.
R^{6a} and R^{6b} are each independently:
i) hydrogen; or
ii) C₁-C₄ linear or C₃-C₄ branched alkyl.
The indices t, w and x are each independently from 0 to 4.

The following are non-limiting examples of units that can substitute for one or more hydrogen atoms on R^{5a}, R^{5b}, R^{7a}, R^{7b}, and R⁸ units. The following substituents, as well as others not herein described, are each independently chosen:
i) C₁-C₁₂ linear, branched, or cyclic alkyl, alkenyl, and alkynyl; methyl (C₁), ethyl (C₂), ethenyl (C₂), ethynyl (C₂), n-propyl (C₃), *iso*-propyl (C₃), cyclopropyl (C₃), 3-propenyl (C₃), 1-propenyl (*also* 2-methylethenyl) (C₃), isopropenyl (*also* 2-methylethen-2-yl) (C₃), prop-2-ynyl (*also* propargyl) (C₃), propyn-1-yl (C₃), n-butyl (C₄), *sec*-butyl (C₄), *iso*-butyl (C₄), *tert*-butyl (C₄), cyclobutyl (C₄), buten-4-yl (C₄), cyclopentyl (C₅), cyclohexyl (C₆);
ii) substituted or unsubstituted C₆ or C₁₀ aryl; for example, phenyl, naphthyl (also referred to herein as naphthylen-1-yl (C₁₀) or naphthylen-2-yl (C₁₀));
iii) substituted or unsubstituted C₆ or C₁₀ alkylenearyl; for example, benzyl, 2-phenylethyl, naphthylen-2-ylmethyl;
iv) substituted or unsubstituted C₁-C₉ heterocyclic rings; as described herein below;
v) substituted or unsubstituted C₁-C₉ heteroaryl rings; as described herein below;
vi) -(CR^{41a}R^{41b})ᵣOR⁴⁰; for example, -OH, -CH₂OH, -OCH₃, -CH₂OCH₃, -OCH₂CH₃, -CH₂OCH₂CH₃, -OCH₂CH₂CH₃, and -CH₂OCH₂CH₂CH₃;
vii) -(CR^{41a}R^{41b} )ᵣC(O)R⁴⁰; for example, -COCH₃, -CH₂COCH₃, -COCH₂CH₃, -CH₂COCH₂CH₃, -COCH₂CH₂CH₃, and -CH₂COCH₂CH₂CH₃;
viii) -(CR^{41a}R^{41b})ᵣC(O)OR⁴⁰; for example, -CO₂CH₃, -CH₂CO₂CH₃, -CO₂CH₂CH₃, -CH₂CO₂CH₂CH₃, -CO₂CH₂CH₂CH₃, and -CH₂CO₂CH₂CH₂CH₃;
xiv) -(CR^{41a}R^{41b})ᵣC(O)N(R⁴⁰)₂; for example, -CONH₂, -CH₂CONH₂, -CONHCH₃, -CH₂CONHCH₃, -CON(CH₃)₂, and -CH₂CON(CH₃)₂;
x) -(CR^{41a}R^{41b})ᵣN(R⁴⁰)₂; for example, -NH₂, -CH₂NH₂, -NHCH₃, -CH₂NHCH₃, -N(CH₃)₂, and -CH₂N(CH₃)₂;
xi) halogen; -F, -Cl, -Br, and -I;
xii) -(CR^{41a}R^{41b})ᵣCN;
xiii) -(CR^{41a}R^{41b})ᵣNO₂;
xiv) -(CH_{j'}X_{k'})ₕCHⱼXₖ; wherein X is halogen, the index j is an integer from 0 to 2, j + k = 3, the index j' is an integer from 0 to 2, j' + k' = 2, the index h is from 0 to 6; for example, -CH₂F, -CHF₂, -CF₃, -CH₂CF₃, -CHFCF₃, -CCl₃, or -CBr₃;
xv) -(CR^{41a}R^{41b})ᵣSR⁴⁰; -SH, -CH₂SH, -SCH₃, -CH₂SCH₃, -SC₆H₅, and -CH₂SC₆H₅;
xvi) -(CR^{41a}R^{41b})ᵣSO₂R⁴⁰; for example, -SO₂H, -CH₂SO₂H, -SO₂CH₃, -CH₂SO₂CH₃, -SO₂C₆H₅, and -CH₂SO₂C₆H₅; and
xvii) -(CR^{41a}R^{41b})ᵣSO₃R⁴⁰; for example, -SO₃H, -CH₂SO₃H, -SO₃CH₃, -CH₂SO₃CH₃, -SO₃C₆H₅, and -CH₂SO₃C₆H₅;
wherein each R⁴⁰ is independently hydrogen, substituted or unsubstituted C₁-C₆ linear, C₃-C₆ branched, or C₃-C₆ cyclic alkyl, phenyl, benzyl, heterocyclic, or heteroaryl; or two R⁴⁰ units can be taken together to form a ring comprising 3-7 atoms; R^{41a} and R^{41b} are each independently hydrogen or C₁-C₄ linear or C₃-C₄ branched alkyl; the index r is from 0 to 4.

One example of L units relates to units having the formula:

-C(O)[C(R^{5a}R^{5b})]ₓNHC(O)-

wherein R^{5a} is hydrogen, substituted or unsubstituted C₁-C₄ alkyl, substituted or unsubstituted phenyl, and substituted or unsubstituted heteroaryl; and the index x is 1 or 2. One embodiment relates to linking units having the formula:
i) -C(O)[C(R^{5a}H)]NHC(O)O-;
ii) -C(O)[C(R^{5a}H)][CH₂]NHC(O)O-;
ii) -C(O)[CH₂][C(R^{5a}H)]NHC(O)O-;
iv) -C(O)[C(R^{5a}H)]NHC(O)-;
v) -C(O)[C(R^{5a}H)][CH₂]NHC(O)-; or
vi) -C(O)[CH₂][C(R^{5a}H)]NHC(O)-;
wherein R^{5a} is:
i) hydrogen;
ii) methyl;
iii) ethyl;
iv) isopropyl;
v) phenyl;
vi) benzyl;
vii) 4-hydroxybenzyl;
viii) hydroxymethyl; or
ix) 1 -hydroxyethyl.

When the index x is equal to 1, this disclosure provides the following non-limiting examples of L units:

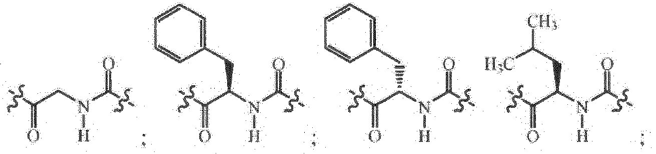

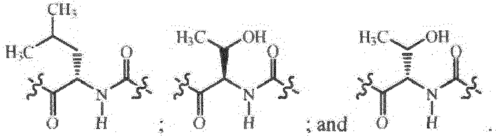

When the index x is equal to 2, this provides the following non-limiting examples of L units:

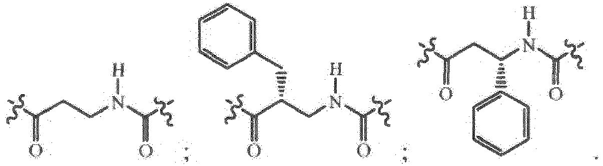

Another example of L units includes units wherein Q is -C(O)-, the indices x and z are equal to 0, w is equal to 1 or 2, a first R*^{6a}* unit chosen from phenyl, 2-fluorophenyl, 3-fluorophenyl, 4-fluorophenyl, 2,3-difluorophenyl, 3,4-difluorophenyl, 3,5-difluorophenyl, 2-chlorophenyl, 3-chlorophenyl, 4-chlorophenyl, 2,3-dichlorophenyl, 3,4-dichlorophenyl, 3,5-dichlorophenyl, 2-hydroxyphenyl, 3-hydroxyphenyl, 4-hydroxyphenyl, 2-methoxyphenyl, 3-methoxyphenyl, 4-methoxyphenyl, 2,3-dimethoxyphenyl, 3,4-dimethoxyphenyl, and 3,5-dimethoxyphenyl; a second R^{6a} unit is hydrogen and R^{6b} units are hydrogen. For example a linking unit having the formula:

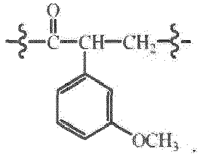

A further example of L includes a first R^{6a} unit as depicted herein above that is a
substituted or unsubstituted heteroaryl unit as described herein above.

A yet further example of L includes units having the formula:

-C(O)[C(R^{6a}R^{6b})]_{w}-;

wherein R^{6a} and R^{6b} are hydrogen and the index w is equal to 1 or 2; said units chosen from:
i) -C(O)CH₂-; and
ii) -C(O)CH₂CH₂-.

Another example of L units includes units having the formula:

-C(O)[C(R^{5a}R^{5b})]ₓC(O)-;

wherein R^{5a} and R^{5b} are hydrogen and the index x is equal to 1 or 2; said units chosen from:
i) -C(O)CH₂C(O)-; and
ii) -C(O)CH₂CH₂C(O)-

Still further examples of L units includes units having the formula:

-C(O)NH[C(R^{5a}R^{5b})]ₓ-;

wherein R^{5a} and R^{5b} are hydrogen and the index w is equal to 0, 1 or 2; said units chosen from:
ii) -C(O)NH-;
ii) -C(O)NHCH₂-; and
iii) -C(O)NHCH₂CH₂-.

A yet still further example of L units includes units having the formula:

-SO₂[C(R^{6a}R^{6b})]_{w}-;

wherein R^{8a} and R^{8b} are hydrogen or methyl and the index w is equal to 0, 1 or 2; said units chosen from:
i) -SO₂-;
ii) -SO₂CH₂-; and
iii) -SO₂CH₂CH₂-.

The disclosed compounds (analogs) are arranged into several Categories to assist the formulator in applying a rational synthetic strategy for the preparation of analogs which are not expressly exampled herein. The arrangement into categories does not imply increased or decreased efficacy for any of the compositions of matter described herein.

A described herein above the disclosed compounds include all pharmaceutically acceptable salt forms. A compound having the formula: 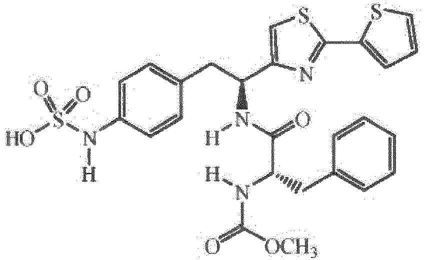 can form salts, for example, a salt of the sulfamic acid: 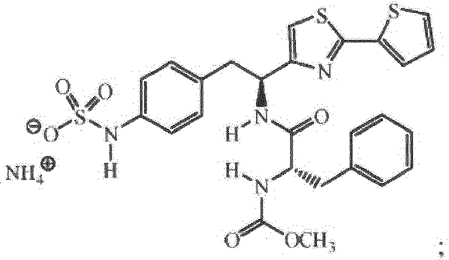 and

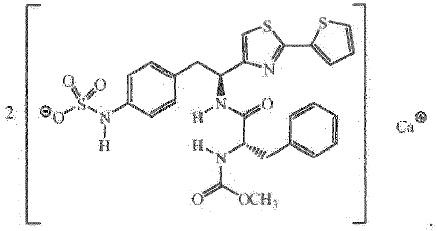

The compounds can also exist in a zwitterionic form, for example: 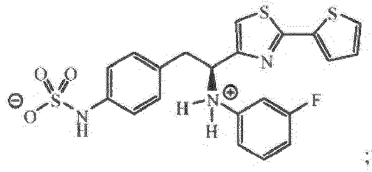 or
as a salt of a strong acid, for example:

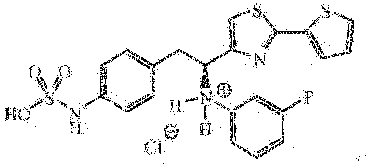

Category I of the present disclosure relates to compounds falling outside the scope of the present invention wherein R is a substituted or unsubstituted thiazol-2-yl unit having the formula: 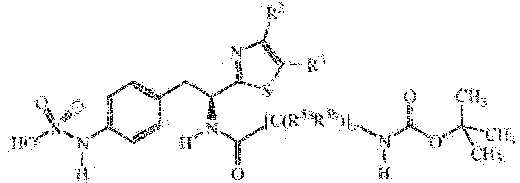 one example of which relates to inhibitors having the formula: 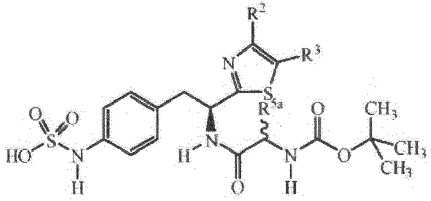 wherein R units are thiazol-2-yl units, that when substituted, are substituted with R² and R³ units. R and R^{5a} units are further described in Table I.

**TABLE I**

| **No.** | **R** | **R^{5a}** |
|---|---|---|
| A1 | thiazol-2-yl | (*S*)-benzyl |
| A2 | 4-methylthiazol-2-yl | (*S*)-benzyl |
| A3 | 4-ethylthiazol-2-yl | (*S*)-benzyl |
| A4 | 4-propylthiazol-2-yl | (*S*)-benzyl |
| A5 | 4-*iso*-propylthiazol-2-yl | (*S*)-benzyl |
| A6 | 4-cyclopropylthiazol-2-yl | (*S*)-benzyl |
| A7 | 4-butylthiazol-2-yl | (*S*)-benzyl |
| A8 | 4-*tert*-butylthiazol-2-yl | (*S*)-benzyl |
| A9 | 4-cyclohexylthiazol-2-yl | (*S*)-benzyl |
| A10 | 4-(2,2,2-trifluoroethyl)thiazol-2-yl | (*S*)-benzyl |
| A11 | 4-(3,3,3-tritluorypropyl)thiazol-2-yl | (*S*)-benzyl |
| A12 | 4-(2,2-difluorocyclopropyl)thiazol-2-yl | (*S*)-benzyl |
| A13 | 4-(methoxymethyl)thiazol-2-yl | (*S*)-benzyl |
| A14 | 4-(carboxylic acid ethyl ester)thiazol-2-yl | (*S*)-benzyl |
| A15 | 4,5-dimethylthiazol-2-yl | (*S*)-benzyl |
| A16 | 4-methyl-5-ethylthiazol-2-yl | (*S*)-benzyl |
| A17 | 4-phenylthiazol-2-yl | (*S*)-benzyl |
| A18 | 4-(4-chlorophenyl)thiazol-2-yl | (*S*)-benzyl |
| A19 | 4-(3,4-dimethylphenyl)thiazol-2-yl | (*S*)-benzyl |
| A20 | 4-methyl-5-phenylthiazol-2-yl | (*S*)-benzyl |
| A21 | 4-(thiophen-2-yl)thiazol-2-yl | (*S*)-benzyl |
| A22 | 4-(thiophen-3-yl)thiazol-2-yl | (*S*)-benzyl |
| A23 | 4-(5-chlorothiophen-2-yl)thiazol-2-yl | (*S*)-benzyl |
| A24 | 5,6-dihydro-4*H*-cyclopenta[*d*]thiazol-2-yl | (*S*)-benzyl |
| A25 | 4,5,6,7-tetrahydrobenzo[*d*]thiazol-2-yl | (*S*)-benzyl |

The compounds encompassed within the first type of Category I of the present disclosure can be prepared by the procedure outlined in Scheme I and described in Example 1 herein below.

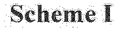

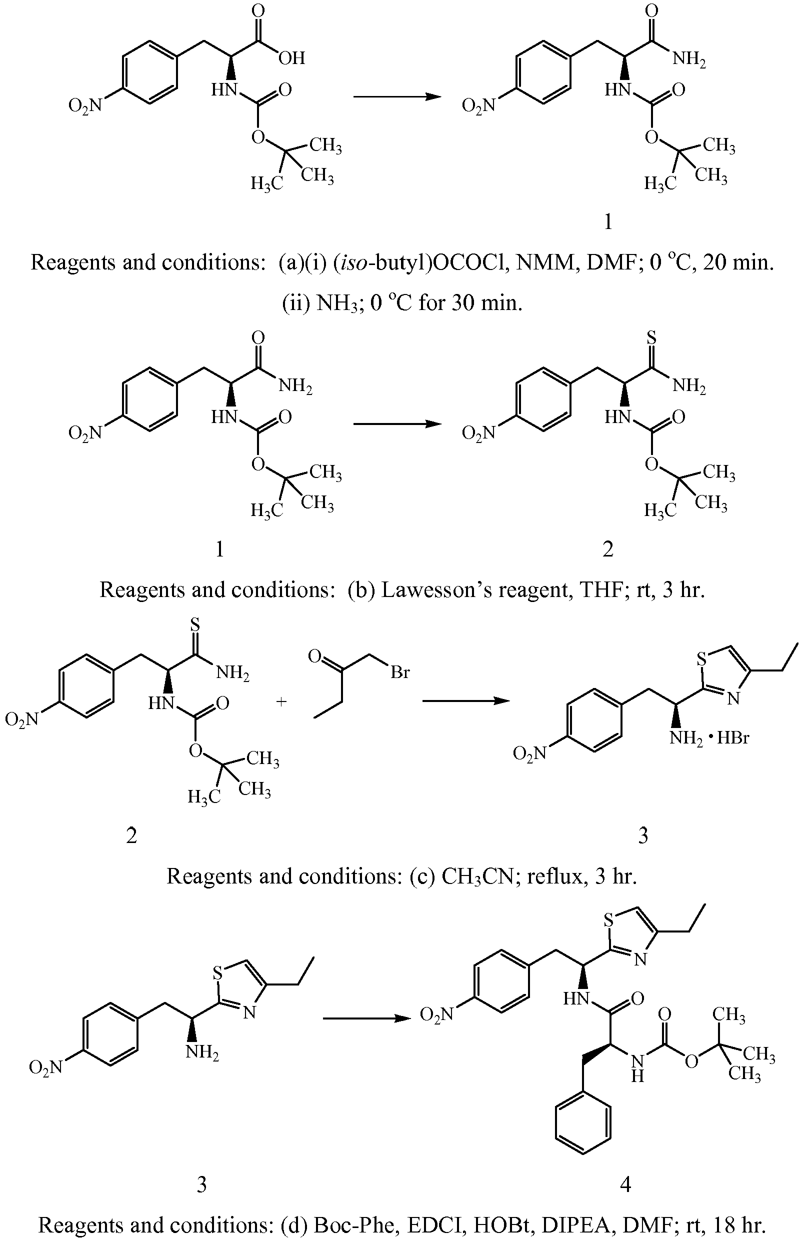

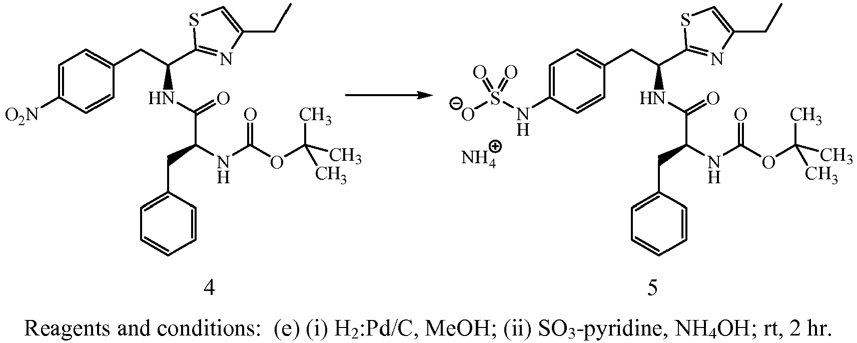

### EXAMPLE 1

### 4-{(S)-2-[(S)-2-(tert-Butoxycarbonylamino)-3-phenylpropanamido]-2-(4-ethylthiazol-2-yl)ethyl}phenylsulfamic acid (5)

Preparation of [1-(S)-carbamoyl-2-(4-nitrophenyl)ethyl-carbamic acid *tert-*butyl ester (1): To a 0 °C solution of 2-(*S*)-*tert*-butoxycarbonylamino-3-(4-nitrophenyl)-propionic acid and *N*-methylmorpholine (1.1 mL, 9.65 mmol) in DMF (10 mL) is added dropwise *iso-*butyl chloroformate (1.25 mL, 9.65 mmol). The mixture is stirred at 0 °C for 20 minutes after which NH₃ (g) is passed through the reaction mixture for 30 minutes at 0 °C. The reaction mixture is concentrated and the residue dissolved in EtOAc, washed successively with 5% citric acid, water, 5% NaHCO₃, water and brine, dried (Na₂SO₄), filtered and concentrated in vacuo to a residue that is triturated with a mixture of EtOAc/petroleum ether to provide 2.2 g (74%) of the desired product as a white solid.

Preparation of [2-(4-nitrophenyl)-1-(*S*)-thiocarbamoylethyl]carbamic acid *tert-*butyl ester (2): To a solution of [1-(*S*)-carbamoyl-2-(4-nitrophenyl)ethyl-carbamic acid *tert*-butyl ester, 1, (0.400 g, 1.29 mmol) in THF (10 mL) is added Lawesson's reagent (0.262 g. 0.65 mmol). The reaction mixture is stirred for 3 hours and concentrated to a residue which is purified over silica to provide 0.350 g (83%) of the desired product. ¹H NMR (300 MHz, CDCl₃) δ 8.29 (s, 1H), 8.10 (d. *J* = 8.4 Hz, 2H), 8.01 (s, 1H), 7.42 (d, *J* = 8.4 Hz, 2H), 5.70 (d, *J* = 7.2 Hz, 1H), 4.85 (d, *J* = 7.2 Hz, 1H), 3.11-3.30 (m, 1H), 1.21 (s, 9H).

Preparation of 1-(S)-(4-ethylthiazol-2-yl)-2-(4-nitrophenyl)ethyl amine (3): A mixture of [2-(4-nitrophenyl)-1-(*S*)-thiocarbamoylethyl]-carbamic acid *tert*-butyl ester, 2, (0.245 g, 0.753 mmol), 1-bromo-2-butanone (0.125 g, 0.828 mmol) in CH₃CN (5 mL) is refluxed 3 hours. The reaction mixture is cooled to room temperature and diethyl ether is added to the solution and the precipitate which forms is removed by filtration. The solid is
dried under vacuum to afford 0.242 g (90% yield) of the desired product. ESI+ MS 278 (M+1).

Preparation of { 1-[1-(4-ethylthiazol-2-y1)-2-(4-nitrophenyl)ethylcarbamoyl]-2-phenylethyl} carbamic acid *tert-*butyl ester (4): To a solution of 1-(S)-(4-ethylthiazol-2-yl)-2-(4-nitrophenyl)ethyl amine hydrobromide, 3, (0.393 g, 1.1 mmol), (S)-(2-*tert-*butoxycarbonylamino)-3-phenylpropionic acid (0.220 g, 0.828 mmol) and 1-hydroxybenzotriazole (HOBt) (0.127 g, 0.828 mmol) in DMF (10 mL) at 0 °C, is added 1-(3-dimethylaminopropyl)-3-ethylcarbodiimide (EDCI) (0.159 g, 0.828 mmol) followed by diisopropylamine (0.204 g, 1.58 mmol). The mixture is stirred at 0 °C for 30 minutes then at room temperature overnight. The reaction mixture is diluted with water and extracted with EtOAc. The combined organic phase is washed with 1 N aqueous HCl, 5 % aqueous NaHCO₃, water and brine, and dried over Na₂SO₄. The solvent is removed *in vacuo* to afford 0.345 g of the desired product which is used without further purification. LC/MS ESI+ 525 (M+1).

Preparation of 4-{(S)-2-[(S)-2-(*tert*-butoxycarbonylarnino)-3-phenylpropanamido]- 2-(4-ethylthiazol-2-yl)ethyl}phenylsulfamic acid ammonium salt (5): { 1-[1-(4-ethylthiazol-2-yl)-2-(4-nitrophenyl)ethylcarbamoyl]-2-phenylethyl} carbamic acid *tert*-butyl ester, 4, (0.345 g) is dissolved in MeOH (4 mL). A catalytic amount of Pd/C (10% w/w) is added and the mixture is stirred under a hydrogen atmosphere 2 hours. The reaction mixture is filtered through a bed of CELITE™ and the solvent is removed under reduced pressure. The crude product is dissolved in pyridine (12 mL) and treated with SO₃-pyridine (0.314 g). The reaction is stirred at room temperature for 5 minutes after which a 7% solution of NH₄OH (50 mL) is added. The mixture is then concentrated and the resulting residue is purified by reverse phase chromatography to afford 0.222 g of the desired product as the ammonium salt. ¹H NMR (CD₃OD): δ 7.50-6.72 (m, 10H), 5.44-5.42 (d, 1H, *J*=6.0 Hz), 4.34 (s, 1H), 3.34-2.79 (m, 4H), 2.83-2.76 (q, 2H, *J*=7.2 Hz), 1.40 (s, 9H), 1.31 (t, 3H, *J*=7.5 Hz).

The disclosed inhibitors can also be isolated as the free acid. A non-limiting example of this procedure is described herein below in Example 4.

The following is a non-limiting example of compounds encompassed within this type of the first Category I of the present disclosure.

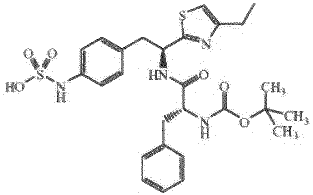

4- {(S)-2-[(*R*)-2-(*tert*-butoxycarbonylamino)-3-phenylpropanamido]-2-(4-ethylthiazol-2-yl)ethyl}phenylsulfamic acid: ¹H NMR (CD₃OD): δ 7.22-7.02 (m, 10H), 5.39 (s, 1H), 4.34 (s, 1H), 3.24-2.68 (m, 6H), 1.37 (s, 9H), 1.30 (t, 3H, *J*=7.5 Hz).

Another example of this type of Category I relates to inhibitors having the formula: 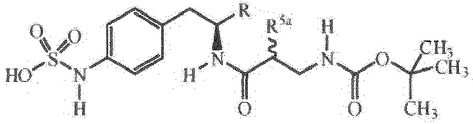 wherein R units and R^{5a} units further described in Table II.

**TABLE II**

| **No.** | **R** | **R^{5a}** |
|---|---|---|
| B26 | thiazol-2-yl | (*S*)-benzyl |
| B27 | 4-methylthiazol-2-yl | (*S*)-benzyl |
| B28 | 4-ethylthiazol-2-yl | (*S*)-benzyl |
| B29 | 4-propylthiazol-2-yl | (*S*)-benzyl |
| B30 | 4-*iso*-propylthiazol-2-yl | (*S*)-benzyl |
| B31 | 4-cyclopropylthiazol-2-yl | (*S*)-benzyl |
| B32 | 4-butylthiazol-2-yl | (*S*)-benzyl |
| B33 | 4-*tert*-butylthiazol-2-yl | (*S*)-benzyl |
| B34 | 4-cyclohexylthiazol-2-yl | (*S*)-benzyl |
| B35 | 4-(2,2,2-trifluoroethyl)thiazol-2-yl | (*S*)-benzyl |
| B36 | 4-(3,3,3-trifluoropropyl)thiazol-2-yl | (*S*)-benzyl |
| B37 | 4-(2,2-difluorocyclopropyl)thiazol-2-yl | (*S*)-benzyl |
| B38 | 4-(methoxymethyl)thiazol-2-yl | (*S*)-benzyl |
| B39 | 4-(carboxylic acid ethyl ester)thiazol-2-yl | (*S*)-benzyl |
| B40 | 4,5-dimethylthiazol-2-yl | (*S*)-benzyl |
| B41 | 4-methyl-5-ethylthiazol-2-yl | (*S*)-benzyl |
| B42 | 4-phenylthiazol-2-yl | (*S*)-benzyl |
| B43 | 4-(4-chlorophenyl)thiazol-2-yl | (*S*)-benzyl |
| B44 | 4-(3,4-dimethylphenyl)thiazol-2-yl | (*S*)-benzyl |
| B45 | 4-methyl-5-phenylthiazol-2-yl | (*S*)-benzyl |
| B46 | 4-(thiophen-2-yl)thiazol-2-yl | (*S*)-benzyl |
| B47 | 4-(thiophen-3-yl)thiazol-2-yl | (*S*)-benzyl |
| B48 | 4-(5-chlorothiophen-2-yl)thiazol-2-yl | (*S*)-benzyl |
| B49 | 5,6-dihydro-4*H*-cyclopenta[*d*]thiazol-2-yl | (*S*)-benzyl |
| B50 | 4,5,6,7-tetrahydrobenzo[*d*]thiazol-2-yl | (*S*)-benzyl |

The compounds of this example can be prepared according to the procedure outlined above in Scheme I and described in Example 1 by substituting the appropriate Boc-β-amino acid for (S)-(2-*tert*-butoxycarbonylamino)-3-phenylpropionic acid in step (d).

The following are non-limiting examples of compounds according to this type:

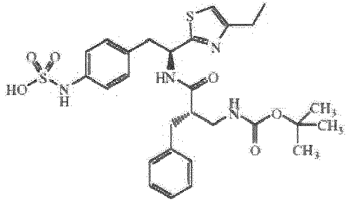

{1-[1-(4-Ethylthiazol-2-yl)-(*S*)-2-(4-sulfoaminophenyl)ethylcarbamoyl]-(*S*)-2-phenylethyl}methyl carbamic acid *tert*-butyl ester: ¹H NMR (300 MHz, MeOH-d₄) δ 8.36 (d, *J* = 8.1 Hz, 1H), 7.04-7.22 (m, 9H), 5.45 (s, 1H), 3.01-3.26 (m, 2H), 2.60-2.88 (m, 4H), 2.33 (s, 3H), 1.30 (s, 9H).

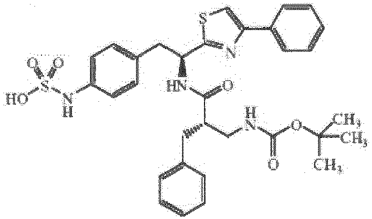

{1-[1-(4-Phenylthiazol-2-yl)-(*S*)-2-(4-sulfoaminophenyl)ethylcarbamoyl]-(*S*)-2-phenylethyl}methyl carbamic acid *tert*-butyl ester: ¹H NMR (300 MHz, MeOH-d₄) δ 8.20 (d, *J* = 8.1 Hz, 1H), 7.96-7.99 (m, 2H), 7.48-7.52 (m, 3H), 7.00-7.23(m, 7H), 6.89 (s, 1H), 5.28 (q, *J* = 7.5 Hz, 1H), 4.33 (t, *J* = 6.6 Hz, 1H), 3.09-3.26 (m, 2H), 3.34 (dd, *J* = 13.2 and 8.4 Hz, 1H), 2.82 (dd, *J* = 13.2 and 8.4 Hz, 1H), 1.38 (s, 9H).

The second type of Category I of the present disclosure relates to compounds falling outside the scope of the claims wherein R is a substituted or unsubstituted thiazol-4-yl having the formula: 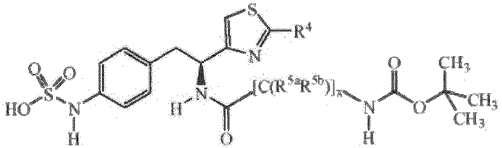 One example of which relates to inhibitors having the formula: 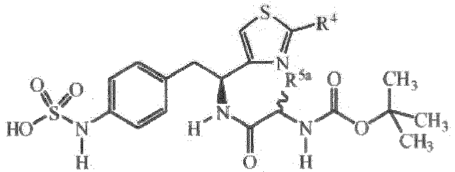 wherein R units and R^{5a} units further described in Table III. **TABLE III**

| **No.** | **R** | **R^{5a}** |
|---|---|---|
| C51 | thiazol-4-yl | (*S*)-benzyl |
| C52 | 2-methylthiazol-4-yl | (*S*)-benzyl |
| C53 | 2-ethylthiazol-4-yl | (*S*)-benzyl |
| C54 | 2-propylthiazol-4-yl | (*S*)-benzyl |
| C55 | 2-*iso*-propylthiazol-4-yl | (*S*)-benzyl |
| C56 | 2-cyclopropylthiazol-4-yl | (*S*)-benzyl |
| C57 | 2-butylthiazol-4-yl | (*S*)-benzyl |
| C58 | 2-*tert*-butylthiazol-4-yl | (*S*)-benzyl |
| C59 | 2-cyclohexylthiazol-4-yl | (*S*)-benzyl |
| C60 | 2-(2,2,2-trifluoroethyl)thiazol-4-yl | (*S*)-benzyl |
| C61 | 2-(3,3,3-trifluoropropyl)thiazol-4-yl | (*S*)-benzyl |
| C62 | 2-(2,2-difluorocyclopropyl)thiazol-4-yl | (*S*)-benzyl |
| C63 | 2-phenylthiazol-4-yl | (*S*)-benzyl |
| C64 | 2-(4-chlorophenyl)thiazol-4-yl | (*S*)-benzyl |
| C65 | 2-(3,4-dimethylphenyl)thiazol-4-yl | (*S*)-benzyl |
| C66 | 2-(thiophen-2-yl)thiazol-4-yl | (*S*)-benzyl |
| C67 | 2-(thiophen-3-yl)thiazol-4-yl | (*S*)-benzyl |
| C68 | 2-(3-chlorothiophen-2-yl)thiazol-4-yl | (*S*)-benzyl |
| C69 | 2-(3-methylthiophen-2-yl)thiazol-4-yl | (*S*)-benzyl |
| C70 | 2-(2-methylthiazol-4-yl)thiazol-4-yl | (*S*)-benzyl |
| C71 | 2-(furan-2-yl)thiazol-4-yl | (*S*)-benzyl |
| C72 | 2-(pyrazin-2-yl)thiazol-4-yl | (*S*)-benzyl |
| C73 | 2-[(2-methyl)pyridin-5-yl]thiazol-4-yl | (*S*)-benzyl |
| C74 | 2-(4-chlorobenzenesulfonylmethyl)thiazol-4-yl | (*S*)-benzyl |
| C75 | 2-(*tert*-butylsulfonylmethyl)thiazol-4-yl | (*S*)-benzyl |

The compounds encompassed within the second type of Category I of the present disclosure can be prepared by the procedure outlined in Scheme II and described in Example 2 herein below.

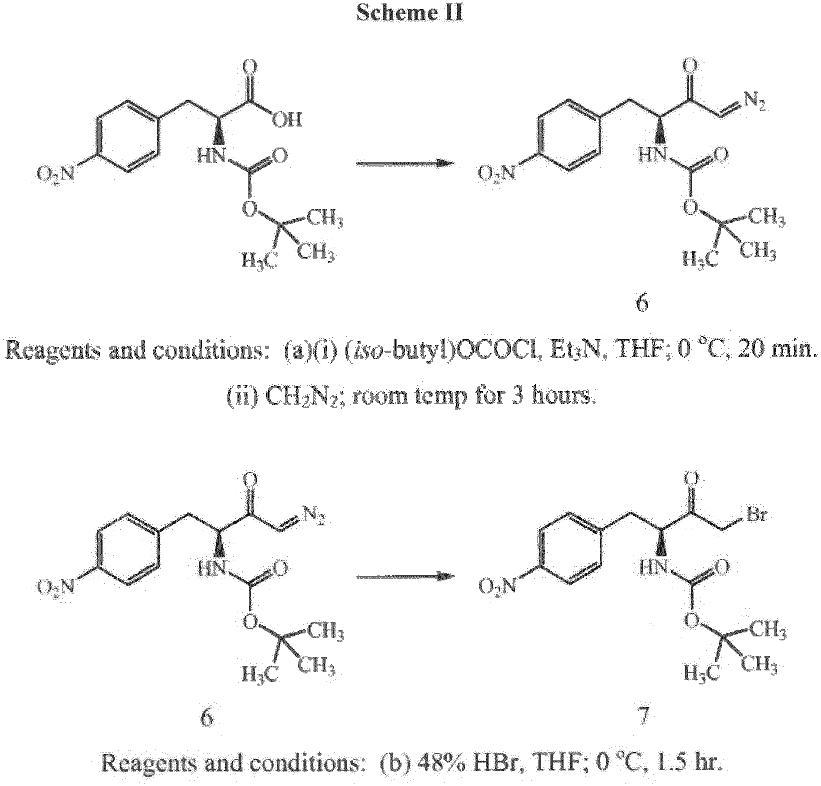

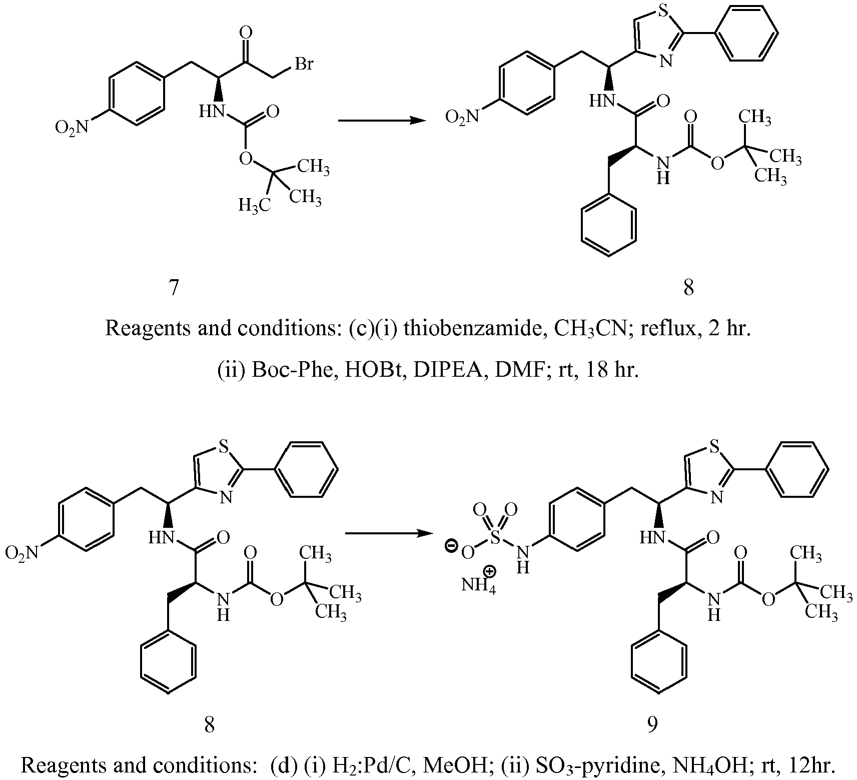

### EXAMPLE 2

### 4-{(S)-2-(S)-2-(tert-Butoxycarbonylamino)-3-phenylpropanamido-2-(2-phenylthiazol-4-yl)}phenylsulfamic acid (9)

Preparation of (S)-[3-diazo-1-(4-nitrobenzyl)-2-oxo-propyl]-carbamic acid *tert-*butyl ester (6): To a 0 °C solution of 2-(*S*)-*tert*-butoxycarbonylamino-3-(4-nitrophenyl)-propionic acid (1.20 g, 4.0 mmol) in THF (20 mL) is added dropwise triethylamine (0.61 mL, 4.4 mmol) followed by *iso*-butyl chloroformate (0.57 mL, 4.4 mmol). The reaction mixture is stirred at 0 °C for 20 minutes and filtered. The filtrate is treated with an ether solution of diazomethane (∼16 mmol) at 0 °C. The reaction mixture is stirred at room temperature for 3 hours then concentrated *in vacuo.* The resulting residue is dissolved in EtOAc and washed successively with water and brine, dried (Na₂SO₄), filtered and concentrated. The residue is purified over silica (hexane/EtOAc 2:1) to afford 1.1 g (82% yield) of the desired product as a slightly yellow solid. ¹H NMR (300 MHz, CDCl₃) δ 8.16 (d, *J* = 8.7 Hz, 2H), 7.39 (d, *J* = 8.7 Hz, 2H), 5.39 (s, 1H), 5.16 (d, *J* = 6.3 Hz, 1H), 4.49 (s, 1H), 3.25 (dd, *J* = 13.8 and 6.6, 1H), 3.06 (dd, *J* = 13.5 and 6.9 Hz, 1H), 1.41 (s, 9H).

Preparation of (*S*)-*tert*-butyl 4-bromo-1-(4-nitrophenyl)-3-oxobutan-2-ylcarbamate (7): To a 0 °C solution of (*S*)-[3-diazo-1-(4-nitrobenzyl)-2-oxo-propyl]-carbamic acid *tert-*butyl ester, 6, (0.350 g, 1.04 mmol) in THF (5 mL) is added dropwise 48% aq. HBr (0.14 mL, 1.25 mmol). The reaction mixture is stirred at 0 °C for 1.5 hours then the reaction is quenched at 0 °C with sat. Na₂CO₃. The mixture is extracted with EtOAc (3x 25 mL) and the combined organic extracts are washed with brine, dried (Na₂SO₄), filtered and concentrated to obtain 0.400 g of the product which is used in the next step without further purification. ¹H NMR (300 MHz, CDCl₃) δ 8.20 (d, *J* = 8.4 Hz, 2H), 7.39 (d, *J* = 8.4 Hz, 2H), 5.06 (d, *J* = 7.8 Hz, 1H), 4.80 (q, *J* = 6.3 Hz, 1H), 4.04 (s, 2H), 1.42 (s, 9H).

Preparation of *tert*-butyl (*S*)-1-(*S*)-2-(4-nitrophenyl)-1-(2-phenylthiazole-4-yl)ethylamino-1-oxo-3-phenylpropan-2-ylcarbamate (8): A mixture of thiobenzamide (0.117 g, 0.85 mmol) and (*S*)-*tert*-butyl 4-bromo-1-(4-nitrophenyl)-3-oxobutan-2-ylcarbamate, 7, (0.300 g, 0.77 mmol) in CH₃CN (4 mL) is refluxed 2 hours. The reaction mixture is cooled to room temperature and diethyl ether is added to precipitate the intermediate 2-(nitrophenyl)-(*S*)-1-(4-phenylthiazol-2-yl)ethylamine which is isolated by filtration as the hydrobromide salt. The hydrobromide salt is dissolved in DMF (3 mL) together with diisoproylethylamine (0.42 mL, 2.31 mmol), 1-hydroxybenzotriazole (0.118 g, 0.79 mmol) and (*S*)-(2-*tert*-butoxycarbonyl-amino)-3-phenylpropionic acid (0.212 g, 0.80 mmol). The mixture is stirred at 0 °C for 30 minutes then at room temperature overnight. The reaction mixture is diluted with water and extracted with EtOAc. The combined organic phase is washed with 1 N aqueous HCl, 5 % aqueous NaHCO₃, water and brine, and dried over Na₂SO₄. The solvent is removed *in vacuo* to afford 0.395 g (90 % yield) of the desired product which is used without further purification. LC/MS ESI+ 573 (M+1).

Preparation of 4- {(*S*)-2-(*S*)-2-(*tert*-butoxycarbonyl)-3 -phenylpropaneamido-2-(2-phenylthiazole-4-yl)}phenylsulfamic acid (9): *tert*-butyl (*S*)-1-(*S*)-2-(4-nitrophenyl)-1-(2-phenylthiazole-4-yl)ethylamino-1-oxo-3-phenylpropan-2-ylcarbamate, 8, (0.360 g) is dissolved in MeOH (4 mL). A catalytic amount of Pd/C (10% w/w) is added and the mixture is stirred under a hydrogen atmosphere 12 hours. The reaction mixture is filtered through a bed of CELITE™ and the solvent is removed under reduced pressure. The crude product is dissolved in pyridine (12 mL) and treated with SO₃-pyridine (0.296 g). The reaction is stirred at room temperature for 5 minutes after which a 7% solution of NH₄OH (10 mL) is added. The mixture is then concentrated and the resulting residue is purified by
reverse phase chromatography to afford 0.050 g of the desired product as the ammonium salt. ¹H NMR (300 MHz, MeOH-d₄) δ 8.20 (d, *J* = 8.1 Hz, 1H), 7.96-7.99 (m, 2H), 7.48-7.52 (m, 3H), 7.00-7.23(m, 7H), 6.89 (s, 1H), 5.28 (q, *J* = 7.5 Hz, 1H), 4.33 (t, *J* = 6.6 Hz, 1H), 3.09-3.26 (m, 2H), 3.34 (dd, *J* = 13.2 and 8.4 Hz, 1H), 2.82 (dd, *J* = 13.2 and 8.4 Hz, 1H), 1.38 (s, 9H).

The first type of Category II of the present disclosure relates to compounds some of which fall within the scope of the invention wherein R is a substituted or unsubstituted thiazol-4-yl unit having the formula: 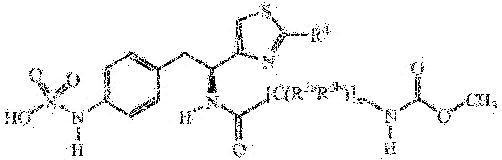 one example of which relates to inhibitors having the formula: 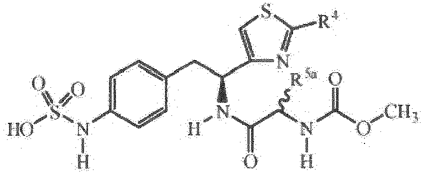 wherein R units are thiazol-4-yl units, that when substituted, are substituted with R⁴ units. R and R^{5a} units are further described in Table IV.

**TABLE IV**

| **No.** | **R** | **R^{5a}** |
|---|---|---|
| D76 | thiazol-4-yl | (*S*)-benzyl |
| D77 | 2-methylthiazol-4-yl | (*S*)-benzyl |
| D78 | 2-ethylthiazol-4-yl | (*S*)-benzyl |
| D79 | 2-propylthiazol-4-yl | (*S*)-benzyl |
| D80 | 2-*iso*-propylthiazol-4-yl | (*S*)-benzyl |
| D81 | 2-cyclopropylthiazol-4-yl | (*S*)-benzyl |
| D82 | 2-butylthiazol-4-yl | (*S*)-benzyl |
| D83 | 2-*tert*-butylthiazol-4-yl | (*S*)-benzyl |
| D84 | 2-cyclohexylthiazol4-yl | (*S*)-benzyl |
| D85 | 2-(2,2,2-trifluoroethyl)thiazol-4-yl | (*S*)-benzyl |
| D86 | 2-(3,3,3-trifluoropropyl)thiazol-4-yl | (*S*)-benzyl |
| D87 | 2-(2.2-difluorocydopropyl)thiazol-4-yl | (*S*)-benzyl |
| D88 | 2-phenylthiazol-4-yl | (*S*)-benzyl |
| D89 | 2-(4-chlorophenyl)thiazol-4-yl | (*S*)-benzyl |
| D90 | 2-(3,4-dimethylphenyl)thiazol-4-yl | (*S*)-benzyl |
| D91 | 2-(thiophen-2-yl)thiazol-4-yl | (*S*)-benzyl |
| D92 | 2-(thiophen-3-yl)thiazol-4-yl | (*S*)benzyl |
| D93 | 2-(3-chlorothiophen-2-yl)thiazol-4-yl | (*S*)-benzyl |
| D94 | 2-(3-methylthiophen-2-yl)thiazol-4-yl | (*S*)-benzyl |
| D95 | 2-(2-methylthiazol-4-yl)thiazol-4-yl | (*S*)-benzyl |
| D96 | 2-(furan-2-yl)thiazol-4-yl | (*S*)-benzyl |
| D97 | 2-(pyrazin-2-yl)thiazol-4-yl | (*S*)-benzyl |
| D98 | 2-[(2-methyl)pyridin-5-yl]thiazol-4-yl | (*S*)-benzyl |
| D99 | 2-(4-chlorobenzenesulfonylmethyl)thiazol-4-yl | (*S*)-benzyl |
| D100 | 2-(*tert*-butylsulfony lmethyl)thiazol-4-yl | (*S*)-benzyl |

The compounds encompassed within the second aspect of Category II of the present disclosure and as defined by the claims can be prepared by the procedure outlined in Scheme III and described in Example 3 herein below.

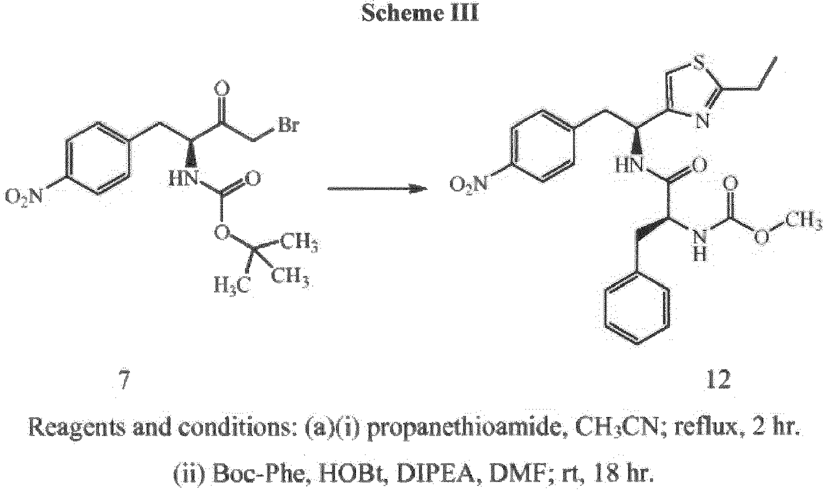

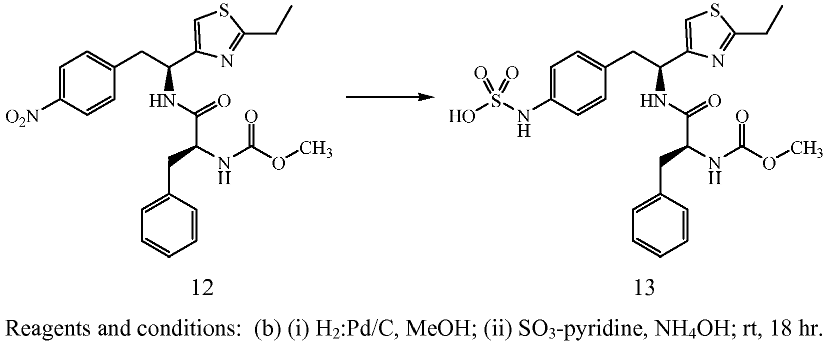

### EXAMPLE 3

### 4-{(S)-2-[(S)-2-(Methoxycarbonylamino)-3-phenylpropanamido]-2-(2-ethylthiazol-4-y|) ethyl}phenylsulfamic acid (13)

Preparation of methyl (*S*)-1-[(*S*)-1-(2-ethylthiazole-4-yl)-2-(4-nitrophenyl)-ethyl]amino-1-oxo-3-phenylpropane-2-ylcarbamate (12): A mixture of propanethioamide (69 mg, 0.78 mmol) and (*S*)-*tert*-butyl 4-bromo-1-(4-nitrophenyl)-3-oxobutan-2-ylcarbamate, 7, (0.300 g, 0.77 mmol) in CH₃CN (4 mL) is refluxed for 2 hours. The reaction mixture is cooled to room temperature and diethyl ether is added to precipitate the intermediate 2-(nitrophenyl)-(*S*)-1-(4-ethylthiazol-2-yl)ethylamine which is isolated by filtration as the hydrobromide salt. The hydrobromide salt is dissolved in DMF (8 mL) together with diisoproylethylamine (0.38 mL, 2.13 mmol), 1-hydroxybenzotriazole (107 mg, 0.71 mmol) and (*S*)-(2-methoxycarbonyl-amino)-3-phenylpropionic acid (175 mg, 0.78 mmol). The mixture is stirred at 0 °C for 30 minutes then at room temperature overnight. The reaction mixture is diluted with water and extracted with EtOAc. The combined organic phase is washed with 1 N aqueous HCl, 5 % aqueous NaHCO₃, water and brine, and dried over Na₂SO₄. The solvent is removed *in vacuo* to afford 0.300g (81% yield) of the desired product which is used without further purification. LC/MS ESI+MS 483 (M+1).

Preparation of 4-((*S*)-2-((*S*)-2-(methoxycarbonylamino)-3-phenylpropanamido)-2-(2-ethylthiazol-4-yl) ethyl)phenylsulfamic acid ammonium salt (13): *tert*-Butyl (*S*)-1-(*S*)-2-(4-nitrophenyl)-1-(2-ethylthiazole-4-yl)ethylamino-1-oxo-3 -phenylpropan-2-ylcarbamate, 12, (0.300g) is dissolved in MeOH (4 mL). A catalytic amount of Pd/C (10% w/w) is added and the mixture is stirred under a hydrogen atmosphere 18 hours. The reaction mixture is filtered through a bed of CELITE™ and the solvent is removed under reduced pressure. The crude product is dissolved in pyridine (12 mL) and treated with SO₃-pyridine (223 mg, 1.40 mmol). The reaction is stirred at room temperature for 5 minutes after which a 7% solution of NH₄OH (12 mL) is added. The mixture is then concentrated and the resulting residue is purified by reverse phase chromatography to afford 25 mg of the desired product as the ammonium salt. ¹H NMR (300 MHz, MeOH-d₄) δ 7.14-7.24 (m, 6H), 6.97-7.0 (m, 4H), 6.62 (s, 1H), 5.10-5.30 (m, 1H), 4.36 (t, *J* = 7.2 Hz, 1H), 3.63 (s, 3H), 3.14 (dd, *J* = 13.5 and 6.3 Hz, 1H), 2.93-3.07 (m, 5H), 2.81 (dd, J = 13.5 and 6.3 HZ, 1H), 1.39 (t, *J* = 7.8 Hz, 3H).

In another iteration of the process of the present disclosure, compound 13, as well as the other analogs which comprise the present disclosure, can be isolated as the free acid by adapting the procedure described herein below.

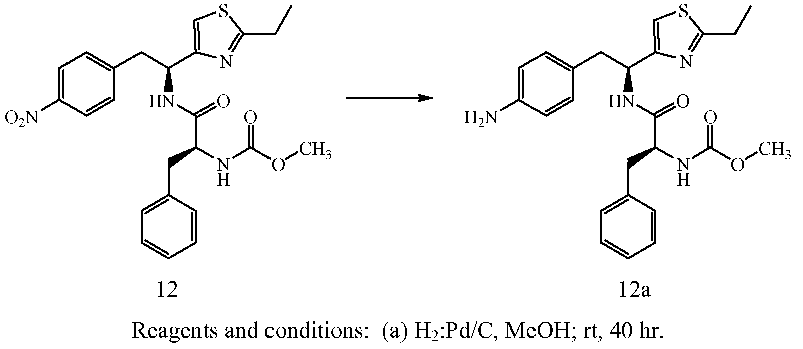

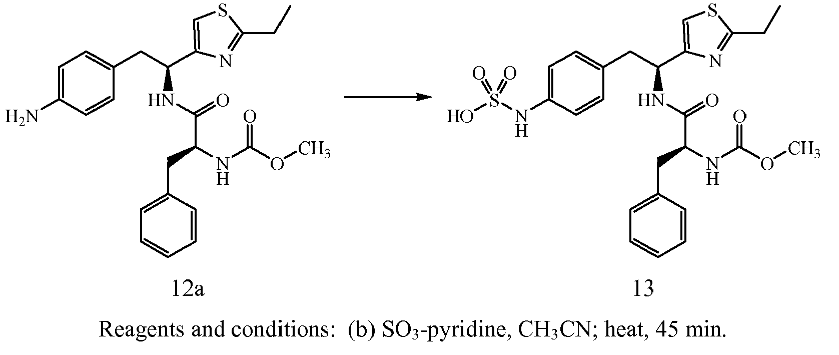

### EXAMPLE 4

### 4-((S)-2-((S)-2-(Methoxycarbonylamino)-3-phenylpropanamido)-2-(2-ethylthiazol-4-yl) ethyl)phenylsulfamic acid [Free Acid Form] (13)

Preparation of {1-[2-(*S*)-(4-(*S*)-aminophenyl)-1-(2-ethylthiazol-4-yl)ethylcarbamoyl]-2-phenylethyl}-carbamic acid methyl ester (12a): A Parr hydrogenation vessel is charged with *tert*-butyl (*S*)-1-(*S*)-2-(4-nitrophenyl)-1-(2-ethylthiazole-4-yl)ethylamino-1-oxo-3-phenylpropan-2-ylcarbamate, 12, (18.05 g, 37.4 mmol, 1.0 eq) and Pd/C (10 % Pd on
C, 50 % wet, Degussa-type E101 NE/W, 2.68 g, 15 wt %) as solids. MeOH (270 mL, 15 mL/g) is added to provide a suspension. The vessel is put on a Parr hydrogenation apparatus. The vessel is submitted to a fill/vacuum evacuate process with N2 (3 × 20 psi) to inert, followed by the same procedure with H₂ (3 × 40 psi). The vessel is filled with H₂ and the vessel is shaken under 40 psi H₂ for -40 hr. The vessel is evacuated and the atmosphere is purged with N₂ (5 × 20 psi). An aliquot is filtered and analyzed by HPLC to insure complete conversion. The suspension is filtered through a pad of celite to remove the catalyst, and the homogeneous yellow filtrate is concentrated by rotary evaporation to afford 16.06 g (95% yield) of the desired product as a tan solid, which is used without further purification.

Preparation of 4-((*S*)-2-((*S*)-2-(methoxycarbonyl)-3-phenylpropanamido)-2-(2-ethylthiazol-4-yl) ethyl)phenylsulfamic acid (13): A 100 mL RBF is charged with {1-[2-(*S*)-(4-(*S*)-aminophenyl)-1-(2-ethylthiazol-4-yl)ethyl-carbamoyl]-2-phenylethyl} -carbamic acid methyl ester, 12a, (10.36 g, 22.9 mmol, 1.0 eq.) prepared in the step described herein above. Acetonitrile (50 mL, 5 mL/g) is added and the yellow suspension is stirred at room temperature. A second 3-necked 500 mL RBF is charged with SO₃ pyr (5.13 g, 32.2 mmol, 1.4 eq.) and acetonitrile (50 mL 5 mL/g) and the white suspension is stirred at room temperature. Both suspensions are gently heated until the reaction solution containing { 1-[2-(*S*)-(4-(*S*)-aminophenyl)-1-(2-ethylthiazol-4-yl)ethyl-carbamoyl]-2-phenylethyl}-carbamic acid methyl ester becomes red-orange in color (typically for this example about 44 °C). This substrate containing solution is poured in one portion into the stirring suspension of SO₃ pyr at 35 °C. The resulting opaque mixture (39 °C) is stirred vigorously while allowed to slowly cool to room temperature. After stirring for 45 min, the reaction is determined to be complete by HPLC. H₂O (200 mL, 20 mL/g) is added to the orange suspension to provide a yellow-orange homogeneous solution having a pH of approximately 2.4. Concentrated H₃PO₄ is added slowly over 12 minutes to lower the pH to approximately 1.4. During this pH adjustment, an off-white precipitate is formed and the solution is stirred at room temperature for 1 hr. The suspension is filtered and the filter cake is washed with the filtrate. The filter cake is air-dried on the filter overnight to afford 10.89 g (89 % yield) of the desired product as a tan solid.

The following are further non-limiting examples of the second aspect of Category II of the present disclosure, some of which are in accordance with the invention.

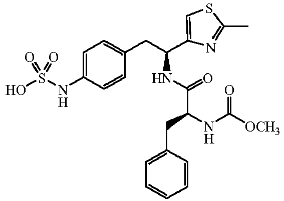

4-{(*S*)-2-[(*S*)-2-(Methoxycarbonylamino)-3-phenylpropanamido]-2-(2-methylthiazol-4-yl)ethyl}phenylsulfamic acid: ¹H NMR (300 MHz, MeOH-d₄) δ 8.15 (d, *J* = 8.4 Hz, 1H), 7.16-7.25 (m, 5H), 6.97-7.10 (m, 4H), 6.61 (s, 1H), 5.00-5.24 (m, 1H), 4.36 (t, *J* = 7.2 Hz, 1H), 3.64 (s, 2H), 3.11-3.19 (s, 1H), 2.92-3.04 (s, 2H), 2.81 (dd, *J* = 13.5 and 8.1 Hz, 1H), 2.75 (s, 3H).

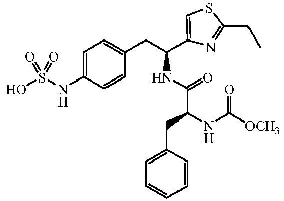

4-{(S)-2-(2-Ethylthiazole-4-yl)-2-[(S)-2-(methoxycarbonylamino)-3-phenylpropan-amido]ethyl}phenylsulfamic acid: ¹H NMR (300 MHz, MeOH-d₄) δ 7.16-7.29 (m, 5H), 7.02-7.12 (m, 4H), 6.83 (s, 1H), 5.10-5.35 (m, 1H), 3.52-3.67(m, 3H), 3.18-3.25 (m, 2H), 3.05 (q, *J* = 7.5 Hz, 2H), 2.82-2.95 (m, 2H), 2.65 (s, 3H), 1.39 (t, *J* = 7.5 Hz, 3H).

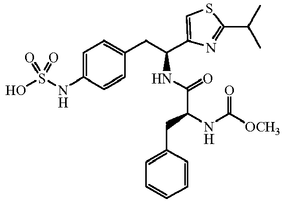

4-{(*S*)-2-(2-Isopropylthiazol-4-yl)-2-[(*S*)-2-(methoxycarbonylamino)-3-phenylpropan-amido]ethyl}phenylsulfamic acid: ¹H NMR (CD₃OD) δ 8.16 (d, 1H, J = 8.7Hz), 7.22-7.13 (m, 3H), 7.07 (d, 1H, J = 8.4Hz), 6.96 (d, 1H, J = 8.1Hz), 6.62 (s, 1H), 5.19 (t, 1H, J = 7.2Hz), 4.36 (t, 1H, J = 7.8Hz), 3.63 (s, 3H), 3.08 (1H, A of ABX, J = 3.6, 14.5Hz), 2.99 (1H, B of ABX, J = 7.2, 13.8Hz), 2.85-2.78 (m, 1H), 1.41 (d, 6H, J = 6.9Hz).

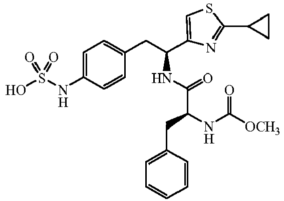

4-{(*S*)-2-(2-Cyclopropylthiazol-4-yl)-2-[(*S*)-2-(methoxycarbonylamino)-3-phenylpropanamido]ethyl}phenylsulfamic acid: ¹H NMR (CD₃OD): δ 7.15-7.02 (m, 5H), 6.96-6.93 (d, 2H, J=8.4 Hz), 6.86-6.83 (d, 2H, J=8.3 Hz), 6.39 (s, 1H), 5.01 (t, 1H, *J*=5.0 Hz), 4.22 (t, 1H, J=7.4 Hz), 3.51 (s, 3H), 2.98-2.69 (m, 2H), 2.22-2.21 (m, 1H), 1.06-1.02 (m, 2H), 0.92-0.88 (m, 2H).

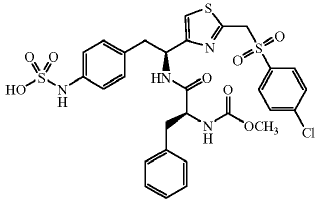

4-{(S)-2-{2-[(4-Chlorophenylsulfonyl)methyl]thiazol-4-yl}-2-[(S)-2-(methoxy-carbonylamino)-3-phenylpropanamido]ethyl}phenylsulfamic acid: ¹H NMR (CD₃OD): δ 7.96-7.93 (d, 2H, *J*=8.6 Hz), 7.83-7.80 (d, 2H, *J*=8.6 Hz), 7.44-7.34 (m, 5H), 7.29-7.27 (d, 2H, *J*=8.4 Hz), 7.14-7.11 (d, 2H, *J*=8.4 Hz), 6.97 (s, 1H), 5.31 (t, 1H, *J*=6.8 Hz), 5.22-5.15 (m, 2H), 4.55 (t, 1H, *J*=7.3 Hz), 3.84 (s, 3H), 3.20-2.96 (m, 4H).

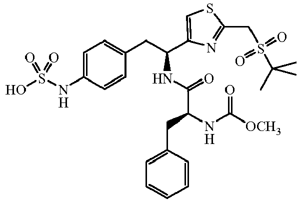

4-{(S)-2-[2-(*tert*-Butylsulfonylmethyl)thiazol-4-yl]-2-[(S)-2-(methoxycarbonyl-amino)-3-phenylpropanamido]ethyl}phenylsulfamic acid: ¹H NMR (CD₃OD): δ 7.40-7.30 (m, 5H), 7.21-7.10 (m, 4H), 7.02 (s, 1H), 5.37 (t, 1H, *J*=6.9 Hz), 5.01-4.98 (m, 2H), 4.51 (t, 1H, *J*=7.1 Hz), 3.77 (s, 3H), 3.34-2.91 (m, 4H), 1.58 (s, 9H).

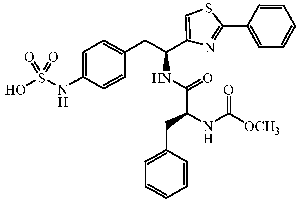

4-{(*S*)-2-[(*S*)-2-(Methoxycarbonylamino)-3-phenylpropionamido]-2-(2-phenylthiazole-4-yl)ethyl}phenylsulfamic acid: ¹H NMR (300 MHz, DMSO-d₆) δ 7.96-7.99 (m, 2H), 7.51-7.56 (m, 3H), 7.13-7.38 (m, 6H), 6.92-6.95 (m, 4H), 5.11-5.16 (m, 1H), 4.32-4.35 (m, 1H), 3.51 (s, 3H), 3.39-3.40 (m, 2H), 3.09-3.19 (m, 1H), 2.92-3.02 (m, 2H), 2.75 (dd, *J* = 10.5 Hz and 9.9 Hz, 1H).

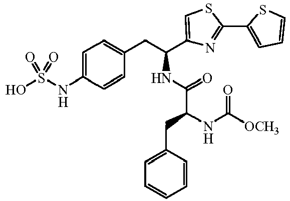

4-{(*S*)-2-[(*S*)-2-(Methoxycarbonylamino)-3-phenylpropanamido]-2-[2-(thiophen-2-yl)thiazol-4-yl]ethyl}phenylsulfamic acid: ¹H NMR (CD₃OD): δ 7.61-7.56 (m, 2H), 7.25-7.01 (m, 10H), 6.75 (s, 1H), 5.24-5.21 (q, 1H, J=7.2 Hz), 4.38 (t, 1H, *J*=7.2 Hz), 3.60 (s, 3H), 3.23-3.14 (m, 1H), 3.08-3.00 (m, 2H), 2.87-2.80 (m, 1H).

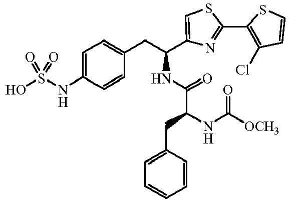

4-{(*S*)-2-[2-(3-Chlorothiophen-2-yl)thiazol-4-yl]-2-[(*S*)-2-(methoxycarbonylamino)-3-phenylpropanamido]ethyl}phenylsulfamic acid: ¹H NMR (CD₃OD): δ 7.78-7.76 (d, 1H, *J*=5.4 Hz), 7.36-7.14 (m, 10H), 7.03 (s, 1H), 5.39 (t, 1H, *J*=6.9 Hz), 4.54 (t, 1H, *J*=7.3 Hz), 3.80 (s, 3H), 3.39-2.98 (m, 4H).

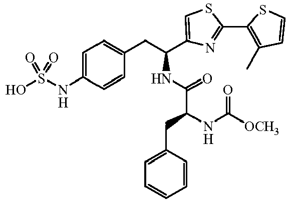

4-{(*S*)-2-[(*S*)-2-(Methoxycarbonylamino)-3-phenylpropanamido]-2-[2-(3-methylthiophen-2-yl)thiazol-4-yl]ethyl}phenylsulfamic acid: ¹H NMR (CD₃OD): δ 7.38 (d, 1H, *J*=5.1 Hz), 7.15-6.93 (m, 10H), 6.73 (s, 1H), 5.17 (t, 1H, *J*=6.9 Hz), 4.31 (t, 1H, *J*= 7.3 Hz), 3.57 (s, 3H), 3.18-3.11 (m, 1H), 3.02-2.94 (m, 2H), 2.80-2.73 (m, 1H), 2.46 (s, 3H).

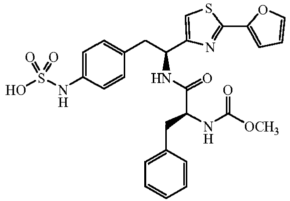

4-{[(*S*)-2-(2-(Furan-2-yl)thiazol-4-yl]-2-[(*S*)-2-(methoxycarbonylamino)-3-phenylpropanamido]ethyl}phenylsulfamic acid: ¹H NMR (CD₃OD): δ 7.54-7.46 (m, 1H), 7.02-6.79 (m, 10H), 6.55-6.51 (m, 1H), 6.44-6.41 (m, 1H), 5.02-5.00 (q, 1H, J=6.4 Hz), 4.16-4.14 (q, 1H, *J*=7.1 Hz), 3.43 (s, 3H), 2.96-2.58 (m, 4H).

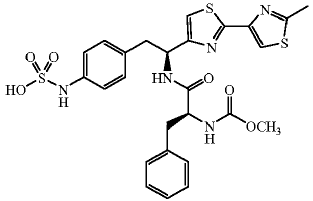

4-{(*S*)-2-[(*S*)-2-(Methoxycarbonylamino)-3-phenylpropanamido]-2-[2-(2-methylthiazole-4-yl)thiazole-4yl]ethyl}phenylsulfamic acid: ¹H NMR (300 MHz, MeOH-d₄) δ 8.27(d, *J* = 5.4 Hz, 1H), 7.97 (s, 1H), 6.99-7.21(m, 8H), 5.18-5.30 (m, 1H), 4.30-4.39 (m, 1H), 3.64 (s, 3H), 3.20 (dd, *J* = 14.1 and 6.6 Hz, 1H), 2.98-3.08(m, 2H), 2.84 (dd, *J* =14.1 and 6.6 Hz, 1H), 2.78 (s, 3H).

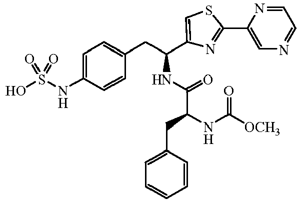

4-{(*S*)-2-[(*S*)-2-(Methoxycarbonylamino)-3-phenylpropanamido]-2-[(2-pyrazin-2-yl)thiazole-4-yl]ethyl}phenylsulfamic acid: ¹H NMR (300 MHz, MeOH-d₄) δ 9.34 (s, 1H), 8.65 (s, 2H), 8.34 (d, *J* = 8.1 Hz, 1H), 7.00-5.16 (m. 9H), 5.30 (q, *J* = 7.2 Hz, 1H), 4.41 (t, *J* = 7.2 Hz, 1H), 3.65 (s, 3H), 3.23 (dd, *J* = 13.8 and 6.9 Hz, 1H), 2.98-3.13 (m, 2H), 2.85 (dd, *J* = 13.8 and 6.9 Hz, 1H).

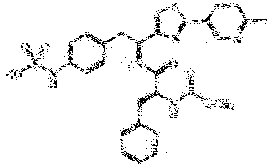

4-{(*S*)-2-[(*S*)-2-(Methoxycarbonylainino)-3-phenylpropanamido]-2-[2-(6-methylpyridin-3-yl)thiazol-4-yl]ethyl}phenylsulfamic acid: ¹H NMR (CD₃OD): δ 8.90 (s, 1H), 8.19-8.13 (m, 1H), 7.39-7.36 (d, 1H, J=8.2 Hz), 7.07-6.88 (m, 9H), 6.79 (s, 1H), 5.17 (t, 1H, J=7.0 Hz), 4.29 (t, 1H, *J*=7.4 Hz), 3.54 (s, 3H), 3.10-2.73 (m, 4H), 2.53 (s, 3H).

Category III of the present disclosure relates to compounds some of which fall within the scope of the invention wherein R is a substituted or unsubstituted thiazol-2-yl unit having the formula: 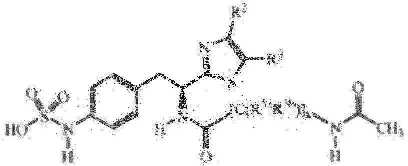 one example of which relates to inhibitors having the formula: 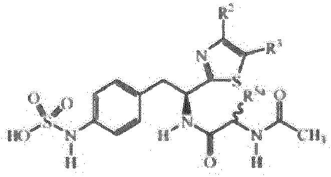 wherein R units are thiazol-2-yl units, that when substituted, are substituted with R² and R³ units. R and R^{5a} units are further described in Table V.

**TABLE V**

| **No.** | **R** | **R^{5a}** |
|---|---|---|
| E101 | thiazol-2-yl | (*S*)-benzyl |
| E102 | 4-methylthiazol-2-yl | (*S*)-benzyl |
| E103 | 4-ethylthiazol-2-yl | (*S*)-benzyl |
| E104 | 4-propylthiazol-2-yl | (*S*)-benzyl |
| E105 | 4-*iso*-propylthiazol-2-yl | (*S*)-benzyl |
| E106 | 4-cyclopropylthiazol-2-yl | (*S*)-benzyl |
| E107 | 4-butylthiazol-2-yl | (*S*)-benzyl |
| E108 | 4-*tert*-butylthiazol-2-yl | (*S*)-benzyl |
| E109 | 4-cyclohexylthiazol-2-yl | (*S*)-benzyl |
| E110 | 4-(2,2,2-trifluoroethyl)thiazol-2-yl | (*S*)-benzyl |
| E111 | 4-(3,3,3-trifluoropropyl)thiazol-2-yl | (*S*)-benzyl |
| E112 | 4-(2,2-difluorocyclopropyl)thiazol-2-yl | (*S*)-benzyl |
| E113 | 4-(methoxymethyl)thiazol-2-yl | (*S*)-benzyl |
| E114 | 4-(carboxylic acid ethyl ester)thiazol-2-yl | (*S*)-benzyl |
| E115 | 4,5-dimethylthiazol-2-yl | (*S*)-benzyl |
| E116 | 4-methyl-5-ethylthiazol-2-yl | (*S*)-benzyl |
| E117 | 4-phenylthiazol-2-yl | (*S*)-benzyl |
| E118 | 4-(4-chlorophenyl)thiazol-2-yl | (*S*)-benzyl |
| E119 | 4-(3,4-dimethylphenyl)thiazol-2-yl | (*S*)-benzyl |
| E120 | 4-methyl-5-phenylthiazol-2-yl | (*S*)-benzyl |
| E121 | 4-(thiophen-2-yl)thiazol-2-yl | (*S*)-benzyl |
| E122 | 4-(thiophen-3-yl)thiazol-2-yl | (*S*)-benzyl |
| E123 | 4-(5-chlorothiophen-2-yl)thiazol-2-yl | (*S*)-benzyl |
| E124 | 5,6-dihydro-4*H*-cyclopenta[*d*]thiazol-2-yl | (*S*)-benzyl |
| E125 | 4,5,6,7-tetrahydrobenzo[*d*]thiazol-2-yl | (*S*)-benzyl |

The compounds encompassed within Category III of the present disclosure can be prepared by the procedure outlined in Scheme IV and described in Example 5 herein below.

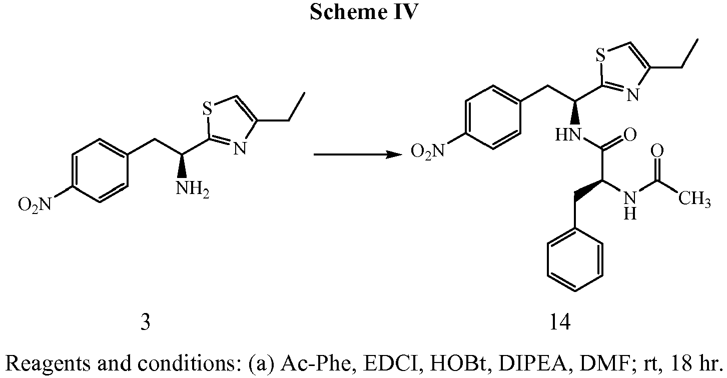

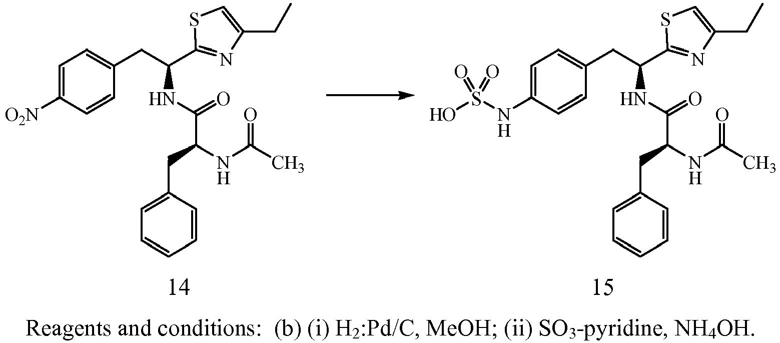

### EXAMPLE 5

### 4-[(S)-2-((S)-2-Acetamido-3-phenylpropanamido)-2-(4-ethylthiazol-2-yl)ethyl]phenylsulfamic acid (15)

Preparation of (*S*)-2-acetamido-*N*-[(*S*)-1-(4-ethylthiazol-2-yl)-2-(4-nitrophenyl)-ethyl]-3-phenylpropanamide (14): To a solution of 1-(*S*)-(4-ethylthiazol-2-yl)-2-(4-nitrophenyl)ethyl amine hydrobromide, 3, (0.343 g, 0.957 mmol), *N*-acetyl-L-phenylalanine (0.218 g), 1-hydroxybenzotriazole (HOBt) (0.161g), diisopropyl-ethylamine (0.26 g), in DMF (10 mL) at 0°, is added 1-(3-dimethylaminopropyl)-3-ethylcarbodiimide (EDCI) (0.201 g). The mixture is stirred at 0 °C for 30 minutes then at room temperature overnight. The reaction mixture is diluted with water and extracted with EtOAc. The combined organic phase is washed with 1 N aqueous HCl, 5 % aqueous NaHCO₃, water and brine, and dried over Na₂SO₄. The solvent is removed *in vacuo* to afford 0.313 g (70 % yield) of the desired product which is used without further purification. LC/MS ESI+ 467 (M+1).

Preparation of 4-((S)-2-((S)-2-acetamido-3-phenylpropanamido)-2-(4-ethylthiazol-2-yl)ethyl)phenylsulfamic acid (15): (*S*)-2-Acetamido-*N*-[(*S*)-1-(4-ethylthiazol-2-yl)-2-(4-nitrophenyl)ethyl]-3-phenylpropanamide, 14, (0.313 g) is dissolved in MeOH (4 mL). A catalytic amount of Pd/C (10% w/w) is added and the mixture is stirred under a hydrogen atmosphere 2 hours. The reaction mixture is filtered through a bed of CELITE™ and the solvent is removed under reduced pressure. The crude product is dissolved in pyridine (12 mL) and treated with SO₃-pyridine (0.320 g). The reaction is stirred at room temperature for 5 minutes after which a 7% solution of NH₄OH (30 mL) is added. The mixture is then concentrated and the resulting residue is purified by reverse phase chromatography to afford 0.215 g of the desired product as the ammonium salt. ¹H NMR (CD₃OD): δ 7.23-6.98 (m, 10H), 5.37 (t, 1H), 4.64 (t, 1H, *J*=6.3 Hz), 3.26-2.74 (m, 6H), 1.91 (s, 3H), 1.29 (t, 3H, *J*=7.5 Hz).

The following are further non-limiting examples of compounds encompassed within Category III of the present disclosure.

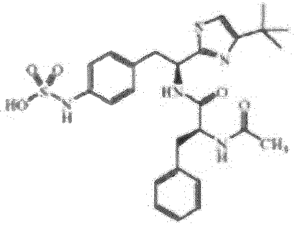

4-[(*S*)-2-((*S*)-2-Acetamido-3-phenylpropanamido)-2-(4-*tert*-butylthiazol-2-yl)ethyl]phenylsulfamic acid: ¹H NMR (300 MHz, CD₃OD): δ 7.22-7.17 (m, 5H), 7.06 (dd, *J*=14.1, 8.4 Hz, 4H), 6.97 (d, *J*=0.9 Hz, 1H), 5.39 (dd, *J*=8.4, 6.0 Hz, 1H), 4.65 (t, *J*=7.2 Hz, 1H), 3.33-3.26 (m, 1H), 3.13-3.00 (m, 3H), 2.80 (dd, *J*=13.5, 8.7 Hz, 1H), 1.91 (s, 3H), 1.36 (s, 9H).

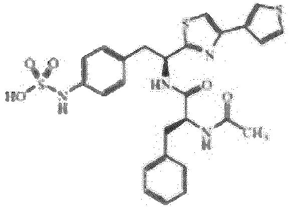

4-{(*S*)-2-((*S*)-2-Acetamido-3-phenylpropanamido)-2- [4-(thiophen-3 -yl)thiazol-2-yl]ethyl)phenylsulfamic acid: ¹H NMR (300 MHz, CD₃OD): δ 8.58 (d, *J*=8.1 Hz, 1H), 7.83-7.82 (m, 1H), 7.57-7.46 (m, 3H), 7.28-6.93 (m, 11H), 5.54-5.43 (m, 1H), 4.69-4.55 (m, 2H), 3.41-3.33 (m, 1H), 3.14-3.06 (3H), 2.86-2.79 (m, 1H), 1.93 (s, 3H).

The first type of Category IV of the present disclosure relates to compounds not falling within the scope of the invention wherein R is a substituted or unsubstituted thiazol-2-yl unit having the formula: 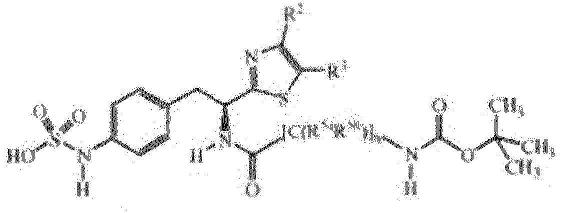 one example of which relates to inhibitors having the formula: 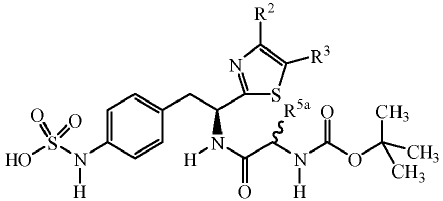 wherein R units and R^{5a} units further described in Table VI.

**TABLE VI**

| **No.** | **R** | **R^{5a}** |
|---|---|---|
| F126 | thiazol-2-yl | hydrogen |
| F127 | 4-methylthiazol-2-yl | hydrogen |
| F128 | 4-ethylthiazol-2-yl | hydrogen |
| F129 | 4-propylthiazol-2-yl | hydrogen |
| F130 | 4-*iso*-propylthiazol-2-yl | hydrogen |
| F131 | 4-cyclopropylthiazol-2-yl | hydrogen |
| F132 | 4-butylthiazol-2-yl | hydrogen |
| F133 | 4-*tert*-butylthiazol-2-yl | hydrogen |
| F134 | 4-cyclohexylthiazol-2-yl | hydrogen |
| F135 | 4,5-dimethylthiazol-2-yl | hydrogen |
| F136 | 4-methyl-5-ethylthiazol-2-yl | hydrogen |
| F137 | 4-phenylthiazol-2-yl | hydrogen |
| F138 | thiazol-2-yl | (*S*)-*iso*-propyl |
| F139 | 4-methylthiazol-2-yl | (*S*)-*iso*-propyl |
| F140 | 4-ethylthiazol-2-yl | (*S*)-*iso*-propyl |
| F141 | 4-propylthiazol-2-yl | (*S*)-*iso*-propyl |
| F142 | 4-*iso*-propylthiazol-2-yl | (*S*)-*iso*-propyl |
| F143 | 4-cyclopropylthiazol-2-yl | (*S*)-*iso*-propyl |
| F144 | 4-butylthiazol-2-yl | (*S*)-*iso*-propyl |
| F145 | 4-*tert*-butylthiazol-2-yl | (*S*)-*iso*-propyl |
| F146 | 4-cyclohexylthiazol-2-yl | (*S*)-*iso*-propyl |
| F147 | 4,5-dimethylthiazol-2-yl | (*S*)-*iso*-propyl |
| F148 | 4-methyl-5-ethylthiazol-2-yl | (*S*)-*iso*-propyl |
| F149 | 4-phenylthiazol-2-yl | (*S*)-*iso*-propyl |
| F150 | 4-(thiophen-2-yl)thiazol-2-yl | (*S*)-*iso*-propyl |

The compounds encompassed within Category IV of the present disclosure can be prepared by the procedure outlined in Scheme V and described in Example 6 herein below.

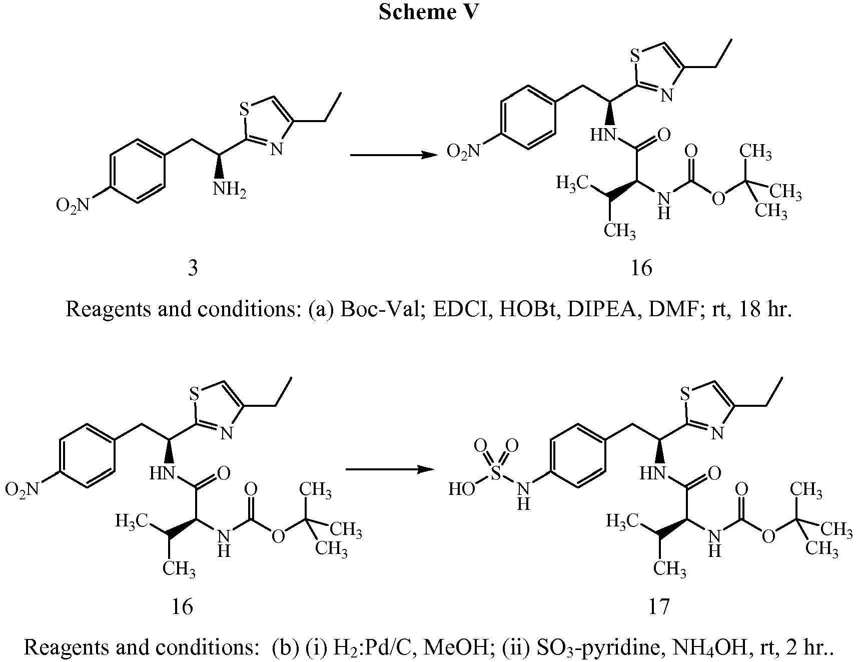

### EXAMPLE 6

### 4-{(S)-2-[(S)-2-(tert-Butoxycarbonylamino)-3-methylbutanamido]-2-(4-ethylthiazol-2-yl)ethyl}phenylsulfamic acid (17)

Preparation of *tert*-butyl (*S*)-1-[(*S*)-(4-ethylthiazol-2-yl)-2-(4-nitrophenyl)ethylamino]-3-methyl-1-oxobutan-2-ylcarbamate (16): To a solution of 1-(S)-(4-ethylthiazol-2-yl)-2-(4-nitrophenyl)ethyl amine hydrobromide, 3, (0.200 g, 0.558 mmol), (*S*)-(2-*tert*-butoxycarbonylamino)-3-methylbutyric acid (0.133 g) and 1-hydroxybenzotriazole (HOBt) (0.094 g) in DMF (5 mL) at 0°, is added 1-(3-dimethylaminopropyl)-3-ethylcarbodiimide (EDCI) (0.118 g) followed by diisopropylamine (0.151 g). The mixture is stirred at 0 °C for 30 minutes then at room temperature overnight. The reaction mixture is diluted with water and extracted with EtOAc. The combined organic phase is washed with 1 N aqueous HCl, 5 % aqueous NaHCO₃, water and brine, and dried over Na₂SO₄. The solvent is removed *in vacuo* to afford 0.219 g (82% yield) of the desired product which is used without further purification. LC/MS ESI+ 477 (M+1).

Preparation of 4- {(*S*)-2-[(*S*)-2-(*tert*-butoxycarbonylamino)-3-methylbutanamido]-2-(4-ethylthiazol-2-yl)ethyl}phenylsulfamic acid (17): *tert*-butyl (*S*)-1-[(*S*)-(4-ethylthiazol-2-yl)-2-(4-nitrophenyl)ethylamino]-3-methyl-1-oxobutan-2-ylcarbamate, 16, (0.219 g) is dissolved in MeOH (4 mL). A catalytic amount of Pd/C (10% w/w) is added and the mixture is stirred under a hydrogen atmosphere 2 hours. The reaction mixture is filtered through a bed of CELITE™ and the solvent is removed under reduced pressure. The crude product is dissolved in pyridine (5 mL) and treated with SO₃-pyridine (0.146 g). The reaction is stirred at room temperature for 5 minutes after which a 7% solution of NH₄OH (30 mL) is added. The mixture is then concentrated and the resulting residue is purified by reverse phase chromatography to afford 0.148 g of the desired product as the ammonium salt. ¹H NMR (CD₃OD): 8 7.08 (s, 4H), 7.02 (s, 1H), 5.43 (s, 1H), 3.85 (s, 1H), 3.28-2.77 (m, 4H), 1.94 (s, 1H), 1.46 (s, 9H), 1.29 (s, 3H, *J*=7.3 Hz), 0.83 (s, 6H).

The following are further non-limiting examples of the second type of Category IV compounds falling outside of the present invention.

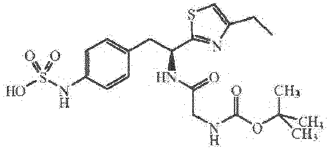

(*S*)-4-{2-[2-(*tert*-Butoxycarbonyl)acetamide]-2-(4-ethylthiazo1-2-yl)ethyl}phenyl-sulfamic acid: ¹H NMR (CD₃OD): δ 7.09-6.91 (m, 5H), 5.30 (t, 1H, *J*=8.4 Hz), 3.60-2.64 (m, 6H), 1.34 (s, 9H), 1.16 (t, 3H, *J*=7.5 Hz).

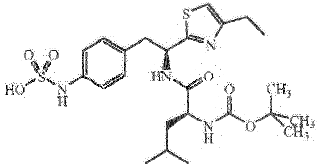

4-{(*S*)-2-[(*S*)-2-(*tert*-Butoxycarbonylamino)-4-methylpentanamido]-2-(4-ethylthiazol-2-yl)ethyl}phenylsulfamic acid: ¹H NMR (CD₃OD) δ 7.19-7.00 (m, 4H), 5.50-5.40 (m, 1H), 4.13-4.06 (m, 1H), 3.32 (1H, A of ABX, J = 7.5, 18Hz), 3.12 (1H, B of ABX, J = 8.1, 13.8Hz), 2.79 (q, 2H, J = 7.8, 14.7Hz), 1.70-1.55 (m, 1H), 1.46 (s, 9H), 1.33 (t, 3H, J = 2.7Hz), 0.92 (q, 6H, J = 6, 10.8Hz).

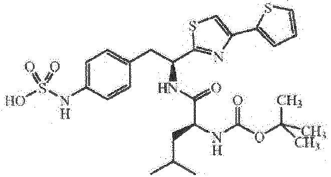

4-{(*S*)-2-[(*S*)-2-(*tert*-Butoxycarbonylamino)-4-methylpentanamido]-2-[2-(thiophen-2-yl)thiazol-4-yl]ethyl}phenylsulfamic acid: ¹H NMR (CD₃OD) δ 8.06 (d, 1H, J = 8.4Hz), 7.61-7.58 (m, 1H), 7.57 (s, 1H), 7.15 (t, 1H, J = 0.6Hz), 7.09-6.98 (m, 6H), 5.30-5.20 (m, 1H), 4.10-4.00 (m, 1H), 3.19-3.13 (m, 2H), 1.63-1.55 (m, 2H), 1.48-1.33 (m, 10H), 0.95-0.89 (m, 6H).

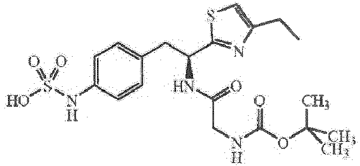

(S)-4- {2-[2-(tert-Butoxycarbonyl)acetamide]-2-(4-ethylthiazol-2-yl)ethyl}-phenylsulfamic acid: ¹H NMR (CD₃OD): δ 7.09-6.91 (m, 5H), 5.30 (t, 1H, *J*=8.4 Hz), 3.60-2.64 (m, 6H), 1.34 (s, 9H), 1.16 (t, 3H, *J*=7.5 Hz).

A further example of Category IV relates to inhibitors having the formula: 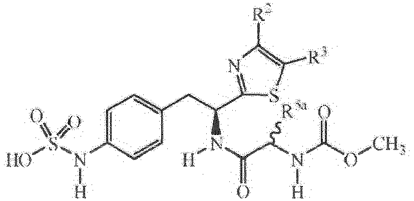 wherein R units and R^{5a} units further described in Table VII.

**TABLE VII**

| **No.** | **R** | **R^{5a}** |
|---|---|---|
| G151 | thiazol-2-yl | hydrogen |
| G132 | 4-methylthiazol-2-yl | hydrogen |
| G153 | 4-ethylthiazol-2-yl | hydrogen |
| G154 | 4-propylthiazol-2-yl | hydrogen |
| G155 | 4-*iso*-propylthiazol-2-yl | hydrogen |
| G156 | 4-cyclopropylthiazol-2-yl | hydrogen |
| G157 | 4-butylthiazol-2-yl | hydrogen |
| G158 | 4-*tert*-butylthiazol-2-yl | hydrogen |
| G159 | 4-cyclohexylthiazol-2-yl | hydrogen |
| G160 | 4,5-dimethylthiazol-2-yl | hydrogen |
| G161 | 4-methyl-5-ethylthiazol-2-yl | hydrogen |
| G162 | 4-phenylthiazol-2-yl | hydrogen |
| G163 | thiazol-2-yl | (*S*)-*iso*-propyl |
| G164 | 4-methylthiazol-2-yl | (*S*)-*iso*-propyl |
| G165 | 4-ethylthiazol-2-yl | (*S*)-*iso*-propyl |
| G166 | 4-propylthiazol-2-yl | (*S*)-*iso*-propyl |
| G167 | 4-*iso*-propylthiazol-2-yl | (*S*)-*iso*-propyl |
| G168 | 4-cyclopropylthiazol-2-yl | (*S*)-*iso*-propyl |
| G169 | 4-butylthiazol-2-yl | (*S*)-*iso*-propyl |
| G170 | 4-*tert*-butylthiazol-2-yl | (*S*)-*iso*-propyl |
| G171 | 4-cyclohexylthiazol-2-yl | (*S*)-*iso*-propyl |
| G172 | 4,5-dimethylthiazol-2-yl | (*S*)-*iso*-propyl |
| G173 | 4-methyl-5-ethylthiazol-2-yl | (*S*)-*iso*-propyl |
| G174 | 4-phenylthiazol-2-yl | (*S*)-*iso*-propyl |
| G175 | 4-(thiophen-2-yl)thiazol-2-yl | (*S*)-*iso*-propyl |

The compounds encompassed within this example of Category IV can be made according to the procedure outlined in Scheme V and described in Example 6 by substituting the corresponding methylcarbamate for the Boc-protected reagent. The following are non-limiting examples of this type.

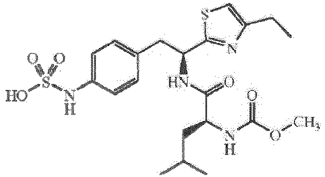

4-{(*S*)-2-(4-Ethylthiazol-2-yl)-2-[(*S*)-2-(methoxycarbonyl)-4-methylpentan-amido]ethyl}phenylsulfamic acid: ¹H NMR (CD3OD) δ 7.12-7.03 (m, 5H), 6.84 (d, 1H, J = 8.4Hz), 5.40 (t, 1H, J = 5.7Hz), 4.16 (t, 1H, J = 6.3Hz), 3.69 (s, 3H), 3.61-3.55 (m, 1H), 3.29-3.27 (m, 1H), 3.14-3.07 (m, 1H), 2.81 (q, 2H, J = 3.9, 1 1.2Hz), 1.66-1.59 (m, 1H), 1.48-1.43 (m, 2H), 1.31 (t, 3H, J = 4.5Hz), 0.96-0.90 (m, 6H).

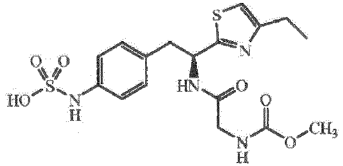

(*S*)-4-{2-(4-Ethylthiazol-2-yl)-2-[2-(methoxycarbonyl)acetamido]ethyl}-phenylsulfamic acid: ¹H NMR (CD₃OD): δ 7.12-7.07 (m, 4H), 7.03 (s, 1H), 5.42 (t, 1H, *J*=5.7 Hz), 3.83-3.68 (q, 2H, *J*=11.4 Hz), 3.68 (s, 3H), 3.34-3.04 (m, 2H), 2.83-2.76 (q, 2H, *J*=7.8 Hz), 1.31 (t, 3H, *J*=7.5 Hz).

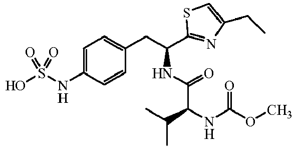

4-{(*S*)-2-(4-Ethylthiazol-2-yl)-2-[(*S*)-2-(methoxycarbonyl)-3-methylbutanamido]-ethyl}phenylsulfamic acid: ¹H NMR (CD₃OD) δ 8.56 (d, 1H, J = 7.8Hz), 7.09 (s, 4H), 7.03 (s, 1H), 5.26-5.20 (m, 1H), 3.90 (d, 1H, J = 7.8Hz), 3.70 (s, 3H), 3.30 (1H, A of ABX, obscured by solvent), 3.08 (1H, B of ABX, J = 9.9, 9Hz), 2.79 (q, 2H, J = 11.1, 7.2Hz), 2.05-1.97 (m, 1H), 1.31 (t, 3H, J = 7.5Hz), 0.88 (s, 3H), 0.85 (s, 3H), 0.79-0.75 (m, 1H).

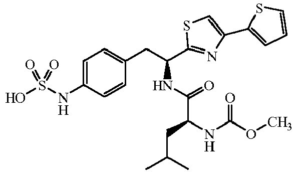

4-{(*S*)-2-[(*S*)-2-(Methoxycarbonyl)-4-methylpentanamido]-2-[2-(thiophen-2-yl)thiazol-4-yl]ethyl}phenylsulfamic acid: ¹H NMR (CD₃OD) δ 8.22 (d, 1H, J = 9Hz), 7.62-7.57 (m, H), 7.15 (t, 1H, J = 0.6Hz), 7.10-6.97 (m, 4H), 5.30-5.20 (m, 1H), 4.16-4.11 (m, 1H), 3.67 (s, 2H), 3.22 (1H, A of ABX, J = 6.9, 13.5Hz), 3.11 (1H, B of ABX, J = 7.8, 13.6Hz), 1.65-1.58 (m, 1H), 1.50-1.45 (m, 2H), 0.95-0.88 (m, 6H).

Category IV of the present disclosure relates to compounds having the formula: 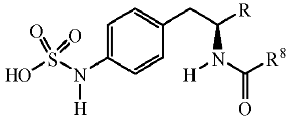 wherein R is a substituted or unsubstituted thiophen-2-yl or thiophen-4-yl unit and non-limiting examples of R² are further described in Table VIII.

**TABLE VIII**

| **No.** | **R** | **R⁸** |
|---|---|---|
| H176 | thiazol-2-yl | -OC(CH₃)₃ |
| H177 | 4-methylthiazol-2-yl | -OC(CH₃)₃ |
| H178 | 4-ethylthiazol-2-yl | -OC(CH₃)₃ |
| H179 | 4-cyclopropylthiazol-2-yl | -OC(CH₃)₃ |
| H180 | 4-*tert*-butylthiazol-2-yl | -OC(CH₃)₃ |
| H181 | 4-cyclohexylthiazol-2-yl | -OC(CH₃)₃ |
| H182 | 4-(2,2,2-trifluoroethyl)thiazol-2-yl | -OC(CH₃)₃ |
| H183 | 4-(3,3,3-trifluoropropyl)thiazol-2-yl | -OC(CH₃)₃ |
| H184 | 4-(2,2-difluorocyclopropyl)thiazol-2-yl | -OC(CH₃)₃ |
| H185 | 4,5-dimethylthiazol-2-yl | -OC(CH₃)₃ |
| H186 | 4-methyl-5-ethylthiazol-2-yl | -OC(CH₃)₃ |
| H187 | 4-phenylthiazol-2-yl | -OC(CH₃)₃ |
| H188 | 4-(4-chlorophenyl)thiazol-2-yl | -OC(CH₃)₃ |
| H189 | 4-(3,4-dimethylphenyl)thiazol-2-yl | -OC(CH₃)₃ |
| H190 | 4-methyl-5-phenylthiazol-2-yl | -OC(CH₃)₃ |
| H191 | 4-(thiophen-2-yl)thiazol-2-yl | -OC(CH₃)₃ |
| H192 | thiazol-4-yl | -OC(CH₃)₃ |
| H193 | 4-methylthiazol-4-yl | -OC(CH₃)₃ |
| H194 | 4-ethylthiazol-4-yl | -OC(CH₃)₃ |
| H195 | 4-cyclopropylthiazol-4-yl | -OC(CH₃)₃ |
| H196 | 4-*tert*-butylthiazol-4-yl | -OC(CH₃)₃ |
| H197 | 4-cyclohexylthiazol-4-yl | -OC(CH₃)₃ |
| H198 | 4-(2,2,2-trifluoroethyl)thiazol-4-yl | -OC(CH₃)₃ |
| H199 | 4-(3,3,3-trifluoropropyl)thiazol-4-yl | -OC(CH₃)₃ |
| H200 | 4-(2,2-difluorocyclopropyl)thiazol-4-yl | -OC(CH₃)₃ |
| H201 | 4,5-dimethylthiazol-4-yl | -OC(CH₃)₃ |
| H202 | 4-methyl-5-ethylthiazol-4-yl | -OC(CH₃)₃ |
| H203 | 4-phenylthiazol-4-yl | -OC(CH₃)₃ |
| H204 | 4-(4-chlorophenyl)thiazol-4-yl | -OC(CH₃)₃ |
| H205 | 4-(3,4-dimethylphenyl)thiazol-4-yl | -OC(CH₃)₃ |
| H206 | 4-methyl-5-phenylthiazol-4-yl | -OC(CH₃)₃ |
| H207 | 4-(thiophen-2-yl)thiazol-4-yl | -OC(CH₃)₃ |
| H208 | thiazol-2-yl | -OCH₃ |
| H209 | 4-methylthiazol-2-yl | -OCH₃ |
| H210 | 4-ethylthiazol-2-yl | -OCH₃ |
| H211 | 4-cyclopropylthiazol-2-yl | -OCH₃ |
| H212 | 4-*tert*-butylthiazol-2-yl | -OCH₃ |
| H213 | 4-cyclohexylthiazol-2-yl | -OCH₃ |
| H214 | 4-(2,2,2-trifluoroethyl)thiazol-2-yl | -OCH₃ |
| H215 | 4-(3,3,3-trifluoropropyl)thiazol-2-yl | -OCH₃ |
| H216 | 4-(2,2-difluorocyclopropyl)thiazol-2-yl | -OCH₃ |
| H217 | 4,5-dimethylthiazol-2-yl | -OCH₃ |
| H218 | 4-methyl-5-ethylthiazol-2-yl | -OCH₃ |
| H219 | 4-phenylthiazol-2-yl | -OCH₃ |
| H220 | 4-(4-chlorophenyl)thiazol-2-yl | -OCH₃ |
| H221 | 4-(3,4-dimethylphenyl)thiazol-2-yl | -OCH₃ |
| H222 | 4-methyl-5-phenylthiazol-2-yl | -OCH₃ |
| H223 | 4-(thiophen-2-yl)thiazol-2-yl | -OCH₃ |
| H224 | thiazol-4-yl | -OCH₃ |
| H225 | 4-methylthiazol-4-yl | -OCH₃ |
| H226 | 4-ethylthiazol-4-yl | -OCH₃ |
| H227 | 4-cyclopropylthiazol-4-yl | -OCH₃ |
| H228 | 4-*tert*-butylthiazol-4-yl | -OCH₃ |
| H229 | 4-cyclohexylthiazol-4-yl | -OCH₃ |
| H230 | 4-(2,2,2-trifluoroethyl)thiazol-4-yl | -OCH₃ |
| H231 | 4-(3,3,3-trifluoropropyl)thiazol-4-yl | -OCH₃ |
| H232 | 4-(2,2-difluorocyclopropyl)thiazol-4-yl | -OCH₃ |
| H233 | 4,5-dimethylthiazol-4-yl | -OCH₃ |
| H234 | 4-methyl-5-ethylthiazol-4-yl | -OCH₃ |
| H235 | 4-phenylthiazol-4-yl | -OCH₃ |
| H236 | 4-(4-chlorophenyl)thiazol-4-yl | -OCH₃ |
| H237 | 4-(3,4-dimethylphenyl)thiazol-4-yl | -OCH₃ |
| H238 | 4-methyl-5-phenylthiazol-4-yl | -OCH₃ |
| H239 | 4-(thiophen-2-yl)thiazol-4-yl | -OCH₃ |
| H240 | thiazol-2-yl | -CH₃ |
| H241 | 4-methylthiazol-2-yl | -CH₃ |
| H242 | 4-ethylthiazol-2-yl | -CH₃ |
| H243 | 4-cyclopropylthiazol-2-yl | -CH₃ |
| H244 | 4-*tert*-butylthiazol-2-yl | -CH₃ |
| H245 | 4-cyclohexylthiazol-2-yl | -CH₃ |
| H246 | 4-(2,2,2-trifluoroethyl)thiazol-2-yl | -CH₃ |
| H247 | 4-(3,3,3-trifluoropropyl)thiazol-2-yl | -CH₃ |
| H248 | 4-(2,2-difluorocyclopropyl)thiazol-2-yl | -CH₃ |
| H249 | 4,5-dimethylthiazol-2-yl | -CH₃ |
| H250 | 4-methyl-5-ethylthiazol-2-yl | -CH₃ |
| H251 | 4-phenylthiazol-2-yl | -CH₃ |
| H252 | 4-(4-chlorophenyl)thiazol-2-yl | -CH₃ |
| H253 | 4-(3,4-dimethylphenyl)thiazol-2-yl | -CH₃ |
| H254 | 4-methyl-5-phenylthiazol-2-yl | -CH₃ |
| H255 | 4-(thiophen-2-yl)thiazol-2-yl | -CH₃ |
| H256 | thiazol-4-yl | -CH₃ |
| H257 | 4-methylthiazol-4-yl | -CH₃ |
| H258 | 4-ethylthiazol-4-yl | -CH₃ |
| H259 | 4-cyclopropylthiazol-4-yl | -CH₃ |
| H260 | 4-*tert*-butylthiazol-4-yl | -CH₃ |
| H261 | 4-cyclohexylthiazol-4-yl | -CH₃ |
| H262 | 4-(2,2,2-trifluoroethyl)thiazol-4-yl | -CH₃ |
| H263 | 4-(3,3,3-trifluoropropyl)thiazol-4-yl | -CH₃ |
| H264 | 4-(2,2-difluorocyclopropyl)thiazol-4-yl | -CH₃ |
| H265 | 4,5-dimethylthiazol-4-yl | -CH₃ |
| H266 | 4-methyl-5-ethylthiazol-4-yl | -CH₃ |
| H267 | 4-phenylthiazol-4-yl | -CH₃ |
| H268 | 4-(4-chlorophenyl)thiazol-4-yl | -CH₃ |
| H269 | 4-(3,4-dimethylphenyl)thiazol-4-yl | -CH₃ |
| H270 | 4-methyl-5-phenylthiazol-4-yl | -CH₃ |
| H271 | 4-(thiophen-2-yl)thiazol-4-yl | -CH₃ |

The compounds encompassed within Category IV of the present disclosure can be prepared by the procedure outlined in VI and described in Example 7 herein below.

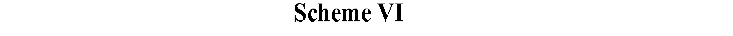

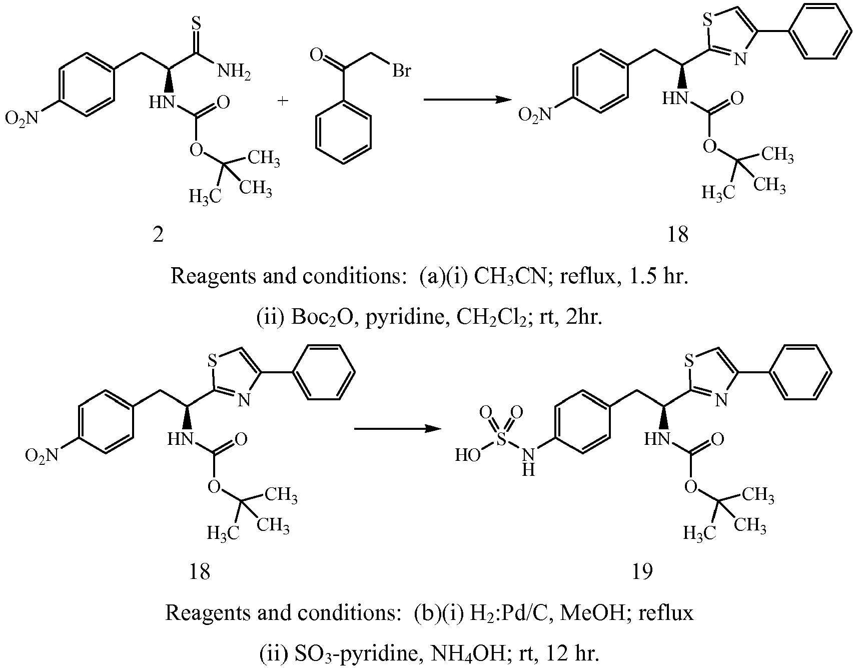

### EXAMPLE 7

### [1-(S)-(Phenylthiazol-2-yl)-2-(4-sulfoaminophenyl)ethyl]-carbamic acid tert-butyl ester (19)

Preparation of [2-(4-nitrophenyl)-1-(*S*)-(4-phenylthiazol-2-yl)ethyl]-carbamic acid *tert-*butyl ester (18): A mixture of [2-(4-nitrophenyl)-1-(*S*)-thiocarbamoylethyl]-carbamic acid *tert-*butyl ester, 2, (0.343 g, 1.05 mmol), 2-bromoacetophenone (0.231 g, 1.15 mmol), in CH₃CN (5 mL) is refluxed 1.5 hour. The solvent is removed under reduced pressure and the residue re-dissolved in CH₂Cl₂ then pyridine (0.24 mL, 3.0 mmol) and BoC₂O (0.24 mL, 1.1 mmol) are added. The reaction is stirred for 2 hours and diethyl ether is added to the solution and the precipitate which forms is removed by filtration. The organic layer is dried (Na₂SO₄), filtered, and concentrated to a residue which is purified over silica to afford 0.176 g (39%) of the desired product ESI+ MS 426 (M+1).

Preparation of [1-(*S*)-(phenylthiazol-2-yl)-2-(4-sulfoaminophenyl)ethyl]-carbamic acid *tert-*butyl ester (19): [2-(4-nitrophenyl)-1-(*S*)-(4-phenylthiazol-2-yl)ethyl]-carbamic acid *tert-*butyl ester, 18, (0.176 g, 0.41 mmol) is dissolved in MeOH (4 mL). A catalytic amount of Pd/C (10% w/w) is added and the mixture is stirred under a hydrogen atmosphere 12 hours. The reaction mixture is filtered through a bed of CELITE™ and the solvent is removed under reduced pressure. The crude product is dissolved in pyridine (12 mL) and treated with SO₃-pyridine (0.195 g, 1.23 mmol). The reaction is stirred at room temperature for 5 minutes after which a 7% solution of NH₄OH (10 mL) is added. The mixture is then concentrated and the resulting residue is purified by reverse phase chromatography to afford 0.080 g of the desired product as the ammonium salt. ¹H NMR (300 MHz, MeOH-d₄) δ 7.93 (d, *J* = 6.0 Hz, 2H), 7.68 (s, 1H), 7.46-7.42 (m, 3H), 7.37-7.32 (m, 1H), 7.14-7.18 (m, 3H), 5.13-5.18 (m, 1H), 3.40 (dd, *J* = 4.5 and 15.0 Hz, 1H), 3.04 (dd, *J* = 9.6 and 14.1 Hz, 1H), 1.43 (s, 9H).

The following are further non-limiting examples of Category IV of the present disclosure.

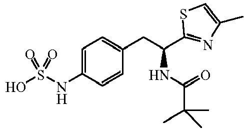

(*S*)-4-(2-(4-Methylthiazol-2-yl)-2-pivalamidoethyl)phenylsulfamic acid: ¹H NMR(CD₃OD): δ 7.31 (s, 4H), 7.20 (s, 1H), 5.61-5.56 (m, 1H), 3.57-3.22 (m, 2H), 2.62 (s, 3H), 1.31 (s, 3H).

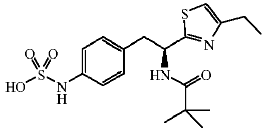

(*S*)-4-(2-(4-Ethylthiazol-2-yl)-2-pivalamidoethyl)phenylsulfamic acid: ¹H NMR (300 MHz, MeOH-d₄) δ 7.92 (d, *J* = 8.1 Hz, 1H), 7.12-7.14 (m, 4H), 7.03 (s, 1H), 5.38-5.46 (m, 1H), 3.3-3.4 (m, 1H), 3.08 (dd, *J* = 10.2 and 13.8 Hz, 1H), 2.79 (q, *J* = 7.2 Hz, 2H), 1.30 (t, *J* = 7.2 Hz, 3H), 1.13 (s, 9H).

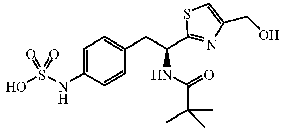

(*S*)-4-(2-(4-(Hydroxymethyl)thiazol-2-yl)-2-pivalamidoethyl)phenylsulfamic acid: ¹H NMR (300 MHz, MeOH-d₄) δ 7.92 (d, *J* = 8.1 Hz, 1H), 7.24 (s, 1H), 7.08 (d, *J* = 8.7 Hz, 2H), 7.00 (d, *J* = 8.7 Hz, 2H), 5.29-5.37 (m, 1H), 4.55 (s, 2H), 3.30 (dd, *J* = 4.8 and 13.5 Hz, 1H), 2.99 (dd, *J* = 10.5 and 13.5 Hz, 1H), 0.93 (s, 9H).

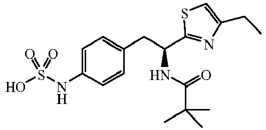

(*S*)-4-(2-(4-(Ethoxycarbonyl)thiazol-2-yl)-2-pivalamidoethyl)phenylsulfamic acid: ¹HNMR (300 MHz, MeOH-d₄) δ 8.30 (s, 1H), 8.04 (d, *J* = 8.1 Hz, 1H), 7.13 (s, 4H), 5.41-5.49 (m, 1H), 4.41 (q, *J* = 7.2 Hz, 2H), 3.43 (dd, *J* = 5.1 and 13.8 Hz, 1H), 3.14 (dd, *J* = 5.7 and 9.9 Hz, 1H), 1.42 (t, *J* = 7.2 Hz, 3H), 1.14 (s, 9H).

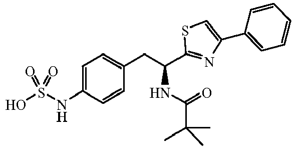

(*S*)-4-(2-(4-Phenylthiazol-2-yl)-2-pivalamidoethyl)phenylsulfamic acid: ¹H NMR (300 MHz, MeOH-d₄) δ 7.94-8.01 (m, 3H), 7.70 (s, 1H), 7.42-7.47 (m, 2H), 7.32-7.47 (m, 1H), 7.13-7.20 (m, 3H), 5.48-5.55 (m, 1H), 3.50 (dd, *J* = 5.1 and 14.1 Hz, 1H), 3.18 (dd, *J* = 10.2 and 14.1 Hz, 1H), 1.17 (s, 9H).

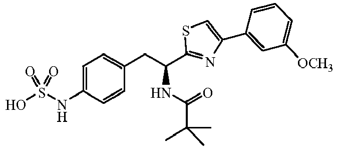

4-((S)-2-(4-(3-Methoxyphenyl)thiazol-2-yl)-2-pivalamidoethyl)phenylsulfamic acid: ¹H NMR (CD₃OD): δ 7.96-7.93 (d, 1H, *J*=8.1 Hz), 7.69 (s, 1H), 7.51-7.49 (d, 2H, *J*=7.9 Hz), 7.33 (t, 1H, *J*=8.0 Hz), 7.14 (s, 4H), 6.92-6.90 (d, 1H, *J*=7.8 Hz), 5.50 (t, 1H, *J*=5.1 Hz), 3.87 (s, 3H), 3.50-3.13 (m, 2H), 1.15 (s, 9H).

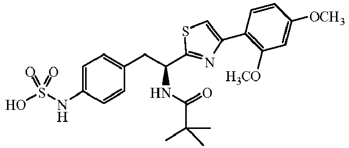

4-((S)-2-(4-(2,4-Dimethoxyphenyl)thiazol-2-yl)-2-pivalamidoethyl)phenylsulfamic acid: ¹HNMR (CD₃OD): δ 8.11-8.09 (d, 1H, *J*=7.8 Hz), 7.96-7.93 (d, 1H, *J*= 8.4 Hz), 7.74 (s, 1H), 7.18-7.16 (m, 4H), 6.67-6.64 (d, 2H, *J*=9.0 Hz), 5.55-5.47 (m, 1H), 3.95 (s, 3H), 3.87 (s, 3H), 3.52-3.13 (m, 2H), 1.17 (s, 9H).

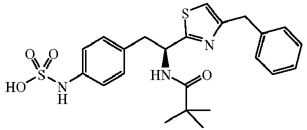

(S)-4-(2-(4-Benzylthiazol-2-yl)-2-pivalamidoethyl)phenylsulfamic acid: ¹H NMR (CD₃OD) δ 7.85 (d, 1H, J = 8.4Hz), 7.38-7.20 (m, 4H), 7.11-7.02 (m, 1H), 7.00 (s, 1H), 5.42-5.37 (m, 1H), 4.13 (s, 2H), 3.13-3.08 (m, 2H), 1.13 (s, 9H).

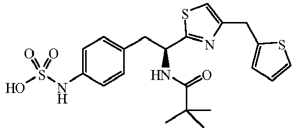

(S)-4-(2-Pivalamido-2-(4-(thiophen-2-ylmethyl)thiazol-2-yl)ethyl)phenylsulfamic acid: ¹HNMR (CD₃OD) δ 7.88-7.85 (d, 1H), 7.38-7.35 (m, 1H), 7.10-7.01 (m, 4H), 7.02 (s, 1H), 5.45-5.38 (m, 1H), 4.13 (s, 2H), 3.13-3.05 (m, 2H), 1.13 (2, 9H).

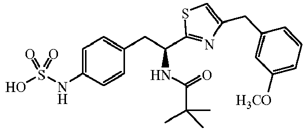

(S)-4-(2-(4-(3-Methoxybenzyl)thiazol-2-yl)-2-pivalamidoethyl)phenylsulfamic acid: ¹H NMR (CD₃OD) δ 7.85 (d, 1H, J = 8.4Hz), 7.25-7.20 (m, 1H), 7.11-7.02 (m, 4H), 7.01 (s, 1H), 6.90-6.79 (m, 2H), 5.45-5.40 (m, 1H), 4.09 (s, 2H), 3.79 (s, 3H), 3.12-3.08 (m, 2H), 1.10 (s, 9H).

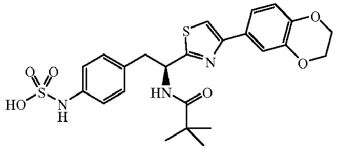

4-((*S*)-2-(4-(2,3-Dihydrobenzo[b][1,4]dioxin-6-yl)thiazol-2-yl)-2-pivalamidoethyl)-phenylsulfamic acid: ¹H NMR (CD₃OD): δ 7.53 (s, 1H), 7.45 (s, 1H), 7.42-7.40 (d, 1H, *J*= 8.4 Hz), 7.19-7.15 (m, 4H), 6.91-6.88 (d, 2H, *J*=8.4 Hz), 5.51-5.46 (m, 1H), 4.30 (s, 4H), 3.51-3.12 (m, 2H), 1.16 (s, 9H).

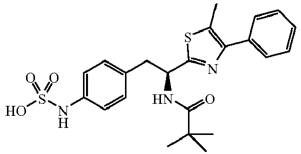

(S)-4-(2-(5-Methyl-4-phenylthiazol-2-yl)-2-pivalamidoethyl)phenylsulfamic acid: ¹H NMR (CD₃OD): δ 7.63-7.60 (d, 2H, *J*=7.1 Hz), 7.49-7.35 (m, 3H), 7.14 (s, 4H), 5.43-5.38 (m, 1H), 3.42-3.09 (m, 2H), 2.49 (s, 3H), 1.14 (s, 9H).

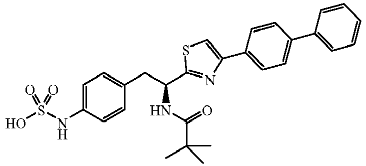

(S)-4- (2-(4-(Biphen-4-yl)thiazol-2-yl)-2-pivalamidoethyl)phenylsulfamic acid: ¹H NMR (CD₃OD): δ 8.04-8.01 (m, 2H), 7.72-7.66 (m, 5H), 7.48-7.35 (m, 3H), 7.15 (s, 4H), 5.50 (t, 1H, *J*=5.0 Hz), 3.57-3.15 (d, 2H), 1.16 (s, 9H).

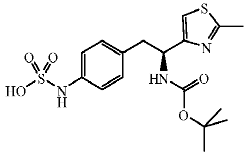

(*S*)-4-(2-*tert*-Butoxycarbonyl-2-(2-methylthaizol-4-yl)-phenylsulfamic acid ¹H NMR (300 MHz, D₂O) δ 6.99-7.002(m, 4H), 6.82 (s, 1H), 2.26 (dd, *J* = 13.8 and 7.2 Hz, 1H), 2.76 (dd, *J* = 13.8 and 7.2 Hz, 1H), 2.48 (s, 3H), 1.17 (s, 9H).

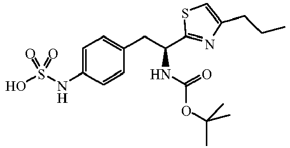

(S)-4-(2-(*tert*-Butoxycarbonyl)-2-(4-propylthiazol-2-yl)ethyl)-phenyl sulfamic acid: ¹H NMR (300 MHz, CD₃OD): δ 7.18-7.02 (m, 5H), 5.06-5.03 (m, 1H), 3.26 (dd, *J*=13.8, 4.8 Hz, 1H), 2.95 (dd, *J*=13.8, 9.3 Hz, 1H), 2.74 (dd, *J*=15.0, 7.2 Hz, 2H), 1.81-1.71 (m, 2H), 1.40 (s, 7H), 1.33 (bs, 2H), 0.988 (t, *J*= 7.5 Hz 3H).

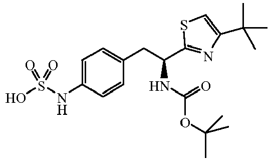

(S)-4-(2-(*tert*-Butoxycarbonyl)-2-(4-*tert*-butylthiazol-2-yl)ethyl)-phenyl sulfamic acid: ¹HNMR (300 MHz, CD₃OD): δ 7.12 (s, 4H), 7.01 (s, 1H), 5.11-5.06 (m, 1H), 3.32-3.25 (m, 1H), 2.96 (m, 1H), 1.42 (s, 8H), 1.38 (s, 9H), 1.32 (s, 1H).

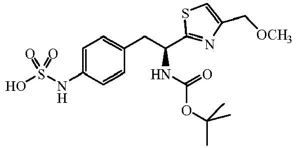

(S)-4-(2-(*tert*-Butoxycarbonylamino)-2-(4-(methoxymethyl)thiazol-2-yl)ethyl)-phenyl sulfamic acid: ¹H NMR (300 MHz, CD₃OD): δ 7.36 (s, 1H), 7.14-7.05 (m, 4H), 5.06 (dd, *J*=9.0, 5.1 Hz, 1H), 4.55 (s, 2H), 3.42 (s, 3H), 3.31-3.24 (m, 1H), 2.97 (dd, *J*=13.8, 9.9 Hz, 1H), 1.47-1.31 (m, 9H).

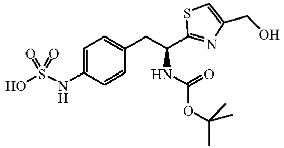

(*S*)-4-(2-*tert*-Butoxycarbonylamino)-2-(4-(2-hydroxymethyl)thiazol-2-yl)ethyl)phenylsulfamic acid: ¹H NMR (300 MHz, MeOH-d₄) δ 7.22-7.25 (m, 1H), 7.09-7.15 (m, 4H), 5.00-5.09 (m, 1H), 4.32-4.35 (m, 1H), 3.87 (t, *J* = 6.6 Hz, 2H), 3.23-3.29 (m, 1H), 3.09-3.18 (m, 1H), 2.98 (t, *J* = 6.6 Hz, 2H), 1.41 (s, 9H).

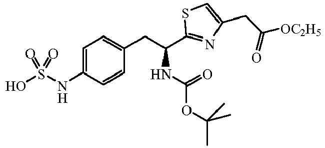

(*S*)-4-(2-*tert*-Butoxycarbonylamino)-2-(4-(2-ethoxy-2-oxoethyl)-thiazole-2-yl)-ethyl)phenylsulfamic acid: ¹H NMR (300 MHz, MeOH-d₄) δ 7.29 (s, 1H), 7.09-7.16 (m, 4H), 5.04-5.09 (m, 1H), 4.20 (q, *J* = 6.9 Hz, 2H), 3.84 (s, 2H), 3.30 (dd, *J* = 4.8 and 14.1 HZ, 1H), 2.97 (dd, *J* = 9.6 Hz and 13.8 Hz, 1H), 1.41 (s, 9H), 1.29 (t, *J* = 7.2 Hz, 3H).

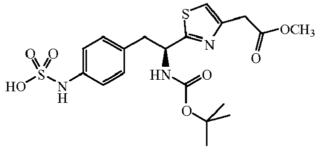

(*S*)-4-(2-(*tert*-Butoxycarbonylamino)-2-(4-(2-methoxy-2-oxoethyl)thiazol-2-yl)ethyl)phenylsulfamic acid: ¹H NMR (300 MHz, MeOH-d₄) δ 7.31 (s, 1H), 7.01-7.16 (m, 4H), 5.04-5.09 (m, 1H), 4.01 (s, 2H), 3.78 (s, 2H), 3.74 (s, 3H), 3.29 (dd, *J* = 5.1 and 13.8 Hz, 1H), 2.99 (dd, *J* = 9.3 and 13.8 Hz, 1H), 1.41 (s, 9H).

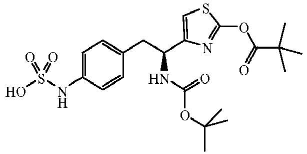

(*S*)-4-(2-(*tert*-Butoxycarbonylamino)-2-(2-(pivaloyloxy)thiazol-4-yl)ethyl)-phenylsulfamic acid: ¹H NMR (300 MHz, D₂O) δ 6.95 (s, 4H), 6.63 (s, 1H), 2.94 (dd, *J* = 13.5 and 4.8 Hz, 1H), 2.75 (dd, *J* =13.5 and 4.8 Hz, 1H), 1.16 (s, 9H), 1.13 (s, 9H).

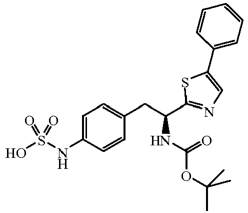

(*S*)-4-(2-(*tert*-Butoxycarbonylamino)-2-(5-phenylthiazol-2-yl)ethyl)-phenyl sulfamic acid: ¹H NMR (300 MHz, CD₃OD): δ 7.98 (s, 1H), 7.62 (d, *J*=7.2 Hz, 2H), 7.46-7.35 (m, 4H), 7.14 (s, 4H), 5.09 (bs, 1H), 3.07-2.99 (m, 2H), 1.43 (s, 9H).

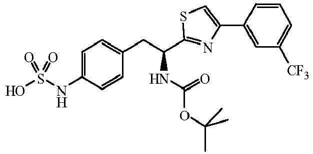

4-((S)-2-(tert-Butoxycarbonylamino)-2-(4-(3-(trifluoromethyl)phenyl)thiazol-2-yl)ethyl)phenyl sulfamic acid: ¹H NMR (300 MHz, CD₃OD): δ 8.28 (s, 1H), 8.22-8.19 (m, 1H),7.89 (s, 1H), 7.65 (d, *J*=5.1 Hz, 2H), 7.45 (d, *J*=8.1 Hz, 1H), 7.15 (s, 4H), 5.17-5.14 (m, 1H), 3.43-3.32 (m, 1H), 3.05 (dd, *J*=14.1, 9.6 Hz, 1H), 1.42 (s, 9H).

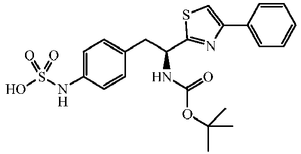

(*S*)-4-(2-(*tert*-Butoxycarbonylamino)-2-(4-phenylthiazol-2-yl)ethyl)-phenyl sulfamic acid: ¹H NMR (300 MHz, CD₃OD): δ 7.98 (s, 1H), 7.94 (d, *J*=7.2 Hz, 2H), 7.46-7.35 (m, 4H), 7.14 (s, 4H), 5.09 (bs, 1H), 3.07-2.99 (m, 2H), 1.43 (s, 9H).

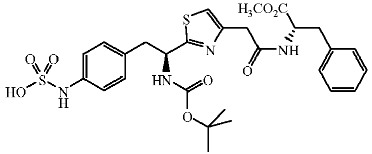

(S,S)-2-(2-{2-[2-*tert*-Butoxycarbonylamino-2-(4-sulfoaminophenyl)ethyl]thiazol-4-yl}acetylamido)-3-phenylpropionic acid methyl ester: ¹H NMR (300 MHz, MeOH-d₄) δ 6.85-6.94 (m, 9H), 6.64 (s, 1H), 4.83 (s, 1H), 4.54-4.58 (m, 1H), 3.49 (s, 3H), 3.39 (s, 2H), 2.80-2.97 (m, 1H), 2.64-2.78 (m, 1H), 1.12 (s, 9H).

(S)-[1-{1-Oxo-4-[2-(1-phenyl-1H-tetrazol-5-sulfonyl)ethyl]-1H-1λ⁴-thiazol-2-yl}-2-(4-sulfamino-phenyl)-ethyl]-carbamic acid *tert-*butyl ester: ¹H NMR (300 MHz, MeOH-d₄) δ 7.22-7.75 (m, 2H), 7.62-7.69 (m, 2H), 7.55 (s, 1H), 7.10-7.20 (m, 5H), 5.25 (m, 1H), 4.27-4.36 (m, 1H), 4.11-4.21 (m, 1H), 3.33-3.44 (m, 4H), 2.84-2.90 (m, 1H), 1.33 (s, 9H).

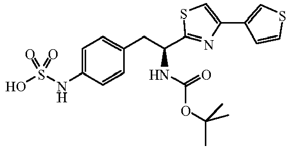

4-((S)- 2-(*tert*-Butoxycarbonylamino)-2-(4-(thiophen-3-yl)thiazol-2-yl)ethyl)phenyl sulfamic acid: ¹H NMR (300 MHz, CD₃OD): δ 7.84 (dd, *J*=3.0, 1.5 Hz, 1H), 7.57-7.55 (m, 2H), 7.47 (dd, *J*=4.8, 3.0 Hz, 1H), 7.15(s, 4H), 5.15-5.10 (m, 1H), 3.39-3.34 (m, 1H), 3.01 (dd, *J*=14.1, 9.6 Hz, 1H), 1.42 (s, 8H), 1.32 (s, 1H).

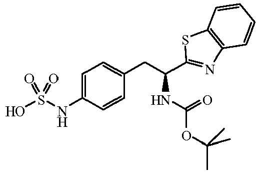

(S)-4-(2-(Benzo[d]thiazol-2-ylamino)-2-(*tert*-butoxycarbonyl)ethyl)phenylsulfamic acid: ¹H NMR (CD₃OD) δ 7.86-7.82 (m, 2H), 7.42 (t, 2H, J=7.1 Hz), 7.33 (t, 1H, J=8.2 Hz), 7.02 (s, 4H), 5.10-5.05 (m, 1H), 2.99-2.91 (m, 2H), 1.29 (s, 9H).

(*S*)-4-(2-*tert*-Butoxycarbonylamino)-2-(2-methylthiazol-4-yl)-phenylsulfamic acid ¹H NMR (300 MHz, D₂O) δ 6.99-7.002(m, 4H), 6.82 (s, 1H), 2.26 (dd, *J* = 13.8 and 7.2 Hz, 1H), 2.76 (dd, *J* = 13.8 and 7.2 Hz, 1H), 2.48 (s, 3H), 1.17 (s, 9H).

The first type of Category V of the present disclosure relates to 2-(thiazol-2-yl) compounds having the formula: 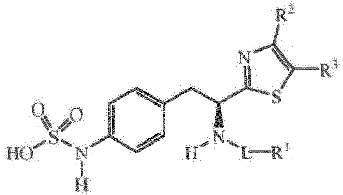 wherein R¹, R², R³, and L are further defined herein in Table IX herein below.

**TABLE IX**

| **No.** | **L** | **R¹** | **R²** | **R³** |
|---|---|---|---|---|
| I272 | -C(O)CH₂- | phenyl | -CH₃ | -H |
| I273 | -C(O)CH₂- | 2-fluorophenyl | -CH₃ | -H |
| I274 | -C(O)CH₂- | 3-fluorophenyl | -CH₃ | -H |
| I275 | -C(O)CH₂- | 4-fluorophenyl | -CH₃ | -H |
| I276 | -C(O)CH₂- | 2.3-difluorophenyl | -CH₃ | -H |
| I277 | -C(O)CH₂- | 3.4-difluoropheny | -CH₃ | -H |
| I278 | -C(O)CH₂- | 3.5-difluorophenyl | -CH₃ | -H |
| I279 | -C(O)CH₂- | 2-chlorophenyl | -CH₃, | -H |
| I280 | -C(O)CH₂- | 3-chlorophenyl | -CH₃ | -H |
| I281 | -C(O)CH₂- | 4-chlorophenyl | -CH₃ | -H |
| I282 | -C(O)CH₂- | 2,3-dichlorophenyl | -CH₃ | -H |
| I283 | -C(O)CH₂- | 3,4-dichlorophenyl | -CH₃ | -H |
| I294 | -C(O)CH₂- | 3,5-dichlorophenyl | -CH₃ | -H |
| I285 | -C(O)CH₂- | 2-hydroxyphenyl | -CH₃ | -H |
| I286 | -C(O)CH₂- | 3-hydroxyphenyl | -CH₃ | -H |
| I287 | -C(O)CH₂- | 4-hydroxypheny | -CH₃ | -H |
| I288 | -C(O)CH₂- | 2-methoxyphenyl | -CH₃ | -H |
| I289 | -C(O)CH₂- | 3-methoxyphenyl | -CH₃ | -H |
| I290 | -C(O)CH₂- | 4-methoxypheny | -CH₃ | -H |
| I291 | -C(O)CH₂- | 2.3-dimethoxyphenyl | -CH₃ | -H |
| I292 | -C(O)CH₂- | 3,4-dimethoxyphenyl | -CH₃ | -H |
| I293 | -C(O)CH₂- | 3.5-dimethoxyphenyl | -CH₃ | -H |
| I294 | -C(O)CH₂- | phenyl | -CH₂CH₃ | -H |
| I295 | -C(O)CH₂- | 2-fluorophenyl | -CH₂CH₃ | -H |
| I296 | -C(O)CH₂- | 3-fluorophenyl | -CH₂CH₃ | -H |
| I297 | -C(O)CH₂- | 4-fluorophenyl | -CH₂CH₃ | -H |
| I298 | -C(O)CH₂- | 2,3-difluorophenyl | -CH₂CH₃ | -H |
| I299 | -C(O)CH₂- | 3,4-difluorophenyl | -CH₂CH₃ | -H |
| I300 | -C(O)CH₂- | 3,5-difluorophenyl | -CH₂CH₃ | -H |
| I301 | -C(O)CH₂- | 2-chlorophenyl | -CH₂CH₃ | -H |
| I302 | -C(O)CH₂- | 3-chlorophenyl | -CH₂CH₃ | -H |
| I303 | -C(O)CH₂- | 4-chlorophenyl | -CH₂CH₃ | -H |
| I304 | -C(O)CH₂- | 2,3-dichlorophenyl | -CH₂CH₃ | -H |
| I305 | -C(O)CH₂- | 3,4-dichlorophenyl | -CH₂CH₃ | -H |
| I306 | -C(O)CH₂- | 3,5-dichlorophenyl | -CH₂CH₃ | -H |
| I307 | -C(O)CH₂- | 2-hydroxyphenyl | -CH₂CH₃ | -H |
| I308 | -C(O)CH₂- | 3-hydroxyphenyl | -CH₂CH₃ | -H |
| I309 | -C(O)CH₂- | 4-hydroxyphenyl | -CH₂CH₃ | -H |
| I310 | -C(O)CH₂- | 2-methoxyphenyl | -CH₂CH₃ | -H |
| I311 | -C(O)CH₂- | 3-methoxyphenyl | -CH₂CH₃ | -H |
| I312 | -C(O)CH₂- | 4-methoxyphenyl | -CH₂CH₃ | -H |
| I313 | -C(O)CH₂- | 2,3 -dimethoxyphenyl | -CH₂CH₃ | -H |
| I314 | -C(O)CH₂- | 3,4-dimethoxyphenyl | -CH₂CH₃ | -H |
| I315 | -C(O)CH₂- | 3,5-dimethoxyphenyl | -CH₂CH₃ | -H |
| I316 | -C(O)CH₂CH₂- | phenyl | -CH₃ | -H |
| I317 | -C(O)CH₂CH₂- | 2-fluorophenyl | -CH₃ | -H |
| I318 | -C(O)CH₂CH₂- | 3-fluorophenyl | -CH₃ | -H |
| I319 | -C(O)CH₂CH₂- | 4-fluorophenyl | -CH₃ | -H |
| I320 | -C(O)CH₂CH₂- | 2,3-difluorophenyl | -CH₃ | -H |
| I321 | -C(O)CH₂CH₂- | 3,4-difluorophenyl | -CH₃ | -H |
| I322 | -C(O)CH₂CH₂- | 3,5-difluorophenyl | -CH₃ | -H |
| I323 | -C(O)CH₂CH₂- | 2-chlorophenyl | -CH₃ | -H |
| I324 | -C(O)CH₂CH₂- | 3-chlorophenyl | -CH₃ | -H |
| I325 | -C(O)CH₂CH₂- | 4-chlorophenyl | -CH₃ | -H |
| I326 | -C(O)CH₂CH₂- | 2,3-dichlorophenyl | -CH₃ | -H |
| I327 | -C(O)CH₂CH₂- | 3,4-dichlorophenyl | -CH₃ | -H |
| I328 | -C(O)CH₂CH₂- | 3,5-dichlorophenyl | -CH₃ | -H |
| I329 | -C(O)CH₂CH₂- | 2-hydroxyphenyl | -CH₃ | -H |
| I330 | -C(O)CH₂CH₂- | 3-hydroxyphenyl | -CH₃ | -H |
| I331 | -C(O)CH₂CH₂- | 4-hydroxyphenyl | -CH₃ | -H |
| I332 | -C(O)CH₂CH₂- | 2-methoxyphenyl | -CH₃ | -H |
| I333 | -C(O)CH₂CH₂- | 3-methoxyphenyl | -CH₃ | -H |
| I334 | -C(O)CH₂CH₂- | 4-methoxyphenyl | -CH₃ | -H |
| I335 | -C(O)CH₂CH₂- | 2,3 -dimethoxyphenyl | -CH₃ | -H |
| I336 | -C(O)CH₂CH₂- | 3,4-dimethoxyphenyl | -CH₃ | -H |
| I337 | -C(O)CH₂CH₂- | 3,5-dimethoxyphenyl | -CH₃ | -H |
| I338 | -C(O)CH₂CH₂- | phenyl | -CH₂CH₃ | -H |
| I339 | -C(O)CH₂CH₂- | 2-fluorophenyl | -CH₂CH₃ | -H |
| I340 | -C(O)CH₂CH₂- | 3-fluorophenyl | -CH₂CH₃ | -H |
| I341 | -C(O)CH₂CH₂- | 4-fluorophenyl | -CH₂CH₃ | -H |
| I342 | -C(O)CH₂CH₂- | 2,3-difluorophenyl | -CH₂CH₃ | -H |
| I343 | -C(O)CH₂CH₂- | 3,4-difluorophenyl | -CH₂CH₃ | -H |
| I344 | -C(O)CH₂CH₂- | 3,5-difluorophenyl | -CH₂CH₃ | -H |
| I345 | -C(O)CH₂CH₂- | 2-chlorophenyl | -CH₂CH₃ | -H |
| I346 | -C(O)CH₂CH₂- | 3-chlorophenyl | -CH₂CH₃ | -H |
| I347 | -C(O)CH₂CH₂- | 4-chlorophenyl | -CH₂CH₃ | -H |
| I348 | -C(O)CH₂CH₂- | 2,3-dichlorophenyl | -CH₂CH₃ | -H |
| I349 | -C(O)CH₂CH₂- | 3,4-dichlorophenyl | -CH₂CH₃ | -H |
| I350 | -C(O)CH₂CH₂- | 3,5-dichlorophenyl | -CH₂CH₃ | -H |
| I351 | -C(O)CH₂CH₂- | 2-hydroxyphenyl | -CH₂CH₃ | -H |
| I352 | -C(O)CH₂CH₂- | 3-hydroxyphenyl | -CH₂CH₃ | -H |
| I353 | -C(O)CH₂CH₂- | 4-hydroxyphenyl | -CH₂CH₃ | -H |
| I354 | -C(O)CH₂CH₂- | 2-methoxyphenyl | -CH₂CH₃ | -H |
| I355 | -C(O)CH₂CH₂- | 3-methoxyphenyl | -CH₂CH₃ | -H |
| I356 | -C(O)CH₂CH₂- | 4-methoxyphenyl | -CH₂CH₃ | -H |
| I357 | -C(O)CH₂CH₂- | 2,3 -dimethoxyphenyl | -CH₂CH₃ | -H |
| 1358 | -C(O)CH₂CH₂- | 3,4-dimethoxyphenyl | -CH₂CH₃ | -H |
| 1359 | -C(O)CH₂CH₂- | 3,5-dimethoxyphenyl | -CH₂CH₃ | -H |

The compounds encompassed within the first type of Category V of the present disclosure can be prepared by the procedure outlined in Scheme VII and described in Example 8 herein below.

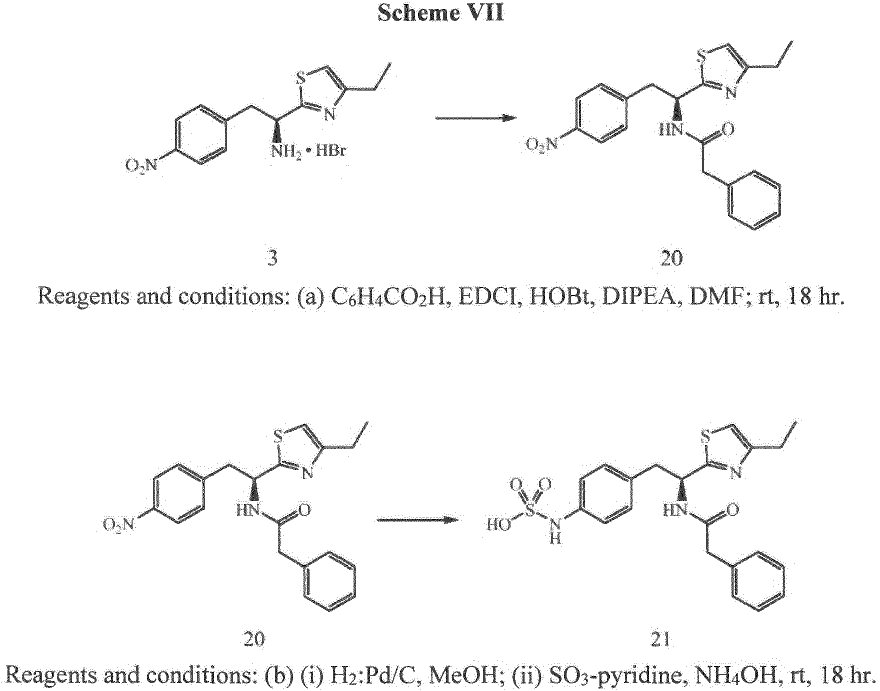

### EXAMPLE 8

### {4-[2-(S)-(4-Ethylthiazol-2-yl)-2-(2-phenylacetylamido)ethyl)phenyl}sulfamic acid (21)

Preparation of *N*-[1-(4-ethylthiazol-2-yl)-2-(4-nitrophenyl)ethyl]-2-phenyl-acetamide (20): To a solution of 1-(S)-(4-ethylthiazol-2-yl)-2-(4-nitrophenyl)ethyl amine hydrobromide, 3, (0.393 g, 1.1 mmol), phenylacetic acid (0.190 g, 1.4 mmol) and 1-hydroxybenzotriazole (HOBt) (0.094 g, 0.70 mmol) in DMF (10 mL) at 0°, is added 1-(3-dimethylaminopropyl)-3-ethylcarbodiimide (EDCI) (0.268g, 1.4 mmol) followed by triethylamine (0.60 mL, 4.2mmol). The mixture is stirred at 0 °C for 30 minutes then at room temperature overnight. The reaction mixture is diluted with water and extracted with EtOAc. The combined organic phase is washed with 1 N aqueous HCl, 5 % aqueous
NaHCO₃, water and brine, and dried over Na₂SO₄. The solvent is removed *in vacuo* to afford 0.260 g (60 % yield) of the desired product which is used without further purification. ESI+ MS 396 (M+1).

Preparation of {4-[2-(S)-(4-ethylthiazol-2-yl)-2-(2-phenylacetylamido)ethyl]-phenyl}sulfamic acid (21): N-[1-(4-ethylthiazol-2-y)-2-(4-nitrophenyl)ethyl]-2-phenyl-acetamide, 20, (0.260 g) is dissolved in MeOH (4 mL). A catalytic amount of Pd/C (10% w/w) is added and the mixture is stirred under a hydrogen atmosphere 18 hours. The reaction mixture is filtered through a bed of CELITE™ and the solvent is removed under reduced pressure. The crude product is dissolved in pyridine (12 mL) and treated with SO₃-pyridine (0.177 g, 1.23). The reaction is stirred at room temperature for 5 minutes after which a 7% solution of NH₄OH (10 mL) is added. The mixture is then concentrated and the resulting residue is purified by reverse phase chromatography to afford 0.136 g of the desired product as the ammonium salt. ¹H NMR (CD₃OD) δ 8.60 (d, 1H, J = 8.1Hz), 7.33-7.23 (m, 3H), 7.16-7.00 (m, 6H), 5.44-5.41 (m, 1H), 3.28 (1H, A of ABX, obscured by solvent), 3.03 (1H, B of ABX, J = 14.1, 9.6Hz), 2.80 (q, 2H, J = 10.5, 7.8Hz) 1.31 (t, 3H, J = 4.6Hz).

The following are non-limiting examples of the first type of Category V of the present disclosure.

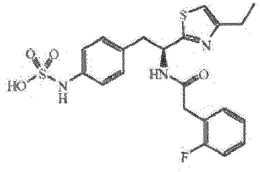

(*S*)-4-(2-(4-Ethylthiazol-2-yl)-2-(2-(2-fluorophenyl)acetamido)ethyl)phenylsuliamic acid: ¹H NMR (CD₃OD) δ 8.65(d, 1H, J = 8.4Hz), 7.29-7.15 (m, 1H), 7.13-7.03 (m, 7H), 5.46-5.42 (m, 1H), 3.64-3.51 (m, 2H), 3.29 (1H), 3.04 (1H, B of ABX, J = 13.8, 9.6Hz), 2.81 (q, 2H, J = 15.6, 3.9Hz), 1.31 (t, 3H, J = 7.8Hz). ¹⁹F NMR (CD₃OD) δ 43.64.

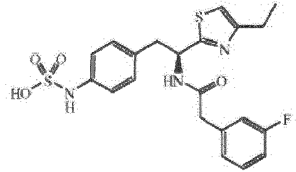

(*S*)-4-(2-(4-Ethylthiazol-2-yl)-2-(2-(3-fluorophenyl)acetamido)ethyl)phenylsulfamic acid: ¹H NMR (CD3OD) δ 8.74 (d, 1H, J = 8.4Hz), 7.32 (q, 1H, J = 6.6, 14.2Hz), 7.10-6.91 (m, 8H), 5.47-5.40 (m, 1H), 3.53 (s, 2H), 3.30 (1H), 3.11 (1H, B of ABX, J = 9.6, 14.1Hz), 2.80 (q, 2H, J = 6.6, 15.1Hz), 1.31 (t, 3H, J = 7.8Hz). 19F NMR δ 47.42.

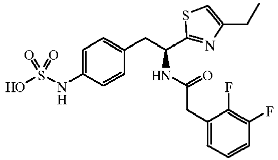

(*S*)-4-(2-(2-(2,3-Difluorophenyl)acetamido)-2-(4-ethylthiazol-2-yl)ethyl)-phenylsulfamic acid: ¹H NMR (CD₃OD) δ 7.16-7.05 (m, 5H), 6.85-6.80 (m, 1H), 5.48-5.43 (m, 1H), 3.63 (s, 2H), 3.38 (1H, A of ABX, obscured by solvent), 3.03 (1H), 2.80 (q, H, J = 15.1, 7.8Hz), 1.31 (t, 3H, J = 7.5Hz).

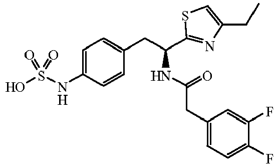

(*S*)-4-(2-(2-(3,4-Difluorophenyl)acetamido)-2-(4-ethylthiazol-2-yl)ethyl)-phenylsulfamic acid: ¹H NMR (CD₃OD) δ 8.75 (d, 1H, J = 7.8Hz), 7.23-7.04 (m, 6H), 6.88-6.84 (m, 1H), 5.44-5.40 (m, 1H), 3.49 (s, 2H), 3.34 (1H), 3.02 (1H, B of ABX, J = 14.1, 9.9Hz), 2.80 (q, 2H, J = 15.1, 7.8Hz), 1.31 (t, 1H, J = 7.5Hz). 19F NMR (CD3OD) δ 22.18, 19.45.

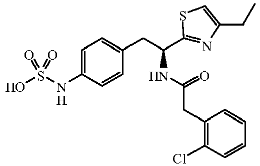

(*S*)-4-(2-(2-(2-Chlorophenyl)acetamido)-2-(4-ethylthiazol-2-yl)ethyl)phenylsulfamic acid: ¹H NMR (CD3OD) δ 7.39-7.36 (m, 1H), 7.27-7.21 (m, 2H), 7.15-6.98 (m, 5H), 5.49-5.44 (m, 1H), 3.69 (d, 2H, J = 11.7Hz), 3.32 (1H), 3.04 (1H, B of ABX, J = 9.3, 13.9Hz), 2.80 (q, 2H, J = 7.8, 15.3Hz), 1.31 (t, 3H, J = 7.5Hz).

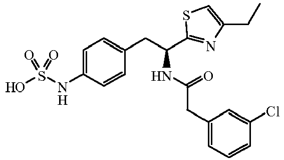

(*S*)-4-(2-(2-(3-Chlorophenyl)acetamido)-2-(4-ethylthiazol-2-yl)ethyl)phenylsulfamic acid: ¹H NMR (CD3OD) δ 7.33-7.23 (m, 3H), 7.13-7.03 (m, 5H), 5.43 (q, 1H, J = 5.1, 9.6Hz), 3.51 (s, 2H), 3.29 (1H), 3.03 (1H, B of ABX, J = 9.9, 14.1Hz), 2.80 (q, 2H, J = 7.5, 15Hz), 1.31 (t, 3H, J = 7.8Hz).

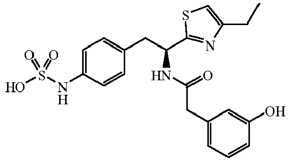

(*S*)-4-(2-(4-Ethylthiazol-2-yl)-2-(2-(3-hydroxyphenyl)acetamido)ethyl)-phenylsulfamic acid: ¹H NMR (CD₃OD) δ 7.16-7.08 (m, 3H), 7.03-7.00 (m, 3H), 6.70-6.63 (m, 2H), 5.42-5.40 (m, 1H), 3.44 (s, 2H), 3.28 (1H, A of ABX, obscured by solvent), 3.04 (B of ABX, J = 14.1, 9.6Hz), 2.89 (q, 2H, J = 15, 7.5Hz), 1.31 (t, 3H, J = 7.5Hz).

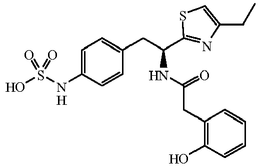

(*S*)-4-(2-(4-Ethylthiazol-2-yl)-2-(2-(2-methoxyphenyl)acetamido)ethyl)-phenylsulfamic acid: ¹H NMR (CD₃OD) δ 8.00 (d, 1H, J = 7.8Hz), 7.26 (t, 1H, J = 13.2Hz), 7.09-7.05 (m, 4H), 7.01 (s, 1H), 6.91-6.89 (m, 4H), 5.44-5.39 (m, 1H), 3.71 (s, 3H), 3.52 (s, 2H), 3.26 (1H, A of ABX, J = 14.1, 5.1Hz), 3.06 (1H B of ABX, J = 13.8, 8.4Hz), 2.80 (q, 2H, J = 8.1, 15.6Hz), 1.31 (t, 3H, J = 1.2Hz).

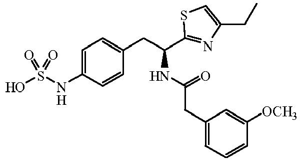

(*S*)-4-{2-(4-Ethylthiazol-2-yl)-2-[2-(3-methoxyphenyl)acetamido]ethyl}-phenylsulfamic acid: ¹H NMR (CD₃OD) δ 8.58 (d, 1H, J = 8.1Hz), 7.21 (t, 1H, J = 7.8Hz), 7.12-7.02 (m, 4H), 6.81 (s, 2H), 6.72 (d, 1H, J = 7.5Hz), 5.45-5.40 (m, 1H), 3.79 (s, 3H), 3.50 (s, 2H), 3.29 (1H, A of ABX, obscured by solvent), 3.08 (1H, B of ABX, J = 11.8, 5.1Hz), 2.80 (q, 2H, J = 15, 7.5Hz), 1.31 (t, 3H, J = 6.6Hz).

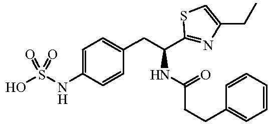

(*S*)-4-(2-(4-Ethylthiazol-2-yl)-2-(3-phenylpropanamido)ethyl)phenylsulfamic acid: ¹H NMR (CD₃OD) δ 8.56 (d, 1H, J = 8.4Hz), 7.25-6.98 (m, 9H), 5.43-5.38 (m, 1H), 3.26 (1H, A of ABX, J = 14.1, 9.6Hz), 2.97 (1H, B of ABX, J = 10.9, 3Hz), 2.58-2.76 (m, 3H), 2.98 (q, 2H, J = 13.8, 7.2Hz), 1.29 (t, 3H, J = 8.7Hz).

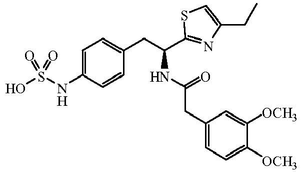

(*S*)-4-(2-(2-(3,4-Dimethoxyphenyl)acetamido)-2-(4-ethylthiazol-2-yl)ethyl)-phenylsulfamic acid: ¹H NMR (CD₃OD) δ 7.12-7.03 (m, 3H), 6.91 (d, 1H, J = 8.4Hz), 6.82 (s, 1H), 6.66 (d, 1H, J = 2.1Hz), 6.63 (d, 1H, J = 2.1Hz), 5.43 (m, 1H), 3.84 (s, 3H), 3.80 (s, 3H), 3.45 (s, 2H), 3.30 (1H), 3.03 (1H, B of ABX, J = 14.1, 9.6Hz), 2.79 (q, 2H, J = 15.1, 7.2Hz), 1.30 (t, 3H, J = 7.2Hz).

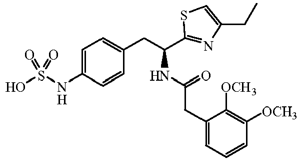

(*S*)-4-(2-(2-(2,3-Dimethoxyphenyl)acetamido)-2-(4-ethylthiazol-2-yl)ethyl)-phenylsulfamic acid: ¹H NMR (CD₃OD) δ 8.31 (d, 1H, J = 7.8Hz), 7.11-6.93 (m, 6H), 6.68 (d, 1H, J = 7.5Hz), 5.49-5.40 (m, 1H), 3.87 (s, 3H), 3.70 (s, 3H), 3.55 (s, 2H), 3.26 (1H, A of ABX, obscured by solvent), 3.06 (1H, B of ABX, J = 13.9, 9Hz), 2.80 (q, 2H, J = 14.8, 7.5Hz), 1.31 (t, 3H, J = 7.5Hz).

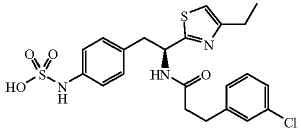

(S)-4-(2-(3-(3-Chlorophenyl)propanamido)-2-(4-ethylthiazol-2-yl)ethyl)phenyl-sulfamic acid: ¹H NMR (CD3OD) δ 7.27-7.18 (m, 3H), 7.13-7.08 (m, 5H), 7.01 (s, 1H), 5.39 (q, 1H, J = 5.1, 9.4Hz), 3.28 (1H, A of ABX, J = 5.1, 14.1Hz), 2.97 (1H, B of ABX, J = 9.3, 13.9Hz), 2.88-2.76 (m, 4H), 2.50 (t, 2H, J = 8.1Hz), 1.31 (t, 3H, J = 7.8Hz).

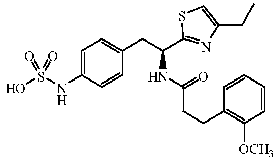

(*S*)-4-(2-(4-Ethylthiazol-2-yl)-2-(3-(2-methoxyphenyl)propanamido)ethyl)-phenylsulfamic acid: ¹H NMR (CD₃OD) δ 7.18-7.08 (m, 6H), 6.92 (d, 1H, J = 8.1Hz), 6.82 (t, 1H, J = 7.5Hz), 5.40-5.35 (m, 1H), 3.25 (1H, A of ABX, J = 15, 5.4Hz), 3.00 (1H, B of ABX, J = 10.5, 7.5Hz), 2.88-2.76 (m, 4H), 2.47 (q, 2H, J = 9.1, 6Hz), 1.31 (t, 3H, J = 7.8Hz).

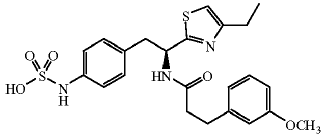

(*S*)-4-(2-(4-Ethylthiazol-2-yl)-2-(3-(3-methoxyphenyl)propanamido)ethyl)-phenylsulfamic acid: ¹H NMR (CD₃OD) δ 7.19-7.00 (m, 5H), 6.75 (s, 1H), 6.73 (s, 1H), 5.42-5.37 (m, 1H), 3.76 (s, 3H), 3.25 (1H, A of ABX, J = 13.9, 5.4Hz), 2.98 (1H, B of ABX, J = 14.1, 9.6Hz), 2.86-2.75 (m, 4H), 2.48 (q, 2H, J = 11.7, 1.2Hz), 1.31 (t, 3H, J = 7.5Hz).

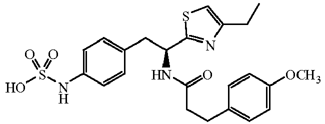

(*S*)-4-(2-(4-Ethylthiazol-2-yl)-2-(3-(4-methoxyphenyl)propanamido)ethyl)-phenylsulfamic acid: ¹H NMR (CD₃OD) δ 7.13-6.99 (m, 7H), 6.82-6.78 (m, 2H), 5.42-5.37 (m, 1H), 3.33 (s, 3H), 3.23 (1H), 2.97 (1H, B of ABX, J = 13.3, 11.4Hz), 2.83-2.75 (m, 4H), 2.49 (q, 2H, J = 6.4, 3.3Hz), 1.31 (t, 3H, J = 7.5Hz).

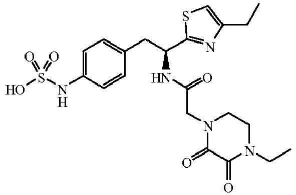

(*S*)-4-{2-[2-(4-Ethyl-2,3-dioxopiperazin-1-yl)acetamido]-2-(4-ethylthiazol-2-yl)ethyl}phenylsulfamic acid: ¹H NMR (CD₃OD) δ 7.14 (s, 4H), 7.08 (s, 1H), 5.56-5.51 (m, 1H), 4.34 (d, 2H, J = 16.2Hz), 3.88 (d, 2H, J = 17.6Hz), 3.59-3.40 (m, 3H), 3.26-3.14 (m, 3H), 2.98 (1H, B of ABX, J = 10.8, 13.9Hz), 2.82 (q, 2H, J = 6.9, 15Hz), 1.32 (t, 3H, J = 7.5Hz), 1.21 (t, 3H, J = 7.2Hz).

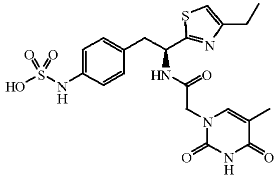

(*S*)-4-{2-(4-Ethylthiazol-2-yl)-2-[2-(5-methyl-2,4-dioxo-3,4-dihydropyrimidin-1(2*H*)-yl)acetamido]ethyl}phenylsulfamic acid: ¹H NMR (CD₃OD): δ 7.13 (s, 1H), 7.06-7.02 (m, 4H), 6.95 (s, 1H), 5.42-5.31 (m, 1H), 4.43-4.18 (dd, 2H, *J*=16.5 Hz), 3.24-2.93 (m, 2H), 2.74-2.69 (q, 2H, *J*=7.3 Hz), 1.79 (s, 3H), 1.22 (t, 3H, *J*=7.5 Hz).

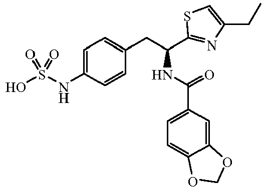

(*S*)-4-[2-(benzo[*d*][1,3]dioxole-5-carboxamido)-2-(4-ethylthiazol-2-yl)ethyl]-phenylsulfamic acid: ¹H NMR (CD₃OD) δ 7.25 (d, 1H, J=6.5 Hz), 7.13 (s, 1H), 7.06 (d, 2H, J=8.5 Hz), 7.00 (d, 2H, J=8.5 Hz), 6.91 (s, 1H), 6.76 (d, 1H, J=8.1 Hz), 5.90 (s, 2H), 5.48 (q, 1H, J=5.0 Hz), 3.32-3.24 (m, 2H), 3.07-2.99 (m, 2H), 2.72 (q, 2H, J=7.5 Hz), 1.21 (t, 3H, J=7.5 Hz).

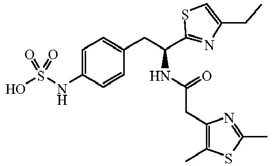

(*S*)-4-{2-[2-(2,5-Dimethylthiazol-4-yl)acetamido]-2-(4-ethylthiazol-2-yl)ethyl}-phenylsulfamic acid: ¹H NMR (CD₃OD): δ 7.10-7.01 (m, 5H), 5.41 (t, 1H, *J*=6.9 Hz), 3.58 (s, 2H), 3.33-3.01 (m, 2H), 2.82-2.75 (q, 2H, *J*=7.5 Hz), 2.59 (s, 3H), 2.23 (s, 3H), 1.30 (t, 3H, *J*=7.5 Hz).

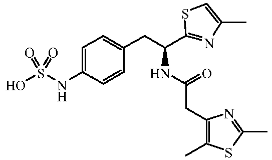

(*S*)-4-{2-[2-(2,4-Dimethylthiazol-5-yl)acetamido]-2-(4-methylthiazol-2-yl)ethyl}-phenylsulfamic acid: ¹H NMR (CD₃OD): δ 8.71-8.68 (d, 1H, J=8.4 Hz), 7.10-7.03 (m, 4H), 7.01 (s, 1H), 5.41 (m, 1H), 3.59 (s, 1H), 3.34-2.96 (m, 2H), 2.59 (s, 3H), 2.40 (s, 3H), 2.23 (s, 3H).

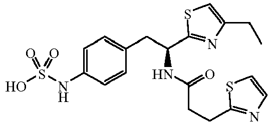

(*S*)-4-{2-(4-Ethylthiazol-2-yl)-2-[3-(thiazol-2-yl)propanamido]ethyl}phenylsulfamic acid: ¹H NMR (CD₃OD): δ 7.67-7.65 (m, 1H), 7.49-7.47 (m, 1H), 7.14-7.08 (m, 4H), 7.04
s, 1H), 5.46-5.41 (q, 1H, J=5.1 Hz), 3.58 (s, 2H), 3.30-3.25 (m, 3H), 3.02-2.67 (m, 5H), 1.31 (t, 3H, J=7.5 Hz).

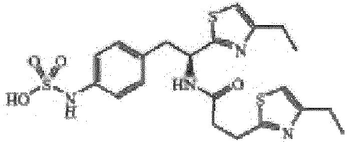

(S)-4-{2-(4-Ethylthiazol-2-yl)-2-[2-(4-ethylthiazol-2-yl)acetamido]ethyl}-phenylsulfamic acid: ¹H NMR (CD₃OD): δ 7.04-6.91 (m, 6H), 5.32 (t, 1H, J=5.4 Hz), 3.25-2.90 (m, 2H), 2.71-2.61 (m, 4H) 1.93 (s, 2H) 1.22-1.14 (m, 6H).

The second type of Category V of the present disclosure relates to 2-(thiazol-4-yl) compounds having the formula: 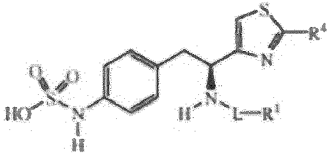 wherein R¹, R⁴, and L are further defined herein in Table X herein below.

**TABLE X**

| **No.** | **L** | **R¹** | **R⁴** |
|---|---|---|---|
| J360 | -C(O)CH₂- | phenyl | methyl |
| J361 | -C(O)CH₂- | phenyl | ethyl |
| J362 | -C(O)CH₂- | phenyl | phenyl |
| J363 | -C(O)CH₂- | phenyl | thiophen-2-yl |
| J364 | -C(O)CH₂- | phenyl | thiazol-2-yl |
| J365 | -C(O)CH₂- | phenyl | oxazol-2-yl |
| J366 | -C(O)CH₂- | phenyl | isoxazol-3-yl |
| J367 | -C(O)CH₂- | 3-chlorophenyl | methyl |
| J368 | -C(O)CH₂- | 3-chlorophenyl | ethyl |
| J369 | -C(O)CH₂- | 3-chlorophenyl | phenyl |
| J370 | -C(O)CH₂- | 3-chlorophenyl | thiophen-2-yl |
| J371 | --C(O)CH₂- | 3-chlorophenyl | thiazol-2-yl |
| J372 | -C(O)CH₂- | 3 -chlorophenyl | oxazol-2-yl |
| J373 | -C(O)CH₂- | 3-chlorophenyl | isoxazol-3-yl |
| J374 | -C(O)CH₂- | 3-methoxyphenyl | methyl |
| J375 | -C(O)CH₂- | 3-methoxyphenyl | ethyl |
| J376 | -C(O)CH₂- | 3 -methoxyphenyl | phenyl |
| J377 | -C(O)CH₂- | 3 -methoxyphenyl | thiophen-2-yl |
| J378 | -C(O)CH₂- | 3 -methoxyphenyl | thiazol-2-yl |
| J379 | -C(O)CH₂- | 3 -methoxyphenyl | oxazol-2-yl |
| J380 | -C(O)CH₂- | 3 -methoxyphenyl | isoxazol-3-yl |
| J381 | -C(O)CH₂- | 3-fluorophenyl | methyl |
| J382 | -C(O)CH₂- | 3-fluorophenyl | ethyl |
| J383 | -C(O)CH₂- | 3-fluorophenyl | phenyl |
| J384 | -C(O)CH₂- | 3-fluorophenyl | thiophen-2-yl |
| J385 | -C(O)CH₂- | 3-fluorophenyl | thiazol-2-yl |
| J386 | -C(O)CH₂- | 3-fluorophenyl | oxazol-2-yl |
| J387 | -C(O)CH₂- | 3-fluorophenyl | isoxazol-3-yl |
| J388 | -C(O)CH₂- | 2,5-dimethylthiazol-4-yl | methyl |
| J389 | -C(O)CH₂- | 2,5-dimethylthiazol-4-yl | ethyl |
| J390 | -C(O)CH₂- | 2,5-dimethylthiazol-4-yl | phenyl |
| J391 | -C(O)CH₂- | 2,5-dimethylthiazol-4-yl | thiophen-2-yl |
| J392 | -C(O)CH₂- | 2,5-dimethylthiazol-4-yl | thiazol-2-yl |
| J393 | -C(O)CH₂- | 2,5-dimethylthiazol-4-yl | oxazol-2-yl |
| J394 | -C(O)CH₂- | 2,5-dimethylthiazol-4-yl | isoxazol-3-yl |
| J395 | -C(O)CH₂- | 2,4-dimethylthiazol-5-yl | methyl |
| J396 | -C(O)CH₂- | 2,4-dimethylthiazol-5-yl | ethyl |
| J397 | -C(O)CH₂- | 2,4-dimethylthiazol-5-yl | phenyl |
| J398 | -C(O)CH₂- | 2,4-dimethylthiazol-5-yl | thiophen-2-yl |
| J399 | -C(O)CH₂- | 2,4-dimethylthiazol-5-yl | thiazol-2-yl |
| J400 | -C(O)CH₂- | 2,4-dimethylthiazol-5-yl | oxazol-2-yl |
| J401 | -C(O)CH₂- | 2,4-dimethylthiazol-5-yl | isoxazol-3-yl |
| J402 | -C(O)CH₂- | 4-ethylthiazol-2-yl | methyl |
| J403 | -C(O)CH₂- | 4-ethylthiazol-2-yl | ethyl |
| J404 | -C(O)CH₂- | 4-ethylthiazol-2-yl | phenyl |
| J405 | -C(O)CH₂- | 4-ethylthiazol-2-yl | thiophen-2-yl |
| J406 | -C(O)CH₂- | 4-ethylthiazol-2-yl | thiazol-2-yl |
| J407 | -C(O)CH₂- | 4-ethylthiazol-2-yl | oxazol-2-yl |
| J408 | -C(O)CH₂- | 4-ethylthiazol-2-yl | isoxazol-3-yl |
| J409 | -C(O)CH₂- | 3-methyl-1,2,4-oxadiazol-5-yl | methyl |
| J410 | -C(O)CH₂- | 3-methyl-1,2,4-oxadiazol-5-yl | ethyl |
| J411 | -C(O)CH₂- | 3-methyl-1,2,4-oxadiazol-5-yl | phenyl |
| J412 | -C(O)CH₂- | 3-methyl-1,2,4-oxadiazol-5-yl | thiophen-2-yl |
| J413 | -C(O)CH₂- | 3-methyl-1,2,4-oxadiazol-5-yl | thiazol-2-yl |
| J414 | -C(O)CH₂- | 3-methyl-1,2,4-oxadiazol-5-yl | oxazol-2-yl |
| J415 | -C(O)CH₂- | 3-methyl-1,2,4-oxadiazol-5-yl | isoxazol-3-yl |
| J416 | -C(O)CH₂CH₂- | phenyl | methyl |
| J417 | -C(O)CH₂CH₂- | phenyl | ethyl |
| J418 | -C(O)CH₂CH₂- | phenyl | phenyl |
| J419 | -C(O)CH₂CH₂- | phenyl | thiophen-2-yl |
| J420 | -C(O)CH₂CH₂- | phenyl | thiazol-2-yl |
| J421 | -C(O)CH₂CH₂- | phenyl | oxazol-2-yl |
| J422 | -C(O)CH₂CH₂- | phenyl | isoxazol-3-yl |
| J423 | -C(O)CH₂CH₂- | 3 -chlorophenyl | methyl |
| J424 | -C(O)CH₂CH₂- | 3-chlorophenyl | ethyl |
| J425 | -C(O)CH₂CH₂- | 3 -chlorophenyl | phenyl |
| J426 | -C(O)CH₂CH₂- | 3 -chlorophenyl | thiophen-2-yl |
| J427 | -C(O)CH₂CH₂- | 3 -chlorophenyl | thiazol-2-yl |
| J428 | -C(O)CH₂CH₂- | 3 -chlorophenyl | oxazol-2-yl |
| J429 | -C(O)CH₂CH₂- | 3-chlorophenyl | isoxazol-3-yl |
| J430 | -C(O)CH₂CH₂- | 3 -methoxyphenyl | methyl |
| J431 | -C(O)CH₂CH₂- | 3 -methoxyphenyl | ethyl |
| J432 | -C(O)CH₂CH₂- | 3 -methoxyphenyl | phenyl |
| J433 | -C(O)CH₂CH₂- | 3 -methoxyphenyl | thiophen-2-yl |
| J434 | -C(O)CH₂CH₂- | 3 -methoxyphenyl | thiazol-2-yl |
| J435 | -C(O)CH₂CH₂- | 3 -methoxyphenyl | oxazol-2-yl |
| J436 | -C(O)CH₂CH₂- | 3 -methoxyphenyl | isoxazol-3-yl |
| J437 | -C(O)CH₂CH₂- | 3-fluorophenyl | methyl |
| J438 | -C(O)CH₂CH₂- | 3-fluorophenyl | ethyl |
| J439 | -C(O)CH₂CH₂- | 3-fluorophenyl | phenyl |
| J440 | -C(O)CH₂CH₂- | 3-fluorophenyl | thiophen-2-yl |
| J441 | -C(O)CH₂CH₂- | 3-fluorophenyl | thiazol-2-yl |
| J442 | -C(O)CH₂CH₂- | 3-fluorophenyl | oxazol-2-yl |
| J443 | -C(O)CH₂CH₂- | 3-fluorophenyl | isoxazol-3-yl |
| J444 | -C(O)CH₂CH₂- | 2,5-dimethylthiazol-4-yl | methyl |
| J445 | -C(O)CH₂CH₂- | 2,5-dimethylthiazol-4-yl | ethyl |
| J446 | -C(O)CH₂CH₂- | 2,5-dimethylthiazol-4-yl | phenyl |
| J447 | -C(O)CH₂CH₂- | 2,5-dimethylthiazol-4-yl | thiophen-2-yl |
| J448 | -C(O)CH₂CH₂- | 2,5-dimethylthiazol-4-yl | thiazol-2-yl |
| J449 | -C(O)CH₂CH₂- | 2,5-dimethylthiazol-4-yl | oxazol-2-yl |
| J450 | -C(O)CH₂CH₂- | 2,5-dimethylthiazol-4-yl | isoxazol-3-yl |
| J451 | -C(O)CH₂CH₂- | 2,4-dimethylthiazol-5-yl | methyl |
| J452 | -C(O)CH₂CH₂- | 2,4-dimethylthiazol-5-yl | ethyl |
| J453 | -C(O)CH₂CH₂- | 2,4-dimethylthiazol-5-yl | phenyl |
| J454 | -C(O)CH₂CH₂- | 2,4-dimethylthiazol-5-yl | thiophen-2-yl |
| J455 | -C(O)CH₂CH₂- | 2,4-dimethylthiazol-5-yl | thiazol-2-yl |
| J456 | -C(O)CH₂CH₂- | 2,4-dimethylthiazol-5-yl | oxazol-2-yl |
| J457 | -C(O)CH₂CH₂- | 2,4-dimethylthiazol-5-yl | isoxazol-3-yl |
| J458 | -C(O)CH₂CH₂- | 4-ethylthiazol-2-yl | methyl |
| J459 | -C(O)CH₂CH₂- | 4-ethylthiazol-2-yl | ethyl |
| J460 | -C(O)CH₂CH₂- | 4-ethylthiazol-2-yl | phenyl |
| J461 | -C(O)CH₂CH₂- | 4-ethylthiazol-2-yl | thiophen-2-yl |
| J462 | -C(O)CH₂CH₂- | 4-ethylthiazol-2-yl | thiazol-2-yl |
| J463 | -C(O)CH₂CH₂- | 4-ethylthiazol-2-yl | oxazol-2-yl |
| J464 | -C(O)CH₂CH₂- | 4-ethylthiazol-2-yl | isoxazol-3-yl |
| J465 | -C(O)CH₂CH₂- | 3-methyl-1,2,4-oxadiazol-5-yl | methyl |
| J466 | -C(O)CH₂CH₂- | 3-methyl-1,2,4-oxadiazol-5-yl | ethyl |
| J467 | -C(O)CH₂CH₂- | 3-methyl-1,2,4-oxadiazol-5-yl | phenyl |
| J468 | -C(O)CH₂CH₂- | 3-methyl-1,2,4-oxadiazol-5-yl | thiophen-2-yl |
| J469 | -C(O)CH₂CH₂- | 3-methyl-1,2,4-oxadiazol-5-yl | thiazol-2-yl |
| J470 | -C(O)CH₂CH₂- | 3-methyl-1,2,4-oxadiazol-5-yl | oxazol-2-yl |
| J471 | -C(O)CH₂CH₂- | 3-methyl-1,2,4-oxadiazol-5-yl | isoxazol-3-yl |

The compounds encompassed within the second type of Category I of the present disclosure can be prepared by the procedure outlined in Scheme II and described in Example 9 herein below.

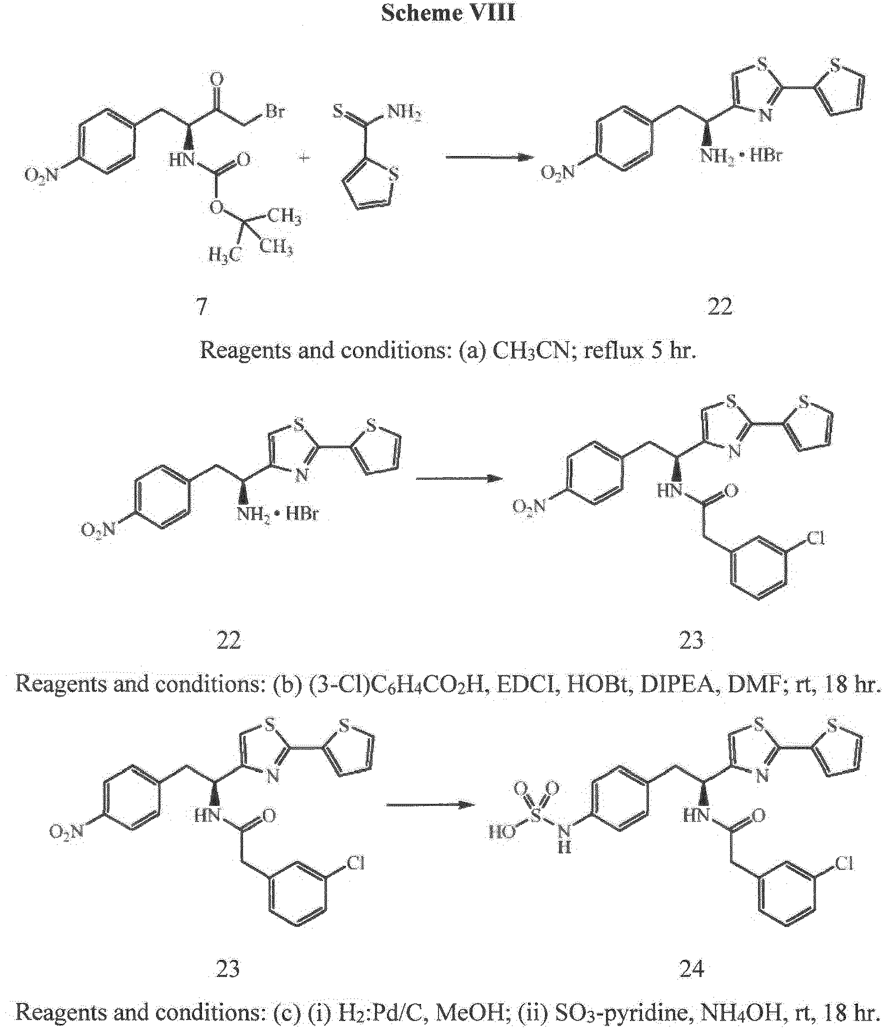

### EXAMPLE 9

### 4-((S)-2-(2-(3-chlorophenyl)acetamido)-2-(2-(thiophen-2-yl)thiazol-4-yl)ethyl)phenylsulfamic acid (24)

Preparation of (*S*)-2-(4-nitrophenyl)-1-[(thiophen-2-yl)thiazol-4-yl]ethanamine hydrobromide salt (22): A mixture of (*S*)-*tert*-butyl 4-bromo-1-(4-nitrophenyl)-3-oxobutan-2-ylcarbamate, 7, (7.74g, 20mmol), and thiophen-2-carbothioic acid amide (3.14g, 22mmol) in CH₃CN (200 mL) is refluxed for 5 hours. The reaction mixture is cooled to room temperature and diethyl ether (50 mL) is added to the solution. The precipitate which forms is collected by filtration. The solid is dried under vacuum to afford 7.14 g (87 % yield) of the desired product. ESI+ MS 332 (M+1).

Preparation of 2-(3-chlorophenyl)-*N*-{(*S*)-2-(4-nitrophenyl)-1-[2-(thiophen-2-yl)thiazol-4-yl]ethyl}acetamide (23): To a solution of 2-(4-nitrophenyl)-1-(2-thiophene2-ylthiazol-4-yl)ethylamine, 22, (0.41 g, 1mmol) 3-chlorophenylacetic acid (0.170g, 1mmol) and 1-hydroxybenzotriazole (HOBt) (0.070g, 0.50mmol) in DMF (5 mL) at 0 °C, is added 1-(3-dimethylaminopropyl)-3-ethylcarbodiimide (EDCI) (0.190g, 1mmol) followed by triethylamine (0.42mL, 3mmol). The mixture is stirred at 0 °C for 30 minutes then at room temperature overnight. The reaction mixture is diluted with water and extracted with EtOAc. The combined organic phase is washed with 1 N aqueous HCl, 5 % aqueous NaHCO₃, water and brine, and dried over Na₂SO₄. The solvent is removed *in vacuo* to afford 0.290 g (60 % yield) of the desired product which is used without further purification. ESI- MS 482 (M-1).

Preparation of {4-[2-(3-chlorophenyl)acetylamino]-2-(2-thiophen-2-ylthiazol-4-yl)ethyl]phenyl}sulfamic acid (24): 2-(3-chlorophenyl)-N- {(S)-2-(4-nitrophenyl)-1-[2-(thiophene2-yl)thiazol-4-yl]ethyl}acetamide, 23, (0.290 g) is dissolved in MeOH (4 mL). A catalytic amount of Pd/C (10% w/w) is added and the mixture is stirred under a hydrogen atmosphere 18 hours. The reaction mixture is filtered through a bed of CELITE™ and the solvent is removed under reduced pressure. The crude product is dissolved in pyridine (12 mL) and treated with SO₃-pyridine (0.157 g). The reaction is stirred at room temperature for 5 minutes after which a 7% solution of NHₐOH is added. The mixture is then concentrated and the resulting residue is purified by reverse phase chromatography to afford 0.078 g of the desired product as the ammonium salt. ¹H NMR (CD3OD) δ 7.61 (d, 1H, J = 3.6Hz), 7.58 (d, 1H, J = 5.1Hz), 7.41-7.35 (m, 1H), 7.28-7.22 (m, 2H), 7.18-6.98 (m, 6H), 5.33 (t, 1H, J = 6.6Hz), 3.70 (d, 2H, J = 3.9Hz), 3.23 (1H, A of ABX, J = 6.6, 13.8Hz), 3.07 (1H, B of ABX, J = 8.1, 13.5Hz).

The following are non-limiting examples of compounds encompassed within the second type of Category V of the present disclosure.

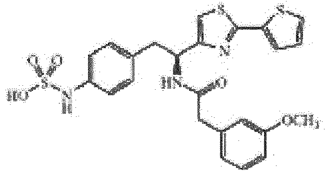

4-((*S*)-2-(2-(3-Methoxyphenyl)acetamido)-2-(2-(thiophene2-yl)thiazol-4-yl)ethyl)-phenylsulfamic acid: ¹H NMR (CD3OD) δ 8.35 (d, 1H, J = 8.7Hz), 7.61-7.57 (m, 2H), 7.25-7.20 (m, 2H), 7.25-7.20 (m, 2H), 7.09 (s, 1H), 7.05 (d, 2H, J = 4.2Hz), 6.99 (d, 1H, J = 8.7Hz), 6.81 (d, 1H, J = 7.8Hz), 6.77 (s, 1H), 5.30-5.28 (m, 1H), 3.76 (s, 3H), 3.51 (s, 2H), 3.20 (1H, A of ABX, J = 6.3, 13.6Hz), 3.06 (1H, B of ABX, J = 8.1, 13.8Hz).

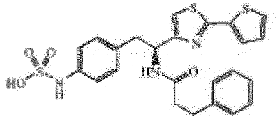

4-{(*S*)-2-(3-Phenylpropanamido)-2-[2-(thiophene2-yl)thiazol-4-yl]ethyl}-phenylsulfamic acid: ¹H NMR (CD3OD) δ 8.30 (d, 1H, J = 9Hz), 7.61-7.56 (m, 2H), 7.26-7.14 (m, 7H), 7.12 (d, 1H, J = 1.5Hz), 7.09 (d, 1H, J = 2.1Hz), 6.89 (s, 1H), 5.28-5.26 (m, 1H), 3.18 (1H, A of ABX, J = 6.2, 13.8Hz), 2.96 (1H, B of ABX, J = 8.4, 13.6Hz).

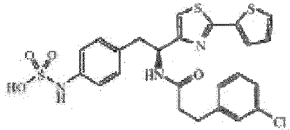

4-{(*S*)-2-(3-(3-Chlorophenyl)propanamido)-2-[2-(thiophene2-yl)thiazol-4-yl]ethyl}phenylsulfamic acid: ¹H NMR (CD₃OD) δ 7.61-7.56 (m, 3H), 7.22-7.14 (m, 6H), 7.08 (d, 1H), 7.00 (d, 1H, J = 77.5Hz), 6.870 (s, 1H), 5.25 (t, 1H, J = 7.8Hz), 3.18 (1H, A of ABX, J = 6.6, 13.8Hz), 2.97 (1H, B of ABX, J = 7.8, 13.8Hz), 2.87 (t, 2H, J = 7.5Hz), 2.51 (t, 2H, J = 7.2Hz).

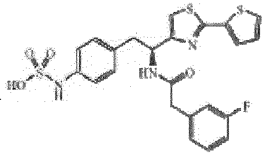

4-{(*S*)-2-[2-(3-Fluorophenyl)acetamido]-2-[2-(thiophen-2-yl)thiazol-4-yl]ethyl}phenylsulfamic acid: ¹H NMR (CD₃OD) δ 7.61-7.57 (m, 2H), 7.32-7.28 (m, 1H), 7.19-7.16 (m, 2H), 7.08 (t, 1H, J = 4.5Hz), 7.02-6.95 (m, 6H), 5.29 (t, 1H, J = 8.1Hz), 3.53 (s, 2H), 3.22 (1H, A of ABX, J = 6.6, 13.9Hz), 3.06 (1H, B of ABX, J = 8.4, 13.6Hz).

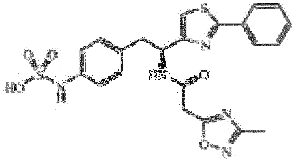

(*S*)-4-{2-[2-(3-Methyl-1,2,4-oxadiazol-5-yl)acetamido]-2-(2-phenylthiazol-4-yl)ethyl}phenylsulfamic acid: ¹H NMR (CD₃OD): δ 7.98-7.95 (m, 2H), 7.48-7.46 (m, 3H), 7.23 (s, 1H), 7.09-7.05 (m, 4H 5.33 (t, 1H, J=7.2 Hz), 3.33-3.06 (m, 2H), 2.35 (s, 3H).

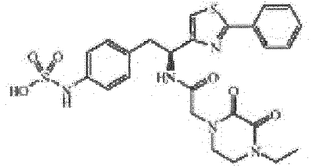

4-{(*S*)-2-[2-(4-ethy)-2,3-dioxopiperazin-1-yl)acetamido]-2-[2-(thiophen-2-yl)thiazo)-4-yl]ethyl}phenylsulfamic acid: ¹H NMR (CD₃OD) δ 7.62 (d, 1H, J = 3Hz), 7.58 (d, 1H, J = 15.6Hz), 7.27 (s, 1H), 7.16 (t, 1H, J = 1.5Hz), 5.42-5.32 (m, 1H), 4.31 (d, 1H, J = 15.6Hz), 3.91 (d, 1H, J = 15.9Hz), 3.60-3.50 (m, 4H), 3.30-3.23 (m, 2H), 2.98 (1H, B of ABX, J = 9.9, 13.8Hz), 1.21 (t, 3H, J = 6.9Hz).

The third type of Category V of the present disclosure relates to compounds having the formula: 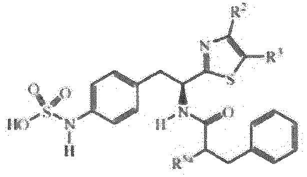 wherein the linking unit L comprises a phenyl unit, said linking group having the formula:

-C(O)[(CR^{5a}H)][(CR^{6a}H)]-

R¹ is hydrogen, R^{6a} is phenyl, R^{5a} is phenyl or substituted phenyl and non-limiting examples of the units R², R³, and R^{5a} are further exemplified herein below in Table XI.

**TABLE XI**

| **No.** | **R²** | **R**³ | **R^{5a}** |
|---|---|---|---|
| K472 | methyl | hydrogen | phenyl |
| K473 | methyl | hydrogen | 2-fluorophenyl |
| K474 | methyl | hydrogen | 3-fluorophenyl |
| K475 | methyl | hydrogen | 4-fluorophenyl |
| K476 | methyl | hydrogen | 3,4-difluorophenyl |
| K477 | methyl | hydrogen | 2-chlorophenyl |
| K478 | methyl | hydrogen | 3-chlorophenyl |
| K479 | methyl | hydrogen | 4-chlorophenyl |
| K480 | methyl | hydrogen | 3,4-dichlorophenyl |
| K481 | methyl | hydrogen | 2-methoxyphenyl |
| K432 | methyl | hydrogen | 3-methoxyphenyl |
| K483 | methyl | hydrogen | 4-methoxyphenyl |
| K484 | ethyl | hydrogen | phenyl |
| K485 | ethyl | hydrogen | 2-fluorophenyl |
| K486 | ethyl | hydrogen | 3-fluorophenyl |
| K487 | ethyl | hydrogen | 4-fluorophenyl |
| K4S8 | ethyl | hydrogen | 3,4-difluorophenyl |
| K489 | ethyl | hydrogen | 2-chlorophenyl |
| K490 | ethyl | hydrogen | 3-chlorophenyl |
| K491 | ethyl | hydrogen | 4-chlorophenyl |
| K492 | ethyl | hydrogen | 3,4-dichlorophenyl |
| K493 | ethyl | hydrogen | 2-methoxyphenyl |
| K494 | ethyl | hydrogen | 3-methoxyphenyl |
| K495 | ethyl | hydrogen | 4-methoxyphenyl |

The compounds encompassed within the third type of Category V of the present disclosure can be prepared by the procedure outlined in Scheme IX and described in Example 10 herein below.

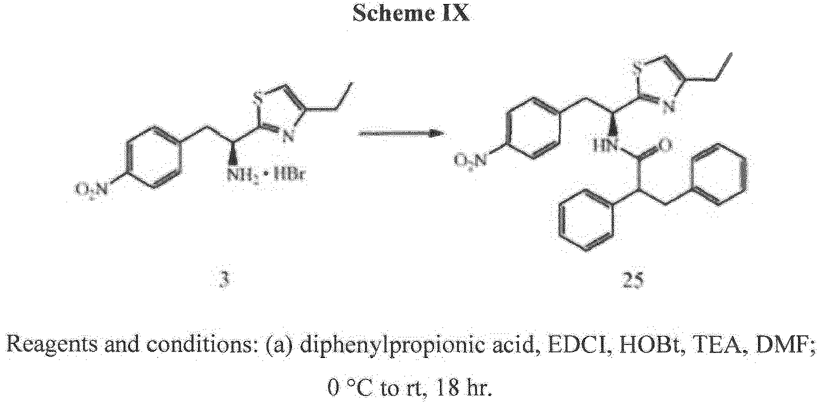

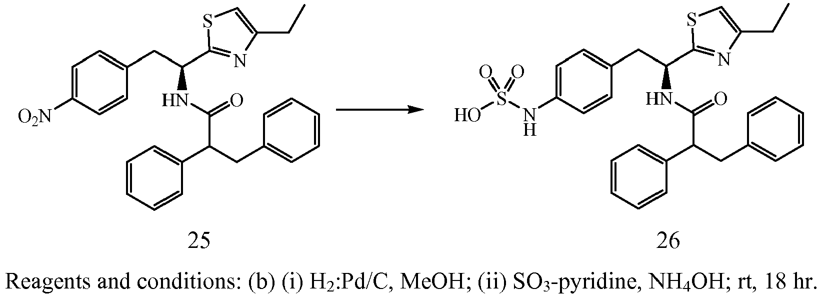

### EXAMPLE 10

### (S)-4-(2-(2,3-Diphenylpropanamido)-2-(4-ethylthiazol-2-yl)ethyl)-phenylsulfamic acid (26)

Preparation of (*S*)-*N*-[1-(4-ethylthiazol-2-yl)-2-(4-nitrophenyl)ethyl]-2,3-diphenyl-propanamide (25): To a solution of 1-(*S*)-(4-ethylthiazol-2-yl)-2-(4-nitrophenyl)ethyl amine hydrobromide, 3, (0.95 g, 2.65 mmol), diphenylpropionic acid (0.60 g, 2.65 mmol) and 1-hydroxybenzotriazole (HOBt) (0.180 g, 1.33 mmol) in DMF (10 mL) at 0°, is added 1-(3-dimethylaminopropyl)-3-ethylcarbodiimide (EDCI) (0.502 g, 2.62 mmol) followed by triethylamine (1.1 mL, 7.95 mmol). The mixture is stirred at 0 °C for 30 minutes then at room temperature overnight. The reaction mixture is diluted with water and extracted with EtOAc. The combined organic phase is washed with 1 N aqueous HCl, 5 % aqueous NaHCO₃, water and brine, and dried over Na₂SO₄. The solvent is removed *in vacuo* to afford 0.903 g (70% yield) of the desired product which is used without further purification.

Preparation of (*S*)-4-(2-(2,3-diphenylpropanamido)-2-(4-ethylthiazol-2-yl)ethyl)phenylsulfamic acid (26) (*S*)-*N*-[1-(4-ethylthiazol-2-yl)-2-(4-nitrophenyl)ethyl]-2,3-diphenyl-propanamide, 25, (0.903 g) is dissolved in MeOH (10 mL). A catalytic amount of Pd/C (10% w/w) is added and the mixture is stirred under a hydrogen atmosphere 18 hours. The reaction mixture is filtered through a bed of CELITE™ and the solvent is removed under reduced pressure. The crude product is dissolved in pyridine (30 mL) and treated with SO₃-pyridine (0.621 g). The reaction is stirred at room temperature for 5 minutes after which a 7% solution of NH₄OH is added. The mixture is then concentrated and the resulting residue is purified by reverse phase chromatography to afford 0.415 g of the desired product as the ammonium salt. ¹H NMR (CD₃OD) δ 8.59-8.52 (m, 1H), 7.37-7.04 (m, 9H), 6.97-6.93 (m, 1H), 6.89-6.85 (m, 2H), 5.36-5.32 (m, 1H), 3.91-3.83 (m, 1H), 3.29 (1H, A of ABX, obscured by solvent), 3.15 (1H, B of ABX, J = 5.4, 33.8Hz), 2.99-2.88 (m, 2H), 2.81-2.69 (m, 2H), 1.32-1.25 (m, 3H).

The precursors of many of the Z units which comprise the third type of Category V are not readily available. The following procedure illustrates an example of the procedure which can be used to provide different R^{5a} units according to the present disclosure. Using the procedure outlined in Scheme X and described in Example 11 the artisan can make modifications without undue experimentation to achieve the R^{5a} units encompassed by the present disclosure.

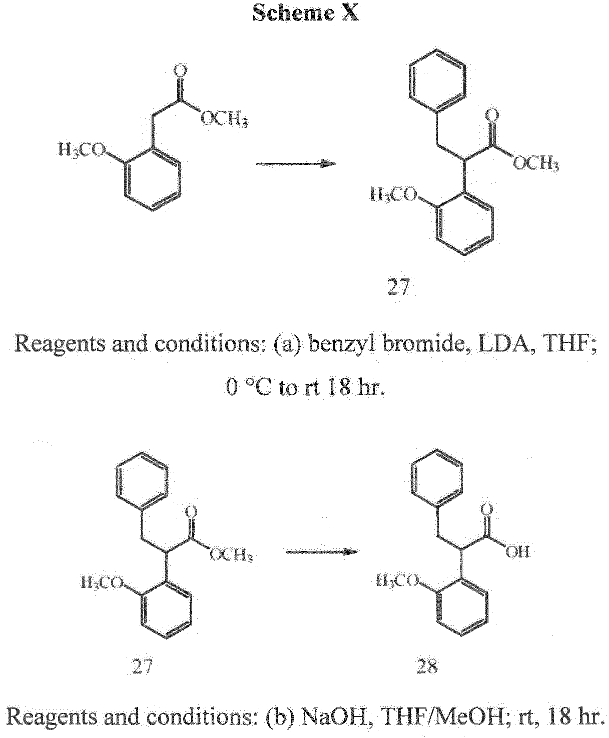

### EXAMPLE 11

### 2-(2-Methoxyphenyl)-3-phenylpropanoic acid (28)

Preparation of methyl 2-(2-methoxyphenyl)-3-phenylpropanoate (27): A 500mL roundbottom flask is charged with methyl 2-(2-methoxyphenyl)acetate (8.496 g, 47 mmol, leq) and THF (200mL). The homogeneous mixture is cooled to 0 °C in an ice bath. Lithium diisopropyl amide (23.5mL of a 2.0M solution in heptane/THF) is added, maintaining a temperature less than 3°C. The reaction is stirred 45 minutes at this reduced temperature. Benzyl bromide (5.6mL, 47mmol, leq) is added dropwise. The reaction is allowed to gradually warm to room temperature and is stirred for 18 hours. The reaction is quenched with 1N HCl and extracted 3 times with equal portions of EtOAc. The combined
extracts are washed with H₂O and brine, dried over Na₂SO₄, filtered, and concentrated. The residue is purified over silica to afford 4.433g (35%) of the desired compound. ESI+ MS 293 (M+Na).

Preparation of 2-(2-methoxyphenyl)-3-phenylpropanoic acid (28): Methyl 2-(2-methoxyphenyl)-3-phenylpropanoate (4.433g, 16mmol, 1eq) is dissolved in 100mL of a 1:1 (v:v) mixture of THF and methanol. Sodium hydroxide (3.28g, 82mmol, 5eq) is added and the reaction mixture is stirred 18 hours at room temperature. The reaction is then poured into H₂O and the pH is adjusted to 2 via addition of IN HCl. A white precipitate forms which is removed by filtration. The resulting solution is extracted with 3 portion of diethyl ether. The extracts are pooled, washed with H₂O and brine, dried over Na₂SO₄, filtered, and concentrated *in vacuo.* The resulting residue is purified over silica to afford 2.107g (51%) of the desired compound. ESI- MS 255 (M-1), 21 1 (M-CO₂H).

Intermediate 28 can be carried forward according to the procedure outlined in Scheme IX and described in Example 10 to produce the following compound according to the third type of Category V.

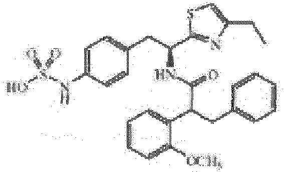

(*S*)-4-{2-(4-Ethylthiazol-2-yl)-2-[2-(2-methoxyphenyl)-3-phenylpropanamido]-ethyl}phenylsulfamic acid: ¹H NMR (CD₃OD) δ 7.32-7.12 (m, 7H), 7.05-7.02 (m, 1H), 6.99-6.83 (m, 4H), 6.80-6.75 (m, 2H), 5.35-5.31 (m, 1H), 4.31-4.26 (m, 1H), 3.75 (s, 3H), 3.20-2.90 (m, 4H), 2.79-2.74 (m, 2H), 1.32-1.25 (m, 3H).

The following are further non-limiting examples of compounds according to the third type of Category I of the present disclosure.

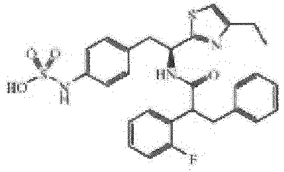

(*S*)-4-{2-(4-Ethylthiazol-2-yl)-2-[2-(3-fluorophenyl)-3-phenylpropanamido]-ethyl}phenylsulfamic acid: ¹H NMR (CD₃OD) δ 7.33-6.87 (m, 14H), 5.39-5.25 (m, 1H), 3.95-3.83 (m, 1H), 3.31-3.10 (m, 1H), 3.05-2.88 (m, 2H), 2.80-2.70 (m, 2H), 1.32-1.23 (m, 3H). ¹⁹F NMR δ 47.59.

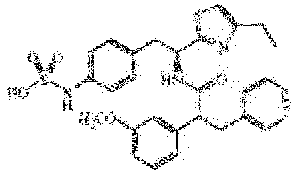

(*S*)-4-{2-(4-Ethylthiazol-2-yl)-2-[2-(3-methoxyphenyl)-3-phenylpropanamido]-ethyl}phenylsulfamic acid: ¹H NMR (CD₃OD) δ 7.85 (d, 1H, J = 8.4Hz), 7.25-7.20 (m, 1H), 7.11-7.02 (m, 4H), 7.01 (s, 1H), 6.90-6.79 (m, 2H), 5.45-5.40 (m, 1H), 4.09 (s, 2H), 3.79 (s, 3H), 3.12-3.08 (m, 2H), 1.10 (s, 9H).

The fourth type of Category V of the present disclosure relates to compounds having the formula: 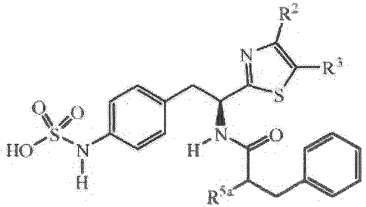 wherein the linking unit L comprises a phenyl unit, said linking group having the formula:

-C(O)[(CR^{5a}H)][(CR^{6a}H]-

R¹ is hydrogen, R^{6a} is phenyl, R^{5a} is substituted or unsubstituted heteroaryl and the units R², R³, and R^{5a} are further exemplified herein below in Table XII.

**TABLE XII**

| **No.** | **R²** | **R³** | **R^{5a}** |
|---|---|---|---|
| L496 | methyl | hydrogen | 3-methyl-1,2,4-oxadiaxol-5-yl |
| L497 | methyl | hydrogen | thiophen-2-yl |
| L498 | methyl | hydrogen | thiazol-2-yl |
| L499 | methyl | hydrogen | oxazol-2-yl |
| L500 | methyl | hydrogen | isoxazol-3-yl |
| L501 | ethyl | hydrogen | 3-methyl-1,2,4-oxadiazol-5-yl |
| L502 | ethyl | hydrogen | thiophen-2-yl |
| L503 | ethyl | hydrogen | thiazol-2-yl |
| L504 | ethyl | hydrogen | oxazol-2-yl |
| L505 | ethyl | hydrogen | isoxazol-3-yl |
| L506 | ethyl | methyl | 3-methyl-1,2,4-oxadiazol-5-yl |
| L507 | ethyl | methyl | thiophen-2-yl |
| L508 | ethyl | methyl | thiazol-2-yl |
| L509 | ethyl | methyl | oxazol-2-yl |
| L510 | ethyl | methyl | isoxazol-3-yl |
| L511 | thiophen-2-yl | hydrogen | 3-methyl-1,2,4-oxadiazol-5-yl |
| L512 | thiophen-2-yl | hydrogen | thiophen-2-yl |
| L513 | thiophen-2-yl | hydrogen | thiazol-2-yl |
| L514 | thiophen-2-yl | hydrogen | oxazol-2-yl |
| L515 | thiophen-2-yl | hydrogen | isoxazol-3-yl |
| L516 | isoxazol-3-yl | hydrogen | 3-methyl-1,2,4-oxadiazol-5-yl |
| L517 | isoxazol-3-yl | hydrogen | thiophen-2-yl |
| L518 | isoxazol-3-yl | hydrogen | thiazol-2-yl |
| L519 | isoxaxol-3-yl | hydrogen | oxazol-2-yl |
| L520 | isoxaxol-3-yl | hydrogen | isoxazol-3-yl |

The compounds encompassed within the fourth type of Category V of the present disclosure can be prepared by the procedure outlined in Scheme V and described in Example 5 herein below.

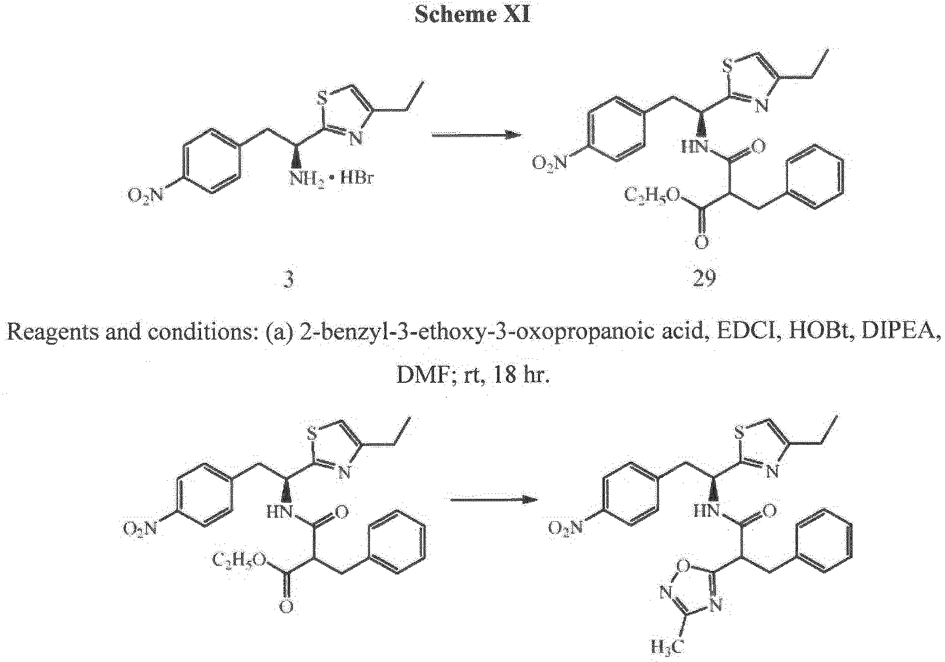

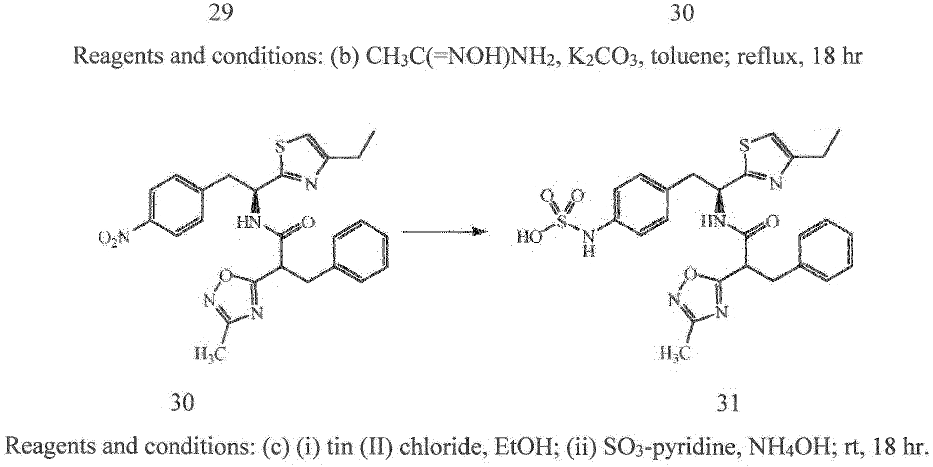

### EXAMPLE 12

### 4-{(S)-2-(4-EthyIthiazol-2-yl)-2-[2-(3-methyl-1,2,4-oxadiazol-5-yl)-3-phenylpropanamido]ethyl}phenylsulfamic acid (31)

Preparation of ethyl-2-benzyl-3-[(S)-1-(4-ethylthiazol-2-yl)-2-(4-nitrophenyl)-ethylamino]-3-oxopropanoate (29): To a solution of 1-(S)-(4-ethylthiazol-2-yl)-2-(4-nitrophenyl)ethyl amine hydrobromide, 3, (0.406 g, 1.13 mmol), 2-benzyl-3-ethoxy-3-oxopropanoic acid (0.277 g) and 1-hydroxybenzotriazole (HOBt) (0.191 g, 1.41 mmol) in DMF (10 mL) at 0°, is added 1-(3-dimethylaminopropyl)-3-ethylcarbodiimide (EDCI) (0.240 g, 1.25 mmol) followed by diisopropylethylamine (DIPEA) (0.306 g). The mixture is stirred at 0 °C for 30 minutes then at room temperature overnight. The reaction mixture is diluted with water and extracted with EtOAc. The combined organic phase is washed with 1 N aqueous HCl, 5 % aqueous NaHCO₃, water and brine, and dried over Na₂SO₄. The solvent is removed *in vacuo* to afford 0.169 g (31 % yield) of the desired product which is used without further purification.

Preparation of *N*-[(*S*)-1-(4-ethylthiazol-2-yl)-2-(4-nitrophenyl)ethyl]-2-(3-methyl-1,2,4-oxadiazol-5-yl)-3-phenylpropanamide (30): Ethyl 2-benzyl-3-((S)-1-(4-ethylthiazol-2-yl)-2-(4-nitrophenyl)ethylamino)-3-oxopropanoate is dissolved in toluene (5 mL) and heated to reflux. Potassium carbonate (80 mg) and acetamide oxime (43 mg) are added, and treated with 80 mg potassium carbonate and 43 mg acetamide oxime at reflux. The reaction mixture is cooled to room temperature, filtered and concentrated. The residue is chromatographed over silica to afford 0.221 g (94%) of the desired product as a yellow oil.

Preparation of 4-{(*S*)-2-(4-ethylthiazol-2-yl)-2-[2-(3-methyl-1,2,4-oxadiazol-5-yl)-3-phenylpropanamido]ethyl}phenylsulfamic acid (31): N-[(S)-1-(4-ethylthiazol-2-yl)-2-(4-nitrophenyl)ethyl]-2-(3-methyl-1,2,4-oxadiazol-5-yl)-3-phenylpropanamide, 30, (0. 221 g) and tin (II) chloride (507 mg, 2.2 mmol) are dissolved in EtOH (25 mL) and the solution is brought to reflux 4 hours. The solvent is removed in vacuo and the resulting residue is dissolved in EtOAc. A saturated solution of NaHCO₃ (50 mL) is added and the solution is stirred 1 hour. The organic layer is separated and the aqueous layer extracted twice with EtOAc. The combined organic layers are dried (Na₂SO₄), filtered and concentrated to a residue which is dissolved in pyridine (0.143 g) and treated with SO₃-pyridine (0.143 g). The reaction is stirred at room temperature for 5 minutes after which a 7% solution of NHaOH is added. The mixture is then concentrated and the resulting residue is purified by reverse phase chromatography to afford 0.07 lg of the desired product as the ammonium salt. ¹H NMR (CD₃OD): δ 7.29-6.87 (m, 10H), 5.38-5.30 (m, 1H), 4.37-4.30 (m, 1H), 3.42-2.74 (m, 6H), 2.38-2.33 (m, 3H), 1.34-1.28 (m, 3H).

Category VI of the present disclosure relates to 2-(thiazol-2-yl) compounds falling outside the scope of the invention having the formula: 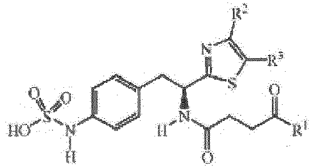 wherein R¹, R², R³, and L are further defined herein in Table XIII herein below.

**TABLE XIII**

| **No.** | **R²** | **R³** | **R¹** |
|---|---|---|---|
| M521 | ethyl | hydrogen | thiophen-2-yl |
| M522 | ethyl | hydrogen | thiazol-2-yl |
| M523 | ethyl | hydrogen | oxazol-2-yl |
| M524 | ethyl | hydrogen | isoxazol-3-yl |
| M525 | ethyl | hydrogen | imidazol-2-yl |
| M526 | ethyl | hydrogen | isoxazol-3-yl |
| M527 | ethyl | hydrogen | oxazol-4-yl |
| M528 | ethyl | hydrogen | isoxazol-4-yl |
| M529 | ethyl | hydrogen | thiophen-4-yl |
| M530 | ethyl | hydrogen | thiazol-4-yl |
| M531 | ethyl | methyl | methyl |
| M532 | ethyl | methyl | ethyl |
| M533 | ethyl | methyl | propyl |
| M534 | ethyl | methyl | *iso*-propyl |
| M535 | ethyl | methyl | butyl |
| M536 | ethyl | methyl | phenyl |
| M537 | ethyl | methyl | benzyl |
| M538 | ethyl | methyl | 2-fluorophenyl |
| M539 | ethyl | methyl | 3-fluorophenyl |
| M540 | ethyl | methyl | 4-fluorophenyl |
| M541 | phenyl | hydrogen | methyl |
| M542 | phenyl | hydrogen | ethyl |
| M543 | phenyl | hydrogen | propyl |
| M544 | phenyl | hydrogen | *iso*-propyl |
| M545 | phenyl | hydrogen | butyl |
| M546 | phenyl | hydrogen | phenyl |
| M547 | phenyl | hydrogen | benzyl |
| M548 | phenyl | hydrogen | 2-fluorophenyl |
| M549 | phenyl | hydrogen | 3-fluorophenyl |
| M550 | phenyl | hydrogen | 4-fluorophenyl |
| M551 | thiophen-2-yl | hydrogen | methyl |
| M552 | thiophen-2-yl | hydrogen | ethyl |
| M553 | thiophen-2-yl | hydrogen | propyl |
| M554 | thiophen-2-yl | hydrogen | *iso*-propyl |
| M555 | thiophen-2-yl | hydrogen | butyl |
| M556 | thiophen-2-yl | hydrogen | phenyl |
| M557 | thiophen-2-yl | hydrogen | benzyl |
| M558 | thiophen-2-yl | hydrogen | 2-fluorophenyl |
| M559 | thiophen-2-yl | hydrogen | 3-fluorophenyl |
| M560 | thiophen-2-yl | hydrogen | 4-fluorophenyl |

The compounds encompassed within Category VI of the present disclosure can be prepared by the procedure outlined in Scheme XII and described in Example 13 herein below.

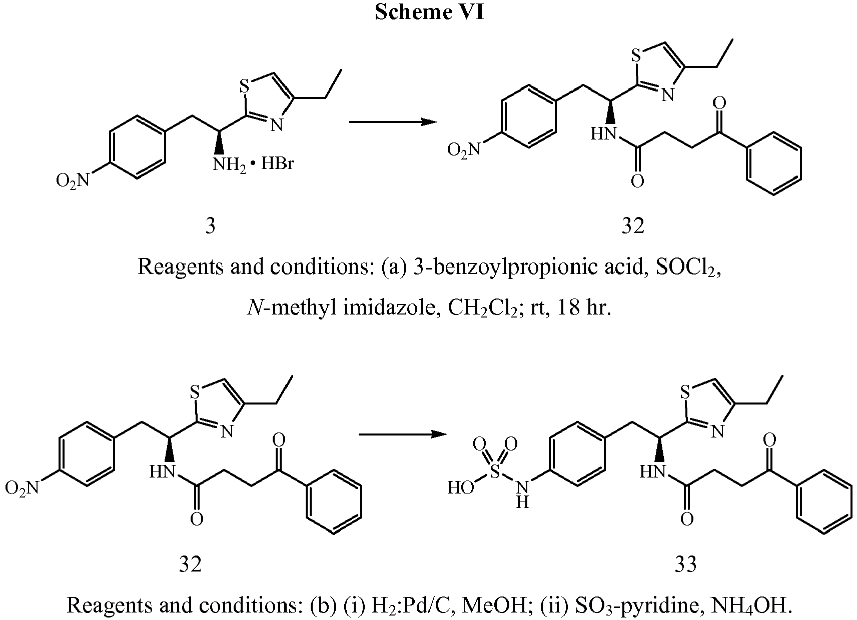

### EXAMPLE 13

### (S)-4-[2-(4-Ethylthiazol-2-yl)-2-(4-oxo-4-phenylbutanamido)ethyl]-phenylsulfamic acid (33)

Preparation of (*S*)-N-[1-(4-ethylthiazol-2-yl)-2-(4-nitrophenyl)ethyl]-4-oxo-4-phenylbutanamide (32): 3-Benzoylpropionic acid (0.250 g) is dissolved in CH₂Cl₂ (5 mL), *N*-methyl imidazole (0.333 mL) is added and the resulting solution is cooled to 0 °C after which a solution of thionyl chloride (0.320 g) in CH₂Cl₂ (2 mL) is added dropwise. After 0.5 hours (*S*)-1-(4-ethylthiazol-2-yl)-2-(4-nitrophenyl)ethanamine, 3, (0.388 g) is added. The reaction is stirred for18 hours at room temperature and then concentrated in vacuo. The resulting residue is dissolved in EtOAc and washed with IN HCl and brine. The solution is dried over Na₂SO₄, filtered, and concentrated and the crude material purified over silica to afford 0.415 g of the desired product.

Preparation of (*S*)-4-[2-(4-ethylthiazol-2-yl)-2-(4-oxo-4-phenylbutanamido)-ethyl]phenylsulfamic acid (33): (*S*)-*N*-[1-(4-ethylthiazol-2-yl)-2-(4-nitrophenyl)ethyl]-2,3-diphenyl-propanamide, 32, (0.2 g) is dissolved in MeOH (15 mL). A catalytic amount of Pd/C (10% w/w) is added and the mixture is stirred under a hydrogen atmosphere 18 hours. The reaction mixture is filtered through a bed of CELITE™ and the solvent is removed under reduced pressure. The crude product is dissolved in pyridine (5 mL) and treated with SO₃-pyridine (0.153 g). The reaction is stirred at room temperature for 5 minutes after which a 7% solution of NHaOH is added. The mixture is then concentrated and the resulting residue is purified by reverse phase chromatography to afford 0.090 g of the desired product as the ammonium salt. ¹H NMR (CD₃OD) δ 8.68 (d, 1H, J=8.2 Hz), 8.00 (d, 2H, J=7.2 Hz), 7.80-7.50 (m, 3H), 7.12 (s, 4H), 7.03 (s, 1H), 5.46-5.38 (m, 1H), 3.29-3.14 (m, 2H), 3.06-2.99 (m, 2H), 2.83 (q, 2H, J=7.5 Hz), 2.69-2.54 (m, 2H), 1.33 (t, 3H, J=7.5 Hz).

The following are non-limiting examples of compounds encompassed within Category II of the present disclosure. The intermediate nitro compounds of the following can be prepared by coupling the appropriate 4-oxo-carboxcylic acid with intermediate 3 under the conditions described herein above for the formation of intermediate 4 of scheme I.

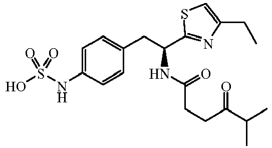

(*S*)-4-(2-(4-Ethylthiazol-2-yl)-2-(5-methyl-4-oxohexanamido)ethyl)phenylsulfamic acid: ¹H NMR (CD₃OD) δ 8.59 (d, 1H, J=8.1 Hz), 7.14 (s, 4H), 7.08 (t, 1H, J=13.0 Hz), 5.40-5.35 (m, 1H), 3.37-3.27 (m, 2H), 3.04-2.97 (m, 1H), 2.83-2.61 (m, 4H), 2.54-2.36 (m, 3H), 1.33 (t, 2H, J=7.3 Hz), 1.09 (dd, 6H, J=7.0, 2.2 Hz).

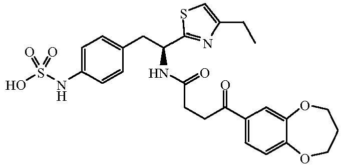

(*S*)-4-{2-[4-(3,4-Dihydro-2H-benzo[b][1,4]dioxepin-7-yl)-4-oxobutanamido]-2-(4-ethylthiazol-2-yl)ethyl}phenylsulfamic acid: ¹H NMR(CD₃OD) δ 8.64 (d, 1H, J=8.4 Hz), 7.60 (d, 2H, J=10.6 Hz), 7.11 (s, 3H), 7.04 (d, 2H, J=5.5 Hz), 5.42-5.40 (m, 1H), 4.30-4.22 (m, 4H), 3.20-2.98 (m, 4H), 2.82 (q, 2H, J=7.3 Hz), 2.67-2.48 (m, 2H), 2.23 (t, 2H, J=5.5 Hz), 1.32 (t, 3H, J=7.3 Hz).

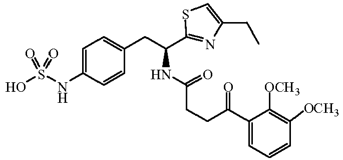

(*S*)-4-{2-[4-(2,3-Dimethoxyphenyl)-4-oxobutanamido]-2-(4-ethylthiazol-2-yl)ethyl}phenylsulfamic acid: ¹H NMR (CD₃OD), δ 8.64 (d, 1H, J=8.1 Hz), 7.21-7.11 (m, 7H), 7.02 (s, 1H), 5.42 (q, 1H, J=5.9 Hz), 3.90 (d, 3H, J=3.3 Hz), 3.88 (d, 3H, J=2.9 Hz), 3.22-3.18 (m, 2H), 3.07-2.99 (m, 2H), 2.83 (q, 2H, J=7.3 Hz), 2.63-2.54 (m, 2H), 1.34 (t, 3H, J=7.69 Hz).

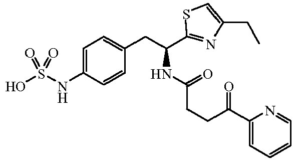

(*S*)-4-{2-(4-Ethylthiazol-2-yl)-2-[4-oxo-4-(pyridin-2-yl)butanamido]ethyl}-phenylsulfamic acid: ¹H NMR (CD₃OD) δ 8.60 (d, 1H, J=12.8 Hz), 7.91-7.81 (m, 2H), 7.48-7.44 (m, 1H), 7.22-7.21 (m, 1H), 6.99 (s, 3H), 6.91 (s, 1H), 5.30 (q, 1H, J=5.4 Hz), 3.36 (q, 2H, J=7.0 Hz), 3.21-3.15 (m, 1H), 2.91-2.85 (m, 1H), 2.74 (q, 2H, J=10.4 Hz), 2.57-2.50 (m, 2H), 1.20 (t, 3H, J=7.5 Hz).

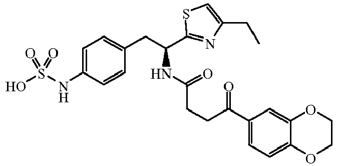

(S)-4-{2-[4-(2,3-dihydrobenzo[b][l,4]dioxin-6-yl)-4-oxobutanamido]-2-(4-ethylthiazol-2-yl)ethyl}phenylsulfamic acid: ¹H NMR (CD₃OD) δ 7.52-7.47 (m,2 H), 7.11(s,4H), 7.03 (s,1H), 6.95 (d, 1H, J=8.4 Hz), 5.41 (q, 1H, J=3.7 Hz), 4.31 (d, 4H, J=5.5 Hz), 3.24-3.12 (m, 2H), 3.06-2.98 (m, 2H), 2.83 (q, 2H, J=7.3 Hz), 2.62-2.53 (m, 2H), 1.33 (t, 3H, J=7.3 Hz).

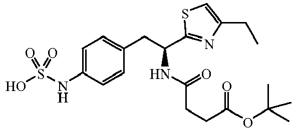

(*S*)-4-[2-(4-tert-butoxy-4-oxobutanamido)-2-(4-ethylthiazol-2-yl)ethyl]phenylsulfamic acid: ¹H NMR (CD₃OD), δ 7.10 (s 4H), 7.02 (s, 1H), 5.41 (q, 1H,
J=3.7 Hz), 3.30-3.25 (m, 1H), 3.06-2.99 (m, 1H), 2.83 (q, 2H, J=7.3 Hz), 2.52-2.40 (m, 4H), 1.42 (s, 9H), 1.33 (t, 3H, J=7.3 Hz).

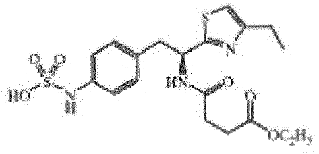

(*S*)-4-[2-(4-ethoxy-4-oxobutanamido)-2-(4-ethylthiazol-2-yl)ethyl]phenylsulfamic acid: ¹H NMR (CD₃OD) δ 8.62 (d, 1H, J=8.4 Hz), 7.10 (s, 4H), 7.02 (s, 1H), 5.40 (q, 1H, 3.7 Hz), 4.15 (q, 2H, J=7.3 Hz), 3.28-3.25 (m, 1H), 3.05-3.02 (m, 1H), 2.82 (q, 2H, J=4.4 Hz), 2.54-2.48 (m, 2H), 1.33 (t, 3H, J=7.3 Hz), 1.24 (t, 3H, J=7.0 Hz).

The first type of Category VII of the present disclosure relates to 2-(thiazol-2-yl) compounds having the formula: 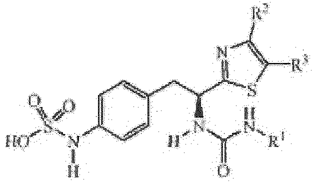 wherein non-limiting examples of R¹, R², and R³ are further described herein below in Table XIV.

**TABLE XIV**

| **No.** | **R²** | **R³** | **R¹** |
|---|---|---|---|
| N561 | methyl | hydrogen | phenyl |
| N562 | methyl | hydrogen | benzyl |
| N563 | methyl | hydrogen | 2-fluorophenyl |
| N564 | methyl | hydrogen | 3-fluorophenyl |
| N565 | methyl | hydrogen | 4-fluorophenyl |
| K566 | methyl | hydrogen | 2-chlorophenyl |
| N567 | methyl | hydrogen | 3-chlorophenyl |
| N568 | methyl | hydrogen | 4-chlorophenyl |
| N569 | ethyl | hydrogen | phenyl |
| N570 | ethyl | hydrogen | benzyl |
| N571 | ethyl | hydrogen | 2-fluorophenyl |
| N572 | ethyl | hydrogen | 3-fluorophenyl |
| N573 | ethyl | hydrogen | 4-fluorophenyl |
| N574 | ethyl | hydrogen | 2-chlorophenyl |
| N575 | ethyl | hydrogen | 3-chlorophenyl |
| N576 | ethyl | hydrogen | 4-chlorophenyl |
| N577 | thiene-2-yl | hydrogen | phenyl |
| N578 | thiene-2-yl | hydrogen | benzyl |
| N579 | thiene-2-yl | hydrogen | 2-fluorophenyl |
| N580 | thiene-2-yl | hydrogen | 3-fluorophenyl |
| N581 | thiene-2-yl | hydrogen | 4-fluorophenyl |
| N582 | thiene-2-yl | hydrogen | 2-chlorophenyl |
| N583 | thiene-2-yl | hydrogen | 3-chlorophenyl |
| N584 | thiene-2-yl | hydrogen | 4-chlorophenyl |

The compounds encompassed within Category VII of the present disclosure can be prepared by the procedure outlined in Scheme XIII and described in Example 14 herein below.

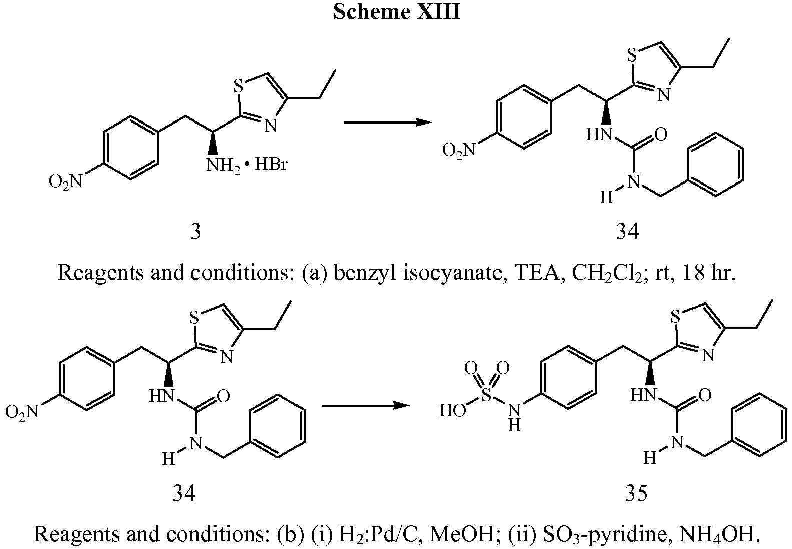

### EXAMPLE 14

### (S)-4-(2-(3-Benzylureido)-2-(4-ethylthiazol-2-yl)ethyl)phenylsulfamic acid (35)

Preparation of (*S*)-1-benzyl-3-[1-(4-ethylthiazol-2-yl)-2-(4-nitrophenyl)ethyl]urea (34): To a solution of 1-(S)-(4-ethylthiazol-2-yl)-2-(4-nitrophenyl)ethyl amine hydrobromide, 3, (0.360 g, 1 mmol) and Et₃N (0.42 mL, 3mmol) in 10 mL CH₂Cl₂ is added benzyl isocyanate (0.12 mL, 1 mmol). The mixture is stirred at room temperature for 18 hours. The product is isolated by filtration to afford 0.425 g (96% yield) of the desired product which is used without further purification.

Preparation of (S)-4-(2-(3-benzylureido)-2-(4-ethylthiazol-2-yl)ethyl)phenylsulfamic acid (35): (S)-1-benzyl-3-[1-(4-ethylthiazol-2-yl)-2-(4-nitrophenyl)ethyl]urea, 34, (0.425 g) is dissolved in MeOH (4 mL). A catalytic amount of Pd/C (10% w/w) is added and the mixture is stirred under a hydrogen atmosphere 18 hours. The reaction mixture is filtered through a bed of CELITE™ and the solvent is removed under reduced pressure. The crude product is dissolved in pyridine (12 mL) and treated with SO₃-pyridine (0.220 g). The reaction is stirred at room temperature for 5 minutes after which a 7% solution of NH₄OH is added. The mixture is then concentrated and the resulting residue is purified by reverse phase chromatography to afford 0.143 g of the desired product as the ammonium salt. ¹H NMR (CD₃OD) δ 7.32-7.30 (m, 2H), 7.29-7.22 (m, 3H), 7.12-7.00 (m, 4H), 6.84 (d, 1H, J = 8.1Hz), 5.35-5.30 (m, 1H), 4.29 (s, 2H), 3.27-3.22 (m, 3H), 3.1 1-3.04 (m, 3H), 2.81 (q, 2H, J = 10.2, 13.0H_{Z}), 1.31 (t, 3H, J = 4.5Hz).

The following is a non-limiting examples of compounds encompassed within the first type of Category VII of the present disclosure.

4-{[(*S*)-2-(2-Ethylthiazol-4-yl)-2-(3-(R)-methoxy-1-oxo-3-phenylpropan-2-yl)ureido]ethyl}phenylsulfamic acid: ¹H NMR (CD₃OD) δ 7.36-7.26 (m, 3H), 7.19-7.17 (m, 2H), 7.10-7.06 (m, 2H), 6.90-6.86 (m, 3H), 5.12-5.06 (m, 1H), 4.60-4.55 (m, 1H), 3.69 (s, 3H) 3.12-2.98 (m, 6H), 1.44-1.38 (m, 3H).

The second type of Category VII of the present disclosure relates to 2-(thiazol-4-yl) compounds having the formula: 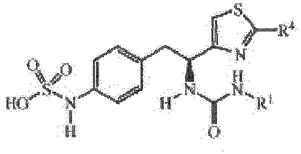 wherein non-limiting examples of R¹ and R⁴ are further described herein below in Table XV.

**TABLE XV**

| **No.** | **R¹** | **R⁴** |
|---|---|---|
| O585 | methyl | methyl |
| O586 | ethyl | methyl |
| O587 | n-propyl | methyl |
| O588 | *iso*-propyl | methyl |
| O589 | phenyl | methyl |
| O590 | benzyl | methyl |
| O591 | 2-fluorophenyl | methyl |
| O592 | 2-chlorophenyl | methyl |
| O593 | thiophen-2-yl | methyl |
| O594 | thiazol-2-yl | methyl |
| O595 | oxazol-2-yl | methyl |
| O596 | isoxazol-3-yl | methyl |
| O597 | methyl | ethyl |
| O598 | ethyl | ethyl |
| O599 | n-propyl | ethyl |
| O600 | *iso*-propyl | ethyl |
| O601 | phenyl | ethyl |
| O602 | benzyl | ethyl |
| O603 | 2-fluorophenyl | ethyl |
| O604 | 2-chlurophenyl | ethyl |
| O605 | thiophen-2-yl | ethyl |
| O606 | thiazol-2-yl | ethyl |
| O607 | oxazol-2-yl | ethyl |
| O608 | isoxazol-3-yl | ethyl |
| O609 | methyl | thiophen-2-yl |
| O610 | ethyl | thiophen-2-yl |
| O611 | n-propyl | thiophen-2-yl |
| O612 | *iso*-propyl | thiophen-2-yl |
| O613 | phenyl | thiophen-2-yl |
| O614 | benzyl | thiophen-2-yl |
| O615 | 2-fluorophenyl | thiophen-2-yl |
| O616 | 2-chlorophenyl | thiophen-2-yl |
| O617 | thiophen-2-yl | thiophen-2-yl |
| O618 | thiazol-2-yl | thiophen-2-yl |
| O619 | oxazol-2-yl | thiophen-2-yl |
| O620 | isoxazol-3-yl | thiophen-2-yl |
| O621 | methyl | thiazol-2-yl |
| O622 | ethyl | thiazol-2-yl |
| O623 | n-propyl | thiazol-2-yl |
| O624 | *iso*-propyl | thiazol-2-yl |
| O625 | phenyl | thiazol-2-yl |
| O626 | benzyl | thiazol-2-yl |
| O627 | 2-fiuorophenyl | thiazol-2-yl |
| O628 | 2-chlorophenyl | thiazol-2-yl |
| O629 | thiophen-2-yl | thiazol-2-yl |
| O630 | thiazol-2-yl | thiazol-2-yl |
| O631 | oxazol-2-yl | thiazol-2-yl |
| O632 | isoxazol-3-yl | thiazol-2-yl |
| O633 | methyl | oxazol-2-yl |
| O634 | ethyl | oxazol-2-yl |
| O635 | | oxazol-2-yl |
| O636 | *iso*-propyl | oxazol-2-yl |
| O637 | phenyl | oxazol-2-yl |
| O638 | benzyl | oxazol-2-yl |
| O639 | 2-fluorophenyl | oxazol-2-yl |
| O640 | 2-chlorophenyl | oxazol-2-yl |
| O641 | thiophen-2-yl | oxazol-2-yl |
| O642 | thiazol-2-yl | oxazol-2-yl |
| O643 | oxazol-2-yl | oxazol-2-yl |
| O644 | isoxazol-3-yl | oxazol-2-yl |

The compounds encompassed within the second type of Category VII of the present disclosure can be prepared by the procedure outlined in Scheme XIV and described in Example 14 herein below.

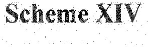

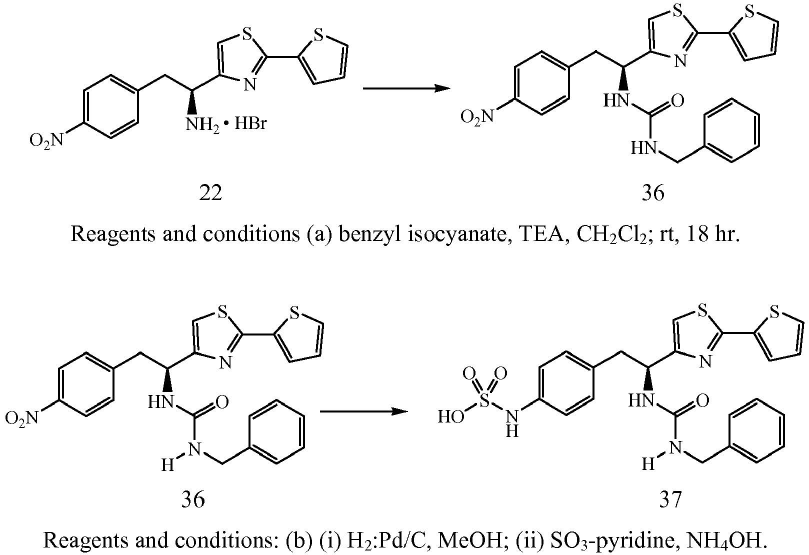

### EXAMPLE 15

### 4-{(S)-2-(3-Benzylureido)-2-[2-(thiophen-2-yl)thiazol-4-yl]ethyl}-phenylsulfamic acid (37)

Preparation of 1-benzyl-3-{(*S*)-2-(4-nitrophenyl)-1-[2-(thiophen-2-yl)thiazol-4-yl]ethyl}urea (36): To a solution of (*S*)-2-(4-nitrophenyl)-1-[(2-thiophen-2-yl)thiazol-4-yl)ethan-amine hydrobromide salt, 8, and Et₃N (0.42mL, 3mmol) in 10 mL DCM is added benzyl isocyanate (0.12mL, 1mmol). The mixture is stirred at room temperature for 18 hours. The product is isolated by filtration to afford 0.445 g (96% yield) of the desired product which is used without further purification.

Preparation of 4-{(*S*)-2-(3-benzylureido)-2-[2-(thiophen-2-yl)thiazol-4-yl]ethyl}phenylsulfamic acid (37): 1-Benzyl-3-{(*S*)-2-(4-nitrophenyl)-1-[2-(thiophen-2-yl)thiazol-4-yl]ethyl}urea, 36, (0.445g) is dissolved in MeOH (10 mL) and CH₂Cl₂ (5 mL). A catalytic amount of Pd/C (10% w/w) is added and the mixture is stirred under a hydrogen atmosphere 18 hours. The reaction mixture is filtered through a bed of CELITE™ and the solvent is removed under reduced pressure. The crude product is dissolved in pyridine (12 mL) and treated with SO₃-pyridine (0.110 g). The reaction is stirred at room temperature for 5 minutes after which a 7% solution of NHaOH is added. The mixture is then concentrated and the resulting residue is purified by reverse phase chromatography to afford 0.080 g of the desired product as the ammonium salt. ¹H NMR (CD₃OD) δ 7.61 (d, 1H, J = 2.1Hz), 7.58 (d, 1H, J = 6Hz), 7.33-7.22 (m, 4H), 7.17-7.14 (m, 1H), 7.09-6.94 (m, 6H), 5.16 (t, 1H, J = 6.6Hz), 4.13 (s, 2H), 3.14-3.11 (m, 2H).

Category VIII of the present disclosure relates to 2-(thiazol-4-yl) compounds having the formula: 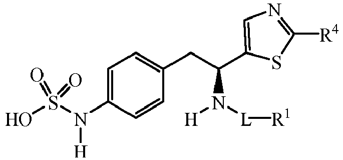 wherein R¹, R⁴, and L are further defined herein in Table XVI herein below.

**TABLE XVI**

| **No.** | **R⁴** | **L** | **R¹** |
|---|---|---|---|
| P645 | methyl | -SO₂- | methyl |
| P646 | ethyl | -SO₂- | methyl |
| P647 | phenyl | -SO₂- | methyl |
| P648 | thiophen-2-yl | -SO₂- | methyl |
| P649 | methyl | -SO₂- | trifluoromethyl |
| P650 | ethyl | -SO₂- | trifluoromethyl |
| P651 | phenyl | -SO₂- | trifluoromethyl |
| P652 | thiophen-2-yl | -SO₂- | trifluoromethyl |
| P653 | methyl | -SO₂- | ethyl |
| P654 | ethyl | -SO₂- | ethyl |
| P655 | phenyl | -SO₂- | ethyl |
| P656 | thiophen-2-yl | -SO₂- | ethyl |
| P657 | methyl | -SO₂- | 2,2,2-trifluoroethyl |
| P658 | ethyl | -SO₂- | 2,2,2-trifluoroethyl |
| P659 | phenyl | -SO₂- | 2,2,2-trifluoroethyl |
| P660 | thiophen-2-yl | -SO₂- | 2,2,2-trifluoroethyl |
| P661 | methyl | -SO₂- | phenyl |
| P662 | ethyl | -SO₂- | phenyl |
| P663 | phenyl | -SO₂- | phenyl |
| P664 | thiophen-2-yl | -SO₂- | phenyl |
| P665 | methyl | -SO₂- | 4-fluorophenyl |
| P666 | ethyl | -SO₂- | 4-fluorophenyl |
| P667 | phenyl | -SO₂- | 4-fluorophenyl |
| P668 | thiophen-2-yl | -SO₂- | 4-fluorophenyl |
| P669 | methyl | -SO₂- | 3,4-dihydro-2H-benzo[b][1,4]oxazin-7-yl |
| P670 | ethyl | -SO₂- | 3,4-dihydro-2H-benzo[b][1,4]oxazin-7-yl |
| P671 | phenyl | -SO₂- | 3,4-dihydro-2H-benzo[b][1,4]oxazin-7-yl |
| P672 | thiophen-2-yl | -SO₂- | 3,4-dihydro-2H-benzo[b][1,4]oxazin-7-yl |
| P673 | methyl | -SO₂- | 1-methyl-1*H-*imidazol-4-yl |
| P674 | ethyl | -SO₂- | 1-methyl-1*H-*imidazol-4-yl |
| P675 | phenyl | -SO₂- | 1-methyl-1*H-*imidazol-4-yl |
| P676 | thiophen-2-yl | -SO₂- | 1-methyl-1*H-*imidazol-4-yl |
| P678 | methyl | -SO₂- | 4-acetamidophenyl |
| P679 | ethyl | -SO₂- | 4-acetamidophenyl |
| P680 | phenyl | -SO₂- | 4-acetamidophenyl |
| P681 | thiophen-2-yl | -SO₂- | 4-acetamidophenyl |
| P682 | methyl | -SO₂CH₂- | phenyl |
| P683 | ethyl | -SO₂CH₂- | phenyl |
| P684 | phenyl | -SO₂CH₂- | phenyl |
| P685 | thiophen-2-yl | -SO₂CH₂- | phenyl |
| P686 | methyl | -SO₂CH₂- | (4-methylcarboxyphenyl)methyl |
| P687 | ethyl | -SO₂CH₂- | (4-methylcarboxyphenyl)methyl |
| P688 | phenyl | -SO₂CH₂- | (4-methylcarboxyphenyl)methyl |
| P689 | thiophen-2-yl | -SO₂CH₂- | (4-methylcarboxyphenyl)methyl |
| P690 | methyl | -SO₂CH₂- | (2-methylthiazol-4-yl)methyl |
| P691 | ethyl | -SO₂CH₂- | (2-methylthiazol-4-yl)methyl |
| P692 | phenyl | -SO₂CH₂- | (2-methylthiazol-4-yl)methyl |
| P693 | thiophen-2-yl | -SO₂CH₂- | (2-methylthiazol-4-yl)methyl |
| P694 | methyl | -SO₂CH₂CH₂- | phenyl |
| P695 | ethyl | -SO₂CH₂CH₂- | phenyl |
| P696 | phenyl | -SO₂CH₂CH₂- | phenyl |
| P697 | thiophen-2-yl | -SO₂CH₂CH₂- | phenyl |

The compounds encompassed within Category VIII of the present disclosure can be prepared by the procedure outlined in Scheme XV and described in Example 16 herein below.

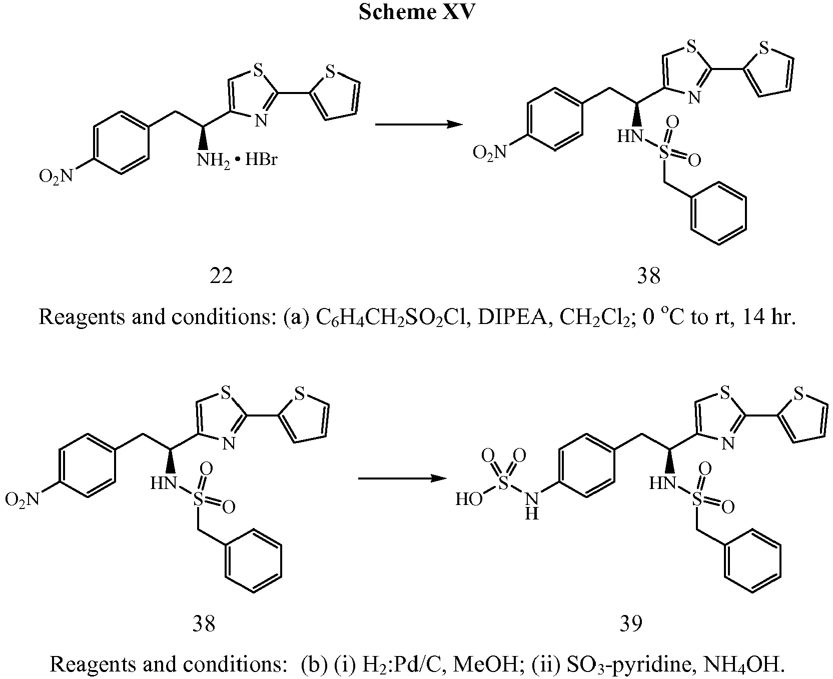

### EXAMPLE 16

### {4-(S)-[2-Phenylmethanesulfonylamino-2-(2-thiophen-2-ylthiazol-4-yl)ethyl]phenyl}sulfamic acid (39)

Preparation of (*S*)-*N*-{2-(4-nitrophenyl)-1-[2-(thiophen-2-yl)thiazol-4-yl]ethyl}-1-phenylmethanesulfonamide (38): To a suspension of 2-(4-nitrophenyl)-1-(2-thiophene2-ylthiazol-4-yl)ethylamine, 8, (330 mg, 0.80 mmol) in CH₂Cl₂ (6 mL) at 0 °C is added diisopropylethylamine (0.30 mL, 1.6 mmol) followed by phenylmethanesulfonyl chloride (167 mg, 0.88 mmol). The reaction mixture is stirred at room temperature for 14 hours. The mixture is diluted with CH₂Cl₂ and washed with sat. NaHCO₃ followed by brine, dried (Na₂SO₄), filtered and concentrated in vacuo. The resulting residue is purified over silica to afford 210 mg of the desired product as a white solid.

Preparation of {4-(*S*)-[2-phenylmethanesulfonylamino-2-(2-thiophen-2-ylthiazol-4-yl)ethyl]phenyl}sulfamic acid (39): (*S*)-*N*-{2-(4-nitrophenyl)-1-[2-(thiophen-2-yl)thiazol-4-yl]ethyl}-1-phenylmethanesulfonamide, 38, (210 mg, 0.41 mmol) is dissolved in MeOH (4 mL). A catalytic amount of Pd/C (10% w/w) is added and the mixture is stirred under a hydrogen atmosphere 18 hours. The reaction mixture is filtered through a bed of CELITE™ and the solvent is removed under reduced pressure. The crude product is dissolved in pyridine (12 mL) and treated with SO₃-pyridine (197 mg, 1.23 mmol). The reaction is stirred at room temperature for 5 minutes after which a 7% solution of NHaOH is added. The mixture is then concentrated and the resulting residue is purified by reverse phase chromatography to afford 0.060 g of the desired product as the ammonium salt. ¹H NMR (300 MHz, MeOH-d₄) δ 7.52-7.63 (m, 6.70-7.28 (m, 11H), 4.75 (t, *J* = 7.2 Hz, 1H), 3.95-4.09 (m, 2H), 3.20 (dd, *J* = 13.5 and 7.8 Hz, 1H), 3.05 (dd, *J* = 13.5 and 7.8 Hz, 1H). 1013770

Intermediates for use in Step (a) of Scheme XV can be conveniently prepared by the procedure outlined herein below in Scheme XVI and described in Example 17.

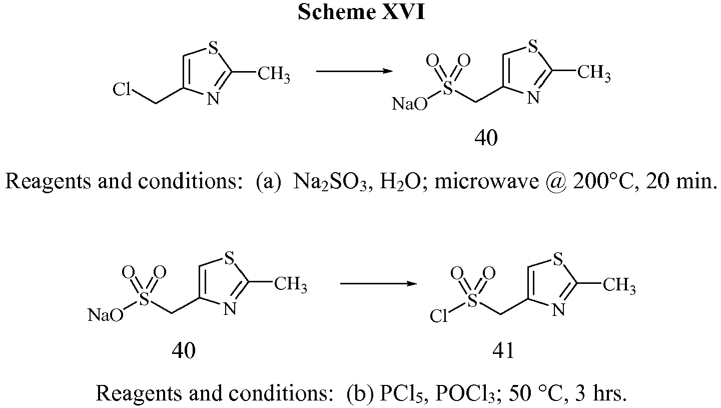

### EXAMPLE 17

### (2-Methylthiazol-4-yl)methanesulfonyl chloride (41)

Preparation of sodium (2-methylthiazol-4-yl)methanesulfonate (40): 4-Chloromethyl-2-methylthiazole (250 mg, 1.69 mmol) is dissolved in H₂O (2 mL) and treated with sodium sulfite (224 mg, 1.78 mmol). The reaction mixture is subjected to microwave irradiation for 20 minutes at 200°C. The reaction mixture is diluted with H₂O (30 mL) and washed with EtOAc (2 x 25 mL). The aqueous layer is concentrated to afford 0.368g of the desired product as a yellow solid. LC/MS ESI+ 194 (M+1, free acid).

Preparation of (2-methylthiazol-4-yl)methanesulfonyl chloride (41): Sodium (2-methylthiazol-4-yl)methanesulfonate, 40, (357 mg, 1.66 mmol) is dissolved in phosphorous oxychloride (6 mL) and is treated with phosphorous pentachloride (345 mg, 1.66 mmol). The reaction mixture is stirred at 50 °C for 3 hours, then allowed to cool to room temperature. The solvent is removed under reduced pressure and the residue is re-dissolved in CH₂Cl₂ (40 mL) and is washed with sat. NaHCO₃ and brine. The organic layer is dried over MgSO₄, filtered, and the solvent removed *in vacuo* to afford 0.095 g of the desired product as a brown oil. LC/MS ESI+ 211 (M+1). Intermediates are obtained in sufficient purity to be carried forward according to Scheme IX without the need for further purification.

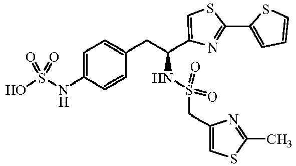

4-{(S)-2-[(2-methylthiazol-4-yl)methylsulfonamido]-2-[2-(thiophen-2-yl)thiazol-4-yl]ethyl}phenylsulfamic acid: ¹H NMR (CD₃OD): δ 7.71-7.66 (m, 2H), 7.27-7.10 (m, 7H), 4.87 (t, 1H, *J*=7.3 Hz), 4.30-4.16 (q, 2H, *J*=13.2 Hz), 3.34-3.13 (m, 2H), 2.70 (s, 3H).

The following are non-limiting examples of compounds encompassed within Category VIII of the present disclosure.

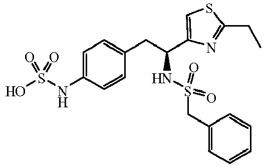

{4-(*S*)-[2-Phenylmethanesulfonylamino-2-(2-ethylthiazol-4-yl)ethyl]phenyl}-sulfamic acid: ¹H NMR (300 MHz, MeOH-d₄) δ 7.27-7.32 (m, 3H), 7.16-7.20 (m, 3H), 7.05-7.6 (m, 2H), 6.96 (d, *J* = 8.4 Hz, 2H), 4.70 (t, *J* = 9.0 Hz, 1H), 3.91-4.02 (m, 2H), 2.95-3.18 (m, 4H), 1.41 (t, *J* = 7.5 Hz, 3H).

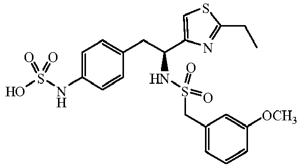

{4-(*S*)-[2-(3-Methoxyphenyl)methanesulfonylamino-2-(2-ethylthiazol-4-yl)ethyl]phenyl}sulfamic acid: ¹H NMR (300 MHz, MeOH-d₄) δ 7.20 (t, *J* = 8.1 Hz. 1H), 6.94-7.08 (m,4H), 6.88-6.94 (m, 3H), 6.75-6.80 (m, 1H), 4.67 (t, *J* = 7.2 Hz, 1H), 3.90-4.0 (m, 2H), 3.76 (s, 3H), 2.95-3.16 (m, 4H), 1.40 (t, *J* = 7.5 HZ, 3H).

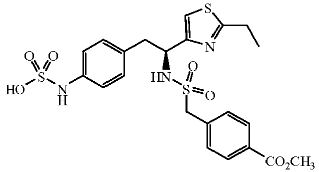

(*S*)-4-{[1-(2-Ethylthiazol-4-yl)-2-(4-sulfoaminophenyl)ethylsulfamoyl]methyl} - benzoic acid methyl ester: ¹H NMR (300 MHz, MeOH-d₄) δ 7.90-7.94- (m, 2H), 7.27-7.30 (m, 2H), 7.06-7.11 (m, 3H), 6.97-7.00 (m, 2H), 4.71 (t, *J* = 7.2 Hz, 1H), 3.95-4.08 (4, 2H), 3.92 (s, 3H), 2.80-3.50 (m, 4H), 1.38-1.44 (m, 3H).

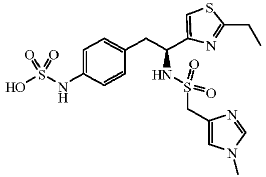

(*S*)-4-[2-(2-Ethylthiazol-4-yl)-2-(1-methyl-1*H*-imidazol-4-sulfonamido)ethyl]-phenylsulfamic acid: ¹H NMR (300 MHz, MeOH-d₄) δ 7.54 (s, 1H, 7.20 (s, 1H), 7.09 (s, 1H), 6.92-7.00 (m, 4H), 4.62 (t, *J* = 5.4 Hz, 1H), 3.70 (s, 3H), 2.98-3.14 (m,3H), 2.79 (dd, *J* = 9.3 and 15.0 Hz, 1H), 1.39 (q, *J* = 7.5 Hz, 3H).

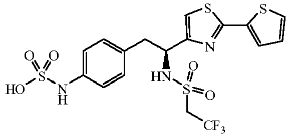

4-{(*S*)-2-[2-(Thiophen-2-yl)thiazol-4-yl]-2-(2,2,2-trifluoroethylsulfonamido)-ethyl}phenylsulfamic acid: ¹H NMR (CD₃OD): δ 7.62-7.56 (m, 2H), 7.22 (s, 1H), 7.16-7.06 (m, 5H), 4.84 (t, 1H, *J*=7.6 Hz), 3.71-3.62 (m, 2H), 3.32-3.03 (m, 2H).

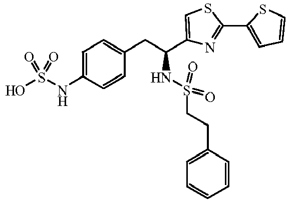

{4-(*S*)-[2-(Phenylethanesulfonylamino)-2-(2thiophen-2-ylthiazol-4-yl)ethyl]-phenyl}sulfamic acid: ¹H NMR (300 MHz, MeOH-d₄) δ 7.56-7.62 (m, 2H), 7.04-7.19(m, 9H), 6.94-6.97 (m, 2H), 4.78 (t, *J* = 7.8 Hz, 1H), 3.22-3.30 (m, 2H)), 3.11 (dd, *J* = 13.5 and 7.8 Hz, 1H), 2.78-2.87 (m, 4H).

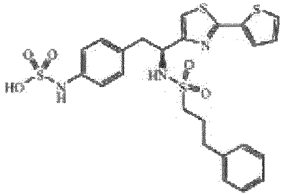

{4-(*S*)-[3-(Phenylpropanesulfonylamino)-2-(2thiophen-2-ylthiazol-4-yl)ethyl]-phenyl}sulfamic acid: ¹H NMR (300 MHz, MeOH-d₄) δ 7.56-7.62 (m, 2H), 6.99-7.17 (m, 10H), 4.72 (t, *J* = 7.8 Hz, 1H), 3.21 (dd, *J* = 13.5 and 7.2 Hz, 1H), 3.02 (dd, *J* = 13.5 and 7.2 Hz, 1H), 2.39-2.64 (m, 4H), 1.65-1.86 (m, 2H).

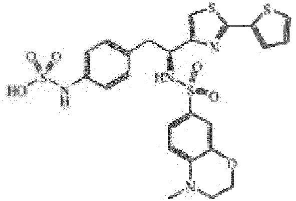

(*S*)-{4-[2-(4-Methyl-3,4-dihydro-2H-benzo[1,4]oxazine-7-sulfonylamino)-2-(2-thiophen-2-ylthiazol-4-yl)ethyl]phenyl}sulfamic acid: ¹H NMR (300 MHz, MeOH-d₄) δ 7.53 (d, J = 5.1 Hz, 1H) 7.48 (d, J=5.1 Hz, 1H), 7.13-7.10 (m, 1H), 7.04 (d, J = 8.4 Hz, 2H), 6.93-6.88 (m, 3H), 6.75 (d, J = 8.1 Hz, 1H), 6.54 (d, J= 8.1 Hz, 1H), 4.61 (t, J = 7.5 Hz, 1H), 4.20-4.08 (m, 2H), 3.14-3.00 (m, 4H), 2.69 (s, 3H).

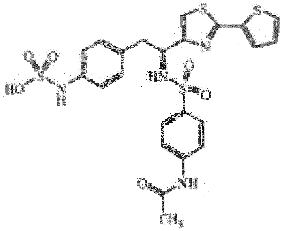

4-{(*S*)-2-(4-acetamidophenylsulfonamido)-2-[2-(thiophen-2-yl)thiazol-4-yl]ethyl}phenylsulfamic acid: ¹H NMR (CD₃OD): δ 7.67-7.52 (m, 6H), 7.24-7.23 (m, 1H), 7.12-7.09 (m, 3H), 7.02-6.99 (m, 2H), 4.70 (t, 1H, J=7.3 Hz), 3.25-3.00 (m, 2H), 2.24 (s, 3H).

The first type of Category IX of the present disclosure relates to compounds having the formula: 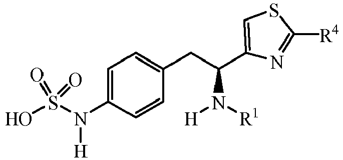 wherein R¹ is a substituted or unsubstituted heteroaryl and R⁴ is C₁-C₆ linear, branched, or cyclic alkyl as further described herein below in Table XVII.

**TABLE XVII**

| **No.** | **R⁴** | **R¹** |
|---|---|---|
| Q698 | -CH₃ | 4-(methoxycarbonyl)thiazol-5-yl |
| Q699 | -CH₃ | 4-[(2-methoxy-2-oxoethyl)carbamoyl]thiazol-5-yl |
| Q700 | -CH₃ | 5-[1-*N*-(2-methoxy-2-oxoethyl)-1-*H*-indol-3-yl]oxazol-2-yl |
| Q701 | -CH₃ | 5-(2-methoxyphenyl)oxazol-2-yl |
| Q702 | -CH₃ | 5-[(*S*)-1-(*tert*-butoxycarbonyl)-2-phenylethyl]oxazol-2-yl |
| Q703 | -CH₃ | 5-[4-(methylcarboxy)phenyl]oxazol-2-yl |
| Q704 | -CH₃ | 5-(3-methoxybenzyl)oxazol-2-yl |
| Q705 | -CH₃ | 5-(4-phenyl)oxazol-2-yl |
| Q706 | -CH₃ | 5-(2-methoxyphenyl)thiazol-2-yl |
| Q707 | -CH₃ | 5-(3-methoxyphenyl)thiazol-2-yl |
| Q708 | -CH₃ | 5-(4-fluorophenyl)thiazol-2-yl |
| Q709 | -CH₃ | 5-(2,4-difluorophenyl)thiazol-2-yl |
| Q710 | -CH₃ | 5-(3-methoxybenzyl)thiazol-2-yl |
| Q711 | -CH₃ | 4-(3-methoxyphenyl)thiazol-2-yl |
| Q712 | -CH₃ | 4-(4-fluorophenyl)thiazol-2-yl |
| Q713 | -CH₂CH₃ | 4-(methoxycarbonyl)thiazol-5-yl |
| Q714 | -CH₂CH₃ | 4-[(2-methoxy-2-oxoethyl)carbamoyl]thiazol-5-yl |
| Q715 | -CH₂CH₃ | 5-[1-*N*-(2-methoxy-2-oxoethyl)-1-*H*-indol-3-yl]oxazol-2-yl |
| Q716 | -CH₂CH₃ | 5-(2-methoxyphenyl)oxazol-2-yl |
| Q717 | -CH₂CH₃ | 5-[(*S*)-1-(*tert*-butoxycarbonyl)-2-phenylethyl]oxazol-2-yl |
| Q718 | -CH₂CH₃ | 5-[4-(methylcarboxy)phenyl]oxazol-2-yl |
| Q719 | -CH₂CH₃ | 5-(3-methoxybenzyl)oxazol-2-yl |
| Q720 | -CH₂CH₃ | 5-(4-phenyl)oxazol-2-yl |
| Q721 | -CH₂CH₃ | 5-(2-methoxyphenyl)thiazol-2-yl |
| Q722 | -CH₂CH₃ | 5-(3-methoxyphenyl)thiazol-2-yl |
| Q723 | -CH₂CH₃ | 5-(4-fluorophenyl)thiazol-2-yl |
| Q724 | -CH₂CH₃ | 5-(2,4-difluorophenyl)thiazol-2-yl |
| Q725 | -CH₂CH₃ | 5-(3-mcthoxybenzyl)thiazol-2-yl |
| Q726 | -CH₂CH₃ | 4-(3-methoxyphenyl)thiazol-2-yl |
| Q727 | -CH₂CH₃ | 4-(4-fluorophenyl)thiazol-2-yl |
| Q728 | cyclopropyl | 4-(methoxycarbonyl)thiazol-5-yl |
| Q729 | cyclopropyl | 4-[(2-methoxy-2-oxoethyl)carbamoyl]thiazol-5-yl |
| Q730 | cyclopropyl | 5-[ 1-*N-*(2-methoxy-2-oxoethyl)-1-*H*-indol-3-y l]oxazol-2-yl |
| Q731 | cyclopropyl | 5-(2-methoxyphenyl)oxazol-2-yl |
| Q732 | cyclopropyl | 5-[(*S*)-1-(*tert-*butoxycarbonyl)-2-phenylethyl]oxazol-2-yl |
| Q733 | cyclopropyl | 5-[4-(methylcarboxy)phenyl]oxazol-2-yl |
| Q734 | cyclopropyl | 5-(3-methoxybenzyl)oxazol-2-yl |
| Q735 | cyclopropyl | 5-(4-phenyl)oxaxol-2-yl |
| Q736 | cyclopropyl | 5-(2-methoxyphenyl)thiazol-2-yl |
| Q737 | cyclopropyl | 5-(3-methoxyphenyl)thiazol-2-yl |
| Q738 | cyclopropyl | 5-(4-fluorophenyl)thiazol-2-yl |
| Q739 | cyclopropyl | 5-(2,4-difluorophenyl)thiazol-2-yl |
| Q740 | cyclopropyl | 5-(3-methoxybenzyl)thiazol-2-yl |
| Q741 | cyclopropyl | 4-(3-methoxyphenyl)thiazol-2-yl |
| Q742 | cyclopropyl | 4-(4-fluoruphenyl)thiazol-2-yl |

Compounds according to the first type of Category IX which comprise a substituted or unsubstituted thiazol-4-yl unit for R¹ can be prepared by the procedure outlined in Scheme XVII and described herein below in Example 18.

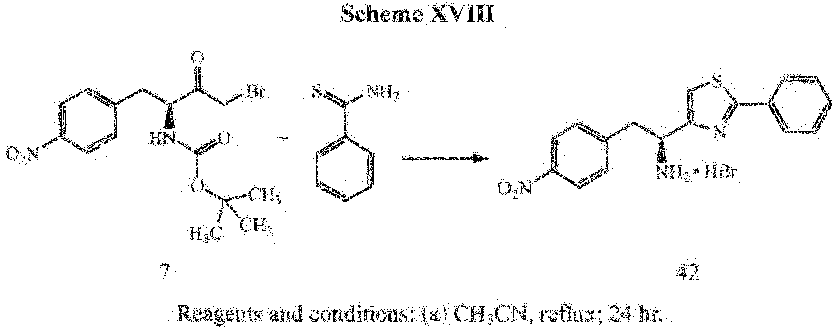

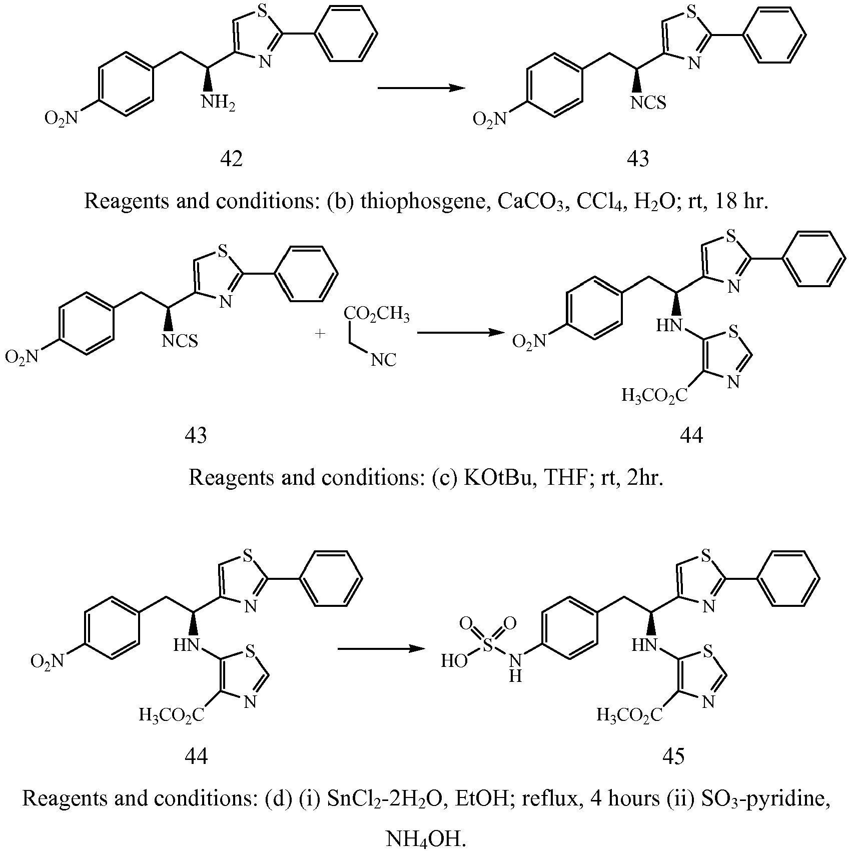

### EXAMPLE 18

### (S)-4-(2-(2-Phenylthiazol-4-yl)2-(4-(methoxycarbonyl)thiazole-5-ylamino)ethyl)phenylsulfamic acid (45)

Preparation of (*S*)-2-(4-nitrophenyl)-1-(2-phenylthiazol-4-yl)ethanamine hydrobromide salt (42): A mixture of (*S*)-*tert*-butyl 4-bromo-1-(4-nitrophenyl)-3-oxobutan-2-ylcarbamate, 7, (1.62 g, 4.17 mmol) and thiobenzamide (0.63 g, 4.60 mmol) in CH₃CN (5 mL) is refluxed for 24 hours. The reaction mixture is cooled to room temperature and diethyl ether (50 mL) is added to the solution. The precipitate which forms is collected by filtration. The solid is dried under vacuum to afford 1.2 g (67 % yield) of the desired product. LC/MS ESI+ 326 (M+1).

Preparation of (*S*)-4-(1-isothiocyanato-2-(4-nitrophenyl)ethyl)-2-phenylthiazole (43): To a solution of (*S*)-2-(4-nitrophenyl)-1-(2-phenylthiazol-4-yl)ethanamine hydrobromide salt, 42, (726 mg, 1.79 mmol) and CaCO₃ (716 mg, 7.16 mmol) in H₂O (2 mL) is added CCl₄ (3 mL) followed by thiophosgene (0.28 mL, 3.58 mmol). The reaction is stirred at room temperature for 18 hours then diluted with CH₂Cl₂ and water. The layers are separated and the aqueous layer extracted with CH₂Cl₂. The combined organic layers are washed with brine, dried (Na₂SO₄) and concentrated *in vacuo* to a residue which is purified over silica (CH₂Cl₂) to afford 480 mg (73 %) of the desired product as a yellow solid. ¹H NMR (300 MHz, CDCl₃) δ 8.15 (d, *J* = 8.7 Hz, 2H), 7.97-7.99 (m, 2H), 7.43-7.50 (m, 3H), 7.34 (d, *J* = 8.7 Hz, 2H), 7.15 (d, *J* = 0.9 Hz, 1H), 5.40-5.95 (m, 1H), 3.60 (dd, *J* = 13.8 and 6.0 Hz, 1H), 3.46 (dd, *J* = 13.8 and 6.0 Hz).

Preparation of (*S*)-methyl 5-[1-(2-phenylthiazol-4-yl)-2-(4-nitrophenyl)-ethylamino]thiazole-4-carboxylate (44): To a suspension of potassium *tert*-butoxide (89 mg, 0.75 mmol) in THF (3 mL) is added methyl isocyanoacetate (65 µL, 0.68 mmol) followed by (*S*)-2-phenyl-4-(1-isothiocyanato-2-(4-nitrophenyl)ethyl)thiazole, 43, (250 mg, 0.68 mmol). The reaction mixture is stirred at room temperature for 2 hours then poured into sat. NaHCO₃. The mixture is extracted with EtOAc (3x 25 mL) and the combined organic layers are washed with brine and dried (Na₂SO₄) and concentrated *in vacuo.* The crude residue is purified over silica to afford 323 mg (∼ 100% yield) of the desired product as a slightly yellow solid. ¹H NMR (300 MHz, CDCl₃) δ 8.09-8.13 (m, 2H), 7.95-7 98 (m, 3H), 7.84 (d, *J* = 1.2 Hz, 1H), 7.44-7.50 (m, 3H), 7.28-7.3 1(m, 2H), 7.96 (d, *J* = 0.6 Hz, 1H), 4.71-4.78(m, 1H), 3.92 (s, 3H), 3.60 (dd, *J* = 13.8 and 6.0 Hz, 1H), 3.45 (dd, *J* = 13.8 and 6.0 Hz, 1H).

Preparation of (*S*)-4-(2-(2-phenylthiazol-4-yl)2-(4-(methoxycarbonyl)thiazole-5-ylamino)ethyl)phenylsulfamic acid (45): (*S*)-methyl 5-[1-(2-phenylthiazol-4-yl)-2-(4-nitrophenyl)-ethylamino]thiazole-4-carboxylate, 44, (323 mg, 0.68 mmol) and tin (II) chloride (612 mg, 2.72 mmol) are dissolved in EtOH and the solution is brought to reflux. The solvent is removed *in vacuo* and the resulting residue is dissolved in EtOAc. A saturated solution of NaHCO₃ is added and the solution is stirred 1 hour. The organic layer is separated and the aqueous layer extracted twice with EtOAc. The combined organic layers are dried (Na₂SO₄), filtered and concentrated to a residue which is dissolved in pyridine (10 mL) and treated with SO₃-pyridine (130 mg, 0.82 mmol). The reaction is stirred at room temperature for 5 minutes after which a 7% solution of NHaOH is added. The mixture is then concentrated and the resulting residue is purified by reverse phase chromatography to afford 0.071g of the desired product as the ammonium salt ¹H NMR
(300 MHz, MeOH-d₄) δ 7.97-8.00 (m, 3H), 7.48-7.52 (m, 3H), 7.22 (s, 1H), 7.03-7.13 (m, 4H), 4.74 (t, *J* = 6.6 Hz, 1H), 3.88 (s, 3H), 3.28-3.42 (m, 2H).

Compounds according to the first type of Category IX which comprise a substituted or unsubstituted thiazol-2-yl unit for R¹ can be prepared by the procedure outlined in Scheme XVIII and described herein below in Example 19. Intermediate 46 can be prepared according to Scheme II and Example 2 by substituting cyclopropane-carbothioic acid amide for thiophen-2-carbothioic acid amide.

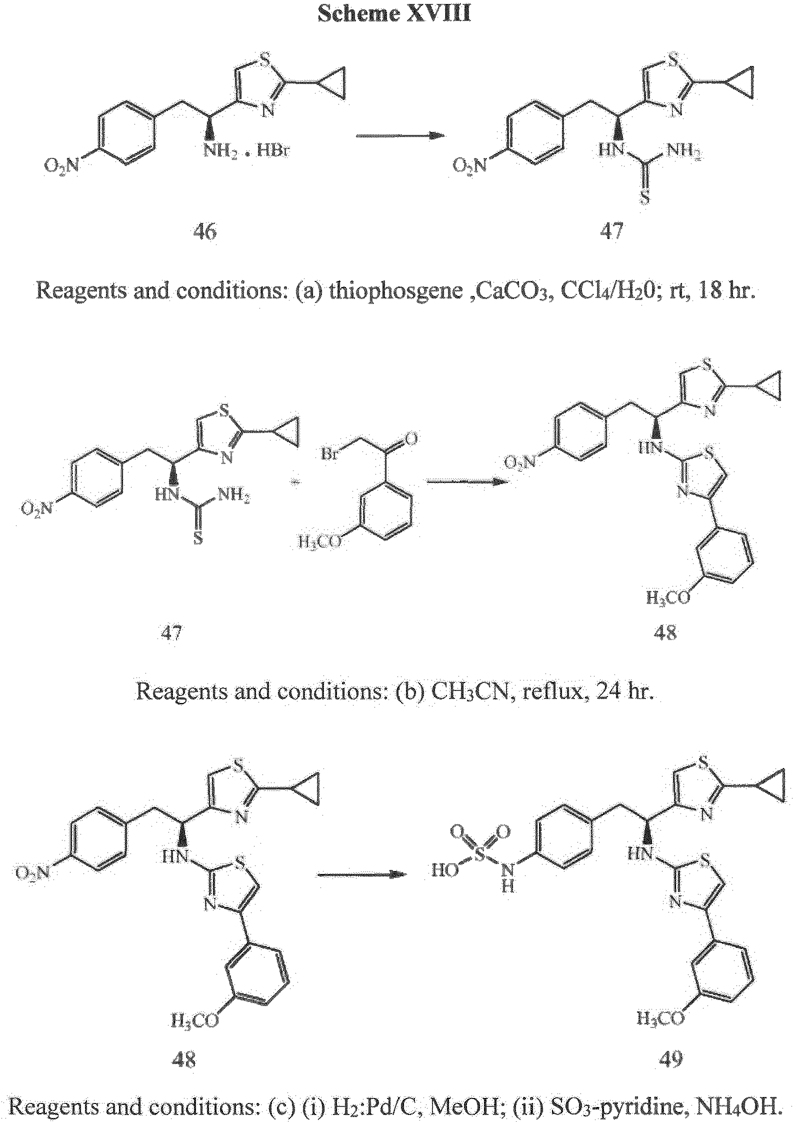

### EXAMPLE 19

### 4-{(S)-2-(2-Cyclopropylthiazol-4-yl)-2-[4-(3-methoxyphenyl)thiazol-2-ylamino]ethyl}phenylsulfamic acid (50)

Preparation of (S)-1-(1-(2-cyclopropylthiazol-4-yl)-2-(4-nitrophenyl)ethyl)-thiourea (47): To a solution of (S)-1-(2-cyclopropylthiazol-4-yl)-2-(4-nitrophenyl)ethan-amine hydrobromide hydrobromide salt, 32, (4.04 g, 10.9 mmol) and CaCO₃ (2.18 g, 21.8 mmol) in CCl₄/water (25 mL/20 mL) is added thiophosgene (1.5 g, 13.1 mmol). The reaction is stirred at room temperature for 18 hours then diluted with CH₂Cl₂ and water. The layers are separated and the aqueous layer extracted with CH₂Cl₂. The combined organic layers are washed with brine, dried (Na₂SO₄) and concentrated *in vacuo* to a residue which is subsequently treated with ammonia (0.5M in 1,4-dioxane, 120 mL) which is purified over silica to afford 2.90 g of the desired product as a red-brown solid. LC/MS ESI- 347 (M-1).

Preparation of (*S*)-4-(3-methoxybenzyl)-N-(1-(2-cyclopropylthiazol-4-yl)-2-(4-nitrophenyl)ethyl)thiazol-2-amine (48): (*S*)-1-(1-(2-Cyclopropylthiazol-4-yl)-2-(4-nitrophenyl)ethyl)-thiourea, 47, (350 mg, 1.00 mmol) and 2-bromo-3'-methoxyacetophenone (253 mg, 1.10 mmol) are combined in 3 mL CH3CN and heated to reflux for 24 hours. The mixture is concentrated and chromatographed to afford 0.172 g of the product as a yellow solid. LC/MS ESI+ 479 (M+1).

Preparation of 4- {(S)-2-(2-cyclopropylthiazol-4-yl)-2-[4-(3-methoxyphenyl)-thiazol-2-ylamino]ethyl}phenylsulfamic acid (49): (S)-4-(3-methoxybenzyl)-*N*-(1-(2-cyclopropylthiazol-4-yl)-2-(4-nitrophenyl)ethyl)thiazol-2-amine, 48, (0.172 g) is dissolved in 10 mL MeOH. A catalytic amount of Pd/C (10% w/w) is added and the mixture is stirred under a hydrogen atmosphere for 18 hours. The reaction mixture is filtered through a bed of CELITE™ and the solvent is removed under reduced pressure. The crude product is dissolved in 5 mL pyridine and treated with SO₃-pyridine (1 14 mg). The reaction is stirred at room temperature for 5 minutes after which 10 mL of a 7% solution of NHaOH is added. The mixture is then concentrated and the resulting residue is purified by reverse-phase chromatography to afford 0.033 g of the desired product as the ammonium salt. ¹H NMR (CD₃OD): δ 7.33-7.22 (m, 3H), 7.10-6.97 (m, 5H), 6.84-6.80 (m, 2H), 5.02 (t, 1H, *J*=6.9 Hz), 3.82 (s, 1H), 3.18 (q, 2H, *J*=7.1 Hz), 2.36 (q, 1H, *J*=4.6 Hz), 1.20-1.13 (m, 2H), 1.04-0.99 (m, 2H).

The following are non-limiting examples of compounds encompassed within the first type of Category IX.

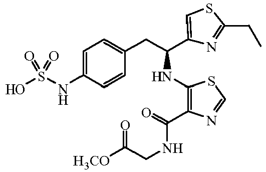

(*S*)-4-(2-(4-((2-Methoxy-2-oxoethyl)carbamoyl)thiazole-5-ylamino)2-(2-ethylthiazole-4-yl)ethyl)phenylsulfamic acid: ¹H NMR (300 MHz, MeOH-d₄) δ 7.91 (s, 1H), 7.08-7.10 (m, 3H), 6.99 (d, *J* = 8.7 Hz, 2H), 4.58 (t, *J* = 6.9 Hz, 1H), 4.11 (d, *J* = 2.7 Hz, 2H), 3.78 (s, 3H), 3.14-3.28 (m, 2H), 3.06 (q, *J* = 7.5 Hz, 2H), 1.41 (t, *J* = 7.5 Hz, 3H).

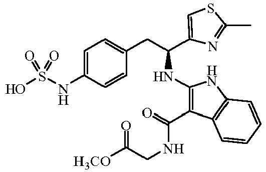

(*S*)-4-(2-{5-[1-*N*-(2-Methoxy-2-oxoethylcarbamoyl)-1-*H*-indol-3-yl]oxazol-2-ylamino}-2-(2-methylthiazol-4-yl)ethyl)phenylsulfamic acid: ¹H NMR (300 MHz, MeOH-d₄) δ 7.63 (d, *J* = 7.8 Hz, 1H), 7.37 (s, 1H), 7.18-7.29 (m, 4H), 7.02-7.16 (m, 4H), 6.85 (s, 1H), 5.04-5.09 (m, 1H), 4.85 (s, 3H), 3.27 (dd, *J* = 13.5 and 8.1 Hz, 1H), 3.10 (m, *J* = 13.5 and 8.1 Hz, 1H), 2.69 (s, 3H).

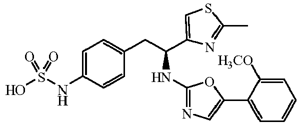

4-((S)-2-(5-(2-Methoxyphenyl)oxazol-2-ylamino)-2-(2-methylthiazol-4-yl)ethyl)phenylsulfamic acid: ¹H NMR (300 MHz, MeOH-d₄) δ 7.52 (dd, *J* = 7.5 and 1.2 Hz, 1H), 6.95-7.24 (m, 10H), 5.04-5.09 (m, 1H), 3.92 (s, 3H), 3.26 (dd, *J* = 13.8 and 8.4 Hz, 1H), 3.10 (dd, *J* = 13.8 and 8.4 Hz, 1H), 2.72 (s, 3H).

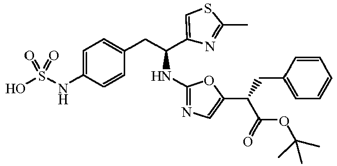

4-((*S*)-2-(5-((*S*)-1-(*tert*-Butoxycarbonyl)-2-phenylethyl)oxazole-2-ylamino)-2-(2-methylthiazole-4-yl)ethyl)phenylsulfamic acid: ¹H NMR (300 MHz, MeOH-d₄) δ 7.03-7.27 (m, 10 H), 6.50 (s, 1H), 4.95-5.00 (m, 1H), 4.76 (t, *J* = 6.9 Hz, 1H), 3.22 (dd, *J* = 14.1 and 6.9 Hz, 1H), 3.00-3.10 (m, 2H), 2.90 (dd, *J* = 14.1 and 6.9 Hz, 1H), 2.72 (s, 3H), 1.37 (s, 9H).

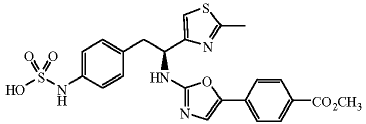

(*S*)-{4-{2-[5-(4-Methoxycarbonyl)phenyl]oxazol-2-ylamino}-2-(2-methylthiazol-4-yl)ethyl}phenylsulfamic acid: ¹H NMR (300 MHz, MeOH-d₄) δ 7.99 (d, *J* = 7.5 Hz, 2H), 7.56-7.59 (m, 2H), 7.23-7.24 (m, 1H), 7.08-7.14 (m, 4H), 6.83 (d, *J* = 10.2 Hz, 1H), 5.08 (t, J = 6.0 Hz, 1H), 3.91 (s, 3H), 3.25-3.35 (m, 1H), 3.09-3.13 (m, 1H), 2.73 (s, 3H).

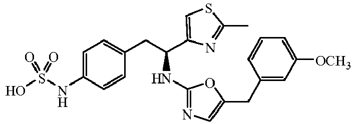

(*S*)-4-(2-(5-(3-Methoxybenzyl)oxazole-2-ylamino)-2-(2-methylthiazole-4-yl)ethyl)phenylsulfamic acid: ¹H NMR (300 MHz, MeOH-d₄) δ 7.03-7.28 (m, 8H), 6.79-6.83 (m, 1H), 5.70 (s, 1H), 4.99-5.06 (m, 2H), 4.41 (d, *J* = 2.1 Hz, 2H), 3.80 (s, 3H), 3.27-3.37 (m, 1H), 3.03-3.15 (m, 1H), 2.71 (s, 3H).

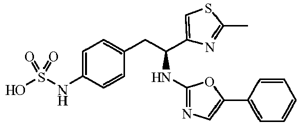

(*S*)-4-(2-(2-Methylthiazole-4-yl)2-(5-phenyloxazole-2-ylamino)ethyl)phenyl-sulfamic acid: ¹H NMR (300 MHz, MeOH-d₄) δ 7.45 (d, J = 8.7 Hz, 2H), 7.33 (t, *J* = 7.8 Hz, 2H), 7.18-7.22 (m, 1H), 7.10-7.14 (m, 6H), 7.04 (s, 1H), 5.04-5.09 (m, 1H), 3.26 (dd, *J* = 13.8 and 6.3 Hz, 1H), 3.10 (dd, *J* = 13.8 and 6.3 Hz, 1H), 2.70 (s, 3H).

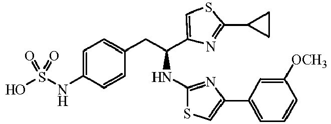

4-((S)-2-(2-Cyclopropylthiazol-4-yl)-2-(4-(3-methoxyphenyl)thiazol-2-ylamino)-ethyl)phenylsulfamic acid: ¹H NMR (CD₃OD): δ 7.33-7.22 (m, 3H), 7.10-6.97 (m, 5H), 6.84-6.80 (m, 2H), 5.02 (t, 1H, *J*=6.9 Hz), 3.82 (s, 1H), 3.18 (q, 2H, *J*=7.1 Hz), 2.36 (q, 1H, *J*=4.6 Hz), 1.20-1.13 (m, 2H), 1.04-0.99 (m, 2H).

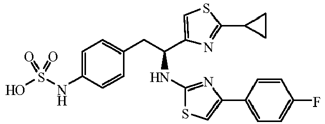

(*S*)-4-(2-(2-cyclopropylthiazol-4-yl)-2-(4-(4-fluorophenyl)thiazol-2-ylamino)ethyl)-phenylsulfamic acid: ¹H NMR (CD₃OD): δ 7.79-7.74 (m, 2H), 7.14-7.03 (m, 7H), 7.21 (s, 1H), 6.79 (s, 1H), 5.08 (t, 1H, *J*=6.6 Hz), 3.29-3.12 (m, 2H), 2.40 (q, 2.40, *J*=5.1 Hz), 1.23-1.18 (m, 2H), 1.08-1.02 (m, 2H).

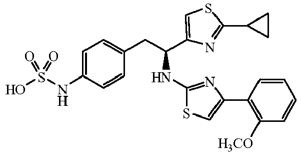

4-((*S*)-2-(2-cyclopropylthiazol-4-yl)-2-(4-(2-methoxyphenyl)thiazol-2-ylamino)-ethyl)phenylsulfamic acid: ¹H NMR (CD₃OD): δ 7.89-7.87 (d, 1H, *J*=7.6 Hz), 7.28 (t, 1H, *J*=7.0 Hz), 7.10-6.96 (m, 8H), 5.03 (t, 1H, *J*=6.9 Hz), 3.90 (s, 1H), 3.19 (q, 2H, *J*=6.6 Hz), 2.38 (q, 1H, *J*=4.8 Hz), 1.21-1.14 (m, 2H), 1.06-1.00 (m, 2H).

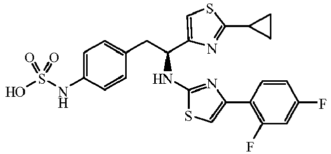

4-((*S*)-2-(2-cyclopropylthiazol-4-yl)-2-(4-(2,4-difluorophenyl)thiazol-2-ylamino)-ethyl)phenylsulfamic acid: ¹H NMR (CD₃OD): δ 8.06-8.02 (q, 2H, *J*=6.9 Hz), 7.12-6.95 (m, 7H), 6.88 (s, 1H), 5.11 (t, 1H, *J*=6.9 Hz), 3.22-3.15 (m, 2H), 2.38 (q, 1H, *J*=4.8 Hz), 1.22-1.15 (m, 2H), 1.06-1.02 (m, 2H).

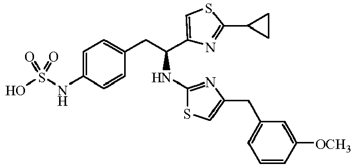

(*S*)-4-(2-(4-(3-methoxybenzyl)thiazol-2-ylamino)-2-(2-cyclopropylthiazol-4-yl)ethyl)phenylsulfamic acid: ¹H NMR (CD₃OD): δ 7.22-7.17 (m, 3H), 7.09-6.97 (m, 5H), 6.78-6.66 (m, 3H), 3.77 (s, 2H), 3.75 (s, 3H), 3.20-3.07 (m, 2H), 2.35 (q, 1H, *J*=4.8 Hz), 1.19-1.13 (m, 2H), 1.03-1.00 (m, 2H).

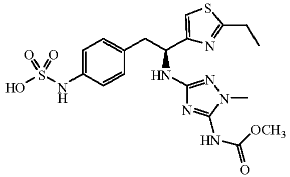

(*S*)- {5-[1-(2-Ethylthiazol-4-yl)-2-(4-sulfoaminophenyl)ethylamino]-2-methyl-2H-[1,2,4]triazole-3-yl}carbamic acid methyl ester: ¹H NMR (300 MHz, MeOH-d₄) δ 6.97-7.08 (m, 5H), 3.71 (s, 3H), 3.51 (s, 3H), 3.15 (dd, *J* = 13.5 and 6.3 Hz, 1H), 3.02-3.07 (m, 3H), 1.40 (t, *J* = 6.6 Hz, 3H).

The second type of Category V of the present disclosure relates to compounds having the formula: 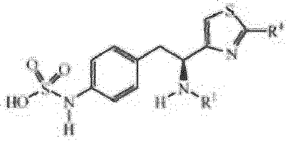 wherein R¹ is a substituted or unsubstituted heteroaryl and R⁴ is substituted or unsubstituted phenyl and substituted or unsubstituted heteroaryl as further described herein below in Table XVIII.

**TABLE XVIII**

| **No.** | **R⁴** | **R¹** |
|---|---|---|
| R743 | phenyl | 4-(methoxycarbonyl)thiazol-5-yl |
| R744 | phenyl | 4-[(2-methoxy-2-oxoethyl)carbamoyl]thiazol-5-yl |
| R745 | phenyl | 5-[1-*N*-(2-methoxy-2-oxoethyl)-1-*H*-indol-3-yl]oxazol-2-yl |
| R746 | phenyl | 5-(2-methoxyphenyl)oxazol-2-yl |
| R747 | phenyl | 5-[(*S*)-1-(*tert*-butoxycarbonyl)-2-phenylethyl]oxazol-2-yl |
| R748 | phenyl | 5-[4-(methylcarboxy)phenyl]oxazol-2-yl |
| R749 | phenyl | 5-(3-methoxybenzyl)oxazol-2-yl |
| R750 | phenyl | 5-(4-phenyl)oxazol-2-yl |
| R751 | phenyl | 5-(2-methoxyphenyl)thiazol-2-yl |
| R752 | phenyl | 5-(3-methoxyphenyl)thiazol-2-yl |
| R753 | phenyl | 5-(4-fluorophenyl)thiazol-2-yl |
| R754 | phenyl | 5-(2,4-difluorophenyl)thiazol-2-yl |
| R755 | phenyl | 5-(3-methoxybenzyl)thiazol-2-yl |
| R756 | phenyl | 4-(3-methoxyphenyl)thiazol-2-yl |
| R757 | phenyl | 4-(4-fluorophenyl)thiazol-2-yl |
| R758 | thiophen-2-yl | 4-(methoxycarbonyl)thiazol-3-yl |
| R759 | thiophen-2-yl | 4-[(2-methoxy-2-oxoethyl)carbamoyl]thiazol-5-yl |
| R760 | thiophen-2-yl | 5-[1-*N*-(2-methoxy-2-oxoethyl)-1-*H*-indol-3--yl]oxazol-2-yl |
| R761 | thiophen-2-yl | 5-(2-methoxyphenyl)oxazol-2-yl |
| R762 | thiophen-2-yl | 5-[(*S*)-1-(*tert*-butoxycarbonyl)-2-phenylethyl]oxazol-2-yl |
| R763 | thiophen-2-yl | 5-[4-(methylcarboxy)phenyl]oxazol-2-yl |
| R764 | thiophen-2-yl | 5-(3-methoxybenzyl)oxazol-2-yl |
| R765 | thiophen-2-yl | 5-(4-phenyl)oxazol-2-yl |
| R766 | thiophen-2-yl | 5-(2-methoxyphenyl)thiazol-2-yl |
| R767 | thiophen-2-yl | 5-(3-methoxyphenyl)thiazol-2-yl |
| R768 | thiophen-2-yl | 5-(4-fluorophenyl)thiazol-2-yl |
| R769 | thiophen-2-yl | 5-(2,4-difluorophenyl)thiazol-2-yl |
| R770 | thiophen-2-yl | 5-(3-methoxybenzyl)thiazol-2-yl |
| R771 | thiophen-2-yl | 4-(3-methoxyphenyl)thiazol-2-yl |
| R772 | thiophen-2-yl | 4-(4-fluorophenyl)thiazol-2-yl |
| R773 | cyclopropyl | 4-(methoxycarbonyl)thiazol-5-yl |
| R774 | cyclopropyl | 4-[(2-methoxy-2-oxoethyl)carbamoyl]thiazol-5-yl |
| R775 | cyclopropyl | 5-[1-*N*-(2-methoxy-2-oxoethyl)-1-*H*-indol-3-yl]oxazol-2-yl |
| R776 | cyclopropyl | 5-(2-methoxyphenyl)oxazol-2-yl |
| R777 | cyclopropyl | 5-[(*S*)-1-(*tert*-butoxycarbonyl)-2-phenylethyl]oxazol-2-yl |
| R778 | cyclopropyl | 5-[4-(methylcarboxy)phenyl]oxazol-2-yl |
| R779 | cyclopropyl | 5-(3-methoxybenzyl)oxazol-2-yl |
| R780 | cyclopropyl | 5-(4-phenyl)oxazol-2-yl |
| R781 | cyclopropyl | 5-(2-methoxyphenyl)thiazol-2-yl |
| R782 | cyclopropyl | 5-(3-methoxyphenyl)thiazol-2-yl |
| R783 | cyclopropyl | 5-(4-fluorophenyl)thiazol-2-yl |
| R784 | cyclopropyl | 5-(2,4-difluorophenyl)thiazol-2-yl |
| R785 | cyclopropyl | 5-(3-methoxybenzyl)thiazol-2-yl |
| R786 | cyclopropyl | 4-(3-methoxyphenyl)thiazol-2-yl |
| R781 | cyclopropyl | 4-(4-fluorophenyl)thiazol-2-yl |

Compounds according to the second type of Category IX which comprise a substituted or unsubstituted thiazol-4-yl unit for R¹ can be prepared by the procedure outlined in Schemes XIX, XX, and XXI and described herein below in Examples 20, 21, and 22.

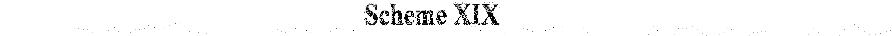

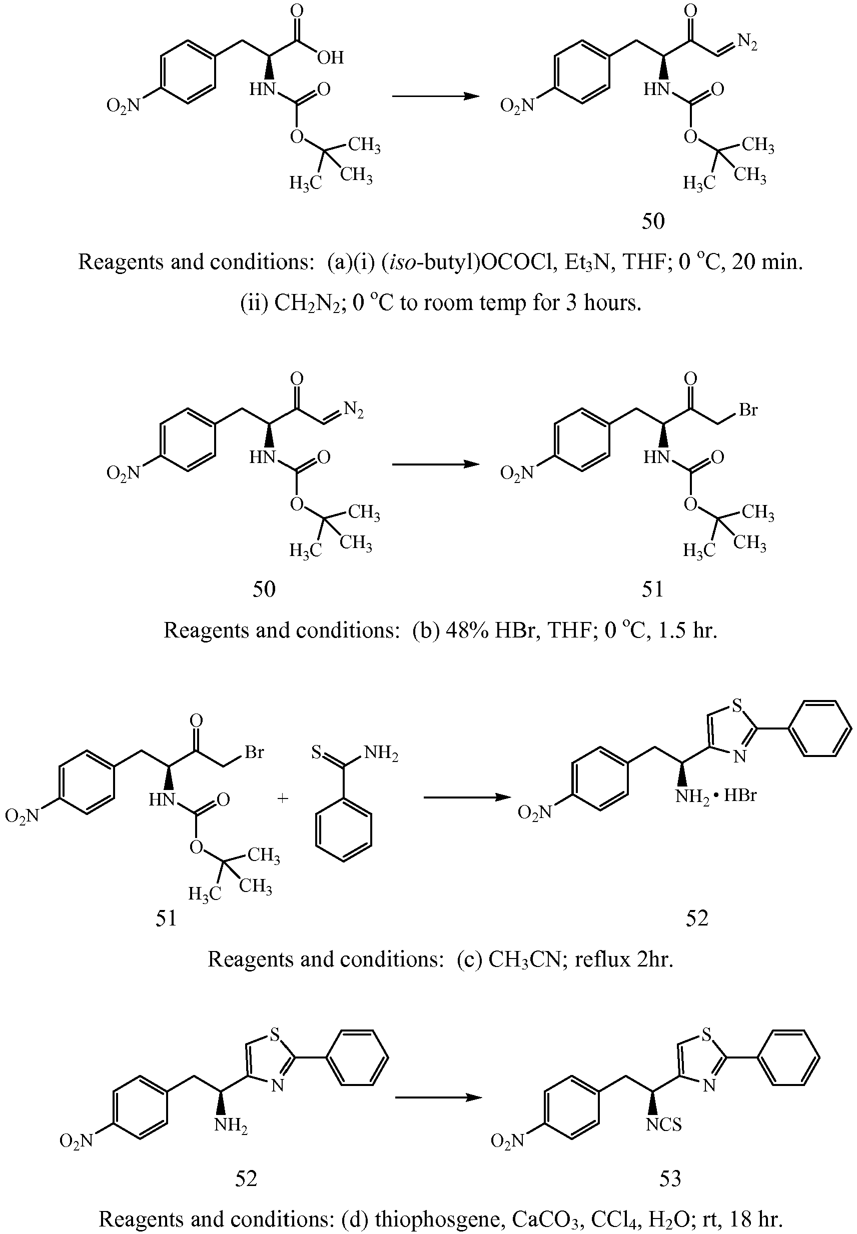

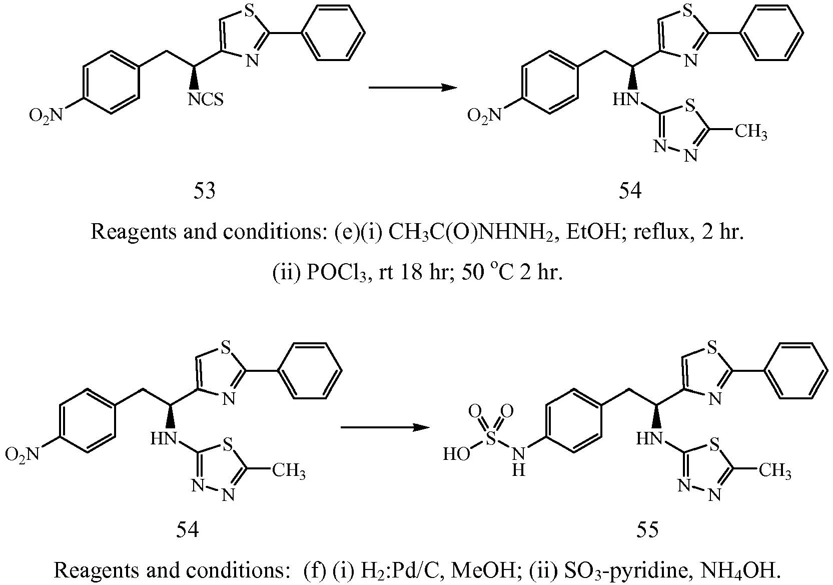

### EXAMPLE 20

### (S)-4-(2-(5-Methyl-1,3,4-thiadiazol-2-ylamino)-2-(2-phenylthiazol-4-yl)ethyl)phenylsulfamic acid (55)

Preparation of [3-diazo-1-(4-nitrobenzyl)-2-oxo-propyl]-carbamic acid *tert-*butyl ester (50): To a 0 °C solution of 2-(*S*)-*tert*-butoxycarbonylamino-3-(4-nitrophenyl)-propionic acid (1.20 g, 4.0 mmol) in THF (20 mL) is added dropwise triethylamine (0.61 mL, 4.4 mmol) followed by *iso*-butyl chloroformate (0.57 mL, 4.4 mmol). The reaction mixture is stirred at 0 °C for 20 minutes then filtered. The filtrate is treated with an ether solution of diazomethane (∼16 mmol) at 0 °C. The reaction mixture is stirred at room temperature for 3 hours and concentrated. The residue is dissolved in EtOAc and washed successively with water and brine, dried (Na₂SO₄), filtered and concentrated *in vacuo.* The resulting residue is purified over silica (hexane/EtOAc 2:1) to afford 1.1 g (82% yield) of the desired product as a slightly yellow solid. ¹H NMR (300 MHz, CDCl₃) δ 8.16 (d, *J* = 8.7 Hz, 2H), 7.39 (d, *J* = 8.7 Hz, 2H), 5.39 (s, 1H), 5.16 (d, *J* = 6.3 Hz, 1H), 4.49 (s, 1H), 3.25 (dd, *J* = 13.8 and 6.6, 1H), 3.06 (dd, *J* = 13.5 and 6.9 Hz, 1H), 1.41 (s, 9H).

Preparation of [3-bromo-1-(4-nitro-benzyl)-2-oxo-propyl]-carbamic acid *tert-*butyl ester (51): To a 0 °C solution of [3-diazo-1-(4-nitrobenzyl)-2-oxo-propyl]-carbamic acid *tert-*butyl ester, 50, (0.350 g, 1.04 mmol) in THF (5 mL) is added dropwise 48% aq. HBr (0.14 mL, 1.25 mmol). The reaction mixture is stirred at 0 °C for 1.5 hours and quenched at 0 °C with saturated aqueous Na₂CO₃. The mixture is extracted with EtOAc (3 x 25 mL) and the combined organic extracts are washed with brine, dried (Na₂SO₄), filtered and concentrated *in vacuo* to afford 0.400 g of the desired product that is used in the next step without further purification. ¹H NMR (300 MHz, CDCl₃) δ 8.20 (d, *J* = 8.4 Hz, 2H), 7.39 (d, *J* = 8.4 Hz, 2H), 5.06 (d, *J* = 7.8 Hz, 1H), 4.80 (q, *J* = 6.3 Hz, 1H), 4.04 (s, 2H), 1.42 (s, 9H).

Preparation of (*S*)-2-(4-nitrophenyl)-1-(2-phenylthiazol-4-yl)ethanamine hydrobromide salt (52): A mixture of [3-bromo-1-(4-nitro-benzyl)-2-oxo-propyl]-carbamic acid *tert-*butyl ester, 51, (1.62 g, 4.17 mmol) and benzothioamide (0.630 g, 4.59 mmol), in CH₃CN (5 mL) is refluxed for 24 hours. The reaction mixture is cooled to room temperature and diethyl ether (50 mL) is added to the solution and the precipitate that forms is collected by filtration. The solid is dried under vacuum to afford 1.059 g (63%) of the desired product. ESI+MS 326 (M+1).

Preparation of (*S*)-4-[1-isothiocyanato-2-(4-nitrophenyl)-ethyl]-2-phenylthiazole (53): To a solution of (*S*)-2-(4-nitrophenyl)-1-(2-phenylthiazol-4-yl)ethanamine hydrobromide salt, 52, (2.03g, 5 mmol) and CaCO₃ (1 g, 10 mmol) in CCl₄/water (10:7.5 mL) is added thiophosgene (0.46 mL, 6 mmol). The reaction is stirred at room temperature for 18 hours then diluted with CH₂Cl₂ and water. The layers are separated and the aqueous layer extracted with CH₂Cl₂. The combined organic layers are washed with brine, dried (Na₂SO₄) and concentrated *in vacuo* to a residue that is purified over silica (CH₂Cl₂) to afford 1.71g (93% yield) of the desired product. ESI+ MS 368 (M+1).

Preparation of (*S*)-5-methyl-*N*-[2-(4-nitrophenyl)-1-(2-phenylthiazol-4-yl)ethyl]-1,3,4-thiadiazol-2-amine (54): A solution of (*S*)-4-[1-isothiocyanato-2-(4-nitrophenyl)-ethyl]-2-phenylthiazole, 53, (332 mg, 0.876 mmol) and acetic hydrazide (65 mg, 0.876 mmol) in EtOH (5 mL) is refluxed for 2 hours. The solvent is removed under reduced pressure, the residue is dissolved in POCl₃ (3 mL) and the resulting solution is stirred at room temperature for 18 hours after which the solution is heated to 50 °C for 2 hours. The solvent is removed *in vacuo* and the residue is dissolved in EtOAc (40 mL) and the resulting solution is treated with IN NaOH until the pH remains approximately 8. The solution is extracted with EtOAc. The combined aqueous layers are washed with EtOAc, the organic layers combined, washed with brine, dried over MgSO₄, filtered, and concentrated *in vacuo* to afford 0.345 g (93% yield) of the desired product as a yellow solid. ¹H NMR (CDCl₃) 8.09 (d, J = 8.4 Hz, 2H), 7.91 (m, 2H), 7.46 (m, 4H), 7.44 (s, 1H), 5.23 (m, 1H), 3.59 (m, 2H), 2.49 (s, 3H). ESI+ MS 424 (M+1).

Preparation of (*S*)-4-[2-(5-methyl-1,3,4-thiadiazol-2-ylamino)-2-(2-phenylthiazol-4-yl)ethyl]phenylsulfamic acid (55): (*S*)-5-Methyl-N-[2-(4-nitrophenyl)-1-(2-phenylthiazol-4-yl)ethyl]-1,3,4-thiadiazol-2-amine, 54, (0.404 g, 0.954 mmol) is dissolved in MeOH (5 mL). Pd/C (50 mg, 10% w/w) is added and the mixture is stirred under a hydrogen atmosphere until the reaction is judged to be complete. The reaction mixture is filtered through a bed of CELITE™ and the solvent removed under reduced pressure. The crude product is dissolved in pyridine (4 mL) and treated with SO₃-pyridine (0.304 g, 1.91 mmol). The reaction is stirred at room temperature for 5 minutes after which a 7% solution of NH₄OH (50 mL) is added. The mixture is then concentrated and the resulting residue is purified by reverse phase preparative HPLC to afford 0.052 g (11% yield) of the desired product as the ammonium salt. ¹H NMR (CD₃OD): δ 8.00-7.97 (m, 2H), 7.51-7.47 (m, 3H), 7.23 (s, 1H), 7.11-7.04 (q, 4H, *J*=9.0 Hz), 5.18 (t, 1H, *J*=7.2 Hz), 3.34-3.22 (m, 2H), 2.50 (s, 3H). ESI- MS 472 (M-1).

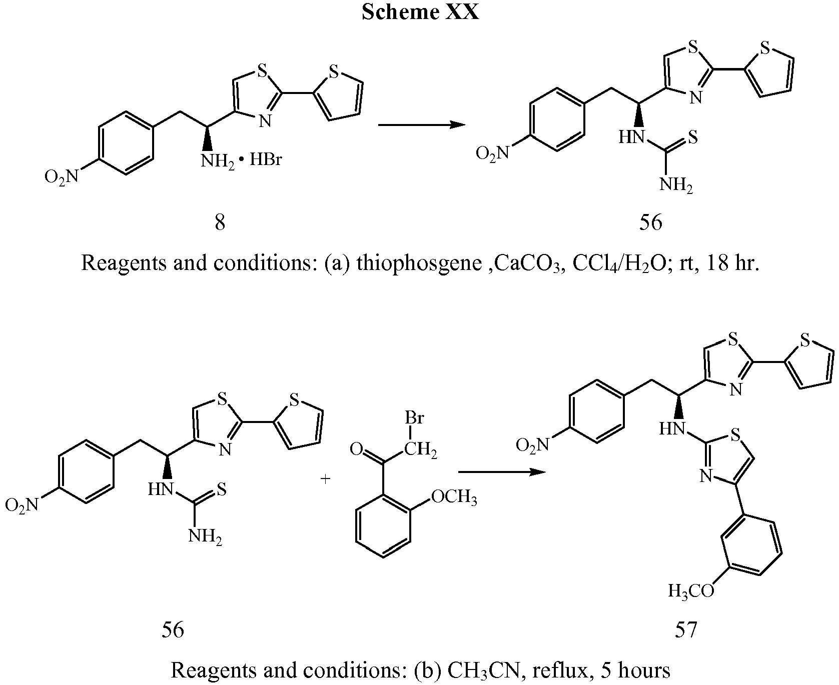

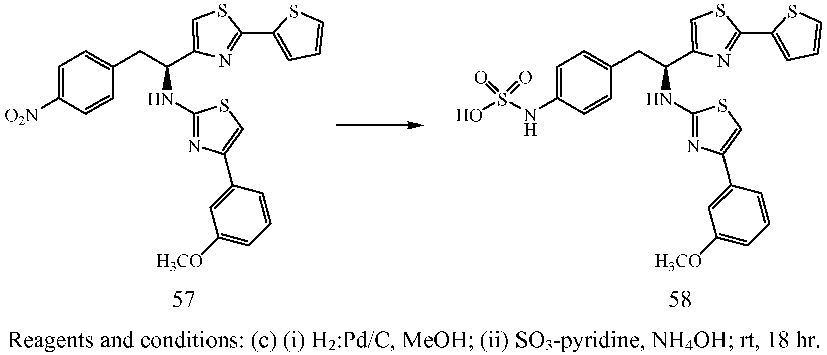

### EXAMPLE 21

### 4-{(S)-2-[4-(2-Methoxyphenyl)thiazol-2-ylamino)-2-[2-(thiophen-2-yl)thiazol-4-yl]ethyl}phenylsulfamic acid (58)

Preparation of (*S*)-1-[1-(thiophen-2-ylthiazol-4-yl)-2-(4-nitrophenyl)ethyl]-thiourea (56): To a solution of (*S*)-2-(4-nitrophenyl)-1-(thiophen-2-ylthiazol-4-yl)ethanamine hydrobromide salt, 8, (1.23 g, 2.98 mmol) and CaCO₃ (0.597 g, 5.96 mmol) in CCl₄/water (10 mL/5 mL) is added thiophosgene (0.412g, 3.58 mmol). The reaction is stirred at room temperature for 18 hours then diluted with CH₂Cl₂ and water. The layers are separated and the aqueous layer extracted with CH₂Cl₂. The combined organic layers are washed with brine, dried (Na₂SO₄) and concentrated *in vacuo* to a residue which is subsequently treated with ammonia (0.5M in 1,4-dioxane, 29.4 mL, 14.7 mmol) which is purified over silica to afford 0.490 g of the desired product as a red-brown solid. ESI+ MS 399 (M+1).

Preparation of 4-(2-methoxyphenyl)-*N*-{(S)-2-(4-nitrophenyl)-1-[2-(thiophen-2-yl)thiazol-4-yl]ethyl}thiazol-2-amine (57): (*S*)-1-[1-(thiophen-2-ylthiazol-4-yl)-2-(4-nitrophenyl)ethyl]-thiourea, 56, (265 mg, 0.679 mmol) is treated with bromo-2'-methoxyacetophenone (171 mg, 0.746 mmol) to afford 0.221 g of the product as a yellow solid. ESI+ MS 521 (M+1).

Preparation on 4- {(S)-2-[4-(2-methoxyphenyl)thiazol-2-ylamino)-2-[2-(thiophen-2-yl)thiazol-4-yl]ethyl}phenylsulfamic acid (58): 4-(2-methoxyphenyl)-*N*-{(S)-2-(4-nitrophenyl)-1-[2-(thiophen-2-yl)thiazol-4-yl]ethyl-4-thiazol-2-amine, 57, (0.229 g) is dissolved in 12 mL MeOH. A catalytic amount of Pd/C (10% w/w) is added and the mixture is stirred under a hydrogen atmosphere for 18 hours. The reaction mixture is filtered through a bed of CELITE™ and the solvent is removed under reduced pressure. The crude product is dissolved in 6 mL pyridine and treated with SO₃-pyridine (140 mg). The reaction is stirred at room temperature for 5 minutes after which 10 mL of a 7% solution of
NH₄OH is added. The mixture is then concentrated and the resulting residue is purified by reverse-phase chromatography to afford 0.033g of the desired product as the ammonium salt. ¹H NMR (CD₃OD): δ 7.96-7.93 (m, 1H), 7.60-7.55 (m, 2H), 7.29-7.23 (m, 1H), 7.18-6.95 (m, 9H), 5.15 (t, 1H, *J*=6.9 Hz), 3.90 (s, 3H), 3.35-3.24 (m, 2H).

Compounds according to the second type of Category IX compounds falling outside the scope of the invention comprise a substituted or unsubstituted oxazol-2-yl unit for R¹ can be prepared by the procedure outlined in Scheme XXI and described herein below in Example 22. Intermediate 39 can be prepared according to Scheme XVII and Example 18.

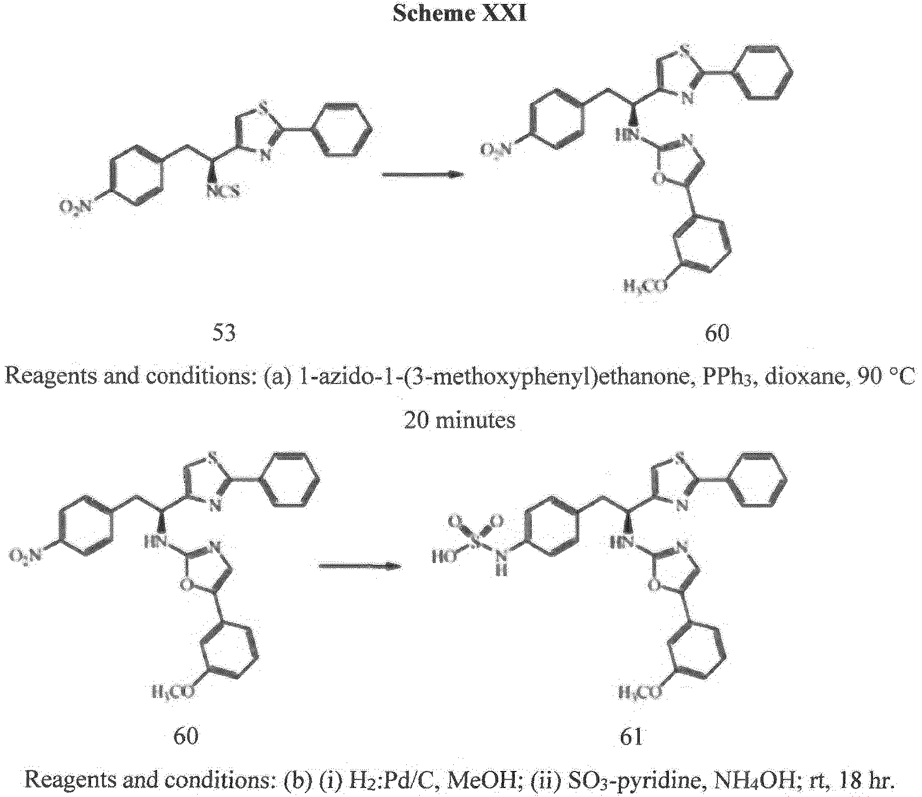

### EXAMPLE 22

### 4-[(S)-2-[5-(3-Methoxyphenyl)oxazole-2-ylamino]-2-(2-phenylthiazole-4-yl)ethyl}phenylsulfamic acid (61)

Preparation of [5-(3-methoxyphenyl)oxazol-2-yl]-[2-(4-nitrophenyl)-1-(2-phenylthiazole-4-yl) ethyl]amine (60): A mixture of (*S*)-4-(isothiocyanato-2-(4-nitrophenyl)ethyl)-2-phenylthiazole, 53, (300 mg, 0.81 mmol), 1-azido-1-(3-methoxyphenyl)ethanone (382 mg, 2.0 mmol) and PPh₃ (0.8 g, polymer bound, ∼3 mmol/g) in dioxane (6 mL) is heated at 90 °C for 20 minutes. The reaction solution is cooled to room temperature and the solvent removed in vacuo and the resulting residue is purified over silica to afford 300 mg (74% yield) of the desired product as a yellow solid. ¹H NMR (300 MHz, MeOH-d₄) δ 8.02 (d, J = 7.2 Hz, 2H), 7.92-7.99 (m, 2H), 7.42-7.47 (m, 3H), 7.22-7.27 (m, 3H), 6.69-7.03 (m, 4H), 6.75-6.78 (m, 1H), 5.26 (t, J = 6.3 Hz, 1H), 3.83 (s, 4H), 3.42-3.45 (m, 2H).

Preparation of 4- {(*S*)-2-[5-(3-methoxyphenyl)oxazole-2-ylamino]-2-(2-phenylthiazole-4-yl)ethyl}phenylsulfamic acid (61): [5-(3-methoxyphenyl)oxazol-2-yl]-[2-(4-nitrophenyl)-1-(2-phenylthiazole-4-yl) ethyl]amine, 60, (300 mg, 0.60 mmol) is dissolved in MeOH (15 mL). A catalytic amount of Pd/C (10% w/w) is added and the mixture is stirred under a hydrogen atmosphere 18 hours. The reaction mixture is filtered through a bed of CELITE™ and the solvent is removed under reduced pressure. The crude product is dissolved in pyridine (10 mL) and treated with SO₃-pyridine (190 mg, 1.2 mmol). The reaction is stirred at room temperature for 5 minutes after which a 7% solution of NH₄OH is added. The mixture is then concentrated and the resulting residue is purified by reverse-phase chromatography to afford 0.042 g of the desired product as the ammonium salt. ¹H NMR (300 MHz, MeOH-d₄) δ 7.99 (d, *J* = 7.5 Hz, 2H), 7.46-7.50 (m, 3H),7.23-7.29 (m, 3H), 7.04-7.12 (m, 6H), 6.78 (dd, *J* = 8.4 and 2.4 Hz, 1H), 5.16 (t, *J* = 6.6 Hz, 1H), 3.81 (s, 3H), 3.29-3.39 (m, 1H), 3.17 (dd, *J* = 13.8 and 8.1 Hz, 1H).

The following are non-limiting examples of the second type of Category IX of the present disclosure. 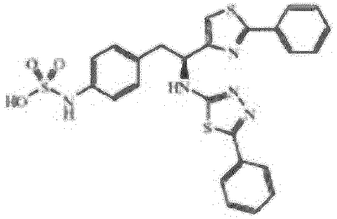 (*S*)-4-(2-(5-Phenyl-1,3,4-thiadiazol-2-ylamino)-2-(2-phenylthiazol-4-yl)ethyl)-phenylsulfamic acid: ¹H NMR (CD₃OD): δ 7.97-7.94 (m, 2H), 7.73-7.70 (m, 2H), 7.44- 7.39 (m, 6H), 7.25 (s, 1H), 7.12 (s, 4H), 5.29 (t, 1H, *J*=6.9 Hz), 3.35-3.26 (m, 2H).

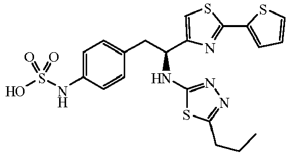

4-((*S*)-2-(5-Propyl-1,3,4-thiadiazol-2-ylamino)-2-(2-(thiophen-2-yl)thiazol-4-yl)ethyl)phenylsulfamic acid: ¹H NMR (CD₃OD): δ 7.59-7.54 (m, 2H), 7.17-7.03 (m, 6H), 5.13 (t, 1H, *J*=7.2 Hz), 3.32-3.13 (m, 2H), 2.81 (t, 2H, *J*=7.4 Hz), 1.76-1.63 (h, 6H, *J*=7.4 Hz), 0.97 (t, 3H, *J*=7.3 Hz).

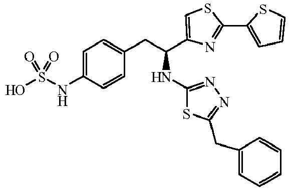

4-((*S*)-2-(5-Benzyl-1,3,4-thiadiazol-2-ylamino)-2-(2-(thiophen-2-yl)thiazol-4-yl)ethyl)phenylsulfamic acid: ¹H NMR (CD₃OD): δ (m, 2H), 7.49-7.45 (m, 2H), 7.26-7.16 (m, 5H), 7.05-6.94 (m, 6H), 5.04 (t, 1H, *J*=7.1 Hz), 4.07 (s, 2H), 3.22-3.04 (m, 2H).

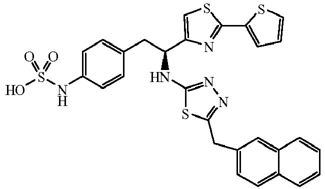

4-((*S*)-2-(5-(Naphthalen-1-ylmethyl)-1,3,4-thiadiazol-2-ylamino)-2-(2-(thiophen-2-yl)thiazol-4-yl)ethyl)phenylsulfamic acid: ¹H NMR (CD₃OD): δ 8.08-8.05 (m, 1H), 7.89-7.80 (m, 2H), 7.55-7.43 (m, 6H), 7.11-7.00 (m, 6H), 5.08 (t, 1H, *J*=7.1 Hz), 4.63 (s, 2H), 3.26-3.08 (m, 2H).

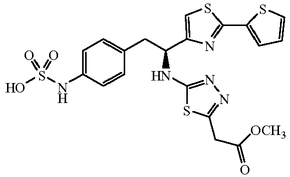

4-((*S*)-2-(5-((Methoxycarbonyl)methyl)-1,3,4-thiadiazol-2-ylamino)-2-(2-(thiophen-2-yl)thiazol-4-yl)ethyl)phenylsulfamic acid: ¹H NMR (CD₃OD): δ 7.48-7.44 (m, 2H), 7.03-6.92 (m, 6H), 5.02 (t, 1H, *J*=7.2 Hz), 4.30 (s, 2H), 3.55 (s, 3H), 3.22-3.02 (m, 2H).

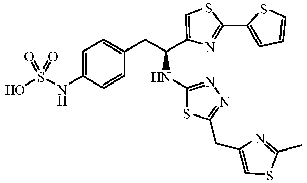

4-((*S*)-2-(5-((2-Methylthiazol-4-yl)methyl)-1,3,4-thiadiazol-2-ylamino)-2-(2-(thiophen-2-yl)thiazol-4-yl)ethyl)phenylsulfamic acid: ¹H NMR (CD₃OD): δ 7.60-7.56 (m, 2H), 7.19 (s, 1H), 7.15-7.12 (m, 2H), 7.09-7.03 (q, 4H, *J*=8.7 Hz), 5.14 (t, 1H, *J*=7.2 Hz), 4.28 (s, 2H), 3.33-3.14 (m, 2H), 2.67 (s, 3H).

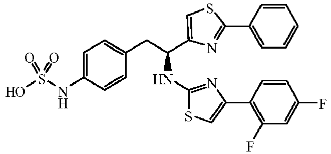

4-{(*S*)-2-[4-(2,4-Difluorophenyl)thiazol-2-ylamino]-2-[2-(thiophen-2-yl)thiazol-4-yl]ethyl}phenylsulfamic acid: ¹H NMR (CD₃OD): δ 8.06-8.02 (q, 1H, *J*=6.8 Hz), 7.59-7.54 (m, 2H), 7.16-7.08 (m, 6H), 7.01-6.88 (m, 4H), 5.20 (t, 1H, *J*=7.0 Hz), 3.36-3.17 (m, 2H).

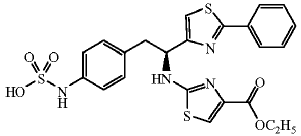

(*S*)-4-{2-[4-(Ethoxycarbonyl)thiazol-2-ylamino]-2-(2-phenylthiazol-4-yl)ethyl}phenylsulfamic acid: ¹H NMR (CD₃OD): δ 8.02-7.99 (m, 2H), 7.54-7.45 (m, 4H), 7.26 (s, 1H), 7.08 (s, 4H), 5.26 (t, 1H, *J*=6.9 Hz), 4.35-4.28 (q, 2H, J=6.9 Hz), 3.38-3.18 (m, 2H), 1.36 (t, 3H, J=7.2 Hz).

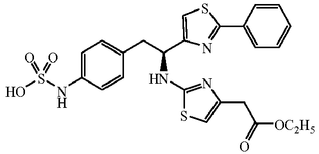

(*S*)-4-{2-[4-(2-Ethoxy-2-oxoethyl)thiazol-2-ylamino]-2-(2-phenylthiazol-4-yl)ethyl}phenylsulfamic acid: ¹H NMR (CD₃OD): δ 7.96 (m, 2H), 7.50-7.46 (m, 3H), 7.21 (s, 1H), 7.10-7.04 (m, 4H), 6.37 (s, 1H), 5.09 (t, 1H, *J*=6.9 Hz), 4.17-4.10 (q, 2H, J=7.1 Hz), 3.54 (s, 2H), 3.35-3.14 (m, 2H), 1.22 (t, 3H, J=7.1 Hz).

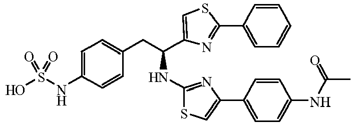

(*S*)-4-{2-[4-(4-acetamidophenyl)thiazol-2-ylamino]-2-(2-phenylthiazol-4-yl)ethyl}phenylsulfamic acid: ¹H NMR (CD₃OD): δ 8.11 (m, 2H), 7.82-7.80 (m, 2H), 7.71-7.61 (m, 6H), 7.40 (s, 1H), 7.23 (s, 4H), 5.32 (t, 1H, J=7.0 Hz), 3.51-3.35 (m, 2H), 2.28 (s, 3H).

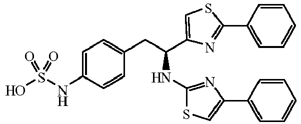

(S)-4-[2-(4-phenylthiazol-2-ylamino)-2-(2-phenylthiazol-4-yl)ethyl]phenylsulfamic acid: ¹H NMR (CD₃OD): δ 8.03-7.99 (m, 2H), 7.75-7.72 (d, 2H, *J*=8.4 Hz), 7.53-7.48 (m, 3H), 7.42 (m, 4H), 7.12 (s, 4H), 6.86 (s, 1H), 5.23 (t, 1H, *J*=7.2 Hz), 3.40-3.27 (m, 2H).

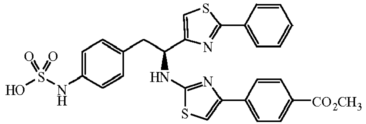

(*S*)-4-{2-[4-(4-(methoxycarbonyl)phenyl)thiazol-2-ylamino]-2-(2-phenylthiazol-4-yl)ethyl}phenylsulfamic acid: ¹H NMR (CD₃OD): δ 8.04-8.00 (m, 4H), 7.92-7.89 (d, 2H, *J*=9.0 Hz), 7.53-7.49 (m, 3H), 7.30 (s, 1H), 7.15 (s, 4H), 7.05 (s, 1H), 5.28 (t, 1H, *J*=6.9 Hz), 3.93 (s, 3H), 3.35-3.24 (m, 2H).

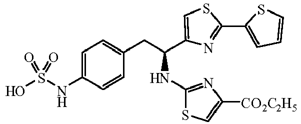

4-{(S)-2-[4-(Ethoxycarbonyl)thiazol-2-ylamino]-2-[2-(thiophen-2-yl)thiazol-4-yl]ethyl}phenylsulfamic acid: ¹H NMR (CD₃OD): δ 7.43-7.38 (m, 2H), 7.26 (s, 1H), 7.00-6.94 (m, 3H), 6.89 (s, 4H), 5.02 (t, 1H, *J*=7.0 Hz), 4.16-4.09 (q, 2H, *J*=7.1 Hz), 3.14-2.94 (m, 2H), 1.17 (t, 3H, *J*=7.1 Hz).

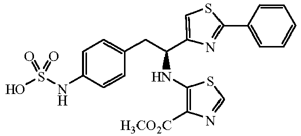

(*S*)-4-[2-(4-(Methoxycarbonyl)thiazol-5-ylamino)-2-(2-phenylthiazole-4-yl)ethyl]phenylsulfamic acid: ¹H NMR (300 MHz, MeOH-d₄) δ 7.97-8.00 (m, 3H), 7.48-7.52 (m, 3H), 7.22 (s, 1H), 7.03-7.13 (m, 4H), 4.74 (t, *J* = 6.6 Hz, 1H), 3.88 (s, 3H), 3.28-3.42 (m, 2H).

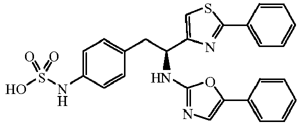

(*S*)-4-[2-(5-Phenyloxazol-2-ylamino)-2-(2-phenylthiazol-4-yl)ethyl]-phenylsulfamic acid: ¹H NMR (300 MHz, MeOH-d₄) δ 7.94-7.96 (m, 2H), 7.45-7.49 (m, 5H), 7.32 (t, *J* = 7.8 Hz, 2H), 7.12 (s, 1H), 7.19 (t, *J* = 7.2 Hz, 1H), 7.12 (s, 4H), 7.05 (s, 1H), 5.15 (t, *J* = 6.4 Hz, 1H), 3.34 (dd, *J* = 14.1 and 8.4 Hz, 1H), 3.18 (dd, *J* = 14.1 and 8.4 Hz, 1H).

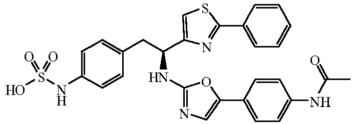

(*S*)-4-{2-[5-(4-Acetamidophenyl)oxazol-2-ylamino]-2-(2-phenylthiazol-4-yl)ethyl}phenylsulfamic acid: ¹H NMR (300 MHz, MeOH-d₄) δ 7.92-7.94 (m, 2H), 7.55-7.58 (m, 2H), 7.39-7.50 (m, 5H), 7.26 (s, 1H), 7.12 (s, 4H), 7.02 (s, 1H0), 5.14 (t, *J* = 7.8 Hz, 1H), 3.13-3.38 (m, 2H), 2.11 (s, 3H).

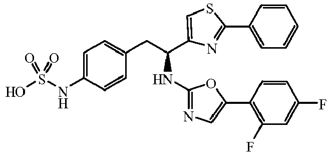

4-((*S*)-2-(5-(2,4-Difluorophenyl)oxazole-2-ylamino)-2-(2-phenylthiazole-4-yl)ethyl)phenylsulfamic acid: ¹H NMR (300 MHz, MeOH-d₄) δ 7.97-7.99 (m, 2H), 7.54-7.62 (m, 1H), 7.45-7.50 (m, 3H), 7.28 (s, 1H), 7.12 (s, 4H), 6.97-7.06 (m, 3H), 5.15-5.20 (m, 1H), 3.28-3.40 (m, 1H), 3.20 (dd, *J* = 13.8 and 8.4 Hz, 1H).

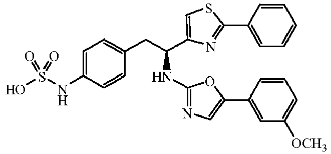

4-{(*S*)-2-[5-(3-Methoxyphenyl)oxazol-2-ylamino]-2-[(2-thiophen-2-yl)thiazole-4-yl]ethyl}phenylsulfamic acid: ¹H NMR (300 MHz, MeOH-d₄) δ 7.55-7.60 (m, 2H), 7.26 (t, *J* = 8.1 Hz, 1H), 7.21 (s, 1H), 7.04-7.15 (m, 8H), 6.77-6.81 (m, 1H), 5.10 (t, *J* = 6.3 Hz, 1H), 3.81 (s, 3H), 3.29-3.36(m, 1H), 3.15 (dd, *J* = 14.1 and 8.4 Hz, 1H).

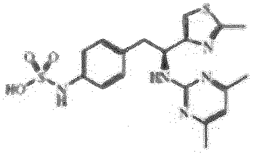

(*S*)-4-[2-(4,6-Dimethylpyrimidin-2-ylamino)-2-(2-methylthiazole-4-yl)ethyl]phenylsulfamic acid: ¹H NMR (300 MHz, MeOH-d₄) δ 7.00-7.10 (m,
5H), 6.44 (s, 1H), 5.50 (t, *J* = 7.2 Hz, 1H), 3.04-3.22 (m, 2H), 2.73 (s, 3H), 2.27 (s, 6H).

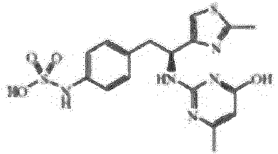

(*S*)-4-[2-(4-Hydroxy-6-methylpyrimidine-2-ylamino)-2-(2-methylthiazole-4-yl)ethyl]phenylsulfamic acid: ¹H NMR (300 MHz, MeOH-d4) δ 7.44 (d, *J* =
8.4Hz,2H), 6.97-7.10 (m, 4H), 5.61 (s, 1H), 5.40-5.49 (m, 1H), 3.10-3.22 (m, 2H), 2.73 (s, 3H), 2.13 (s, 3H).

The first type of Category X of the present disclosure relates to compounds having the formula: 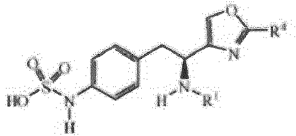 wherein R¹ is heteroaryl nd R⁴ is further described herein below in Table XIX.

**TABLE XIX**

| **No.** | **R⁴** | **R¹** |
|---|---|---|
| S788 | phenyl | 4-(methoxycarbonyl)thiazol-5-yl |
| S789 | phenyl | 4-[(2-methoxy-2-oxoethyl)carbamoyl]thiazol-5-yl |
| S790 | phenyl | 5-[1-*N*-(2-methoxy-2-oxoethyl)-1-*H*-indol-3-yl}oxazol-2-yl |
| S791 | phenyl | 5-(2-methoxyphenyl)oxazol-2-yl |
| S792 | phenyl | 5-[(*S*)-1-(*tert*-butoxycarbonyl)-2-phenylethyl]oxazol-2-yl |
| S793 | phenyl | 5-[4-(methylcarboxy)phenyl]oxazol-2-yl |
| S794 | phenyl | 5-(3-methoxybenzyl)oxazol-2-yl |
| S793 | phenyl | 5-(4-phenyl)oxazol-2-yl |
| S796 | phenyl | 5-(2-methoxyphenyl)thiazol-2-yl |
| S797 | phenyl | 5-(3-methoxyphenyl)thiazol-2-yl |
| S798 | phenyl | 5-(4-fluorophenyl)thiazol-2-yl |
| S799 | phenyl | 5-(2,4-difluorophenyl)thiazol-2-yl |
| S800 | phenyl | 5-(3-methoxybenzyl)thiazol-2-yl |
| S801 | phenyl | 4-(3-methoxyphenyl)thiazol-2-yl |
| S802 | phenyl | 4-(4-fluorophenyl)thiazol-2-yl |
| S803 | thiophen-2-yl | 4-(methoxycarbonyl)thiazol-5-yl |
| S804 | thiophen-2-yl | 4-[(2-methoxy-2-oxoethyl)carbamoyl]thiazol-5-yl |
| S805 | thiophen-2-yl | 5-[1-*N*-(2-methoxy-2-oxoethyl)-1-*H*-indol-3-yl]oxazol-2-yl |
| S806 | thiophen-2-yl | 5-(2-methoxyphenyl)oxazol-2-yl |
| S807 | thiophen-2-yl | 5-[(*S*)-1-(*tert*-butoxycarbonyl)-2-phenylethyl]oxazol-2-yl |
| S808 | thiophen-2-yl | 5-[4-(methylcarboxy)phenyl]oxazol-2-yl |
| S809 | thiophen-2-yl | 5-(3-methoxybenzyl)oxazol-2-yl |
| S810 | thiophen-2-yl | 5-(4-phenyl)oxazol-2-yl |
| S811 | thiophen-2-yl | 5-(2-methoxyphenyl)thiazol-2-yl |
| S812 | thiophen-2-yl | 5-(3-methoxyphenyl)thiazol-2-yl |
| S813 | thiophen-2-yl | 5-(4-fluorophenyl)thiazol-2-yl |
| S814 | thiophen-2-yl | 5-(2,4-difluorophenyl)thiazol-2-yl |
| S815 | thiophen-2-yl | 5-(3-methoxybenzyl)thiazol-2-yl |
| S816 | thiophen-2-yl | 4-(3-methoxyphenyl)thiazol-2-yl |
| S817 | thiophen-2-yl | 4-(4-fluorophenyl)thiazol-2-yl |
| S818 | cyclopropyl | 4-(methoxycarbonyl)thiazol-5-yl |
| S819 | cyclopropyl | 4-[(2-methoxy-2-oxoethyl)carbomoyl]thiazol-5-yl |
| S820 | cyclopropyl | 5-[1-*N*-(2-methoxy-2-oxoethyl)-1-*H*-indol-3-yl]oxazol-2-yl |
| S821 | cyclopropyl | 5-(2-methoxyphenyl)oxazol-2-yl |
| S822 | cyclopropyl | 5-[(*S*)-1-(*tert*-butoxycarbonyl)-2-phenylethyl]oxazol-2-yl |
| S823 | cyclopropyl | 5-[4-(methylcarboxy)phenyl]oxazol-2-yl |
| S824 | cyclopropyl | 5-(3-methoxybenzyl)oxazol-2-yl |
| S825 | cyclopropyl | 5-(4-phenyl)oxazol-2-yl |
| S826 | cyclopropyl | 5-(2-methoxyphenyl)thiazol-2-yl |
| S827 | cyclopropyl | 5-(3-methoxyphenyl)thiazol-2-yl |
| S828 | cyclopropyl | 5-(4-fluorophenyl)thiazol-2-yl |
| S829 | cyclopropyl | 5-(2,4-difluorophenyl)thiazol-2-yl |
| S830 | cyclopropyl | 5-(3-methoxybenzyl)thiazol-2-yl |
| S831 | cyclopropyl | 4-(3-methoxyphenyl)thiazol-2-yl |
| S832 | cyclopropyl | 4-(4-fluorophenyl)thiazol-2-yl |

Compounds according to the first type of Category X can be prepared by the procedure outlined in Scheme XXII and described herein below in Example 23.

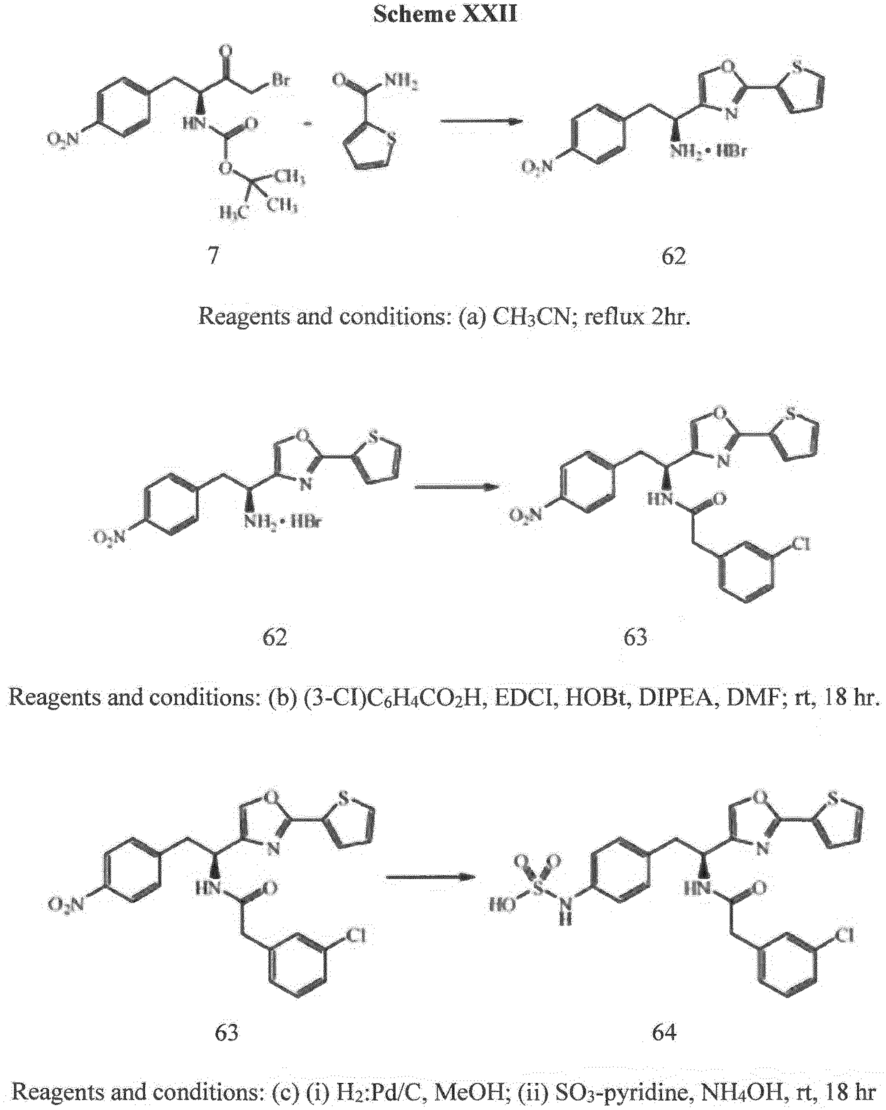

### EXAMPLE 23

### 4-((S)-2-(2-(3-Chlorophenyl)acetamido)-2-(2-(thiophen-2-yl)oxazol-4-yl)ethyl)phenylsulfamic acid (64)

Preparation of (*S*)-2-(4-nitrophenyl)-1-[(thiophen-2-yl)oxazol-4-yl]ethanamine hydrobromide salt (62): A mixture of (*S*)-tert-butyl 4-bromo-1-(4-nitrophenyl)-3-oxobutan-2-ylcarbamate, 7, (38.7 g, 100 mmol), and thiophen-2-carboxamide (14 g, 110 mmol) (available from Alfa Aesar) in CH₃CN (500 mL) is refluxed for 5 hours. The reaction mixture is cooled to room temperature and diethyl ether (200 mL) is added to the solution. The precipitate which forms is collected by filtration. The solid is dried under vacuum to afford the desired product which can be used for the next step without purification.

Preparation of 2-(3-chlorophenyl)-*N*-{(S)-2-(4-nitrophenyl)-1- [2-(thiophen-2-yl)oxazol-4-yl]ethyl}acetamide (63): To a solution of (S)-2-(4-nitrophenyl)-1-[(thiophen-2-yl)oxazol-4-yl]ethanamine HBr, 47, (3.15 g, 10 mmol) 3-chlorophenyl-acetic acid (1.70 g, 10 mmol) and 1-hydroxybenzotriazole (HOBt) (0.70g, 5.0 mmol) in DMF (50 mL) at 0 °C, is added 1-(3-dimethylaminopropyl)-3-ethylcarbodiimide (EDCI) (1.90 g, 10 mmol) followed by triethylamine (4.2 mL, 30 mmol). The mixture is stirred at 0 °C for 30 minutes then at room temperature overnight. The reaction mixture is diluted with water and extracted with EtOAc. The combined organic phase is washed with 1 N aqueous HCl, 5 % aqueous NaHCO₃, water and brine, and dried over Na₂SO₄. The solvent is removed in vacuo to afford the desired product which is used without further purification.

Preparation of -((*S*)-2-(2-(3-chlorophenyl)acetamido)-2-(2-(thiophen-2-yl)oxazol-4-yl)ethyl)phenylsulfamic acid (64): 2-(3-chlorophenyl)-*N*-{(*S*)-2-(4-nitrophenyl)-1-[2-(thiophen-2-yl)oxazol-4-yl]ethyl}acetamide, 63, (3 g) is dissolved in MeOH (4 mL). A catalytic amount of Pd/C (10% w/w) is added and the mixture is stirred under a hydrogen atmosphere 18 hours. The reaction mixture is filtered through a bed of CELITE™ and the solvent is removed under reduced pressure. The crude product is dissolved in pyridine (12 mL) and treated with SO₃-pyridine (0.157 g). The reaction is stirred at room temperature for 5 minutes after which a 7% solution of NH₄OH is added. The mixture is then concentrated and the resulting residue can be purified by reverse phase chromatography to afford the desired product as the ammonium salt.

The second type of Category X of the present disclosure relates to compounds having the formula: 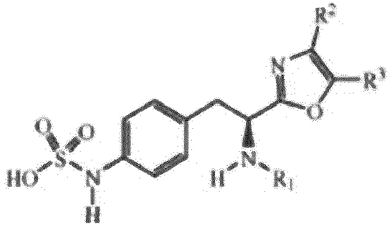 wherein R¹ is aryl and R² and R³ are further described herein below in Table XX.

**TABLE XX**

| **No.** | **R²** | **R³** | **R¹** |
|---|---|---|---|
| T833 | methyl | hydrogen | phenyl |
| T834 | methyl | hydrogen | benzyl |
| T835 | methyl | hydrogen | 2-fluorophenyl |
| T836 | methyl | hydrogen | 3-fluorophenyl |
| T837 | methyl | hydrogen | 4-fluorophenyl |
| T838 | methyl | hydrogen | 2-chlorophenyl |
| T839 | methyl | hydrogen | 3-chlorophenyl |
| T840 | methyl | hydrogen | 4-chlorophenyl |
| T841 | ethyl | hydrogen | phenyl |
| T842 | ethyl | hydrogen | benzyl |
| T843 | ethyl | hydrogen | 2-fluorophenyl |
| T844 | ethyl | hydrogen | 3-fluorophenyl |
| T845 | ethyl | hydrogen | 4-fluorophenyl |
| T846 | ethyl | hydrogen | 2-chlorophenyl |
| T847 | ethyl | hydrogen | 3-chlorophenyl |
| T848 | ethyl | hydrogen | 4-chlorophenyl |
| T849 | thien-2-yl | hydrogen | phenyl |
| T850 | thien-2-yl | hydrogen | benzyl |
| T851 | thien-2-yl | hydrogen | 2-fluorophenyl |
| T852 | thien-2-yl | hydrogen | 3-fluorophenyl |
| T853 | thien-2-yl | hydrogen | 4-fluorophenyl |
| T854 | thien-2-yl | hydrogen | 2-chlorophenyl |
| T855 | thien-2-yl | hydrogen | 3-chlorophenyl |
| T856 | thiene-2-yl | hydrogen | 4-chlorophenyl |

Compounds according to the second type of Category X can be prepared by the procedure outlined in Scheme XXIII and described herein below in Example 24.

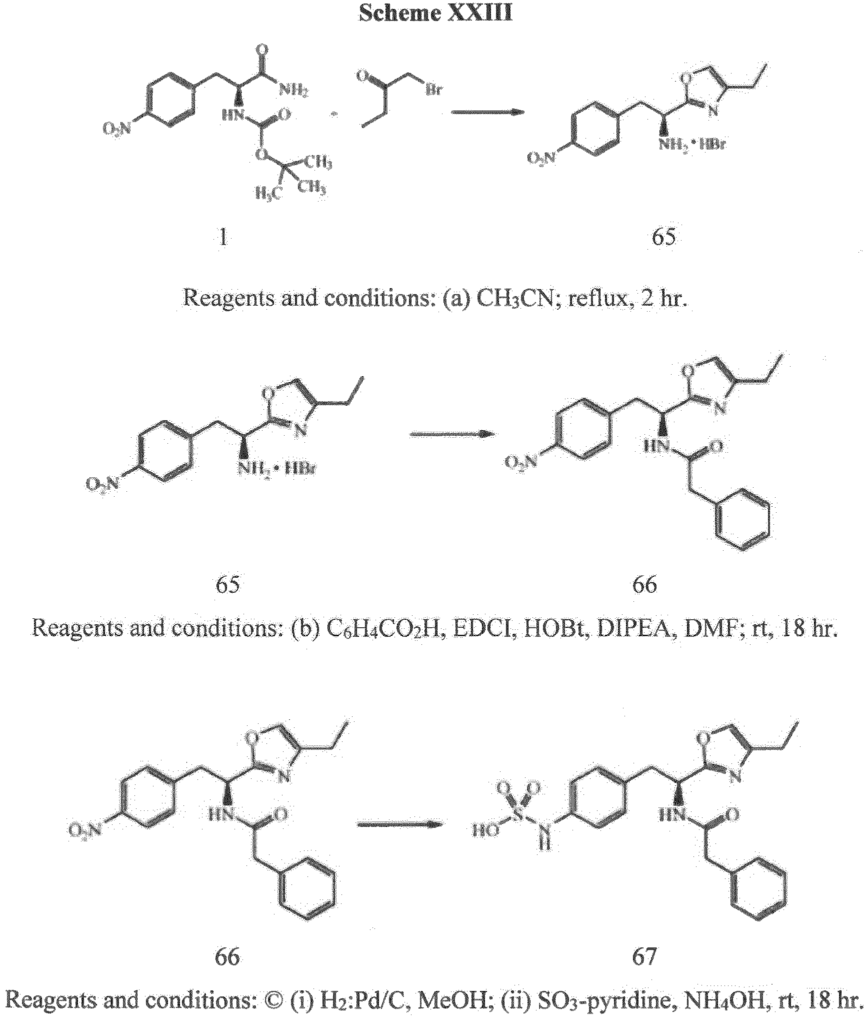

### EXAMPLE 24

### {4-[2-(S)-(4-Ethyloxazol-2-yl)-2-phenylacetylaminoethyl] -phenyl}sulfamic acid (67)

Preparation of (*S*)-1-(4-ethyloxazol-2-yl)-2-(4-nitrophenyl)ethanamine (65): A mixture of [1-(S)-carbamoyl-2-(4-nitrophenyl)ethyl-carbamic acid *tert*-butyl ester, 1, (10 g, 32.3mmol) and 1-bromo-2-butanone (90%, 4.1 mL, 36 mmol) in CH₃CN (500 mL) is refluxed for 18 hours. The reaction mixture is cooled to room temperature and diethyl ether is added to the solution and the precipitate which forms is removed by filtration and is used without further purification.

Preparation of *N*-[1-(4-ethyloxazol-2-yl)-2-(4-nitrophenyl)ethyl]-2-phenyl-acetamide (66): To a solution of (S)-1-(4-ethyloxazol-2-yl)-2-(4-nitrophenyl)ethanamine, 65, (2.9 g, 11 mmol), phenylacetic acid (1.90 g, 14 mmol) and 1-hydroxybenzotriazole (HOBt) (0.94 g, 7.0 mmol) in DMF (100 mL) at 0 ° C, is added 1-(3-dimethylamino-propyl)-3-ethylcarbodiimide (EDCI) (2.68g, 14 mmol) followed by triethylamine (6.0 mL, 42mmol). The mixture is stirred at 0 °C for 30 minutes then at room temperature overnight. The reaction mixture is diluted with water and extracted with EtOAc. The combined organic phase is washed with 1 N aqueous HCl, 5 % aqueous NaHCO₃, water and brine, and dried over Na₂SO₄. The solvent is removed *in vacuo* to afford the desired product which is used without further purification.

Preparation of {4-[2-(S)-(4-ethyloxazol-2-yl)-2-phenylacetylaminocthyl]-phenyl}sulfamic acid (67): *N*-[1-(4-ethyloxazol-2-yl)-2-(4-nitrophenyl)ethyl]-2-phenyl-acetamide, 66, (0.260 g) is dissolved in MeOH (4 mL). A catalytic amount of Pd/C (10% w/w) is added and the mixture is stirred under a hydrogen atmosphere 18 hours. The reaction mixture is filtered through a bed of CELITE™ and the solvent is removed under reduced pressure. The crude product is dissolved in pyridine (12 mL) and treated with SO₃-pyridine (0.177 g, 1.23). The reaction is stirred at room temperature for 5 minutes after which a 7% solution of NH₄OH (10 mL) is added. The mixture is then concentrated and the resulting residue is purified by reverse phase chromatography to afford the desired product as the ammonium salt.

Non-limiting examples of the HPTP-β (IC₅₀ µM) activity for the disclosed compounds are listed in Table XXI. Compounds according to the present invention are identified as AA6, AA28 and AA34. HPTP-β inhibition can be tested by any method chosen by the formulator, for example, Amarasinge K.K. et al., "Design and Synthesis of Potent, Non-peptidic Inhibitors of HPTPbeta" Bioorg Med Chem Lett. 2006 Aug 15;16(16):4252-6. Epub 2006 Jun 12. Erratum in: Bioorg Med Chem Lett. 2008 Aug 15; 18(16):4745.. Evidokimov, Artem G [corrected to Evdokimov, Artem G]: PMID: 16759857; and Klopfenstein S. R. et al. "1,2,3,4-Tetrahydroisoquinolinyl Sulfamic Acids as Phosphatase PTP1B Inhibitors" Bioorg Med Chem Lett. 2006 Mar 15;16(6): 1574-8, both of which are included herein by reference in their entirety.

**TABLE XXI**

| **No.** | **Compound** | **HPTPβ IC₅₀ µM** |
|---|---|---|
| AA1 | 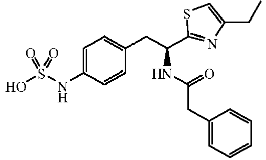 | 0.000157 |
| | (*S*)-{4-[2-(4-Ethylthiazol-2-yl)-2-(phenylacetylamino)ethyl]-phenyl}sulfamic acid | |
| AA2 | 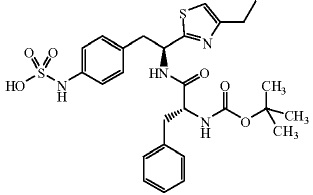 | 0.004 |
| | 4-{(*S*)-2-[(*R*)-2-(*tert*-butoxycarbonylamino)-3-phenylpropanamido]-2-(4-ethylthiazol-2-yl)ethyl}phenylsulfamic acid | |
| AA3 | 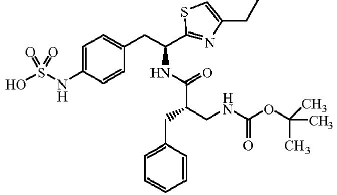 | 0.031 |
| | {1-[1-(5-Ethylthiazol-2-yl)-(*S*)-2-(4-sulfoaminophenyl)ethylcarbamoyl]-(*S*)-2-phenylethyl}methyl carbamic acid *tert*-butyl ester | |
| AA4 | 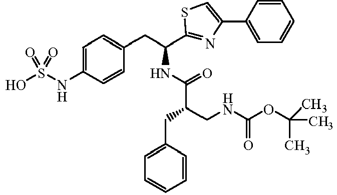 | <5x10⁻⁸ |
| | {1-[1-(5-phenylthiazol-2-yl)-(*S*)-2-(4-sulfoaminophenyl)ethylcarbamoyl]-(*S*)-2-phenylethyl}methyl carbamic acid *tert*-butyl ester | |
| AA5 | 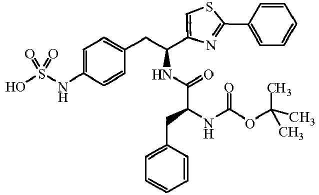 | <5x10⁻⁸ |
| | 4-{(*S*)-2-(*S*)-2-(*tert*-Butoxycarbonylamino)-3-phenylpropanamido-2-(2-phenylthiazol-4-yl)}phenylsulfamic acid | |
| AA6 | 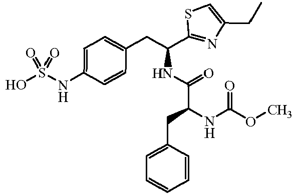 | 0.000162 |
| | 4-{(*S*)-2-(4-Ethylthiazol-2-yl)-2-[(*S*)-2-(methoxycarbonylamino)-3-phenylpropanamido]ethyl}phenylsulfamic acid | |
| AA7 | 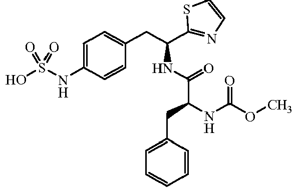 | 0.006 |
| | 4-{(*S*)-2-[(*S*)-2-(Methoxycarbonylamino)-3-phenylpropanamido]-2-(thiazol-2-yl)ethyl}phenylsulfamic acid | |
| AA8 | 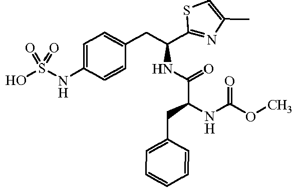 | 0.001 |
| | 4-{(*S*)-2-[(*S*)-2-(Methoxycarbonylamino)-3-phenylpropanamido]-2-(4-methylthiazol-2-yl)ethyl}phenylsulfamic acid | |
| AA9 | 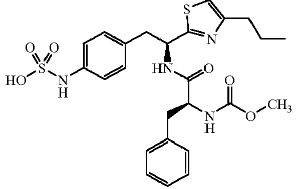 | 0.0001 |
| | 4-{(*S*)-2-[(*S*)-2-(Methoxycarbonylamino)-3-phenylpropanamido]-2-(4-propylthiazol-2-yl)ethyl}phenylsulfamic acid | |
| AA10 | 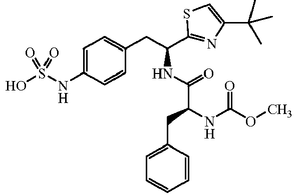 | 0.0002 |
| | 4-{(*S*)-2-(4-*tert*-Butylthiazol-2-yl)-2-[(*S*)-2-(methoxycarbonylamino)-3-phenylpropanamido]ethyl}phenylsulfamic acid | |
| AA11 | 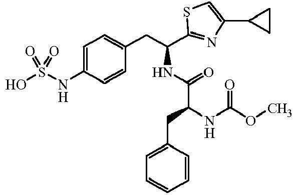 | 0.00001 |
| | 4-{(*S*)-2-(4-Cyclopropylthiazol-2-yl)-2-[(*S*)-2-(methoxy-carbonylamino)-3-phenylpropanamido]ethyl}phenylsulfamic acid | |
| AA12 | 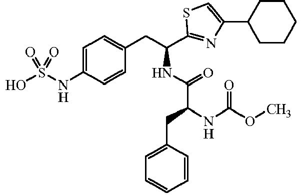 | <5x10⁻⁸ |
| | 4-{(*S*)-2-(4-Cyclohexylthiazol-2-yl)-2-[(*S*)-2-(methoxycarbonylamino)-3-phenyl-propanamido]ethyl}phenylsulfamic acid | |
| AA13 | 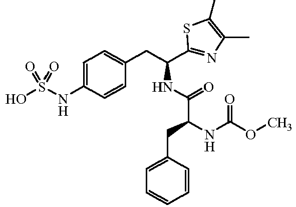 | 0.001 |
| | 4-{(*S*)-2-(4,5-Dimethylthiazol-2-yl)-2-[(*S*)-2-(methoxycarbonylamino)-3-phenyl-propanamido]ethyl}phenylsulfamic acid | |
| AA14 | 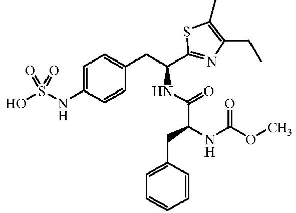 | 0.0001 |
| | 4-{(S)-2-(4-Ethyl-5-methylthiazol-2-yl)-2-[(S)-2-(methoxy-carbonylamino)-3-phenyl-propanamido]ethyl}phenylsulfamic acid | |
| AA15 | 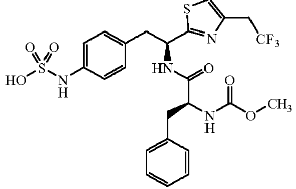 | 0.0003 |
| | 4-{(*S*)-2-[(S)-2-(Methoxycarbonylamino)-3-phenylpropanamido]-2-[4-(2,2,2-trifluoroethyl)thiazol-2-yl]ethyl}phenylsulfamic acid | |
| AA16 | 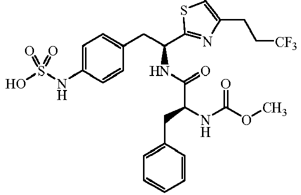 | 0.00008 |
| | 4-{(*S*)-2-[(*S*)-2-(Methoxycarbonylamino)-3-phenylpropanamido)-2-[4-(3,3,3-trifluoropropyl)thiazol-2-yl]ethyl}phenylsulfamic acid | |
| AA17 | 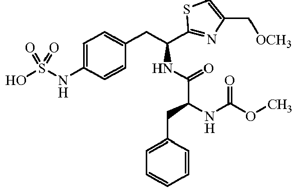 | 0.001 |
| | 4-{(*S*)-2-[(*S*)-2-(Methoxycarbonylamino)-3-phenylpropanamido]-2-[4-(methoxymethyl)thiazol-2-yl]ethyl}phenylsulfamic acid | |
| AA18 | 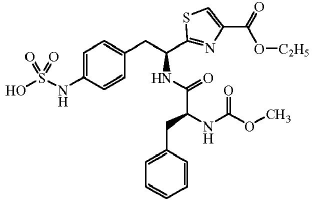 | 0.0002 |
| | 4-{(*S*)-2-(4-(Ethoxycarbonyl)thiazol-2-yl)-2-[(*S*)-2-(methoxy-carbonylamino)-3-phenylpropanamido]ethyl}phenylsulfamic acid | |
| AA19 | 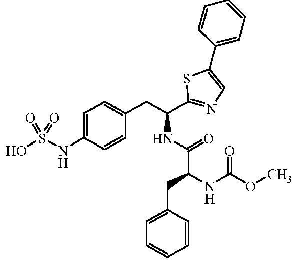 | 0.0003 |
| | 4-{(*S*)-2-[(*S*)-2-(Methoxycarbonylamino)-3-phenylpropanamido]-2-(5-phenylthiazol-2-yl)ethyl}phenylsulfamic acid | |
| AA20 | 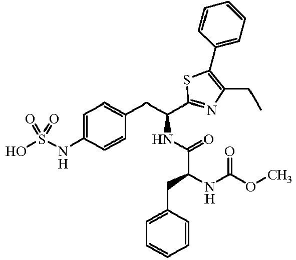 | <5x10⁻⁸ |
| | 4-{(*S*)-2-(4-Ethyl-5-phenylthiazol-2-yl)-2-[(*S*)-2-(methoxy-carbonylamino)-3-phenyl-propanamido]ethyl}phenylsulfamic acid | |
| AA21 | 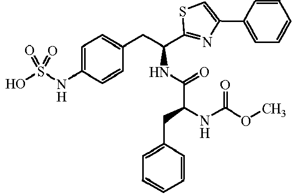 | <2x10⁻⁶ |
| | 4-{(*S*)-2-[(*S*)-2-(Methoxycarbonylamino)-3-phenylpropanamido]-2-(4-phenylthiazol-2-yl)ethyl}phenylsulfamic acid | |
| AA22 | 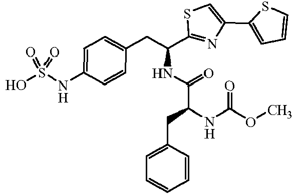 | <5x10⁻⁸ |
| | 4-{(*S*)-2-[(*S*)-2-(Methoxycarbonylamino)-3-phenylpropanamido]-2-[4-(thiophen-2-yl)thiazol-2-yl]ethyl}phenylsulfamic acid | |
| AA23 | 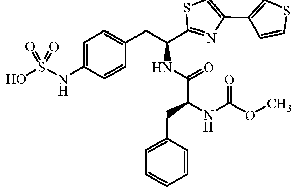 | 0.00009 |
| | 4-{(*S*)-2-[(*S*)-2-(Methoxycarbonylamino)-3-phenylpropanamido]-2-[4-(thiophen-3-yl)thiazol-2-yl]ethyl}phenylsulfamic acid | |
| AA24 | 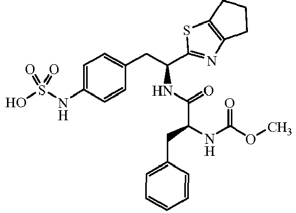 | 0.001 |
| | 4-{(*S*)-2-(5,6-Dihydro-4*H*-cyclopenta[*d*]thiazol-2-yl)-2-[(*S*)-2-(methoxycarbonylamino)-3 - phenylpropanamido]ethyl}phenylsulfamic acid | |
| AA25 | 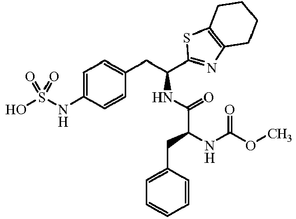 | 0.0004 |
| | 4-{(*S*)-2-[(*S*)-2-(Methoxycarbonylamino)-3-phenylpropanamido]-2-(4,5,6,7-tetrahydrobenzo[*d*]thiazol-2-yl)ethyl}phenylsulfamic acid | |
| AA26 | 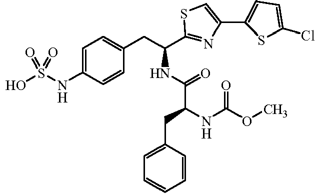 | <5x10⁻⁸ |
| | 4-{(*S*)-2-[4-(5-Chlorothiophen-2-yl)thiazol-2-yl]-2-[(*S*)-2-(methoxycarbonylamino)-3-phenylpropanamido]ethyl}phenylsulfamic acid | |
| AA27 | 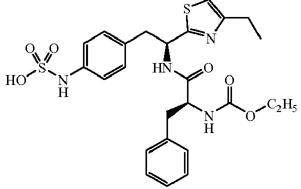 | 0.00014 |
| | 4-{(*S*)-2-[(*S*)-2-(Ethoxycarbonylamino)-3-phenylpropanamido]-2-(4-ethylthiazol-2-yl)ethyl}phenylsulfamic acid | |
| AA28 | 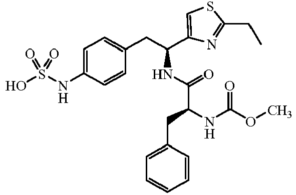 | 0.0001 |
| | 4-{(*S*)-2-[(*S*)-2-(Methoxycarbonylamino)-3-phenylpropanamido]-2-(2-ethylthiazol-4-yl)ethyl}phenylsulfamic acid | |
| AA29 | 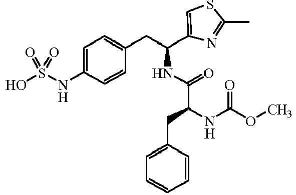 | 0.001 |
| | 4-{(*S*)-2-[(*S*)-2-(Methoxycarbonylamino)-3-phenylpropanamido]-2-(2-methylthiazol-4-yl)ethyl}phenylsulfamic acid | |
| AA30 | 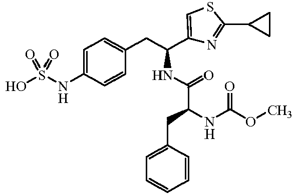 | 0.0002 |
| | 4-{(*S*)-2-(2-Cyclopropylthiazol-4-yl)-2-[(*S*)-2-(methoxy-carbonylamino)-3-phenylpropanamido]ethyl}phenylsulfamic acid | |
| AA31 | 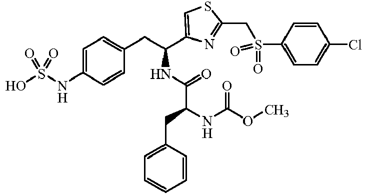 | 0.00008 |
| | 4-{(S)-2-{2-[(4-Chlorophenylsulfonyl)methyl]thiazol-4-yl}-2-[(S)-2-(methoxycarbonylamino)-3 - phenylpropanamido]ethyl}phenylsulfamic acid | |
| AA32 | 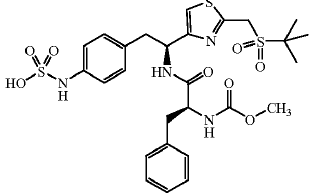 | 0.002 |
| | 4-{(S)-2-[2-(*tert*-Butylsulfonylmethyl)thiazol-4-yl]-2-[(S)-2-(methoxycarbonylamino)-3 - phenylpropanamido]ethyl}phenylsulfamic acid | |
| AA33 | 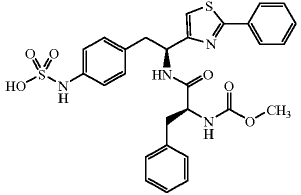 | 7x10⁻⁷ |
| | 4-{(*S*)-2-[(*S*)-2-(Methoxycarbonylamino)-3-phenylpropionamido]-2-(2-phenylthiazole-4-yl)ethyl}phenylsulfamic acid | |
| AA34 | 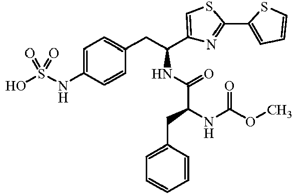 | 5x10⁻⁸ |
| | 4-{(*S*)-2-[(*S*)-2-(Methoxycarbonylamino)-3-phenylpropanamido]-2-[2-(thiophen-2-yl)thiazol-4-yl]ethyl}phenylsulfamic acid | |
| AA35 | 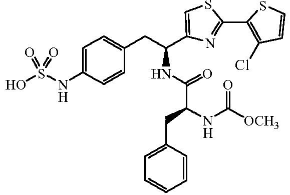 4-{(*S*)-2-[2-(3-Chlorothiophen-2-yl)thiazol-4-yl]-2-[(*S*)-2-(methoxycarbonylamino)-3 - phenylpropanamido]ethyl}phenylsulfamic acid | <5x10⁻⁸ |
| AA36 | 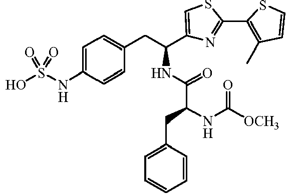 | <5x10⁻⁸ |
| | 4-{(*S*)-2-[(*S*)-2-(Methoxycarbonylamino)-3-phenylpropanamido]-2-[2-(3-methylthiophen-2-yl)thiazol-4-yl]ethyl}phenylsulfamic acid | |
| AA37 | 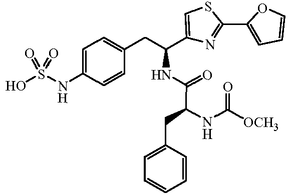 | 0.0004 |
| | 4-{[(*S*)-2-(2-(Furan-2-yl)thiazol-4-yl]-2-[(*S*)-2-(methoxy-carbonylamino)-3-phenylpropanamido]ethyl}phenylsulfamic acid | |
| AA38 | 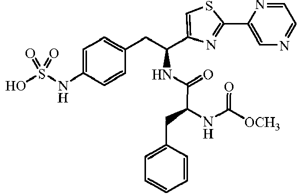 | 0.003 |
| | 4-{(*S*)-2-[(*S*)-2-(Methoxycarbonylamino)-3-phenylpropanamido]-2-[2-(pyrazin-2-yl)thiazol-4-yl]ethyl}phenylsulfamic acid | |
| AA39 | 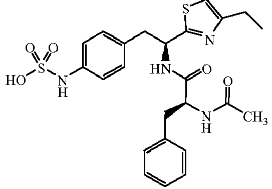 | 0.001 |
| | 4-[(*S*)-2-((*S*)-2-Acetamido-3-phenylpropanamido)-2-(4-ethylthiazol-2-yl)ethyl]phenylsulfamic acid | |
| AA40 | 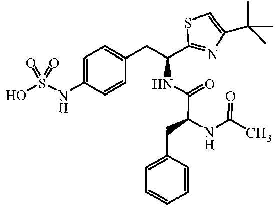 | 0.0003 |
| | 4-[(*S*)-2-((*S*)-2-Acetamido-3-phenylpropanamido)-2-(4-*tert-*butylthiazol-2-yl)ethyl]phenylsulfamic acid | |
| AA41 | 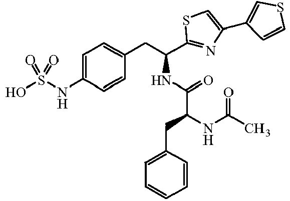 | 0.00024 |
| | 4-{(*S*)-2-((*S*)-2-Acetamido-3-phenylpropanamido)-2-[4-(thiophen-3-yl)thiazol-2-yl]ethyl}phenylsulfamic acid | |
| AA42 | 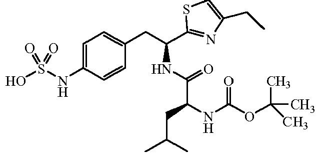 | 0.006 |
| | 4-{(*S*)-2-[(*S*)-2-(*tert*-Butoxycarbonylamino)-3-methylbutanamido]-2-(4-ethylthiazol-2-yl)ethyl}phenylsulfamic acid | |
| AA43 | 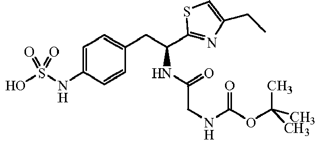 | 0.028 |
| | (*S*)-4-{2-[2-(*tert*-Butoxycarbonylamino)acetamido]-2-(4-ethylthiazol-2-yl)ethyl}phenylsulfamic acid | |
| AA44 | 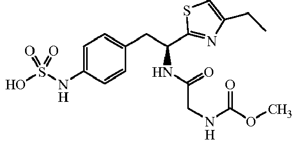 | 0.020 |
| | (*S*)-4-{2-(4-Ethylthiazol-2-yl)-2-[2-(methoxycarbonylamino)acetamido]ethyl}phenylsulfamic acid | |
| AA45 | 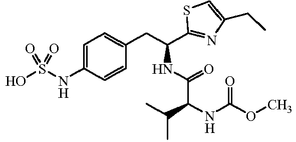 | 0.003 |
| | 4-{(*S*)-2-(4-Ethylthiazol-2-yl)-2-[(*S*)-2-(methoxycarbonylamino)-3-methylbutanamido]-ethyl}phenylsulfamic acid | |
| AA46 | 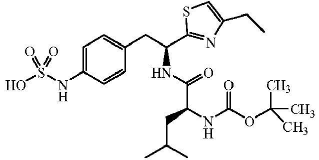 | 0.001 |
| | 4-{(*S*)-2-[(*S*)-2-(*tert*-Butoxycarbonylamino)-4-methylpentanamido]-2-(4-ethylthiazol-2-yl)ethyl}phenylsulfamic acid | |
| AA47 | 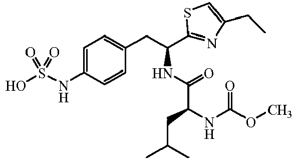 | 0.0003 |
| | 4-{(*S*)-2-(4-Ethylthiazol-2-yl)-2-[(*S*)-2-(methoxycarbonylamino)-4-methylpentanamido]ethyl}phenylsulfamic acid | |
| AA48 | 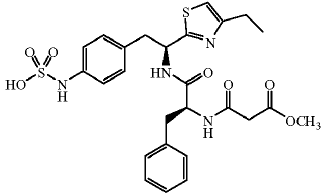 | 0.0003 |
| | 4-((*S*)-2-(4-Ethylthiazol-2-yl)-2-{(*S*)-2-[2-(methoxycarbonylamino)-acetamido]-3-phenylpropanamido}ethyl)phenylsulfamic acid | |
| AA49 | 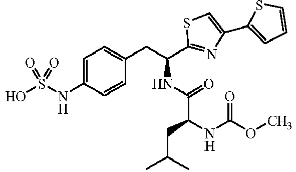 | <5x10⁻⁸ |
| | 4-{(*S*)-2-[(*S*)-2-(Methoxycarbonylamino)-4-methylpentanamido]-2-[2-(thiophen-2-yl)thiazol-4-yl]ethyl}phenylsulfamic acid | |
| AA50 | 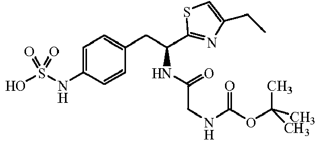 | 0.028 |
| | (*S*)-4-{2-[2-(*tert*-Butoxycarbonylamino)acetamido]-2-(4-ethylthiazol-2-yl)ethyl}-phenylsulfamic acid | |
| AA51 | 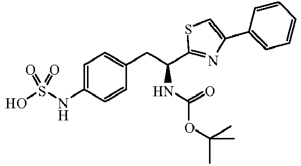 | 0.049 |
| | [1-(*S*)-(Phenylthiazol-2-yl)-2-(4-sulfoaminophenyl)ethyl]-carbamic acid tert-butyl ester | |
| AA52 | 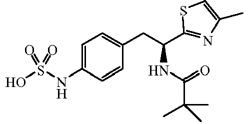 | 0.112 |
| | (*S*)-4-(2-(4-Methylthiazol-2-yl)-2-pivalamidoethyl)phenyl-sulfamic acid | |
| AA53 | 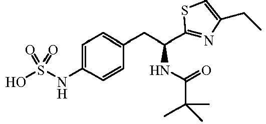 | 0.085 |
| | *(S)*-4-(2-(4-Ethylthiazol-2-yl)-2-pivalamidoethyl)phenyl-sulfamic acid | |
| AA54 | 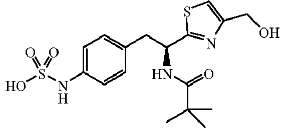 | 0.266 |
| | *(S)*-4-{2-[4-(hydroxymethyl)thiazol-2-yl]-2-pivalamidoethyl}phenylsulfamic acid | |
| AA55 | 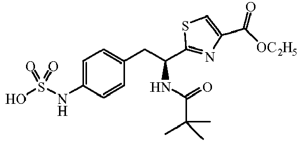 | 0.584 |
| | *(S)*-4-{[2-(4-Ethoxycarbonyl)thiazol-2-yl]-2-pivalamidoethyl}phenylsulfamic acid | |
| AA56 | 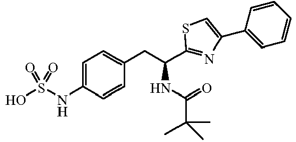 | 0.042 |
| | *(S)*-4-(2-(4-Phenylthiazol-2-yl)-2-pivalamidoethyl)phenylsulfamic acid | |
| AA57 | 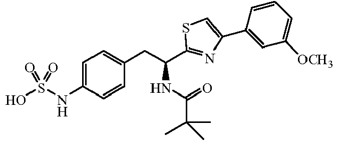 | 0.110 |
| | 4-((*S*)-2-(4-(3-Methoxyphenyl)thiazol-2-yl)-2-pivalamidoethyl)phenylsulfamic acid | |
| AA58 | 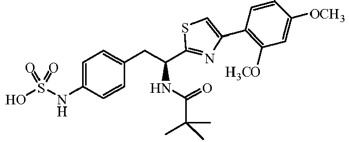 | 0.086 |
| | 4-((*S*)-2-(4-(2,4-Dimethoxyphenyl)thiazol-2-yl)-2-pivalamidoethyl)phenyl-sulfamic acid | |
| AA59 | 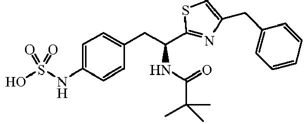 | 0.113 |
| | (*S*)-4-(2-(4-Benzylthiazol-2-yl)-2-pivalamidoethyl)phenylsulfamic acid | |
| AA60 | 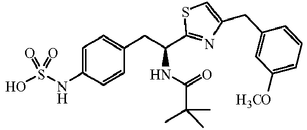 | 0.132 |
| | (S)-4-(2-(4-(3-Methoxybenzyl)thiazol-2-yl)-2-pivalamidoethyl)phenylsulfamic acid | |
| AA61 | 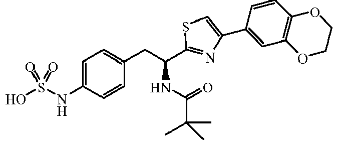 | 0.138 |
| | 4-((S)-2-(4-(2,3-Dihydrobenzo[b][1,4]dioxin-6-yl)thiazol-2-yl)-2-pivalamidoethyl)phenylsulfamic acid | |
| AA62 | 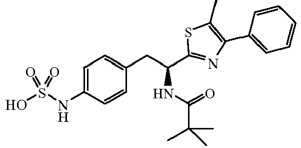 | 0.098 |
| | (S)-4-(2-(5-Methyl-4-phenylthiazol-2-yl)-2-pivalamidoethyl)phenylsulfamic acid | |
| AA63 | 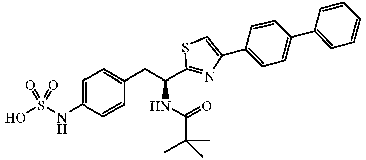 | 0.381 |
| | (S)-4- (2-(4-(Biphen-4-yl)thiazol-2-yl)-2-pivalamidoethyl)phenylsulfamic acid | |
| AA64 | 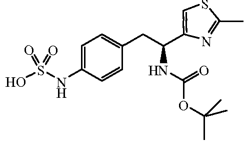 | 0.033 |
| | (*S*)-4-(2-*tert*-Butoxycarbonylamino)-2-(2-methylthiazol-4-yl)ethyl)phenylsulfamic acid | |
| AA65 | 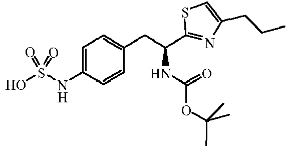 | 0.04 |
| | (S)-4-(2-(*tert*-Butoxycarbonylamino)-2-(4-propylthiazol-2-yl)ethyl)phenyl sulfamic acid | |
| AA66 | 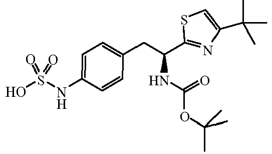 | 0.027 |
| | (S)-4-(2-(*tert*-Butoxycarbonylamino)-2-(4-*tert*-butylthiazol-2-yl)ethyl)phenyl sulfamic acid | |
| AA67 | 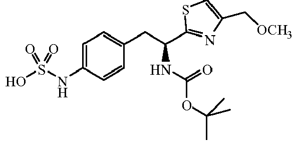 | 0.18 |
| | (S)-4-(2-(*tert*-Butoxycarbonylamino)-2-(4-(methoxymethyl)thiazol-2-yl)ethyl)-phenyl sulfamic acid | |
| AA68 | 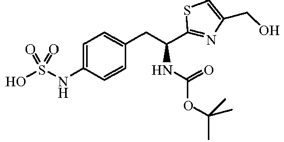 | 0.644 |
| | (*S*)-4-(2-(*tert*-Butoxycarbonylamino)-2-(4-(hydroxymethyl)thiazol-2-yl)ethyl)phenylsulfamic acid | |
| AA69 | 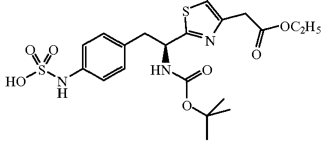 | 0.167 |
| | *(S)*-4-(2-*tert*-Butoxycarbonylamino)-2-(4-(2-ethoxy-2-oxoethyl)thiazol-2-yl)ethyl)phenylsulfamic acid | |
| AA70 | 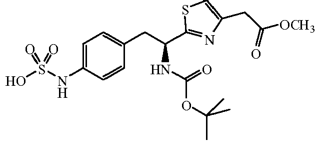 | 0.132 |
| | (*S*)-4-(2-(*tert*-Butoxycarbonyl)-2-(4-(2-(2-methoxy-2-oxoyethyl amino)-2-oxoethyl)thiazole-2-yl)ethyl)phenylsulfamic acid | |
| AA71 | 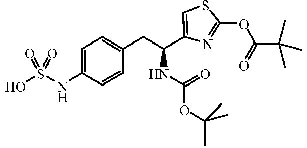 | 0.555 |
| | (S)-4-(2-(*tert*-Butoxycarbonylamino)-2-(2-pivalamidothiazol-4-yl)ethyl)phenylsulfamic acid | |
| AA72 | 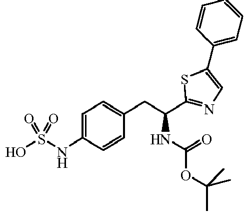 | 0.308 |
| | (S)-4-(2-(*tert*-Butoxycarbonylamino)-2-(5-phenylthiazol-2-yl)ethyl)-phenyl sulfamic acid | |
| AA73 | 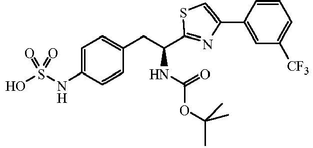 | 0.253 |
| | 4-((S)-2-(*tert*-Butoxycarbonylamino)-2-(4-(3-(trifluoromethyl)phenyl)thiazol-2-yl)ethyl)-phenyl sulfamic acid | |
| AA74 | 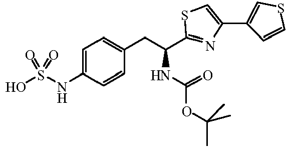 | 0.045 |
| | 4-((S)-2-(*tert*-Butoxycarbonylamino)-2-(4-(thiophen-3-yl)thiazol-2-yl)ethyl)phenyl sulfamic acid | |
| AA75 | 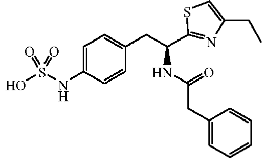 | 0.05 |
| | (*S*)-{4-[2-(4-Ethylthiazol-2-yl)-2-(phenylacetylamido)ethyl]-phenyl}sulfamic acid | |
| AA76 | 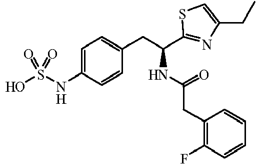 | 0.012 |
| | (*S*)-4-(2-(4-Ethylthiazol-2-yl)-2-(2-(2-fluorophenyl)acetamido)ethyl)phenyl-sulfamic acid | |
| AA77 | 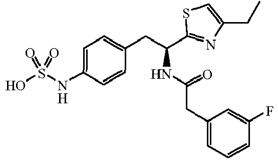 | 0.0003 |
| | (*S*)-4-(2-(4-Ethylthiazol-2-yl)-2-(2-(3-fluorophenyl)acetamido)ethyl)phenyl-sulfamic acid | |
| AA78 | 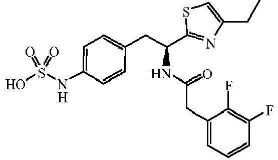 | 0.028 |
| | (*S*)-4-(2-(2-(2,3-Difluorophenyl)acetamido)-2-(4-ethylthiazol-2-yl)ethyl)phenyl-sulfamic acid | |
| AA79 | 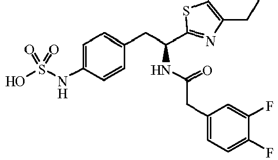 | 0.075 |
| | (*S*)-4-(2-(2-(3,4-Difluorophenyl)acetamido)-2-(4-ethylthiazol-2-yl)ethyl)phenyl-sulfamic acid | |
| AA80 | 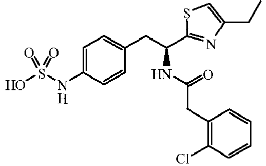 | 0.056 |
| | (*S*)-4-(2-(2-(2-Chlorophenyl)acetamido)-2-(4-ethylthiazol-2-yl)ethyl)phenyl-sulfamic acid | |
| AA81 | 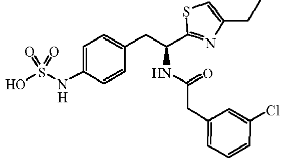 | 0.033 |
| | (*S*)-4-(2-(2-(3-Chlorophenyl)acetamido)-2-(4-ethylthiazol-2-yl)ethyl)phenyl-sulfamic acid | |
| AA82 | 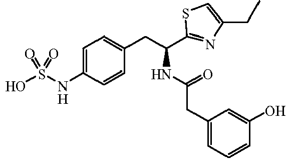 | 0.04 |
| | (*S*)-4-(2-(4-Ethylthiazol-2-yl)-2-(2-(3-hydroxyphenyl)acetamido)ethyl)phenyl-sulfamic acid | |
| AA83 | 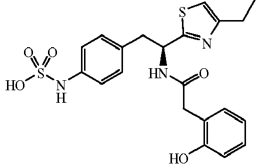 | 0.014 |
| | (*S*)-4-(2-(4-Ethylthiazol-2-yl)-2-(2-(2-methoxyphenyl)acetamido)ethyl)phenyl-sulfamic acid | |
| AA84 | 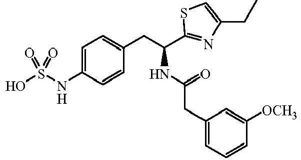 | 0.008 |
| | (*S*)-4-(2-(4-Ethylthiazol-2-yl)-2-(2-(3-methoxyphenyl)acetamido)ethyl)phenyl-sulfamic acid | |
| AA85 | 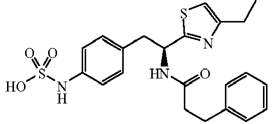 | 0.002 |
| | (*S*)-4-(2-(4-Ethylthiazol-2-yl)-2-(3-phenylpropanamido)ethyl)phenylsulfamic acid | |
| AA86 | 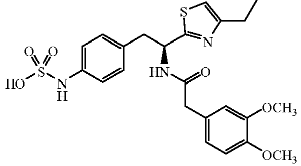 | 0.028 |
| | (*S*)-4-(2-(2-(3,4-Dimethoxyphenyl)acetamido)-2-(4-ethylthiazol-2-yl)ethyl)-phenylsulfamic acid | |
| AA87 | 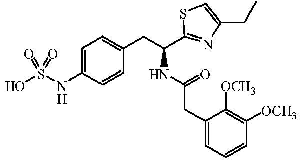 | 0.037 |
| | (*S*)-4-(2-(2-(2,3-Dimethoxyphenyl)acetamido)-2-(4-ethylthiazol-2-yl)ethyl)-phenylsulfamic acid | |
| AA88 | 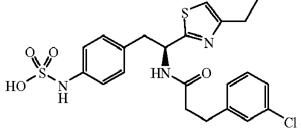 | 0.0002 |
| | (*S*)-4-(2-(3-(3-Chlorophenyl)propanamido)-2-(4-ethylthiazol-2-yl)ethyl)phenyl-sulfamic acid | |
| AA89 | 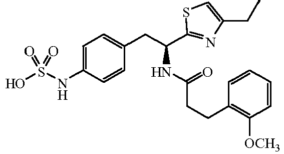 | 0.003 |
| | (*S*)-4-(2-(4-Ethylthiazol-2-yl)-2-(3-(2-methoxyphenyl)propanamido)ethyl)phenyl-sulfamic acid | |
| AA90 | 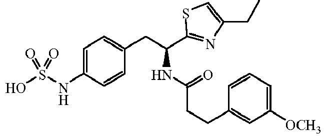 | 0.01 |
| | (*S*)-4-(2-(4-Ethylthiazol-2-yl)-2-(3-(3-methoxyphenyl)propanamido)ethyl)phenyl-sulfamic acid | |
| AA91 | 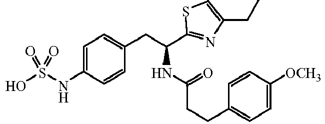 | 0.006 |
| | (*S*)-4-(2-(4-Ethylthiazol-2-yl)-2-(3-(4-methoxyphenyl)propanamido)ethyl)phenyl-sulfamic acid | |
| AA92 | 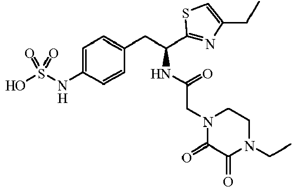 | 0.002 |
| | (*S*)-4-{2-[2-(4-Ethyl-2,3-dioxopiperazin-1-yl)acetamide]-2-(4-ethylthiazol-2-yl)ethyl}phenylsulfamic acid | |
| AA93 | 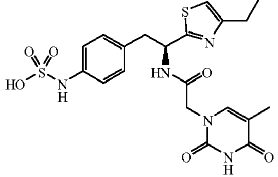 | 0.002 |
| | (*S*)-4-{2-(4-Ethylthiazol-2-yl)-2-[2-(5-methyl-2,4-dioxo-3,4-dihydropyrimidin-1(2*H*)-yl)acetamide]ethyl}phenylsulfamic acid | |
| AA94 | 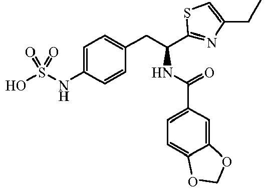 | 0.042 |
| | (*S*)-4-[2-(Benzo[*d*][1,3]dioxole-5-carboxamido)-2-(4-ethylthiazol-2-yl)ethyl]phenylsulfamic acid | |
| AA95 | 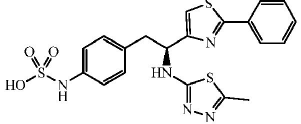 | 0.003 |
| | (*S*)-4-(2-(5-methyl-1,3,4-thiadiazol-2-ylamino)-2-(2-phenylthiazol-4-yl)ethyl)phenylsulfamic acid | |
| AA96 | 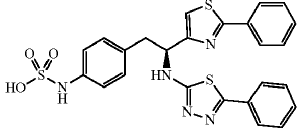 | 0.046 |
| | (*S*)-4-(2-(5-Phenyl-1,3,4-thiadiazol-2-ylamino)-2-(2-phenylthiazol-4-yl)ethyl)-phenylsulfamic acid | |
| AA97 | 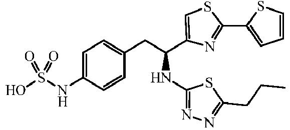 | 0.0002 |
| | 4-((*S*)-2-(5-Propyl-1,3,4-thiadiazol-2-ylamino)-2-(2-(thiophen-2-yl)thiazol-4-yl)ethyl)phenylsulfamic acid | |
| AA98 | 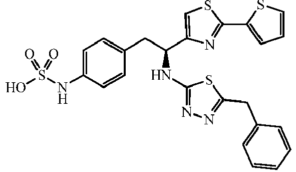 | 0.0006 |
| | 4-((*S*)-2-(5-Benzyl-1,3,4-thiadiazol-2-ylamino)-2-(2-(thiophen-2-yl)thiazol-4-yl)ethyl)phenylsulfamic acid | |
| AA99 | 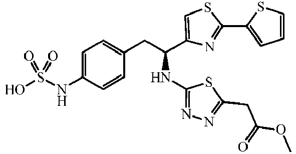 | 0.002 |
| | 4-((*S*)-2-(5-((Methoxycarbonyl)methyl)-1,3,4-thiadiazol-2-ylamino)-2-(2-(thiophen-2-yl)thiazol-4-yl)ethyl)phenylsulfamic acid | |
| AA100 | 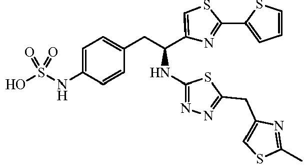 | 9x10⁻⁶ |
| | 4-((*S*)-2-(5-((2-Methylthiazol-4-yl)methyl)-1,3,4-thiadiazol-2-ylamino)-2-(2-(thiophen-2-yl)thiazol-4-yl)ethyl)phenylsulfamic acid | |

### METHODS

Disclosed are methods for the treatment of diseases or conditions of the eye, especially retinopathies, ocular edema and ocular neovascularization. Non-limiting examples of these diseases or conditions include diabetic macular edema, age-related macular degeneration (wet form), choroidal neovascularization, diabetic retinopathy, ocular ischemia, uveitis, retinal vein occlusion (central or branch), ocular trauma, surgery induced edema, surgery induced neovascularization, cystoid macular edema, ocular ischemia, uveitis, and the like. These diseases or conditions are characterized by changes in the ocular vasculature whether progressive or non-progressive, whether a result of an acute disease or condition, or a chronic disease or condition.

One aspect of the disclosed methods relates to diseases that are a direct or indirect result of diabetes, *inter alia,* diabetic macular edema and diabetic retinopathy. The ocular vasculature of the diabetic becomes unstable over time leading to conditions such as non-proliferative retinopathy, macular edema, and proliferative retinopathy. As fluid leaks into the center of the macula, the part of the eye where sharp, straight-ahead vision occurs, the buildup of fluid and the associated protein begin to deposit on or under the macula. This results in swelling that causes the subject's central vision to gradually become distorted. This condition is referred to as "macular edema." Another condition that may occur is non-proliferative retinopathy in which vascular changes, such as microaneurysms, outside the macular region of the eye may be observed.

These conditions may or may not progress to diabetic proliferative retinopathy which is characterized by neovascularization. These new blood vessels are fragile and are susceptible to bleeding. The result is scaring of the retina, as well as occlusion or total blockage of the light pathway through the eye due to the over formation of new blood vessels. Typically subjects having diabetic macular edema are suffering from the non-proliferative stage of diabetic retinopathy; however, it is not uncommon for subjects to only begin manifesting macular edema at the onset of the proliferative stage.

Diabetic retinopathy is the most common cause of vision loss in working-aged Americans (Klein R et al., "The Wisconsin Epidemiologic Study of Diabetic Retinopathy. II. Prevalence and risk of diabetic retinopathy when age at diagnosis is less than 30 years," Arch. Ophthalmol. 1984, 102:520-526). Severe vision loss occurs due to tractional retinal detachments that complicate retinal neovascularization (NV), but the most common cause of moderate vision loss is diabetic macular edema (DME). The pathogenesis of diabetic macular edema is not completely understood, but hypoxia is a contributing factor (Nguyen QD et al., "Supplemental inspired oxygen improves diabetic macular edema; a pilot study," Invest. Ophthalmol. Vis. Sci. 2003, 45:617-624). *Vascular endothelial growth factor* (*Vegf*) is a hypoxia-regulated gene and VEGF levels are increased in hypoxic or ischemic retina. Injection of VEGF into mouse eyes causes breakdown of the inner blood-retinal barrier (*See,* Derevjanik NL et al. Quantitative assessment of the integrity of the blood-retinal barrier in mice, Invest. Ophthalmol. Vis. Sci. 2002, 43:2462-2467) and sustained release of VEGF in the eyes of monkeys causes macular edema (Ozaki H et al., "Intravitreal sustained release of VEGF causes retinal neovascularization in rabbits and breakdown of the blood-retinal barrier in rabbits and primates," Exp Eye Res 1997, 64:505-517). This combination of observations in patients and animal models led to the hypothesis that VEGF plays an important role in the pathogenesis of diabetic macular edema. This hypothesis has been confirmed by several clinical trials that have shown that VEGF antagonists reduce foveal thickening and improve vision in patients with diabetic macular edema (Nguyen QD et al., "Vascular endothelial growth factor is a critical stimulus for diabetic macular edema," Am. J. Ophthalmol. 2006, 142:961-969; and Nguyen QD et al. "Primary End Point (Six Months) Results of the Ranibizumab for Edema of the mAcula in Diabetes (READ-2) Study," Ophthalmology 2009, 116:2175-2181).

The effects of VEGF on vascular endothelial cells are modulated by Tie2 receptors, which are selectively expressed on vascular endothelial cells and are required for embryonic vascular development (Dumont DJ et al., "Dominant-negative and targeted null mutations in the endothelial receptor tyrosine kinase, tek, reveal a critical role in vasculogenesis of the embryo," Genes Dev. 1994, 8:1897-1909). Angiopoietin 1 (Ang1) binds Tie2 with high affinity and initiates phosphorylation and downstream signaling (Davis S et al., "Isolation of angiopoietin-1, a ligand for the TIE2 receptor, by secretion-trap expression cloning," Cell 1996, 87:1161-1169). Mice deficient in Ang1 die around E12.5 with vascular defects similar to, but less severe than those seen in Tie2-deficient mice. Angiopoietin 2 (Ang2) binds Tie2 with high affinity, but does not stimulate phosphorylation in cultured endothelial cells. It acts as a competitive inhibitor of Ang1 and transgenic mice overexpressing Ang2 have a phenotype similar to Ang1-deficient mice. Several lines of evidence indicate that Ang2 is a developmentally- and hypoxia-regulated permissive factor for VEGF-induced neovascularization in the retina (Hackett SF et al., "Angiopoietin 2 expression in the retina: upregulation during physiologic and pathologic neovascularization," J. Cell. Physiol. 2000, 184:275-284). Double transgenic *Tet*/*opsin*/*ang2* and *Tet*/*opsin*/*ang1* mice with inducible expression of Ang2 or Ang1, respectively, have also helped to elucidate the role of Tie2 in the retina (Nambu H et al., "Angiopoietin 1 inhibits ocular neovascularization and breakdown of the blood-retinal barrier," Gene Ther. 2004, 11:865-873). In mice with ischemic retinopathy, increased expression of Ang2 when VEGF is high (P12-17) increases retinal neovascularization, but increased expression at P20 when VEGF levels have come down, hastens regression of retinal neovascularization and findings were similar in other models of ocular neovascularization. In contrast, increased expression of Ang1 suppressed neovascularization and reduced vascular leakage in several model. Therefore, Ang2 reduces stabilizing signals from the matrix making endothelial cells dependent upon VEGF and other soluble stimulators; when VEGF is high, neovascularization is stimulated and when VEGF is low, neovascularization regresses. In contrast, Ang1 increases stabilizing signals from the matrix and makes the vasculature unresponsive to soluble stimulators like VEGF.

Angiopoietin 2 binds Tie2, but does not stimulate phosphorylation and therefore acts as an antagonist under most circumstances. In the eye, angiopoietin 2 is upregulated at sites of neovascularization and acts as a permissive factor for VEGF. Increased expression of VEGF in the retina does not stimulate sprouting of neovascularization from the superficial or intermediate capillary beds of the retina or the choriocapillaris, but does stimulate sprouting from the deep capillary bed where there is constitutive expression of angiopoietin 2 (Hackett SF et al., "Angiopoietin-2 plays an important role in retinal angiogenesis," J. Cell. Physiol. 2002, 192:182-187). Co-expression of VEGF and angiopoietin 2 at the surface of the retina causes sprouting of neovascularization from the superficial retinal capillaries (Oshima Y et al., "Angiopoietin-2 enhances retinal vessel sensitivity to vascular endothelial growth factor," J. Cell. Physiol. 2004, 199:412-417). In double transgenic mice with inducible expression of angiopoietin 2 in the retina, expression of angiopoietin 2 when VEGF levels were high markedly enhanced neovascularization and expression of angiopoietin 2 when VEGF levels were low caused regression of neovascularization. In double transgenic mice with inducible expression of angiopoietin 1, the induced expression of angiopoietin 1 in the retina strongly suppressed VEGF-induced vascular leakage or neovascularization (Nambu H et al., "Angiopoietin 1 inhibits ocular neovascularization and breakdown of the blood-retinal barrier," Gene Ther. 2004, 11:865-873). In fact, in mice with high expression of VEGF in the retina which develop severe NV and retinal detachment, angiopoietin 1 is able to prevent the VEGF-induced detachments.

Regulation of Tie2 also occurs through an endothelial-specific phosphatase, vascular endothelial protein tyrosine phophatase (VE-PTP) in mice (Fachinger G et al., "Functional interaction of vascular endothelial-protein-tyrosine phosphatase with the angiopoietin receptor Tie-2," Oncogene 1999, 18:5948-5943) and its human orthologue human protein tyrosine phosphatase-β (HPTP-β) (Krueger NX et al., "Structural diversity and evolution of human receptor-like protein tyrosine phosphatases," EMBO J. 1990, 9:3241-3252). Mice deficient in VE-PTP die at E10 with severe defects in vascular remodeling and maturation of developing vasculature. Silencing of HPTP-β in cultured human endothelial cells, enhances Ang1-induced phosphorylation of Tie2 and survival-promoting activity while hypoxia increases expression of HPTP-β and reduces Ang1-induced phosphorylation of Tie2 (Yacyshyn OK et al., "Thyrosine phosphatase beta regulates angiopoietin-Tie2 signaling in human endothelial cells," Angiogenesis 2009, 12:25-33).

Diabetic retinopathy, if left untreated, can lead ultimately to blindness. Indeed, diabetic retinopathy is the leading cause of blindness in working-age populations.

Therefore, the disclosed methods relate to preventing, treating, controlling, abating, and/or otherwise minimizing ocular neovascularization in a subject having diabetes or a subject diagnosed with diabetes. In addition, subjects having or subjects diagnosed with diabetes can be alerted to or can be made aware of the risks of developing diabetes-related blindness, therefore the present methods can be used to prevent or delay the onset of nonproliferative retinopathy in subjects known to be at risk. Likewise, the present methods can be used for treating subjects having or being diagnosed with non-proliferative diabetic retinopathy to prevent progression of the condition.

The disclosed methods relate to preventing or controlling ocular neovascularization or treating a disease or condition that is related to the onset of ocular neovascularization by administering to a subject one or more or the disclosed compounds.

One method relates to treating or preventing ocular neovascularization by administering to a subject an effective amount of one or more of the disclosed compounds or pharmaceutically acceptable salts thereof. One example relates to a method for treating ocular edema and neovascularization comprising administering to a subject a composition comprising:
a) an effective amount of one or more of the disclosed compounds or pharmaceutically acceptable salts thereof; and
b) one or more carriers or compatible excipients.

The disclosed methods also relate to preventing or controlling ocular edema or treating a disease or condition that is related to the onset of ocular edema by administering to a subject one or more or the disclosed compounds.

One aspect of this method relates to treating or preventing ocular edema by administering to a subject an effective amount of one or more of the claimed compounds or pharmaceutically acceptable salts thereof. One embodiment of this aspect relates to a method for treating ocular edema comprising administering to a subject a composition comprising:
a) an effective amount of one or more of the claimed compounds or pharmaceutically acceptable salts thereof; and
b) one or more carriers or compatible excipients.

Another disclosed method relates to preventing or controlling retinal edema or retinal neovascularization or treating a disease or condition that is related to the onset of retinal edema or retinal neovascularization by administering to a subject one or more or the disclosed compounds. This method relates to treating or preventing retinal edema or retinal neovascularization by administering to a subject an effective amount of one or more of the disclosed compounds or pharmaceutically acceptable salts thereof, including treating retinal edema or retinal neovascularization comprising administering to a subject a composition comprising:
a) an effective amount of one or more of the disclosed compounds or pharmaceutically acceptable salts thereof; and
b) one or more carriers or compatible excipients.

A further disclosed method relates to treating, preventing or controlling diabetic retinopathy or treating a disease or condition that is related to the onset of diabetic retinopathy by administering to a subject one or more or the disclosed compounds.

This method relates to treating or preventing diabetic retinopathy by administering to a subject an effective amount of one or more of the disclosed compounds or pharmaceutically acceptable salts thereof, including a method for treating diabetic retinopathy comprising administering to a subject a composition comprising:
a) an effective amount of one or more of the disclosed compounds or pharmaceutically acceptable salts thereof; and
b) one or more carriers or compatible excipients.

Another relates to a method for treating or preventing non-proliferative retinopathy comprising administering to a subject a composition comprising:
a) an effective amount of one or more of the disclosed compounds or pharmaceutically acceptable salts thereof; and
b) one or more carriers or compatible excipients.

Another example relates to a method for treating or preventing non-proliferative retinopathy comprising administering to a subject a composition comprising:
a) an effective amount of one or more of the disclosed compounds or pharmaceutically acceptable salts thereof; and
b) one or more carriers or compatible excipients.

Yet a further disclosed method relates to preventing or controlling diabetic macular edema or treating a disease or condition that is related to the onset of diabetic macular edema by administering to a subject one or more or the claimed compounds.

One aspect of this method relates to treating or preventing diabetic macular edema by administering to a subject an effective amount of one or more of the claimed compounds or pharmaceutically acceptable salts thereof. One embodiment of this aspect relates to a method for treating diabetic macular edema comprising administering to a subject a composition comprising:
a) an effective amount of one or more of the claimed compounds or pharmaceutically acceptable salts thereof; and
b) one or more carriers or compatible excipients.

Any of the disclosed diseases or conditions described herein can be treated or prevented by administering to a subject from about 0.01 mg/kg to about 500 mg/kg of the disclosed compounds or pharmaceutically acceptable salts thereof. One iteration of this embodiment relates to a method for treating ocular edema and/or neovascularization comprising administering to a subject from about 0.01 mg/kg to about 50 mg/kg of the disclosed compounds or pharmaceutically acceptable salts thereof. Another iteration of this embodiment relates to administering to a subject from about 0.1 mg/kg to about 10 mg/kg by weight of the subject being treated, one or more of the disclosed compounds or pharmaceutically acceptable salts thereof. A further iteration of this embodiment relates to a method for treating or preventing diseases or conditions related to ocular edema and/or neovascularization comprising administering to a subject from about 1 mg/kg to about 10 mg/kg by weight of the subject one or more of the disclosed compounds or pharmaceutically acceptable salts thereof. A yet another iteration of this embodiment relates to a method for treating or preventing diseases or conditions related to ocular edema and/or neovascularization comprising administering to a subject from about 5 mg/kg to about 10 mg/kg by weight of the subject one or more of the disclosed compounds or pharmaceutically acceptable salts thereof. In a further iteration of this embodiment relates to a method for treating or preventing diseases or conditions related to ocular edema and/or neovascularization comprising administering to a subject from about 1 mg/kg to about 5 mg/kg by weight of the subject one or more of the disclosed compounds or pharmaceutically acceptable salts thereof. In a yet further iteration of this embodiment relates to a method for treating or preventing diseases or conditions related to ocular edema and/or neovascularization comprising administering to a subject from about 3 mg/kg to about 7 mg/kg by weight of the subject one or more of the disclosed compounds or pharmaceutically acceptable salts thereof.

Further disclosed are methods of treating or preventing one or more of the diseases or conditions described herein above related to ocular edema and/or neovascularization that are the result of administration of another pharmaceutically active agent. As such, this aspect relates to a method comprising administering to a subject a composition comprising:
a) an effective amount of one or more of the claimed compounds or pharmaceutically acceptable salts thereof;
b) one or more pharmaceutically active agents; and
c) one or more carriers or compatible excipients.

The methods of the present disclosure may be combined with the standard of care, including but not limited to laser treatment.

Disclosed herein are formulations comprising the disclosed compounds as eye drops, a form of drug delivery that is pharmaceutically-acceptable to patients, convenient, safe, with an onset of action of several minutes. A standard eye drop used in therapy according to federal regulatory practice is sterile, is isotonic (i.e., a pH of about 7.4 for patient comfort), and, if to be used more than once, contains a preservative but has a limited shelf life after opening, usually one month. If the eye drops are packaged in a sterile, single use only unit-dose dispenser the preservative can be omitted.

One method of eye drop formulation comprises the purest form of the disclosed compound (e.g., greater than 99% purity), and mix the compound with purified water and adjust for physiological pH and isotonicity. Examples of buffering agents to maintain or adjust pH include, but are not limited to, acetate buffers, citrate buffers, phosphate buffers and borate buffers. Examples of tonicity adjustors are sodium chloride, mannitol and glycerin. Other pharmaceutically acceptable ingredients can also be added.

The formulated solution is then aliquoted into either a plurality of discrete, sterile disposable cartridges each of which is suitable for unit dosing, or a single cartridge for unit dosing. Such a single disposable cartridge may be, for example, a conical or cylindrical specific volume dispenser, with a container having side-walls squeezable in a radial direction to a longitudinal axis in order to dispense the container contents therefrom at one end of the container. Such disposable containers are currently used to dispense eye drops at 0.3 to 0.4 mL (e.g., Lens Plus™ and Refresh Plus™) per unit dosing, and are ideally adaptable for the delivery of eye drops.

Ophthalmic eye-drop solutions are also packaged in multidose form, for example, as a plastic bottle with an eye-dropper (e.g., Visine™ Original). In such formulations, preservatives are required to prevent microbial contamination after opening of the container. Suitable preservatives include, but are not limited to: benzalkonium chloride, thimerosal, chlorobutanol, methyl paraben, propyl paraben, phenylethyl alcohol, edetate disodium, sorbic acid, polyquatemium-1, or other agents known to those skilled in the art, and all of which are contemplated for use in the present invention. Such preservatives are typically employed at a level of from 0.001 to about 1.0% weight/volume.

Eye drops provide a pulse entry of the drug, but the drug is rapidly diluted by tears and flushed out of the eye. Polymers can be added to ophthalmic solutions in order to increase the viscosity of the vehicle; this prolongs contact with the cornea, often enhancing bioavailability. The types of polymers permitted by the Federal Food and Drug Administration in ophthalmic solutions are defined concentrations of cellulose derivatives (methylcellulose, hydroxyethylcellulose, hydroxypropylcellulose and carboxymethylcellulose)- , dextran 70, gelatin, polyols, glycerin, polyethylene glycol 300, polyethylene glycol 400, polysorbate 80, propylene glyclol, polyvinyl alcohol and povidone, all of which (singly or in combination) are contemplated for use in the present invention.

In certain clinical conditions, the eye drop solutions can be formulated with other pharmaceutical agents, in order to attenuate the irritancy of the other ingredient and to facilitate clinical response. Such agents may include, but are not limited to, a vasoconstrictor such as phenylephrine, oxymetazoline, napthazoline or tetrahydrozoline; a mast-cell stabilizer such as olopatadine; an antihistamine such as azelastine; an antibiotic such as tetracycline; a steroidal anti-inflammatory drug such as betamethasone; a non-steroidal anti-inflammatory drug such as diclofenac; an immunomodulator such as imiquimod or interferons; and antiviral agents such as valaciclovir, cidofovir and trifluridine. The doses used for the above described purposes will vary, but will be in an effective amount to suppress discomfort, itch, irritation, or pain in the eye. When the compositions are dosed topically, the "pharmaceutically effective amount" of compound can generally be in a concentration range of from 0.05 mg/mL to about 500 mg/mL, with 1 to 4 drops administered as a unit dose 1 to 4 times per day. The most common method of ocular drug delivery is the instillation of drops into the lower eyelid (i.e., "eye drops"). About 70% of prescriptions for eye medication are for eye drops. This is due to factors such as expense, ease of bulk manufacture, and patient compliance, as well as effective and uniform
drug delivery. A key requirement is that the formulation be sterile and produced in a sterile environment. An ideal disclosed compound for use in ophthalmic solutions should be soluble and/or miscible in aqueous media at normal ocular pH and tonicity. Moreover, the disclosed compounds should be stable, non-toxic, long acting, and sufficiently potent to counteract dilution of drug concentration by blinking and tearing.

Also disclosed are methods from treating retinal neovascularization. Established retinal neovascularization can be treated by topically applying a composition comprising:
a) from about 0.05 mg/mL to about 500 mg/mL of one or more of the disclosed compounds; and
b) a pharmaceutically acceptable carrier.

In one example of this method, the composition comprises:
a) from about 0.5 mg/mL to about 50 mg/mL of one or more of the disclosed compounds; and
b) a pharmaceutically acceptable carrier.

In another example of this method, the composition comprises:
a) from about 0.05 mg/mL to about 5 mg/mL of one or more of the disclosed compounds; and
b) a pharmaceutically acceptable carrier.

In a further example of this method, the composition comprises:
a) from about 1 mg/mL to about 10 mg/mL of one or more of the disclosed compounds; and
b) a pharmaceutically acceptable carrier.

In a yet another example of this method, the composition comprises:
a) from about 5 mg/mL to about 50 mg/mL of one or more of the disclosed compounds; and
b) a pharmaceutically acceptable carrier.

In addition to the pharmaceutically acceptable carrier, these examples can comprise one or more pharmaceutically acceptable adjunct ingredients. Still further, the compositions can comprise:
In one example of this method, the composition comprises:
   a) from about 0.5 mg/mL to about 50 mg/mL of one or more of the disclosed compounds;
   b) an effective amount of one or more pharmaceutically active ingredients; and
   c) a pharmaceutically acceptable carrier.

Non-limiting examples of pharmaceutically active agents suitable for combination with the disclosed compounds include anti-infectives, *i.e.,* aminoglycosides, anti viral agents, antimicrobials, and the like; anticholinergics/antispasmotics; antidiabetic agents; antihypertensive agents; antineoplastics; cardiovascular agents; central nervous system agents; coagulation modifiers; hormones; immunologic agents; immunosuppressive agents; ophthalmic preparations; and the like.

The disclosed methods include administration of the disclosed compounds in combination with a pharmaceutically acceptable carrier. By "pharmaceutically acceptable" is meant a material that is not biologically or otherwise undesirable, *i.e.,* the material may be administered to a subject without causing any undesirable biological effects or interacting in a deleterious manner with any of the other components of the pharmaceutical formulation in which it is contained. The carrier would naturally be selected to minimize any degradation of the active ingredient and to minimize any adverse side effects in the subject, as would be well known to one of skill in the art. In another aspect, many of the disclosed compounds can be used prophylactically, *i.e.,* as a preventative agent, either neat or with a pharmaceutically acceptable carrier. The ionic liquid compositions disclosed herein can be conveniently formulated into pharmaceutical compositions composed of neat ionic liquid or in association with a pharmaceutically acceptable carrier. *See e.g.,* Remington's Pharmaceutical Sciences, latest edition, by E.W. Martin Mack Pub. Co., Easton, PA, which discloses typical carriers and conventional methods of preparing pharmaceutical compositions that can be used in conjunction with the preparation of formulations of the compounds described herein and which is incorporated by reference herein. Such pharmaceutical carriers, most typically, would be standard carriers for administration of compositions to humans and non-humans, including solutions such as sterile water, saline, and buffered solutions at physiological pH. Other compounds can be administered according to standard procedures used by those skilled in the art. For example, pharmaceutical compositions can also include one or more additional active ingredients such as antimicrobial agents, anti-inflammatory agents, anesthetics, and the like. Examples of pharmaceutically-acceptable carriers include, but are not limited to, saline, Ringer's solution and dextrose solution. The pH of the solution is preferably from about 5 to about 8, and more preferably from about 7 to about 7.5. Further carriers include sustained release preparations such as semipermeable matrices of solid hydrophobic polymers containing the disclosed compounds, which matrices are in the form of shaped articles, e.g., films, liposomes, microparticles, or microcapsules. It will be apparent to those persons skilled in the art that certain carriers can be more preferable depending upon, for instance, the route of administration and concentration of composition being administered. Other compounds can be administered according to standard procedures used by those skilled in the art.

The disclosed method also relates to the administration of the disclosed compounds and compositions. Administration can be systemic via subcutaneous or i.v. administration; or the HPTP-β inhibitor will be administered directly to the eye, e.g., local. Local methods of administration include, for example, by eye drops, subconjunctival injections or implants, intravitreal injections or implants, sub-Tenon's injections or implants, incorporation in surgical irrigating solutions, etc.

The disclosed methods relate to administering the disclosed compounds as part of a pharmaceutical composition. Compositions suitable for topical administration are known to the art (see, for example, US Patent Application 2005/0059639). In various embodiments, compositions of the invention can comprise a liquid comprising an active agent in solution, in suspension, or both. As used herein, liquid compositions include gels. In one embodiment, the liquid composition is aqueous. Alternatively, the composition can take form of an ointment. In another embodiment, the composition is an *in situ* gellable aqueous composition. In iteration, the composition is an *in situ* gellable aqueous solution. Such a composition can comprise a gelling agent in a concentration effective to promote gelling upon contact with the eye or lacrimal fluid in the exterior of the eye. Aqueous compositions of the invention have ophthalmically compatible pH and osmolality. The composition can comprise an ophthalmic depot formulation comprising an active agent for subconjunctival administration. The microparticles comprising active agent can be embedded in a biocompatible pharmaceutically acceptable polymer or a lipid encapsulating agent. The depot formulations may be adapted to release all or substantially all the active material over an extended period of time. The polymer or lipid matrix, if present, may be adapted to degrade sufficiently to be transported from the site of administration after release of all or substantially all the active agent. The depot formulation can be a liquid formulation, comprising a pharmaceutical acceptable polymer and a dissolved or dispersed active agent. Upon injection, the polymer forms a depot at the injections site, e.g. by gelifying or precipitating. The composition can comprise a solid article that can be inserted in a suitable location in the eye, such as between the eye and eyelid or in the conjuctival sac, where the article releases the active agent. Solid articles suitable for implantation in the eye in such fashion generally comprise polymers and can be bioerodible or non-bioerodible.

In one embodiment of the disclosed methods, a human subject with at least one visually impaired eye is treated with 2-4000 µg of a disclosed compound via intravitreal injection. Improvement of clinical symptoms are monitored by one or more methods known to the art, for example, indirect ophthalmoscopy, fundus photography, fluorescein angiopathy, electroretinography, external eye examination, slit lamp biomicroscopy, applanation tonometry, pachymetry, optical coherence tomography and autorefaction. Subsequent doses can be administered weekly or monthly, e.g., with a frequency of 2-8 weeks or 1-12 months apart.

The disclosed compositions include administration of the disclosed compounds in combination with a pharmaceutically acceptable carrier. By "pharmaceutically acceptable" is meant a material that is not biologically or otherwise undesirable, *i.e.,* the material may be administered to a subject without causing any undesirable biological effects or interacting in a deleterious manner with any of the other components of the pharmaceutical formulation in which it is contained. The carrier would naturally be selected to minimize any degradation of the active ingredient and to minimize any adverse side effects in the subject, as would be well known to one of skill in the art. In another aspect, many of the disclosed compounds can be used prophylactically, *i.e.,* as a preventative agent, either neat or with a pharmaceutically acceptable carrier. The ionic liquid compositions disclosed herein can be conveniently formulated into pharmaceutical compositions composed of neat ionic liquid or in association with a pharmaceutically acceptable carrier. *See e.g.,* Remington's Pharmaceutical Sciences, latest edition, by E.W. Martin Mack Pub. Co., Easton, PA, which discloses typical carriers and conventional methods of preparing pharmaceutical compositions that can be used in conjunction with the preparation of formulations of the compounds described herein and which is incorporated by reference herein. Such pharmaceutical carriers, most typically, would be standard carriers for administration of compositions to humans and non-humans, including solutions such as sterile water, saline, and buffered solutions at physiological pH. Other compounds can be administered according to standard procedures used by those skilled in the art. For example, pharmaceutical compositions can also include one or more additional active ingredients such as antimicrobial agents, anti-inflammatory agents, anesthetics, and the like. Examples of pharmaceutically-acceptable carriers include, but are not limited to, saline, Ringer's solution and dextrose solution. The pH of the solution is preferably from about 5 to about 8, and more preferably from about 7 to about 7.5. Further carriers include sustained release preparations such as semipermeable matrices of solid hydrophobic polymers containing the disclosed compounds, which matrices are in the form of shaped articles, e.g., films, liposomes, microparticles, or microcapsules. It will be apparent to those persons skilled in the art that certain carriers can be more preferable depending upon, for instance, the route of administration and concentration of composition being administered. Other compounds can be administered according to standard procedures used by those skilled in the art.

Pharmaceutical formulations can include additional carriers, as well as thickeners, diluents, buffers, preservatives, surface active agents and the like in addition to the compounds disclosed herein. Pharmaceutical formulations can also include one or more additional active ingredients such as antimicrobial agents, anti-inflammatory agents, anesthetics, and the like.

For the purposes of the present disclosure the term "excipient" and "carrier" are used interchangeably throughout the description of the present disclosure and said terms are defined herein as, "ingredients which are used in the practice of formulating a safe and effective pharmaceutical composition."

The formulator will understand that excipients are used primarily to serve in delivering a safe, stable, and functional pharmaceutical, serving not only as part of the overall vehicle for delivery but also as a means for achieving effective absorption by the recipient of the active ingredient. An excipient may fill a role as simple and direct as being an inert filler, or an excipient as used herein may be part of a pH stabilizing system or coating to insure delivery of the ingredients safely to the stomach. The formulator can also take advantage of the fact the compounds of the present disclosure have improved cellular potency, pharmacokinetic properties, as well as improved oral bioavailability.

The term "effective amount" as used herein means "an amount of one or more of the disclosed compounds, effective at dosages and for periods of time necessary to achieve the desired or therapeutic result." An effective amount may vary according to factors known in the art, such as the disease state, age, sex, and weight of the human, animal being treated or route of administration. Although particular dosage regimes may be described in examples herein, a person skilled in the art would appreciated that the dosage regime may be altered to provide optimum therapeutic response. For example, several divided doses may be administered daily or the dose may be proportionally reduced as indicated by the exigencies of the therapeutic situation. In addition, the compositions of the present disclosure can be administered as frequently as necessary to achieve a therapeutic amount.

The disclosed compounds can also be present in liquids, emulsions, or suspensions for delivery of active therapeutic agents in aerosol form to cavities of the body such as the nose, throat, or bronchial passages. The ratio of disclosed compound to the other compounding agents in these preparations will vary as the dosage form requires.

Depending on the intended mode of administration, the pharmaceutical compositions administered as part of the disclosed methods can be in the form of solid, semi-solid or liquid dosage forms, such as, for example, tablets, suppositories, pills, capsules, powders, liquids, suspensions, lotions, creams, gels, or the like, preferably in unit dosage form suitable for single administration of a precise dosage. The compositions will include, as noted above, an effective amount of one or more of the disclosed compounds in combination with a pharmaceutically acceptable carrier and, in addition, can include other medicinal agents, pharmaceutical agents, carriers, adjuvants, diluents, etc.

For solid compositions, conventional nontoxic solid carriers include, for example, pharmaceutical grades of mannitol, lactose, starch, magnesium stearate, sodium saccharin, talc, cellulose, glucose, sucrose, magnesium carbonate, and the like.

Liquid pharmaceutically administrable compositions can, for example, be prepared by dissolving, dispersing, etc., an active compound as described herein and optional pharmaceutical adjuvants in an excipient, such as, for example, water, saline aqueous dextrose, glycerol, ethanol, and the like, to thereby form a solution or suspension. If desired, the pharmaceutical composition to be administered can also contain minor amounts of nontoxic auxiliary substances such as wetting or emulsifying agents, pH buffering agents and the like, for example, sodium acetate, sorbitan monolaurate, triethanolamine sodium acetate, triethanolamine oleate, etc. Actual methods of preparing such dosage forms are known, or will be apparent, to those skilled in this art; for example see *Remington's Pharmaceutical Sciences,* referenced above.

### KITS

Also disclosed are kits comprising the compounds and compositions to be delivered into a human, mammal, or cell. The kits can comprise one or more packaged unit doses of a composition comprising one or more compounds to be delivered into a human, mammal, or cell. The unit dosage ampoules or multi-dose containers, in which the compounds to be delivered are packaged prior to use, can comprise a hermetically sealed container enclosing an amount of polynucleotide or solution containing a substance suitable for a pharmaceutically effective dose thereof, or multiples of an effective dose. The compounds can be packaged as a sterile formulation, and the hermetically sealed container is designed to preserve sterility of the formulation until use.

### PROCEDURES

### Measurement and Quantization of Retinal Edema and Neovascularization and the Effective Inhibition of Ocular Edema and New Blood Vessel Formation by the disclosed HPTP-β Inhibitors

The following studies were conducted to measure the effect of the disclosed compounds on vascular leak and neovascularization of retina tissue.

### Study 1

Twenty (20) 21 day old Rhodopsin/VEGF transgenic mice having constitutive expression of VEGF in their retinal neurons as disclosed by Tobe T et al., in "Evolution of neovascularization in mice with over expression of vascular endothelial growth factor in photoreceptors" Invest Ophthalmol Visual Sci. 1998; 39:180-188 mg/kg, were divided into two equal groups. Each group received subcutaneous injections as follows: **Group 1** received 10 mg/kg/injection (high dose) of a compound from Table XXI; and **Group 2** received injections of vehicle. Two injections were given to each animal on Day 1 while one injection was given to each animal on Day 2.

Two hours after the third and final dose on Day 2, the animals were euthanized, mice were euthanized, retinas removed, and immunohistochemical staining for albumin was done. Briefly, eyes were harvested and fixed in 10% PBS-buffered formalin for 2 hours at room temperature. Retinas were dissected and put in PBS in an Eppendorf tube and blocked with 8% normal donkey serum with 0.05% triton for 1 hour. A goat anti-mouse albumin antibody (Abcam) was added at a 1:150 dilution and stained at RT for 2 hours. After 3 washes with PBST, a donkey anti-goat antibody conjugated with DyLight 593 (from Jackson ImmunoResearch) was added and stained at RT for 50 minutes avoiding light. After 3 washes with PBST, retinas were mounted and imaged by fluorescence microscopy. Areas of albumin staining were assessed using image analysis software (ImagePro Plus 5).

### Study 2

Twenty (20) 21 day old Rhodopsin/VEGF transgenic mice having constitutive expression of VEGF in their retinal neurons were divided into two equal groups. Each group received subcutaneous injections as follows: **Group 1** received 3 mg/kg/injection (low dose) of a compound from Table XXI; and **Group 2** received injections of vehicle. Two injections were given to each animal on Day 1 while one injection was given to each animal on Day 2.

Two hours after the final dose on Day 2 the animals were euthanized and ocular samples were prepared as described in **Study 1** above. **Figure 1B** is a histogram showing the relative concentrations of albumin found on the surfaces of the retinas of the control animals versus the animals treated with 3 mg/kg/dose. Comparing **Figure 1A** to **Figure 1B** mg/kg, these data indicate that the animals treated with a lower dose of 3 mg/kg/injection had less neovascularization than the animals dosed with 3 mg/kg/dose.

**Figure 2A** and **Figure 2B** are representative photomicrographs of the immunohistochemically stained retinas obtained from the sacrificed animals described herein above. **Figure 2B** is a photomicrograph of the retina of a vehicle-treated transgenic mouse. Arrowheads indicate focal, perivasular deposits of albumin (an indicator of vascular leak). As can be seen in these micrographs, the control animal showed significant deposition of albumin in the retina whereas the retina of a transgenic mouse treated with 3 mg/kg/dose showed a marked reduction of perivasular deposits of albumin. **(****Figure 2B****).**

### Study 3

Thirty (30) Rhodopsin/VEGF transgenic mice having constitutive expression of VEGF in their retinal neurons were divided into three equal groups. At day 14 post natal each group received subcutaneous injections as follows: **Group 1** received 10 mg/kg/injection (high dose) of a compound from Table XXI; **Group 2** received 3 mg/kg/injection (low dose) of a compound from Table XXI; and **Group 3** received injections of vehicle. Each animal received two injections per day for 7 days.

At P21, mice were sacrificed; eyes were harvested and fixed in 10% PBS-buffered formalin for 2 hours at room temperature. Retinas were dissected and put in PBS in an Eppendorf tube and stained with GSA-Lectin conjugated with FITC for 2 hours at room temperature. After 3 washes with PBST, retinas were mounted and imaged by fluorescence microscopy. Areas of retinal neovascularization were assessed using image analysis software (ImagePro Plus 5).

**Figure 3A** (arrows) shows the significant level of sprouting of new blood vessels (neovascular tufts) in the retina of the vehicle-treated animals while **Figure 3B** shows the marked reduction of neovascular tufts in the retinas of animals treated with compound D91.

**Figure 4** compares the relative amounts of neovascularization found in this study between animals treated with 10 mg/kg/injection, 3 mg/kg/injection and vehicle. Consistent with the data shown in Figures 1A and 1B, animals dosed with 3 mg/kg/injection had significantly reduced levels of retinal neovascularization than the animals dosed with 10 mg/kg/injection and vehicle.

### VE-PTP (HPTP-β) is upregulated in retinal endothelial cells participating in neovascularization

C57BL/6 mice were treated in accordance with the Association for Research in Vision and Ophthalmology Guidelines on the care and use of animals in research. The mice were placed in 75% oxygen at postnatal day (P) 7 and returned to room air on P12 to create retinopathy of prematurity (ROP). Postnatal day (P) 17, mice with oxygen-induced ischemic retinopathy have areas of ischemic retina and develop neovascularization on the surface of the retina. The mice were euthanized and eyes were fixed in 4% paraformaldehyde at room temperature for 4 hours and retinas were dissected. After blocking with 10% normal goat serum for 1 hour, retinas were incubated with 1:200 rabbit anti-VE-PTP (mouse orthologue of HPTPβ) antibody at for 3 hours. After washing, retinas were incubated with 1:800 goat anti-rabbit antibody conjugated with Cy-3 (Jackson Immuno Laboratory) and then counterstained with FITC-conjugated Griffonia Simplicifolia lectin (GSA). Retinas were flat mounted and examined by fluorescence microscopy (Axioskop; Zeiss, Thornwood, NY).

At P17, retinas from mice with ROP and retinal neovascularization (NV) were dissected and immunofluorescently stained for VE-PTP/HPTP-β and also stained with FITC-labeled Griffonia Simplicifolia (GSA) lectin. As can be seen in **Figures A and D,** the GSA staining shows clumps of neovascularization on the surface of the retina with some faint staining of retinal vessels in the background. As depicted in **Figures B and C,** there was strong staining for HPTP-β in clumps of retinal neovascularization on the surface of the retina and faint staining of some underlying retinal vessels, primarily feeder vessels leading to the neovascularization.

Fluorescence microscopy with the green channel showed clumps of GSA-stained neovascularization on the surface of the retina with some faint staining of retinal vessels in the background **(Figures A and D).** The retina from a room air (RA) control mouse showed normal retinal vessels with no neovascularation **(Figure G).** There was strong staining for HPTP-β in clumps of retinal neovascularization on the surface of the retina and faint staining of some underlying retinal vessels, primarily feeder vessels leading to the neovascularization **(Figures B and C).** There was no detectable staining of retinal vessels in the non-ischemic retinas of RA control mice **(Figures H and I).** Therefore, VE-PTP/ HPTP-β is upregulated in retinal endothelial cells participating in neovascularization.

### Blockade of VE-PTP (HPTP-β) with a disclosed HPTP-β inhibitor promotes phosphorylation of Tie2 in the retinal endothelial cells in vivo

Transgenic mice in which the rhodopsin promoter drives expression of *Vegf* in photoreceptors (*rho*/*VEGF* mice) were used as a model of subretinal neovascularization. At P7, increased levels of VEGF are detectable in photoreceptors, at P14 there are sprouts of neovascularizatiion extending from the deep capillary bed of the retina into the subretinal space, and at P21 there are several clumps of neovascularization in the subretinal space. Rho/VEGF mice were given a subcutaneous injection of vehicle or 10 mg/kg of a compound from Table XXI at P21. Twelve hours after injection 12 hours after injection, mice were euthanized. Eyes were fixed in 4% paraformaldehyde at room temperature for 4 hours and retinas were dissected. After blocking with 10% normal goat serum for 1 hour, retinas were incubated with 1:200 rabbit anti-phospho-TIE2 antibody (R&D, Minneapolis, MN, USA) at room temperature for 3 hours. After washing, retinas were incubated with goat anti-rabbit antibody conjugated with Cy-3. The retinas were immunostained for phosphorylated Tie2 and counter-stained with Griffonia Simplicifolia (GSA) lectin which labels vascular cells. Retinas from control (vehicle-treated rho/VEGF mice) showed light phosphoTie staining in neovasculatization **(****Figures 6A to 6C****).** Retinas from the compound from Table XXI-treated rho/VEGF mice showed strong phosphoTie2 staining in neovascularization and faint staining of some vessels within the retina **(****Figures 6D** **to6 F).** These results indicate that a compound from Table XXI promotes phosphorylation of Tie2 in retinal endothelial cells, particularly in those participating in neovascularization.

### Suppression of ischemia-induced retinal neovascularization

Mice with oxygen-induced ischemic retinopathy, a model predictive of effects in proliferative diabetic retinopathy, were given an intraocular injection of 3 µg of a compound from Table XXI in one eye and vehicle in the fellow eye. At P17, there was little nevascularization on the surface of the retina in eyes treated with a compound from Table XXI **(****Figure 7A****)** compared to retinas from eyes treated with vehicle **(****Figure 7B****).** Measurement of the mean area of retinal neovascularization on the surface of the retina by image analysis confirmed that intraocular application of the compound from Table XXI caused a significant reduction in retinal neovascularization **(****Figure 7C****)** (p=0.019 by unpaired t-test).

### Suppression of subretinal neovascularization in rho/VEGF transgenic mice

The subretinal neovascularization that occurs in *rho*/*VEGF* mice is similar to what has been termed retinal angiomatous proliferation (RAP) which occurs in 30% of patients with neovascular AMD (*See,* Yannuzzi LA et al., "Retinal angiomatous proliferation in age-related macular degeneration," Retina 2001, 21:416-434). Efficacy in this model has predicted a good outcome in patients with neovascular age-related macular degeneration. Hemizygous rho/VEGF transgenic mice were given daily subcutaneous injections of vehicle containing 0, 3, or 10 mg/kg of a compound from Table XXI starting at postnatal day (P) 15. At P21, mice that had been treated with vehicle showed many clumps of subretinal neovascularization **(****Figure 8A****),** while mice that had been treated with 3 mg/kg **(****Figure 8B****)** or 10 mg/kg of a compound from Table XXI **(****Figure 8C****)** had fewer buds of neovascularization. Compared to mice treated with vehicle, the mean area of subretinal neovascularization was significantly less in mice treated with either dose of a compound from Table XXI **(****Figure 8D****).** Intraocular injection of a compound from Table XXI also strongly suppressed subretinal neovascularization in rho/VEGF mice **(****Figure 8****, Frames E to G).**

### The disclosed compounds suppress choroidal neovascularization

A mouse model of choroidal neovascularization that is predictive of a compound's effect in patients with neovascular AMD, subcutaneous injections of 20 or 40 mg/kg a compound from Table XXI significantly reduced choroidal neovascularization **(****Figure 9****, Frames A to C).** Intraocular injection of 3 µg (p=0.0009) or 5 µg of a compound from Table XXI (p=0.022), but not 1 µg significantly suppressed choroidal neovascularization compared to injection of vehicle **(****Figure 9 D)****.** (See, Saishin Y et al., "VEGF-TRAPR1R2 suppresses choroidal neovascularization and VEGF-induced breakdown of the blood-retinal barrier," J. Cell. Physiol. 2003, 195:241-248 and Heier JS et al., "The 1-year results of CLEAR-IT 2, a phase 2 study of vascular endothelial growth factor trap-eye dosed as needed after 12-week fixed dosing," Ophthalmology 2011, 118:1098-1106.)

### The disclosed compounds reduce VEGF-induced retinal vascular leakage

Sustained delivery of VEGF in the vitreous cavity of primates causes macular edema (Ozaki, 1997) and VEGF has been validated as a critical target in diabetic macular edema (Nguyen, 2006 and Nguyen, 2009). In *rho*/*VEGF* transgenic mice, the excess production of VEGF in photoreceptors causes leakage of plasma and serum proteins into the retina. In the healthy retina, there is little serum protein, for example, albumin. Therefore immunohistochemical staining for albumin was used to assess for breakdown of the blood-retinal barrier. There was little staining for albumin seen in the retinas of rho/VEGF mice treated with 10 mg/kg of a compound from Table XXI **(****Figure 10****, Frames A to C),** while the retinas of vehicle-treated mice showed strong staining for albumin surrounding new vessels and mild diffuse staining elsewhere **(****Figure 10****, Frames D to F).** The mean area of albumin staining was significantly reduced in rho/VEGF mice injected with 3 mg/kg (n=10, p=0.03) or 10 mg/kg (n=10, p=0.04) of a compound from Table XXI compared to corresponding controls (n=8 for each, **Figure 10 B)****.**

### The disclosed compounds prevent retinal detachment in Tet/opsin/VEGF double transgenic mice

*Tet*/*opsin*/*VEGF* double transgenic mice represent an extremely aggressive model of ocular neovascularization and vascular leakage. When treated with doxycycline they develop severe neovascularization and exudative retinal detachment. When given injections of doxycycline, double transgenic mice with doxycycline-inducible expression of VEGF express 10-fold higher levels of VEGF than rho/VEGF transgenic mice and develop severe neovascularization and exudative retinal detachments within 3-5 days.

Double hemizygous *Tet*/*opsin*/*VEGF* mice were pretreated for 3 days with twice a day subcutaneous injections of 3 (n=10), 10 (n=8), or 50 mg /kg (n=10) of a compound from Table XXI or vehicle (n=10) and then treatment was continue for 4 days during which 50 mg/kg of doxycycline was also injected. After 4 days, mice were euthanized and eyes were frozen in optical cutting temperature (OCT) embedding solution. Ten micron ocular sections through the optic nerve were stained with Hoechst (1:1,000; Sigma, St. Louis, MO). Sections were examined by light microscopy and the total length of the retina and the length of the retina that was detached was measured by image analysis with the investigator masked with respect to treatment group. The percentage the retina that was detached was computed.

Near total retinal detachments occurred in all mice treated with vehicle **(****Figure 11A****, column 1 and** **Figure 11B****, column 1)** and there was little difference in mice treated with 3 mg/kg of a compound from Table XXI **(****Figure 11C****).** As indicated in **Figure 11A****, column 2 and 3 and** **Figure 11C****,** mice treated with 10 or 50 mg/kg had a significant reduction in percentage of retinal detachment per section (p=0.04295 and p<0.0001). All mice treated with 50 mg/kg of a compound from Table XXI had completely attached retinas.

### Disclosed compounds cause regression of VEGF induced retinal neovascularization.

Using the same procedures as described herein above, the eyes of Rho/VEGF mice were treated subcutaneously twice a day with 10 mg/kg of a compound from Table XXI or with vehicle beginning on P21 and concluding on P27. **Figure 12A** depicts the retina of a control animal receiving only vehicle while **Figure 12B** depicts the retina of an animal treated with a compound from Table XXI. As seen in the photographs and as summarized in **Figure 12C****,** animals treated with a compound from Table XXI had a decreased area of retinal neovascularization.

### Disclosed compounds prevent VEGF induced retinal neovascularization when administered topically.

Using the same procedures as described herein above, the eyes of Rho/VEGF mice were treated with a topically applied composition as disclosed herein three times a day with such that 30 mg/ml of a compound from Table XXI is delivered beginning on P21 and concluding on P27. Control mice were treated topically with vehicle only. **Figure 13A** depicts the retina of a control animal receiving only vehicle while **Figure 13B** depicts the retina of an animal treated with a compound from Table XXI. As seen in the photographs and as summarized in **Figure 13C****,** animals treated with a compound from Table XXI had a decreased area of retinal neovascularization.

### Mouse model of oxygen-induced ischemic retinopathy

Ischemic retinopathy was produced in C57BL/6 mice by a method described by Smith LEH et al., "Oxygen-induced retinopathy in the mouse," Invest. Ophthalmol. Vis. Sci. 1994,35: 101-111. Postnatal day (P) 7 mice and their mothers were placed in an airtight incubator and exposed to an atmosphere of 75 ± 3% oxygen for 5 days. Oxygen was continuously monitored with a PROOX model 110 oxygen controller (Reming Bioinstruments Co., Redfield, NY). At P12, mice were returned to room air and under a dissecting microscope, a Harvard Pump Microinjection System and pulled glass pipettes were used to give a 1 µl intraocular injection of 3 µg of a compound from Table XXI in one eye and vehicle in the fellow eye. At P17, the area of neovascularization on the surface of the retina was measured at P17 as previously described by Shen J et al., "In vivo immunostaining demonstrates macrophages associate with growing and regressing vessels," Invest. Ophthalmol. Vis. Sci. 2007, 48:4335-4341. Briefly, mice were given an intraocular injection of 1 µl containing 0.5 µg rat anti-mouse PECAM antibody (Pharmingen, San Jose, CA) and after 12 hours they were euthanized and eyes were fixed in 10% formalin for 4 hours. Retinas were dissected, incubated for 40 minutes in 1:500 goat anti-rat IgG conjugated with Alexa488 (Invitrogen, Carlsbad, CA), washed, and whole mounted. An observer masked with respect to treatment group examined the slides with a Nikon Fluorescence microscope and measured the area of neovascularization per retina by computerized image analysis using ImagePro Plus software (Media Cybernetics, Silver Spring, MD).

### Transgenic mice with increased expression of VEGF in photoreceptors

Transgenic mice in which the rhodopsin promoter drives expression of VEGF in photoreceptors (rho/VEGF mice) have onset of VEGF expression at P7 and starting at P10 develop sprouts of neovascularization from the deep capillary bed of the retina that grow through the photoreceptor layer and form an extensive network of new vessels in the subretinal space. Between P15 and P21, hemizygous rho/VEGF mice were given a subcutaneous injection of 3 mg/kg or 10 mg/kg of a compound from Table XXI or vehicle twice a day. In another experiment at P15 and P17, mice were given an intraocular injection of 3 µg of a compound from Table XXI in one eye and vehicle in the fellow eye. At P21, the mice were euthanized and eyes were fixed in 4% paraformaldehyde for 6 hours. Retinas were dissected and blocked with 3% bovine serum albumin in PBS for one hour. FITC conjugated GSA was used to stain the retinas at room temperature for 2 hours and then the retinas were flat mounted with the photoreceptor side up and examined by fluorescence microscopy. The area of subretinal neovascularization was measured by image analysis with the investigator masked with respect to treatment group.

### Laser-induced choroidal neovascularization model

Choroidal neovascularization was generated as previously described. Adult C57BL/6 mice had rupture of Bruch's membrane in 3 locations in each eye and then were given subcutaneous injections of 10 or 30 mg/kg of a compound from Table XXI or vehicle twice a day for 14 days. In another experiment, mice were given an intraocular injection of 1 µl containing 3 or 5 µg of a compound from Table XXI in one eye and vehicle in the fellow eye immediately after rupture of Bruch's membrane and 7 days later. Fourteen days after laser, mice were perfused with fluorescein-labeled dextran (2×10⁶ average MW, Sigma, St. Louis, MO) and choroidal flat mounts were examined by fluorescence microscopy. The area of choroidal neovascularization at each Bruch's membrane rupture site was measured by image analysis by an observer masked with respect to treatment group. The area of choroidal neovascularization at the 3 rupture sites in one eye were averaged to give one experimental value.

### Immunofluorescent staining for serum albumin to assess vascular leakage

At P20, rho/VEGF mice were given a subcutaneous injection of 3 or 10 mg/kg of a compound from Table XXI or vehicle which was repeated 12 hours later. At P21, a third injection was given and then and 2 hours later, mice were euthanized, retinas were dissected and immunofluorescently stained for albumin as previously described by Lima e Silva R et al., "Agents that bind annexin A2 suppress ocular neovascularization," J. Cell. Physiol. 2010, 225:855-864. The vessels were labeled by counterstaining with GSA lectin. Retinas were flat mounted, examined by fluorescence microscopy, and the area of albumin staining was measured by image analysis with the investigator masked with respect to treatment group.

Other advantages which are obvious and which are inherent to the invention will be evident to one skilled in the art. It will be understood that certain features and sub-combinations are of utility and may be employed without reference to other features and sub-combinations. This is contemplated by and is within the scope of the claims. Since many possible embodiments may be made of the invention without departing from the scope thereof, it is to be understood that all matter herein set forth or shown in the accompanying drawings is to be interpreted as illustrative and not in a limiting sense.

## Claims

1. A compound for use in treating an ocular edema of an eye of a subject, wherein the compound has the formula selected from the group consisting of: 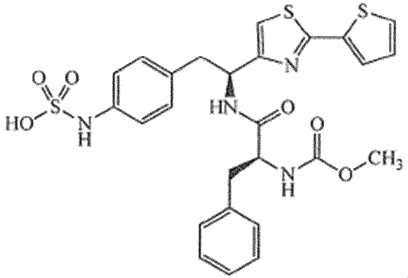 or a pharmaceutically acceptable salt thereof; 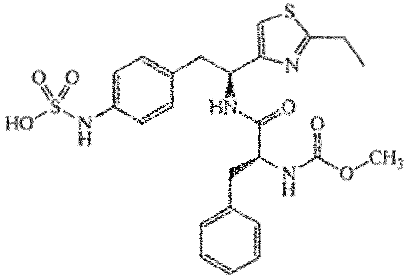 or a pharmaceutically acceptable salt thereof; and 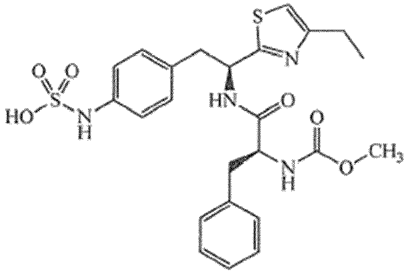 or a pharmaceutically acceptable salt thereof.

2. A compound for use according to Claim 1, wherein the compound has the formula:

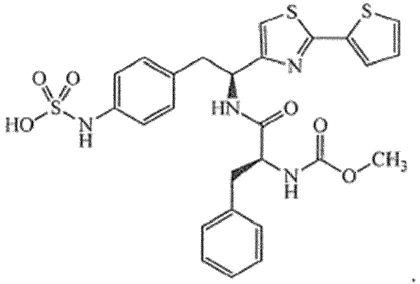

3. A compound for use according to Claim 1, wherein the compound has the formula:

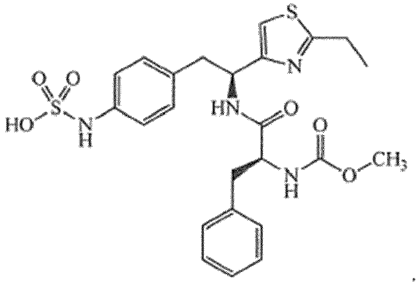

4. A compound for use according to Claim 1, wherein the compound is the formula:

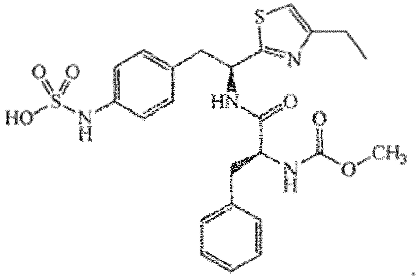

5. A compound having the formula as defined in any one of Claims 1 to 4 or a pharmaceutically acceptable salt thereof, for use according to claim 1, wherein the ocular edema is diabetic macular edema.

6. Pharmaceutical preparation comprising a compound as defined in one of Claims 1 to 4 or a pharmaceutically acceptable salt thereof, and one or more carriers or compatible excipients, for use in treating ocular edema.

## Patentansprüche

1. Verbindung zur Verwendung beim Behandeln eines Okularödems eines Auges in einem Subjekt, wobei die Verbindung eine Formel aufweist, die ausgewählt ist aus der Gruppe bestehend aus: 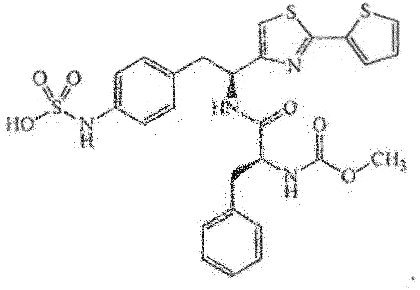 oder einem pharmazeutisch unbedenklichen Salz davon; 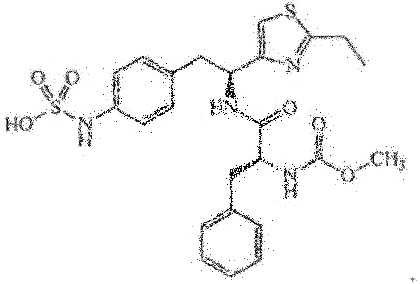 oder einem pharmazeutisch unbedenklichen Salz davon; und 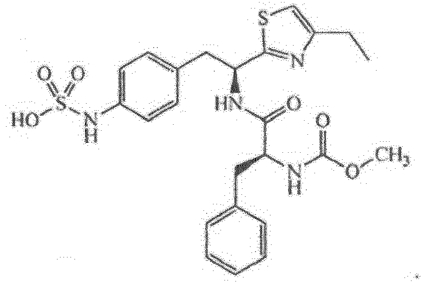 oder einem pharmazeutisch unbedenklichen Salz davon.

2. Verbindung zur Verwendung nach Anspruch 1, wobei die Verbindung die folgende Formel aufweist:

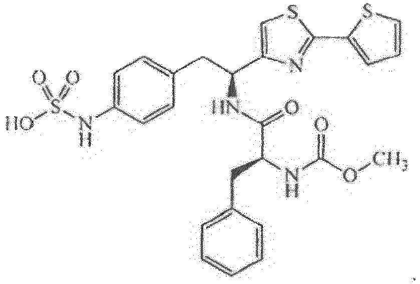

3. Verbindung zur Verwendung nach Anspruch 1, wobei die Verbindung die folgende Formel aufweist:

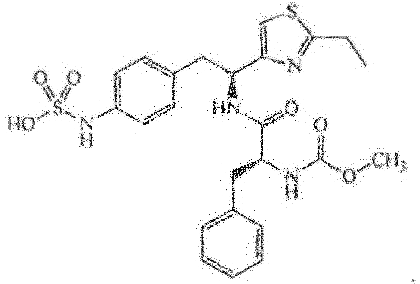

4. Verbindung zur Verwendung nach Anspruch 1, wobei die Verbindung die folgende Formel ist:

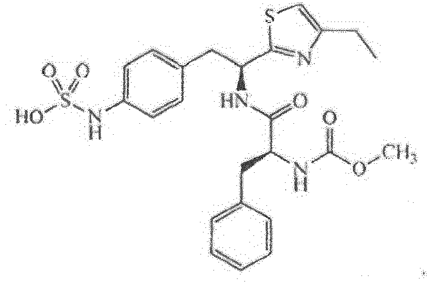

5. Verbindung mit der Formel nach einem der Ansprüche 1 bis 4 oder pharmazeutisch unbedenkliches Salz davon zur Verwendung nach Anspruch 1, wobei es sich bei dem Okularödem um diabetisches Makulaödem handelt.

6. Pharmazeutisches Präparat, umfassend eine Verbindung nach einem der Ansprüche 1 bis 4 oder ein pharmazeutisch unbedenkliches Salz davon und einen oder mehrere Träger oder verträgliche Hilfsstoffe zur Verwendung beim Behandeln von Okularödem.

## Revendications

1. Composé pour une utilisation dans le traitement d'un oedème oculaire d'un oeil chez un patient, lequel composé a une formule choisie dans le groupe constitué par : 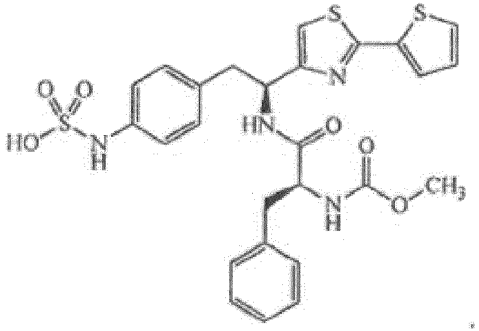 ou sel pharmaceutiquement acceptable de celui-ci ; 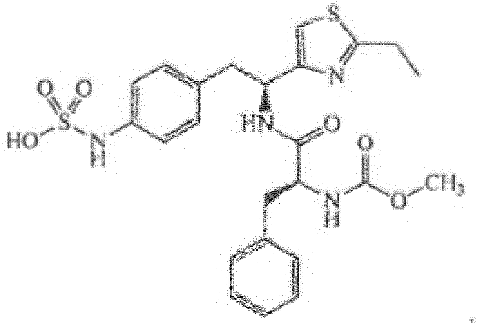 ou sel pharmaceutiquement acceptable de celui-ci ; et 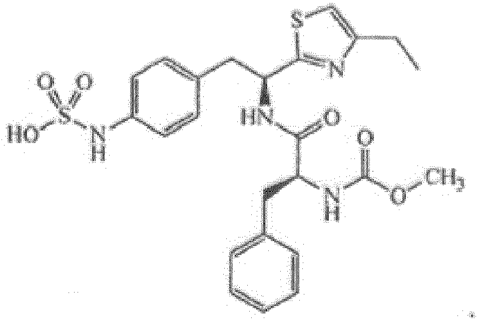 ou sel pharmaceutiquement acceptable de celui-ci.

2. Composé pour une utilisation selon la revendication 1, lequel composé a la formule :

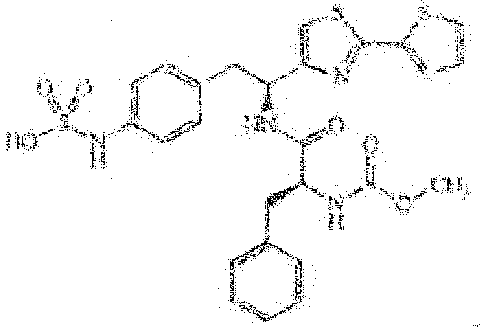

3. Composé pour une utilisation selon la revendication 1, lequel composé a la formule :

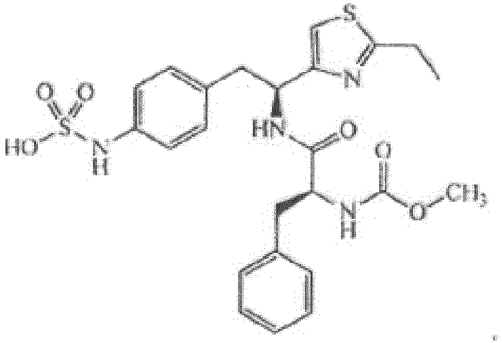

4. Composé pour une utilisation selon la revendication 1, lequel composé a la formule :

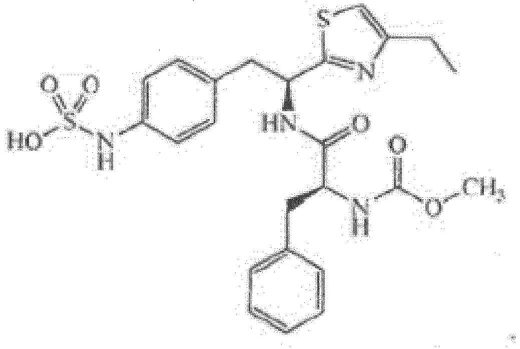

5. Composé ayant la formule telle que définie dans l'une quelconque des revendications 1 à 4 ou sel pharmaceutiquement acceptable de celui-ci, pour une utilisation selon la revendication 1, où l'oedème oculaire est l'oedème maculaire diabétique.

6. Préparation pharmaceutique comprenant un composé tel que défini dans l'une des revendications 1 à 4 ou un sel pharmaceutiquement acceptable de celui-ci, et un ou plusieurs vecteurs ou excipients compatibles, pour une utilisation dans le traitement de l'oedème maculaire.
